# EUROPEAN PATENT SPECIFICATION

(11) **EP 1 760 147 B1**
(45) Date of publication and mention of the grant of the patent: **24.12.2008**
(21) Application number: 06119019.5
(22) Date of filing: 16.08.2006
(51) Int. Cl.: C12N 9/10, C12N 15/54, G01N 33/68

(54) **Crystal structures of carnitine palmitoyltransferase-2 (CPT-2) and uses thereof**
Kristallstrukturen von Carnitine Palmitoyltransferase-2 (CPT-2) und deren Verwendung
Structures cristallines de carnitine palmitoyltransferase-2 (CPT-2) et leurs utilisations

(30) Priority: 29.08.2005 EP 05107883
(43) Date of publication of application: 07.03.2007
(73) Proprietor: F. Hoffmann-La Roche AG, 4070 Basel (CH)
(72) Inventor: Benz, Joerg, 79618 Rheinfelden (DE); Gsell, Bernard, 68480 Lutter (FR); Hennig, Michael, 79576 Weil am Rhein (DE); Rufer, Arne, 69118 Heidelberg (DE); Stihle, Martine, 68730 Michelbach-le-Bas (FR); Thoma, Ralf, 79639 Grenzach-Wyhlen (DE)

(56) References cited:
- BROWN N F ET AL: "Catalytically important domains of rat carnitine palmitoyltransferase II as determined by site-directed mutagenesis and chemical modification. Evidence for a critical histidine residue." THE JOURNAL OF BIOLOGICAL CHEMISTRY. 22 JUL 1994, vol. 269, no. 29, 22 July 1994 (1994-07-22), pages 19157-19162, XP002413715 ISSN: 0021-9258
- RAMSAY R R ET AL: "Selective modulation of carnitine long-chain acyltransferase activities. Kinetics, inhibitors, and active sites of COT and CPT-II." ADVANCES IN EXPERIMENTAL MEDICINE AND BIOLOGY. 1999, vol. 466, 1999, pages 103-109, XP009076781 ISSN: 0065-2598
- ZHENG GUOLU ET AL: "Identification by mutagenesis of a conserved glutamate (Glu487) residue important for catalytic activity in rat liver carnitine palmitoyltransferase II." THE JOURNAL OF BIOLOGICAL CHEMISTRY. 1 NOV 2002, vol. 277, no. 44, 1 November 2002 (2002-11-01), pages 42219-42223, XP002413863 ISSN: 0021-9258
- BLUNDELL T L ET AL: "HIGH-THROUGHPUT CRYSTALLOGRAPHY FOR LEAD DISCOVERY IN DRUG DESIGN" NATURE REVIEWS. DRUG DISCOVERY, NATURE PUBLISHING GROUP, BASINGSTOKE, GB, vol. 1, no. 1, January 2002 (2002-01), pages 45-54, XP009023187 ISSN: 1474-1784
- RUFER ET AL: "The Crystal Structure of Carnitine Palmitoyltransferase 2 and Implications for Diabetes Treatment" STRUCTURE, CURRENT BIOLOGY LTD., PHILADELPHIA, PA, US, vol. 14, no. 4, April 2006 (2006-04), pages 713-723, XP005554546 ISSN: 0969-2126

## Description

The carnitine palmitoyltransferase (CPT) system facilitates the import of long-chain fatty acids into mitochondria of higher eukaryotes. CPT-1 is integrated into the outer mitochondrial membrane and catalyzes the transfer of long chain acyl moieties from acyl-CoA to carnitine. The reverse reaction is carried out by CPT-2, a protein located on the matrix side of the inner mitochondrial membrane. Modulation of the catalytic activity of tissue-specific CPT-1 isoforms is in the focus of developing novel drugs against non-insulin dependent (type 2) diabetes mellitus and obesity.

Crystal structures of proteins are useful to identify and optimize compounds that bind to the active site of enzymes. So far, the crystal structure of CPT-2 had not been solved.

The present invention relates to the structure of full-length rat CPT-2 alone or in complex with the reversible CPT inhibitor ST1326, a substrate analog mimicking palmitoyl carnitine. The structure of CPT-2 in complex with ST1326 provides insight into the role of residues conserved in CPT-1/2 and short chain acyltransferases in protein-ligand interaction.

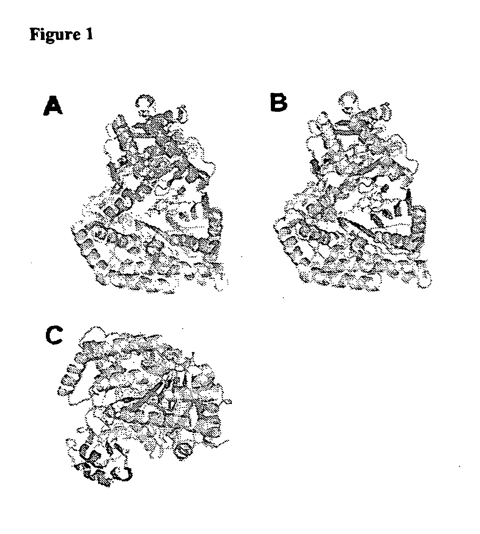

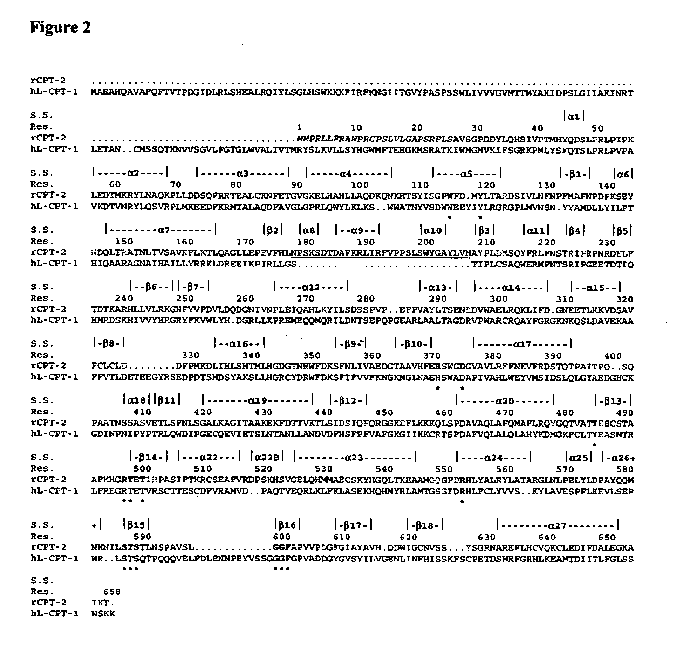

The structure of CPT-2 was determined in three different crystal forms at a resolution of 2.0 Å and 1.6 Å for the apo enzyme and 2.5 Å resolution for the complex with ST1326 (see Examples for details and Table 1).
The overall fold of CPT-2 can be subdivided in an amino-terminal (residues 111-440) and a carboxy-terminal (residues 441-658, plus 32-110) domain. These domains consist of six central anti-parallel β-strands, two of which (β1, β16) mediate the major domain contact, and surrounding α-helices (Figures 1/ 2). The CPT-2 secondary structure contains 27 α-helices and 18 β-strands. The loop connecting helices 22 and 23 adopts a helical conformation in the apo structure and is therefore designated helix 22B. The corresponding region of the ST1326 complex structure is located close to a crystal contact, which may interfere with secondary structure formation. A 30 amino acid insert uniquely found in CPT-2 when compared to other carnitine acyltransferases (29 amino acids in the case of L-CPT-1, Figure 2) protrudes from the amino-terminal domain.

The electron density for the entire catalytic core of rat CPT-2 is well defined. The first 26 amino-terminal amino acids of the construct used for crystallization comprising the His-tag and five residues of the actual CPT-2 sequence are disordered in the complex and both the apo structures. The last residues with interpretable electron density at the carboxy-termini are Lys 654 (ST1326 complex) and Ile 656 (apo, P4₃2₁2), while the full carboxy-terminus is visible in chain A of the orthorhombic (C222₁) apo structure.

Apo CPT-2 and the ST1326 complex crystallized in different crystal forms but the structures have similar conformations as indicated by a r.m.s. distance of 0.38 Å between equivalent Cα atoms. In all three structures the active site residue Arg 498 is in the generously allowed region of the Ramachandran plot and Leu 129 as well as Asn 230 do not comply with favoured geometry of the Ramachandran plot, but unambiguous electron density is visible for these residues. Leu 129 is the second residue in a type II reverse turn (equivalent to Ile 116 in mouse CrAT, PDB code 1t7n), whereas Asn 230 is located in a β-turn whose geometry may be distorted due to interaction with neighboring Asp 297 and Arg 124. Mutation of Arg 124 is associated with inherited CPT-2 deficiency (see below).

Thus, the present invention relates to three crystal forms of the protein CPT-2. One crystal of CPT-2 has unit cell dimensions of a = b = 66Å to 70 Å, c = 302 Å to 312 Å; α = β = γ = 90°, and the crystal has a P4₃2₁2 symmetry. In another embodiment, the crystal has unit cell dimensions of a=93 Å to 97 Å, b= 94 Å to 98 Å, c = 304 Å to 312 Å; α = β = γ = 90°, and the crystal has a C222₁ symmetry. The present invention also relates to a co-crystal of protein CPT-2 and a ligand bound to the active site of CPT-2, wherein the co-crystal has unit cell dimensions of a = 83 Å to 87 Å, b = 94 Å to 98 Å, c from 121 Å to 127 Å; α = β = γ = 90°, and the crystal has a P2₁2₁2₁ symmetry.

**Table 1 Data collection and refinement statistics**

| | | apo, high resolution | apo2 | CPT-II / ST1316 |
|---|---|---|---|---|
| **Data collection** Space group Cell dimensions | | C222₁ | P4₃2₁2 | P2₁2₁2₁ |
| | *a, b, c* (Å) | 95.2, 97.3, 310.4 | 67.6, 67.6, 307.3 | 85.8, 96.2, 124.3 |
| | α, β, γ (°) | 90.0, 90.0, 90.0 | 90.0, 90.0, 90.0 | 90.0, 90.0, 90.0 |
| Resolution (Å) * | | 23.0 - 1.6 | 23.0 - 2.0 | 50.0 - 2.24 |
| | | (1.69 - 1.60) | (2.12 - 2.00) | (2.38 - 2.24) |
| *R*_{merge} | | 6.2 (21.2) | 3.1 (7.4) | 16.7 (41.1) |
| *I*/σ*I* | | 14.04 (7.24) | 28.04 (14.80) | 9.57 (3.20) |
| Completeness (%) | | 99.7 (99.8) | 99.9 (100) | 92.9 (81.8) |
| Redundancy | | 14.7 (17.3) | 21.8 (18.5) | 6.9 (6.2) |
| | | | | |
| **Refinement** | | | | |
| Resolution (Å) | | 23-0-1.6 | 23.0 - 2.0 | 15.0-2.5 |
| No. reflections | | 179022 | 46957 | 32831 |
| *R*_{work} /*R*_{free} | | 16.5 / 19.4 | 17.9 / 23.5 | 24.1 / 29.6 |
| No. atoms (all) | | 11449 | 5502 | 5143 |
| | Protein | 10085 | 4977 | 4963 |
| | Ligand/ion | n/a* | n/a | 28 |
| | Water | 1364 | 525 | 152 |
| *B*-factors (overall) | | 20.1 | 21.9 | 41.0 |
| | Protein | 18.7 | 21.0 | 41.4 |
| | Ligand/ion | n/a* | n/a | 27.6 |
| | Water | 30.1 | 30.7 | 31.6 |
| R.m.s deviations | | | | |
| | Bond lengths (Å) | 0.014 | 0.01 | 0.013 |
| | Bond angles (°) | 1.225 | 1.161 | 1.502 |

| | | | | |
|---|---|---|---|---|
| * The βOG detergent molecule (mean B= 54.9 Å²), the two C16 alkyl-moieties (mean B = 23.3 Å²) and the two residual, unspecifically bound CoA molecules (mean B = 37.7 Å²) were not included in the statistic. | | | | |

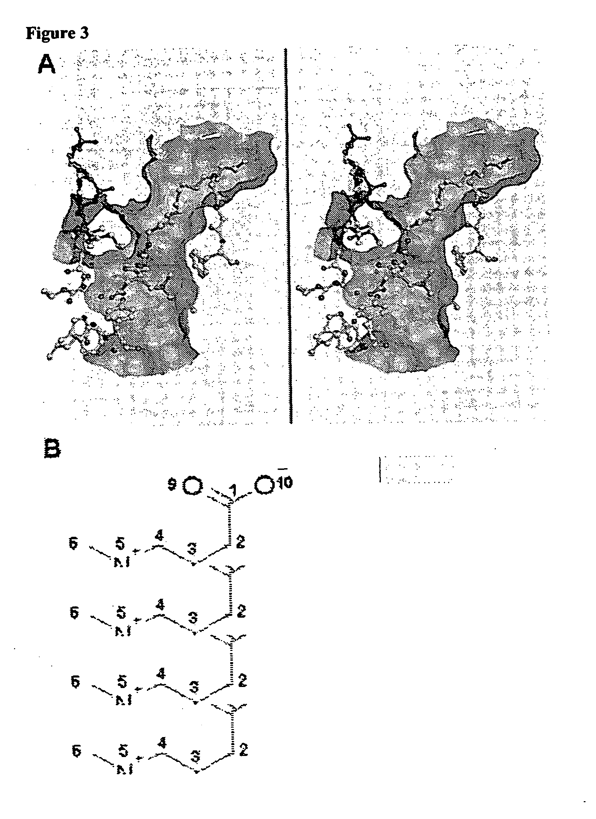

The CPT inhibitor ST1326 binds to the active site of CPT-2 which is located in a tunnel penetrating CPT-2 at the domain interface (Figure 3). ST1326 is a non-cleavable analog of palmitoyl-carnitine, the physiological substrate of CPT-2. The acyl- and carnitine tunnel of the tripartite (Nic a' Bhaird, N., et al., Comparison of the active sites of the purified carnitine acyltransferases from peroxisomes and mitochondria by using a reaction-intermediate analogue. Biochem J., 294, 645-651 (1993)) active site of CPT-2 are occupied by ST1326, whereas the CoA-tunnel can be assigned by homology modeling based on the complex structure of CrAT with CoA (Figure 3). The hydrophilic aminocarnitine head group of ST1326 is tightly bound in a hydrogen-bond network. The catalytic base His 372 forms a hydrogen bond with the amino-nitrogen (N11) of ST1326, which substitutes the ester oxygen of the native ligand palmitoyl-carnitine. Ser 590 of the Ser-Thr-Ser motif conserved among carnitine acyltransferases makes a hydrogen bond to the carbamoyl-oxygen (013) of the ligand. Tyr 486, Ser 488 and Thr 499 of the carboxy-terminal domain are directly hydrogen-bonded to the carboxyl oxygens (O9 and O10) of ST1326. Hydrogen bonds to the guanidinium group of Arg 554 further stabilize the orientation of Tyr 486 and Thr 499. Residues Trp 116, Tyr 120 and Asp 376 of the amino-terminal domain and a concertedly fixed water molecule are also part of the hydrogen bond network binding to the carboxy group of ST1326. Arg 498 forms a strong hydrogen bridge with the side chain of Asp 376 and its guanidinium group interacts with the main chain carbonyl oxygen of Ser 373 in the catalytic loop, thereby rendering the active site residues in a position ideal for catalysis. The positively charged tertiary amine of ST1326 is stabilized by cation-pi interactions with the conserved Phe 602.

The hydrophobic tunnel that accommodates the aliphatic tetradecanoyl tail of ST1326 is lined by residues of β-strands 1 and 16, which form an anti-parallel β-sheet at the domain interface, and the two carboxy-terminal β-strands 17 and 18. A simulated annealing *fofc* map contoured at 2.5 σ shows clear electron density for the ligand ST1326 bound to the active site. The β-strands forming the hydrophobic tunnel are moved apart in order to make room for the extended hydrophobic tail of the substrate analog ST1326 compared to the closed arrangement in CrAT and CrOT (PDB codes 1ndb, 1t7n and 1x18). The glycine residue Gly 600 at a position where bulkier residues are found in CrAT (Met 564) and CrOT (Gln 552) allows binding of LCFA carnitine-derivatives in CPT-2, thereby determining substrate specificity. In contrast to CrAT and CrOT the acyl-tunnel opens to the surface in CPT-2.

Glu 487 and Glu 500 of CPT-2, which are conserved throughout the carnitine acyltransferases, have been implicated in substrate binding and catalysis by means of mutant analysis (Zheng, G., et al., Identification by mutagenesis of a conserved glutamate (Glu487) residue important for catalytic activity in rat liver carnitine palmitoyltransferase II. J. Biol. Chem., 277, 42219-42223 (2002)). The crystal structure of CPT-2 reveals that Glu 487 indeed is located in the part of the active site tunnel that accommodates CoA. Glu 487 together with the highly conserved Asp 464 form a negatively charged patch that is probably required for guiding substrates to the active site. The side chain of Glu 500 interacts with the main chain of conserved Arg 554, which is a crucial component of the hydrogen network required for binding the carnitine moiety of acyltransferase substrates.

In the apo structures the side chains of Tyr 120 and the catalytic His 372 are disordered despite the higher quality of the data set and resolution of the structure analysis. Those residues in the active site that interact with the hydrophilic aminocarnitine head group of ST1326 as well as Ser 590 are moved away from the ligand binding, but the overall shape of the active site tunnel is preformed in the apo enzyme.

The clinically heterogeneous disease CPT-2 deficiency is caused by various mutations in the CPT-2 gene and is inherited in an autosomal recessive manner (Bonnefont, J-P. et al. Carnitine palmitoyltransferases 1 and 2: biochemical, molecular and medical aspects. Mol. Aspects Med., 25, 495-520 (2004); Bonnefont, J.P. et al. Carnitine palmitoyltransferase deficiencies. Mol. Genet. Metab., 68, 424-440 (1999)). Two different manifestations of the disorder can be distinguished based on the time of onset, i.e., the early onset (neonatal or infantile) and the more frequent adult form of CPT-2 deficiency. The early onset form is characterized by a severe symptomatology including cardiomyopathy and hypoketotic hypoglycemia and has been linked to acute liver failure in sudden infant death syndrome (Demaugre, F. et al. Infantile form of carnitine palmitoyltransferase II deficiency with hepatomuscular symptoms and sudden death. Physiopathological approach to carnitine palmitoyltransferase II deficiencies. J. Clin. Invest., 87, 859-864 (1991)). Clinical signs of adult CPT-2 deficiency are recurrent myalgia and myoglobinuria in response to fasting and exercise.

More than 30 point mutations in the coding region for CPT-2 leading to single amino acid exchanges in the enzyme have been identified in addition to deletions/insertions causing frame shifts or truncation of the protein. Whereas truncation of the CPT-2 gene inevitably leads to a severe neonatal presentation of CPT-2 deficiency by loss of CPT-2 function, a graduated correlation of genotype and severity of clinical phenotype becomes obvious for those miss-sense mutations that have been described in a homozygous state (Table 2) (Bonnefont, J.P. et al. Carnitine palmitoyltransferase deficiencies. Mol. Genet. Metab., 68, 424-440 (1999); Thuillier, L. et al. Correlation between genotype, metabolic data, and clinical presentation in carnitine palmitoyltransferase 2 (CPT2) deficiency. Hum. Mutat., 21, 493-501 (2003)). An exception to this correlation is the Arg 631 Cys mutation, which has been identified in homozygous patients both with the adult as well as with the infantile form of CPT-2 deficiency. The crystal structure of CPT-2 allows mapping and interpretation of the effects of the described mutations (summarized in Table 2). Most (60 %) cases of adult CPT-2 deficiency are associated with a Ser 113 Leu mutation. Ser 113 is located at the amino-terminus of helix α5 close to the domain interface. The side chain of Ser 113 does not make any direct contacts to surrounding amino acids but mutation of this residue to a larger, hydrophobic Leu alters the interaction with the neighboring Phe 117. This changes the position and environment of the catalytically important residues Trp 116 and Arg 498, rendering the enzyme less active (Taroni, F. et al. Identification of a common mutation in the carnitine palmitoyltransferase II gene in familial recurrent myoglobinuria patients. Nat. Genet., 4, 314-320 (1993)).

Asp 213 and Glu 487 are affected by naturally occurring mutations and have been determined to be important for CPT-2 function by biochemical analyses (Zheng, G., et al., Identification by mutagenesis of a conserved glutamate (Glu487) residue important for catalytic activity in rat liver carnitine palmitoyltransferase II. J. Biol. Chem., 277, 42219-42223 (2002); Liu, H., et al., Cysteine-scanning mutagenesis of muscle carnitine palmitoyltransferase I reveals a single cysteine residue (Cys-305) is important for catalysis. J. Biol. Chem., 280, 4524-4531 (2005)). Asp 213 is located in a loop between β3 and α10 of CPT-2 and aligns with a cysteine conserved in all human CPT-1 isoforms. Mutation of this cysteine to Ala fully abolishes enzyme activity in human M-CPT-1, indicating that this position is crucial for structural integrity in all CPT isoforms. In CPT-2 the side chain of Asp 213 makes a strong interaction with the main chain nitrogen of His 496. This is important for the positioning Arg 498 and Arg 499 which are involved in substrate binding. Mutation of Glu 487 in β13 to aspartic acid leads to an almost complete loss of CPT-2 activity. This mutation could interfere with CoA binding as Glu 487 is part of the CoA-tunnel surface. From the CPT-2 crystal structure it can be predicted that an aspartic acid at position 487 would form a strong hydrogen bond with the side chain of Thr 589 of the conserved STS motif, thereby distorting the geometry of the active site. A Glu 487 Lys exchange is one of 6 mutations identified in CPT-2 deficiency that cause disruption of internal salt bridges or hydrogen-bond interactions which are fully conserved in CPT-1, CrAT and CrOT (Table 2). A guanidinium nitrogen of Arg 296 makes a strong (2.7 Å) contact with a side chain oxygen of Asp 353, which is conserved in carnitine acyltransferases. The equivalent residue in M-CPT-1, Asp 454, has been proposed to be part of a catalytic triad (Liu, H., et al., Cysteine-scanning mutagenesis of muscle carnitine palmitoyltransferase I reveals a single cysteine residue (Cys-305) is important for catalysis. J. Biol. Chem., 280, 4524-4531 (2005)), whereas the CPT-2 crystal structure implicates this residue to form a conserved salt bridge. None of the reported mutants associated with CPT-2 deficiency is located in the insert, although they are distributed uniformly in the tertiary structure.

Table 2 Residues mutated in human CPT-2 deficiency. All the affected residues are conserved between rat and human CPT-2. Residue numbering for the human and rat isoforms of CPT-II are identical. Homozygous mutations labeled A are associated with the late onset, adult form of CPT-2 deficiency, those labeled I with the early onset, infantile presentation.

| Mutation | Effect on | | Conservation | | |
|---|---|---|---|---|---|
| P50H | structure of amino-terminus | A | CPT-1 | CrAT | CrOT |
| L72F | structure of amino-terminus (M 194 in 1CPT-I; V58, A43, i.e. small hydrophobic residue, in CrAT and CrOT respectively) | | M & B | | |
| | | | CPT-1 | | |
| S113L | enviroment of catalytically important Trp 116 and Arg 498, domain interface (most frequent mutation found in adult CPT-2 deficiency) | A | CPT-1 | CrAT | |
| Y120C | substrate (carnitine moiety) binding | | CPT-1 | CrAT | CrOT |
| R124Q R124X | folding of amino-terminal domain conserved interaction with D232 | | CPT-1 | CrAT | CrOT |
| N146T | folding of amino-terminal domain | | | | |
| R151Q | folding of amino-terminal domain | I | CPT-1 | | CrOT |
| R161W | domain interface and interaction with amino-terminus | A | | | |
| K164X | interaction with amino-terminus (basic residue in CPT-1) | | CPT-1 | | |
| | | | R or H | | |
| E174 K | folding of amino-terminal domain (residue interacts with R382), altered surface of CoA binding site | A | | CrAT | |
| Y210D | close to insertion, i.e. involved in membrane association | | | | |
| D213G | domain interface, substrate binding (long range disruption of hydrogen network), C305 in human muscle CPT-1 (...) | | | | CrOT |
| M214T | domain interface and interaction with amino terminus | | | CrAT | CrOT |
| P227L | folding of amino-terminal domain | I | CPT-1 | CrAT | CrOT |
| K274M | folding of amino-terminal domain | | | | |
| Y290X | folding of amino-terminal domain | | | | |
| R296Q R296X | folding of amino-terminal domain conserved interaction with D353 | | CPT-1 | CrAT | CrOT |
| D328G | folding of amino-terminal domain conserved interaction with R350 | I | CPT-1 | CrAT | CrOT |
| | | | (E) | | (E279 ) |
| R382K | folding of amino-terminal domain (residue interacts with E174) | I | CPT-1 (H) | | |
| F383Y | folding of amino-terminal domain | I | | | |
| F448L | folding of carboxy-terminal domain, surface at entry of acyl-tunnel | | CPT-1 | CrAT | CrOT |
| F454X | substrate (CoA moiety) binding | | | CrAT | |
| | | | | (D420 ) | |
| Y479F | folding of carboxy-terminal domain | | | CrAT | |
| E487K | surface of CoA binding site (...) conserved interaction with H539 | | CPT-1 | CrAT | CrOT |
| 1502T | substrate binding (long range disruption of hydrogen network) | | CPT-1 | CrAT | CrOT |
| | | | (V) | | (V455 ) |
| R503C | folding of carboxy-terminal-domain, surface at entry of carnitine-tunnel conserved interaction with D553 | | CPT-1 | CrAT | CrOT |
| G549D | folding of carboxy-terminal domain, substrate binding (long range disruption of hydrogen network), | | CPT-1 | CrAT | CrOT |
| Q550R | folding of carboxy-terminal domain, possibly binding of CoA | | B | | |
| | | | CPT-1 | | |
| | | | (Q648) | | |
| D553N | substrate binding (long range disruption of hydrogen network), surface at entry of carnitine-tunnel, conserved interaction with R503 | | CPT-1 | CrAT | CrOT |
| P604S | domain interface, substrate binding (alignment of F602) | | CPT-1 | CrAT | CrOT |
| D608H | folding of carboxy-terminal domain interaction with H584 (c | | L & M | CrAT | |
| | | | CPT-1 | | |
| Y628S | domain interface | I | | | CrAT |
| R631C | folding of carboxy-terminal domain | I/A | | | |

The apo, derivative and co-crystals of the invention can be obtained by techniques well-known in the art of protein crystallography, including batch, (modified) microbatch, liquid bridge, dialysis, vapor diffusion and hanging drop methods (see e.g. McPherson, 1982, Preparation and Analysis of Protein Crystals, John Wiley, NY; McPherson, 1990, Eur. J. Biochem. 189:1-23; Webber, 1991, Adv. Protein Chem. 41:1-36; Crystallization of Nucleic Acids and Proteins, Edited by Arnaud Ducruix and Richard Giege, Oxford University Press; Protein Crystallization Techniques, Strategies, and Tips, Edited by Terese Bergfors, International University Line, 1999). Generally, the apo- or co-crystals of the invention are grown by placing a substantially pure CPT-2 polypeptide in an aqueous buffer containing a precipitant at a concentration just below that necessary to precipitate the protein. Water is then removed from the solution by controlled evaporation to produce crystallizing conditions, which are maintained until crystal growth ceases.

As used herein, a "buffered, aqueous solution" refers to a solution based on water which comprises a substance which minimizes changes in the pH of the solution when an acid or base is added to the solution.

As used herein, a "a molar excess" refers to a larger number of ligand molecules than the number of protein molecules.

As used herein, a "vapor diffusion" refers to a protein crystallization method well known in the art wherein the crystallization solution is allowed to equilibrate with the precipitant reservoir solution through the gas phase only.

In a preferred embodiment of the invention, apo or co-crystals are grown by vapor diffusion. In this method, the polypeptide/precipitant solution is allowed to equilibrate in a closed container with a larger aqueous reservoir having a precipitant concentration optimal for producing crystals. Generally, less than about 10 µL of substantially pure polypeptide solution is mixed with an equal or similar volume of reservoir solution, giving a precipitant concentration about half that required for crystallization. This solution is suspended as a droplet underneath a coverslip, which is sealed onto the top of a reservoir. The sealed container is allowed to stand, from one day to one year, usually for about 2-6 weeks, until crystals grow.

For crystals of the invention, it has been found that hanging drops containing about 2-5 µl of rat CTP-2 [3.75 - 50 mg/ml rat CPT-2 in 25 mM Tris/HCl pH 8.0,0.15 M NaCl, 2mM TCEP, 1% (w/v) n-octy-β-D-glucopyranoside (βOG) or 0.33 % (w/v) CHAPS or 1.15 mM n-dodecyl-β-D-maltoside (DBM) and 0.02 % (w/v) NaN₃] grown at room temperature (21°C +/- 2 °C) and an equal or similar amount of reservoir solution (Index 21, 37, 61, 62, 69,75, 76, 79, 88, 90, 91, 92, Hampton Research, as well as solutions derived from these by altering the pH or the concentrations of their ingredients) suspended over 0.33 to 1.0 mL reservoir buffer for about 1-7 days provided crystals suitable for high resolution X-ray structure determination. Particularly preferred conditions were: a (modified) microbatch assay with about 0.1 µl - 1 µl of rat CTP-2 (13.5 mg/ml in 25 mM Tris/HCl pH 8.0, 0.15 M NaCl, 2mM TCEP and 1% (w/v) β-D-OG) pre-incubated with a 3-fold molar excess of ST1326 as well as hanging drop assays with I µl to 5 µl rat CPT-2 (12 mg/ml, from conventional expression or as protein labeled with seleno-methionene, in 25 mM Tris/HCl pH 8.0,0.15 M NaCl, 2mM TCEP and 1% (w/v) β-D-OG) mixed with 0.5 µl to 2 µl of Index 91 (Hampton Research) precipitant solution over a 0.33 ml to 1.0 ml reservoir solution (Index 91, Hampton Research).

All crystal grown in hanging drops were submerged in the respective mother liquor supplemented with 20 % (w/v) to 30 % (w/v) polyethylene glycol 200 (PEG 200) and appropriate ligand-concentration in the case of complex structures prior to freezing in liquid nitrogen or mounting on a goniometer equipped with a liquid nitrogen cooling device. The crystals grown using the microbatch method were either frozen as described for the crystals grown in hanging drops or the mother liquor and oil surrounding the crystal was exchanged against 100 % (v/v) paraffin oil.

Diffraction data typically extending to 2.0 Å was collected from the frozen crystals at the synchrotron beamline X10SA (PX1) or X10SA (PX2) at the SLS, Villigen, Switzerland. Under optimum conditions, data extending to 1.6 Å was recorded.

Of course, those having skill in the art will recognize that the above-described crystallization conditions can be varied. Such variations may be used alone or in combination, and include polypeptide solutions containing polypeptide concentrations between 1 mg/mL and 60 mg/mL, any commercially available buffer systems which can maintain pH from about 3.0 to about 11.0, reducing components in various concentrations (dithiothreitol, dithioeritol, β-mercapto ethanol or other art-recognized equivalents), stabilizing components like sugar or glycerols in concentrations between 0% (w/v) and 30% (w/v) or substitution of other ligands known to bind CPT-2. Precipitant and reservoir solutions containing polyethylene glycol (PEG) concentrations between about 1 % (w/v) and about 30 % (w/v), polyethylene glycol average molecular weights between about 200 and about 20,000 daltons, and temperature ranges between 4°C and 30°C.

Thus, the present invention also relates to a process for co-crystallizing CPT-2 with a ligand that binds to the active site, the process comprising providing a buffered, aqueous solution of 3.75 to 50 mg/ml of CPT-2, adding a molar excess of the ligand to the aqueous solution of CPT-2, and growing crystals by vapor diffusion or microbatch using a buffered reservoir solution of 0 % to 30 % (w/v) PEG, wherein the PEG has an average molecular weight of 200 Da to 20000 Da. The PEG may be added as monomethyl ester. PEG may be used of an average molecular weight of 500 Da to 5,000 Da. The buffered reservoir solution further comprises 0 M to 2 M tri-ammonium citrate pH 7, 0 M to 1 M L-proline, 0 M to 1M trimethylamine-N-oxide, 0 M to 1 M ammonium sulfate, 0 M to 1 M lithium sulfate, 0 M to 1 M ammonium acetate, 0 M to 1 M sodium or magnesium formate and 0 M to 1 M D/L-malic acid pH 7. Said microbatch may be modified. The present invention further relates to crystals produced by the process hereinbefore described.

Derivative crystals of the invention can be obtained by soaking apo or co-crystals in mother liquor containing salts of heavy metal atoms, according to procedures known to those of skill in the art of X-ray crystallography.

Co-crystals of the invention can be obtained by soaking an apo crystal or a crystal of CPT-2 with a low affinity ligand in mother liquor or any other stabilizing solution containing a ligand that binds to the active site, or can be obtained by co-crystallizing the CPT-2 polypeptide in the presence of one or more ligands that bind to the active site.

Methods for obtaining the three-dimensional structure of the crystalline CPT-2 described herein, as well as the atomic structure coordinates, are well-known in the art (see, e.g., D. E. McRee, Practical Protein Crystallography, published by Academic Press, San Diego (1993), and references cited therein).

The crystals of the invention, and particularly the atomic structure coordinates obtained therefrom, have a wide variety of uses. For example, the crystals and structure coordinates described herein are particularly useful for identifying compounds that inhibit or generally modify the activity of CPT-2 as an approach towards developing new therapeutic agents.

The structure coordinates described herein can be used as phasing models in determining the crystal structures of additional native or mutated CPT-2, as well as the structures of co-crystals of such CPT-2 with inhibitors or activators bound. The structure coordinates, as well as models of the three-dimensional structures obtained therefrom, can also be used to aid the elucidation of solution-based structures of native or mutated CPT-2, such as those obtained via NMR. Thus, the crystals and atomic structure coordinates of the invention provide a convenient means for elucidating the structures and functions of CPT-2.

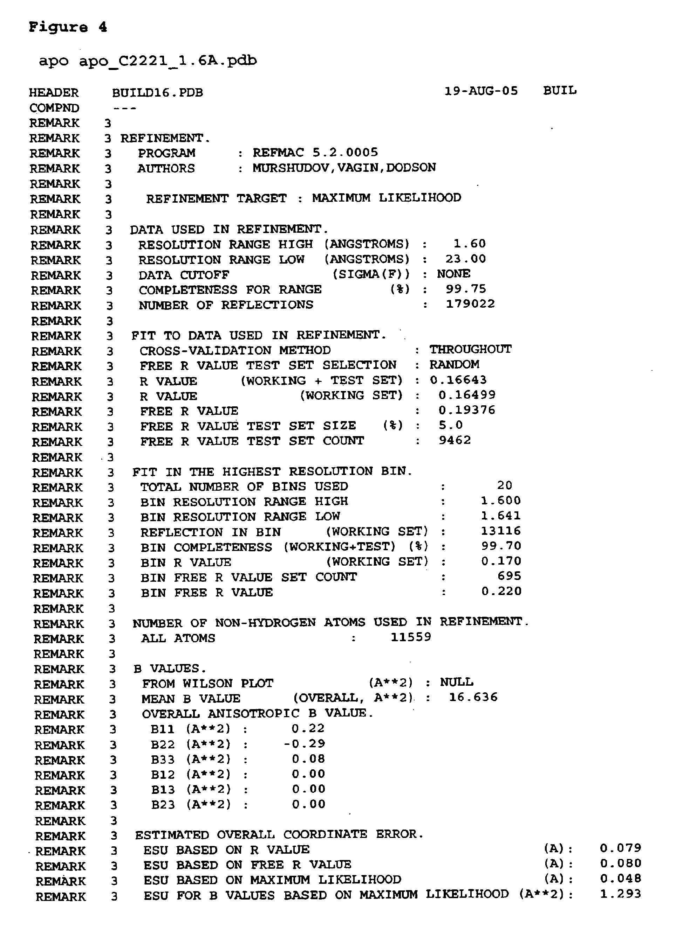

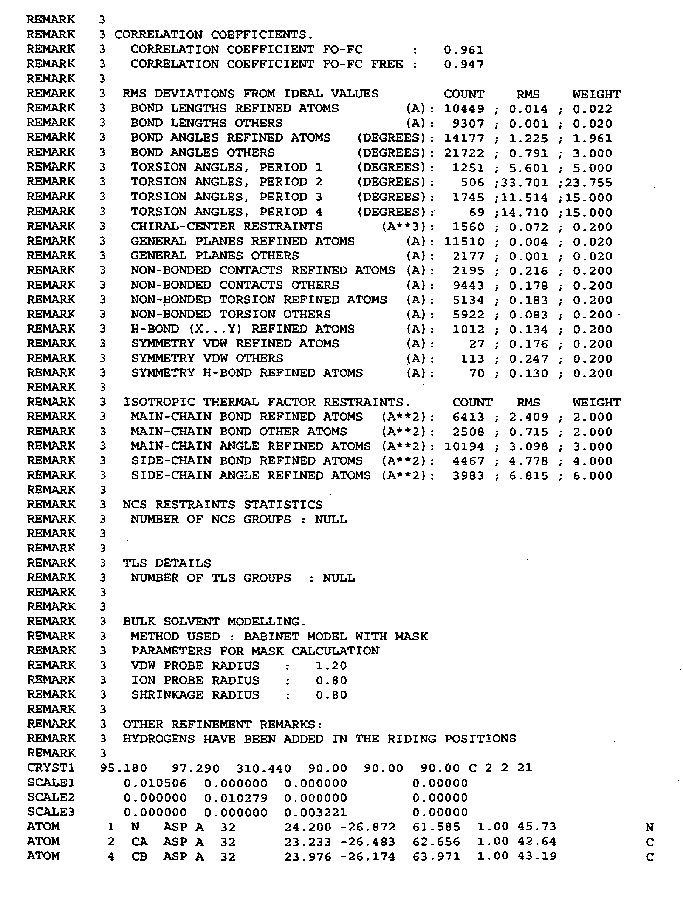

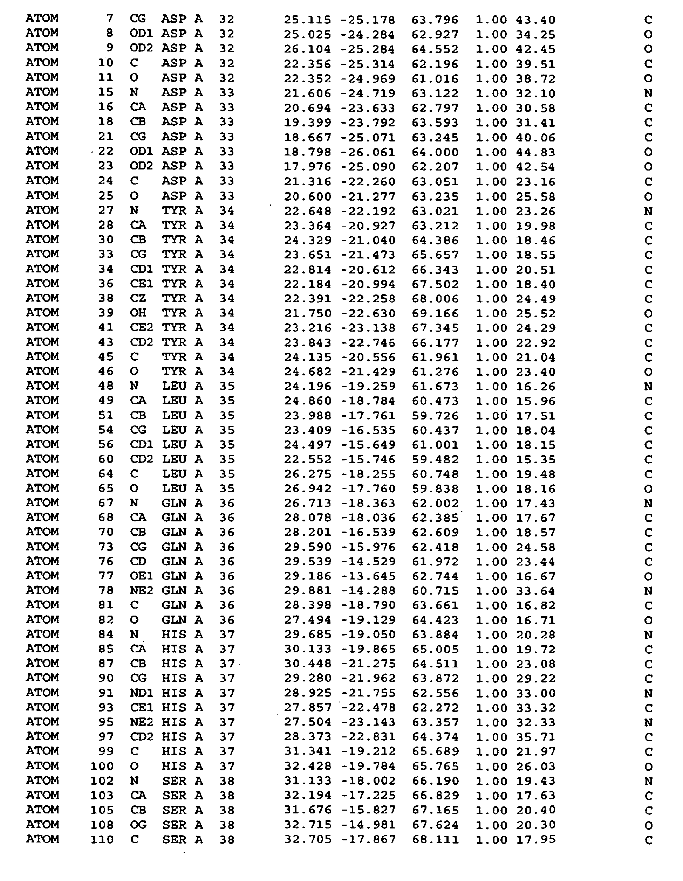

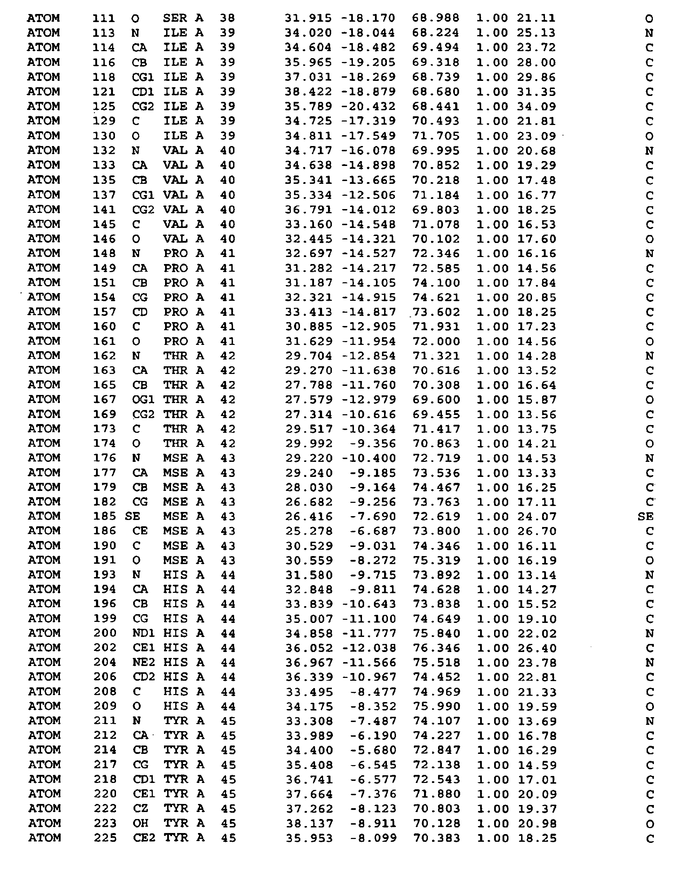

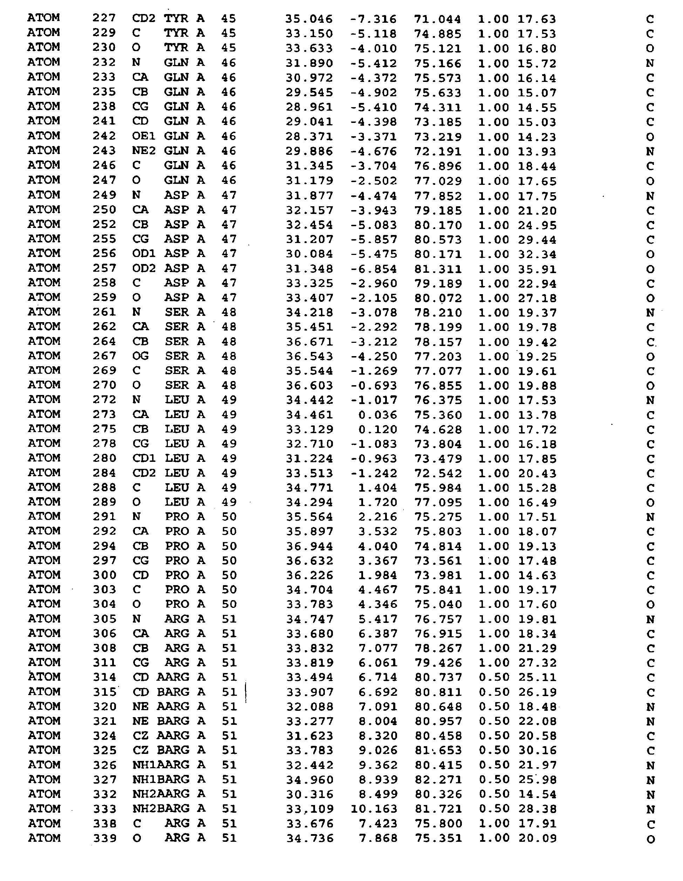

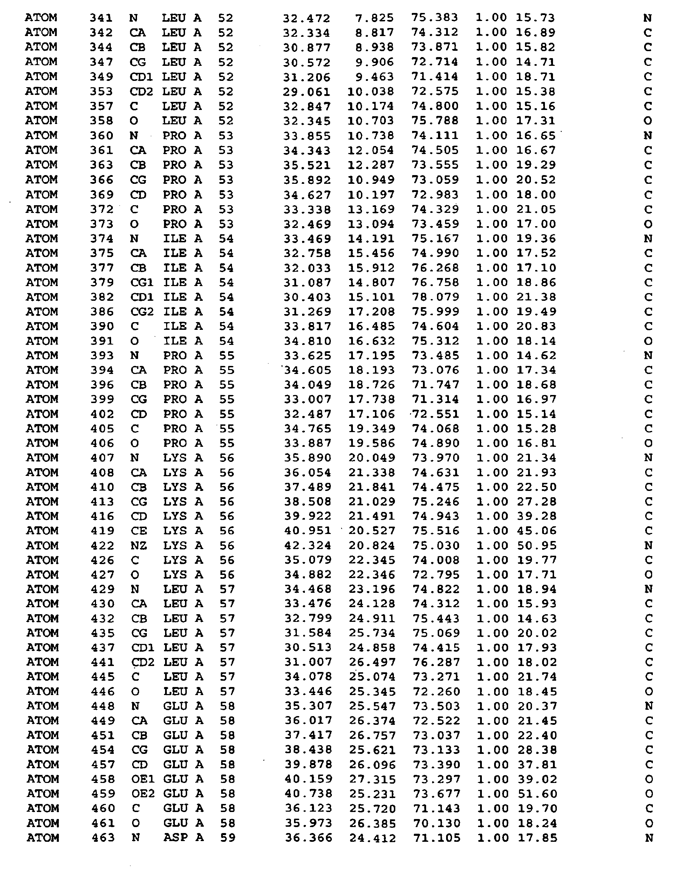

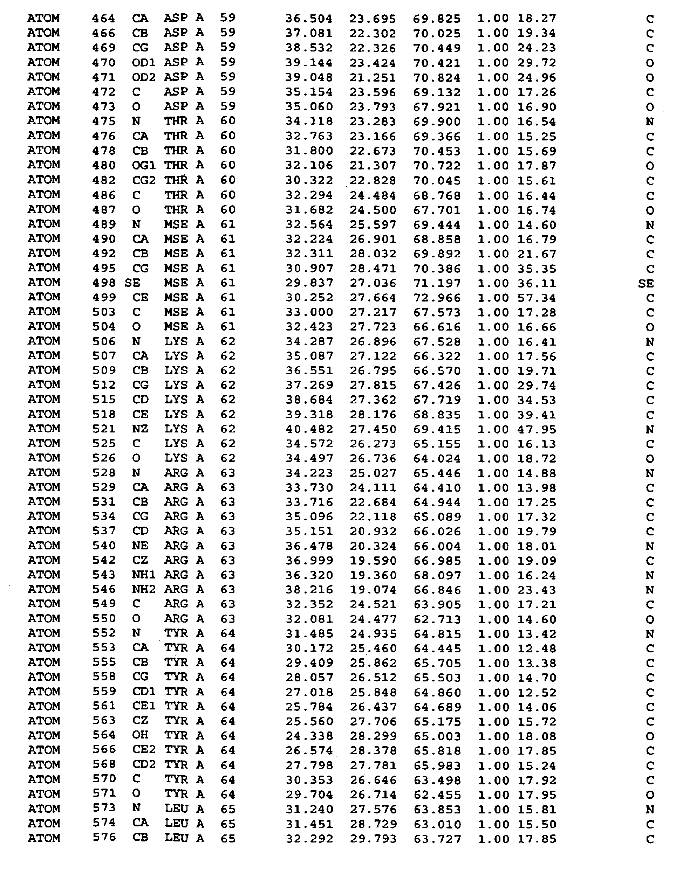

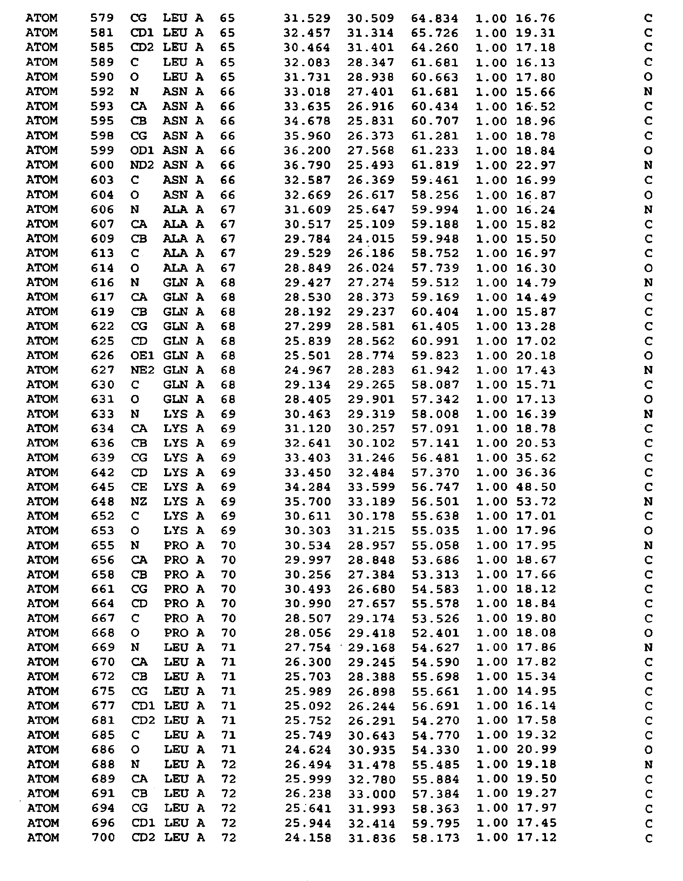

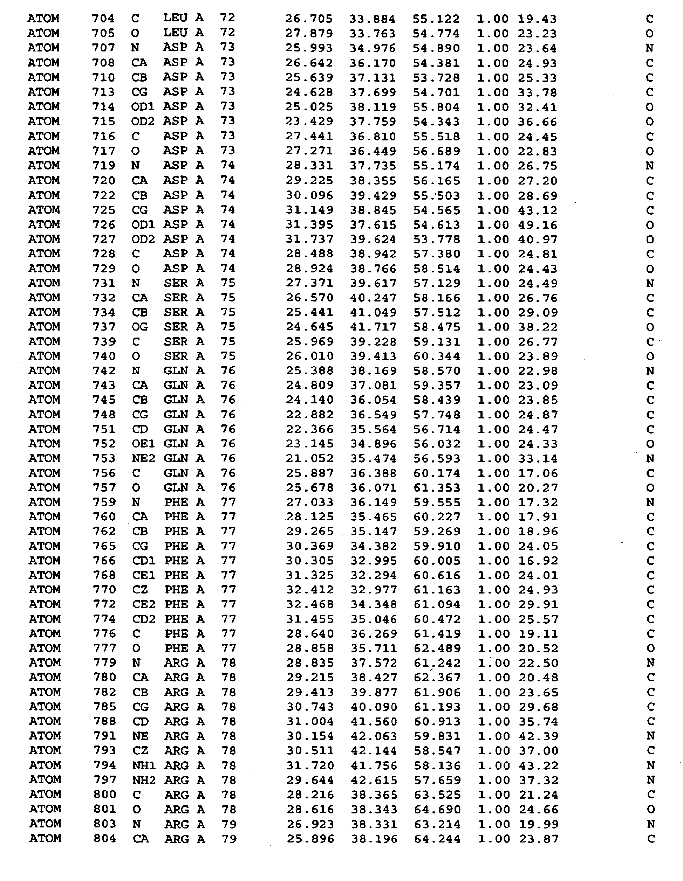

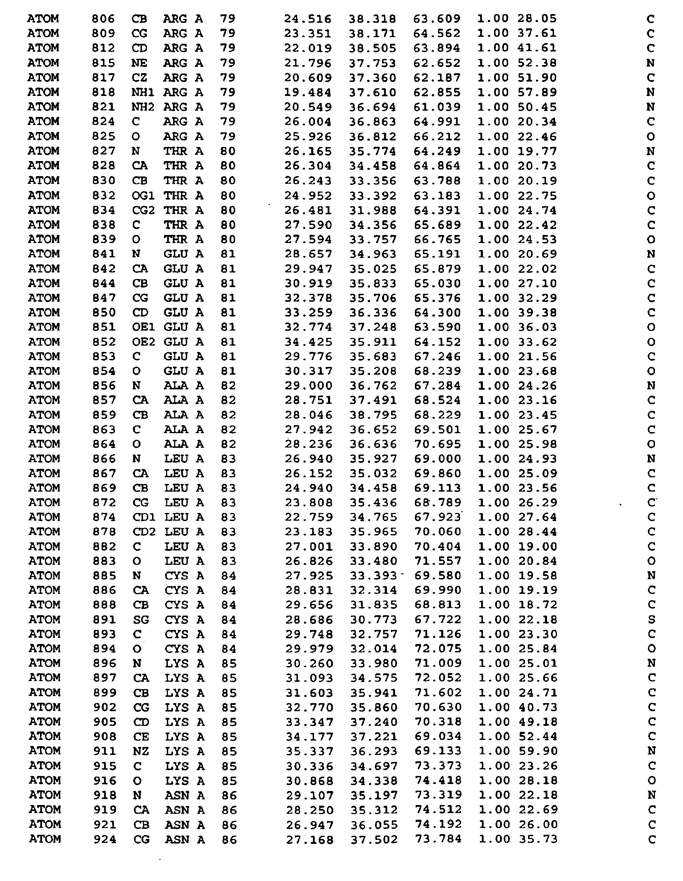

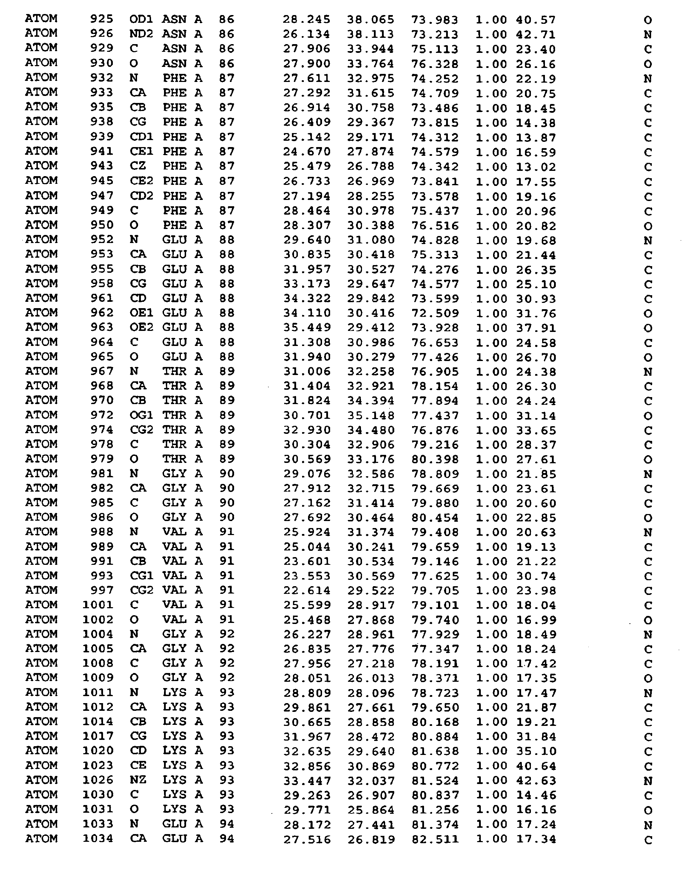

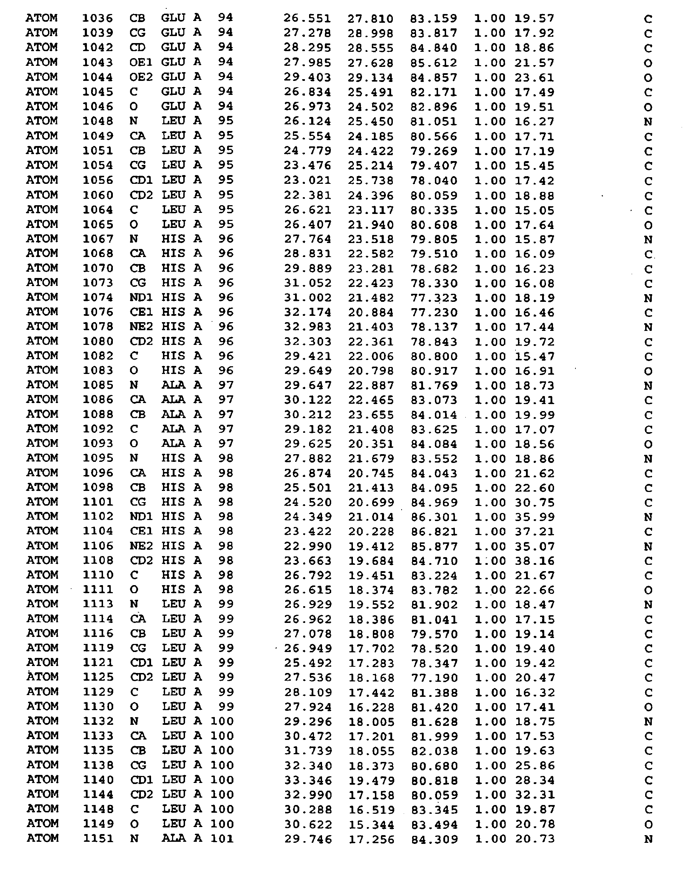

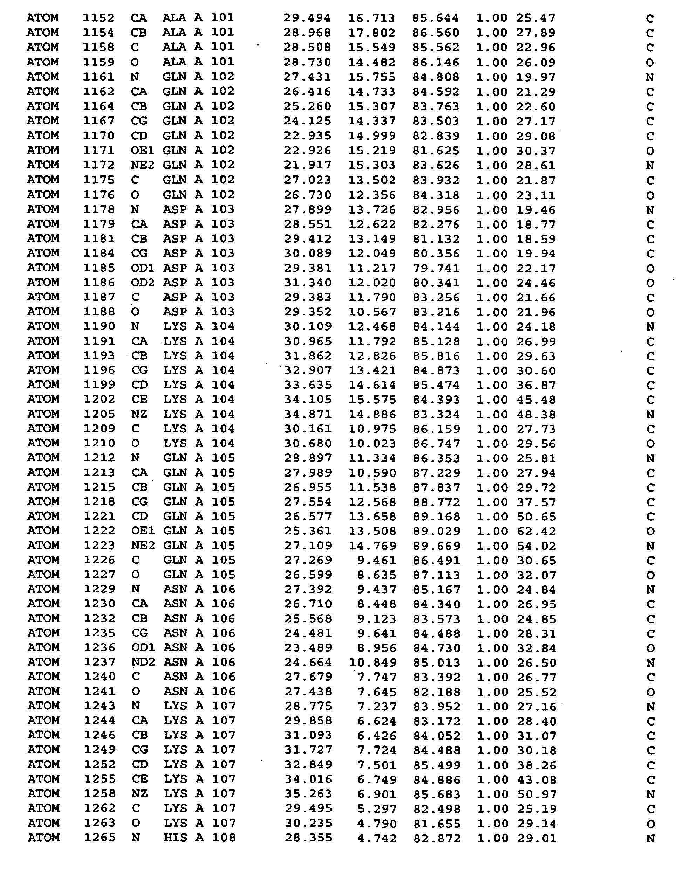

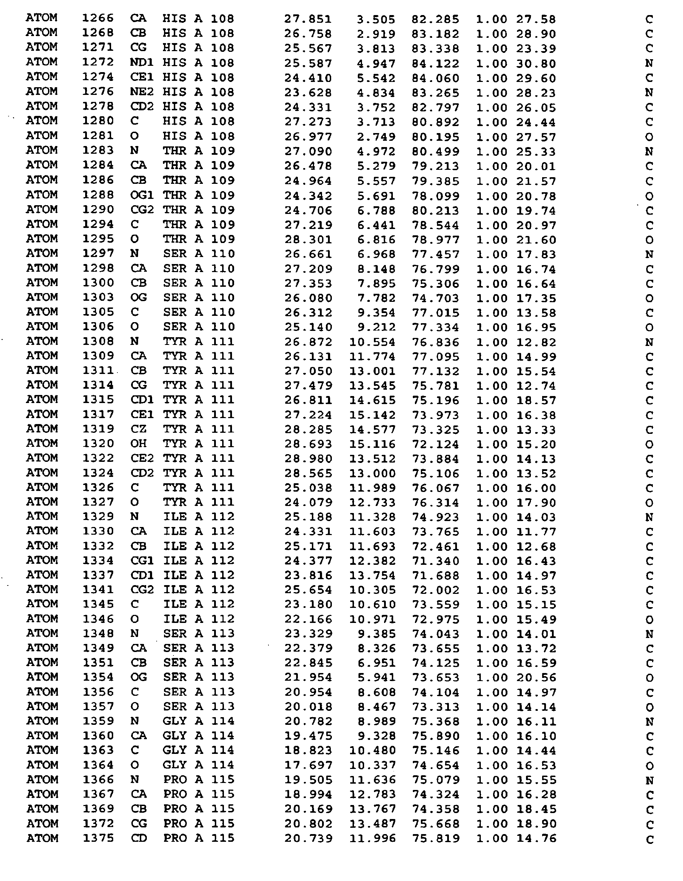

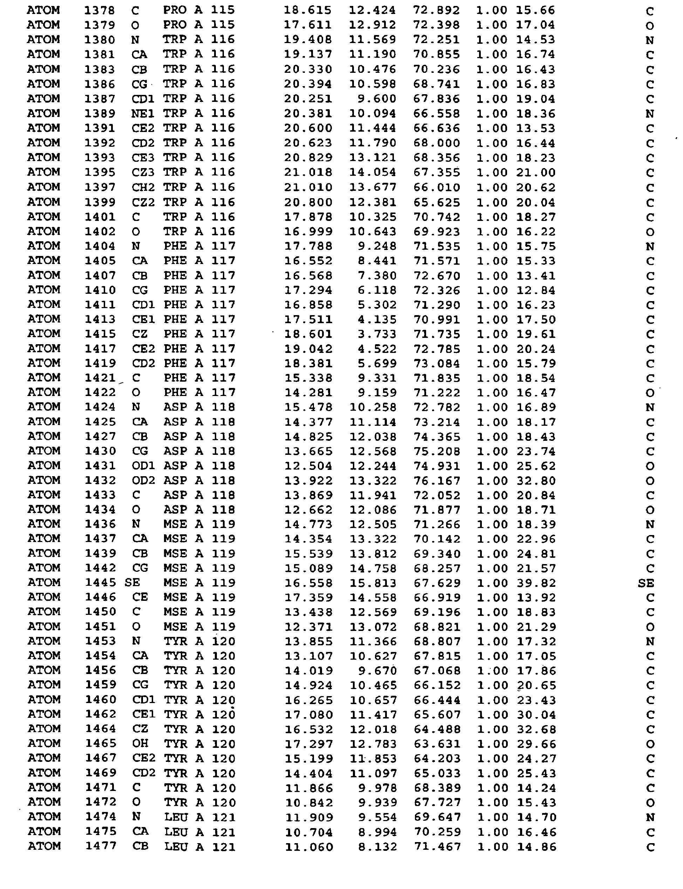

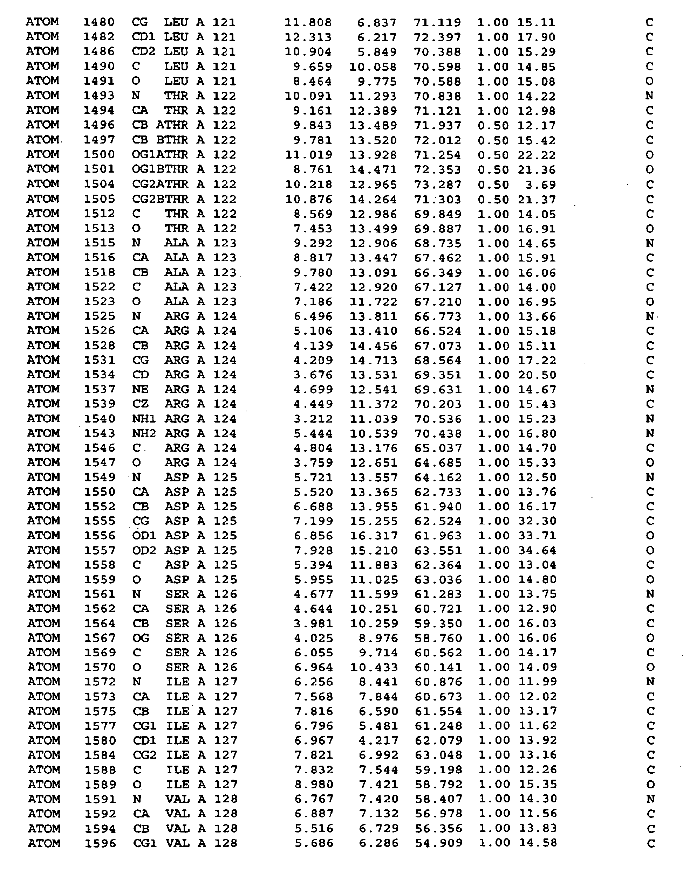

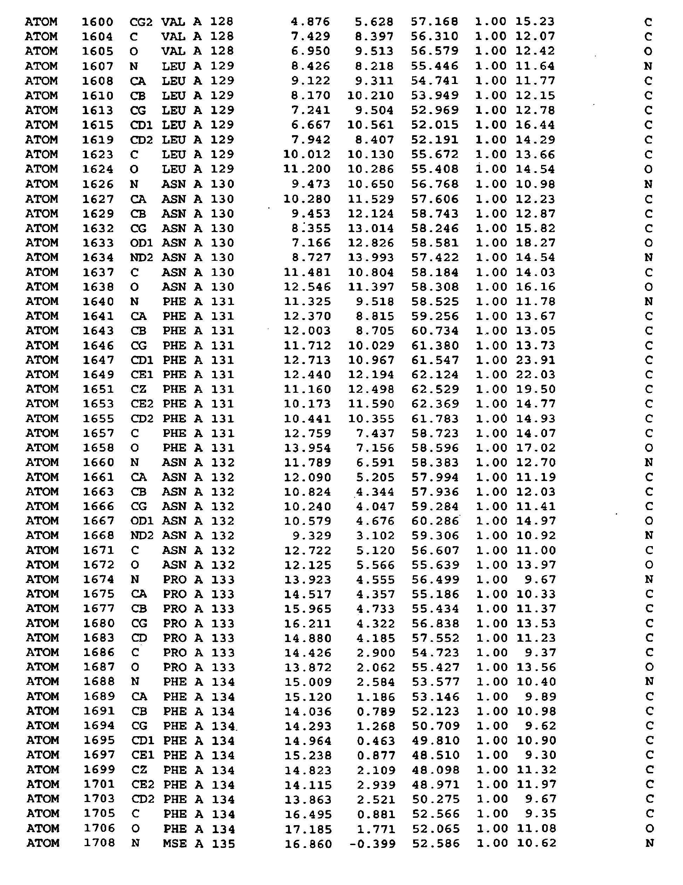

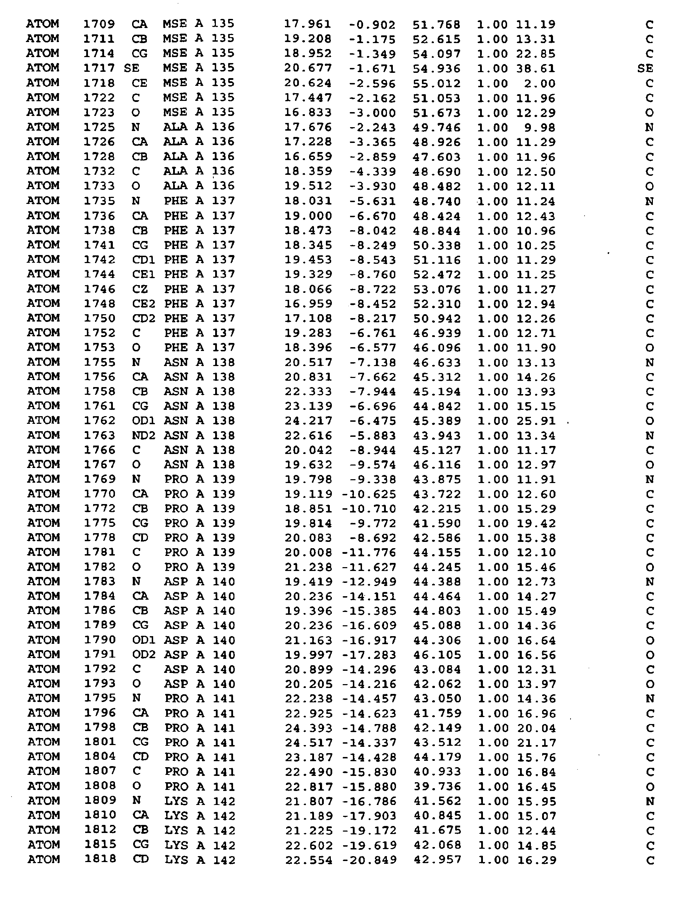

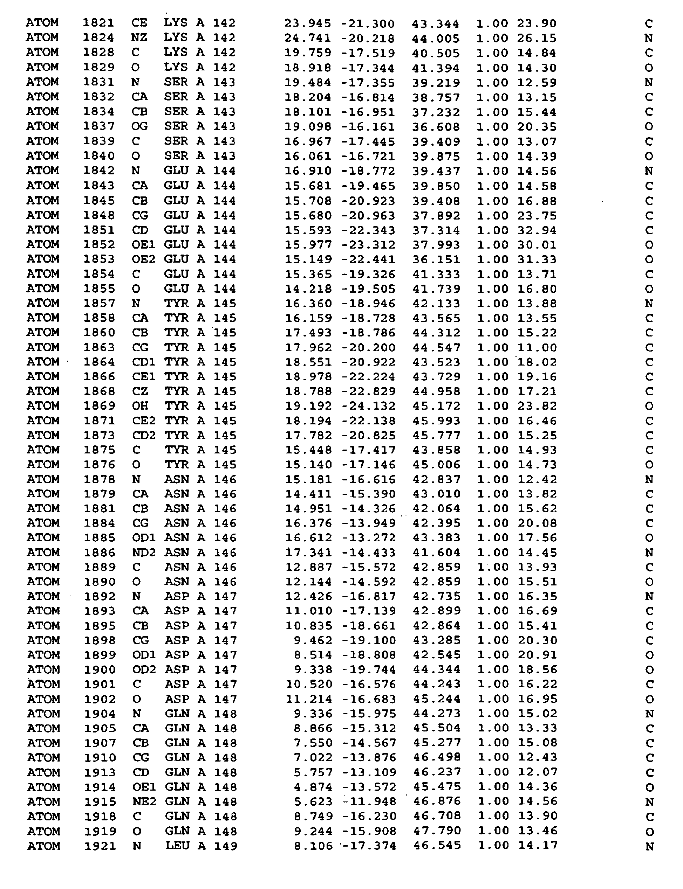

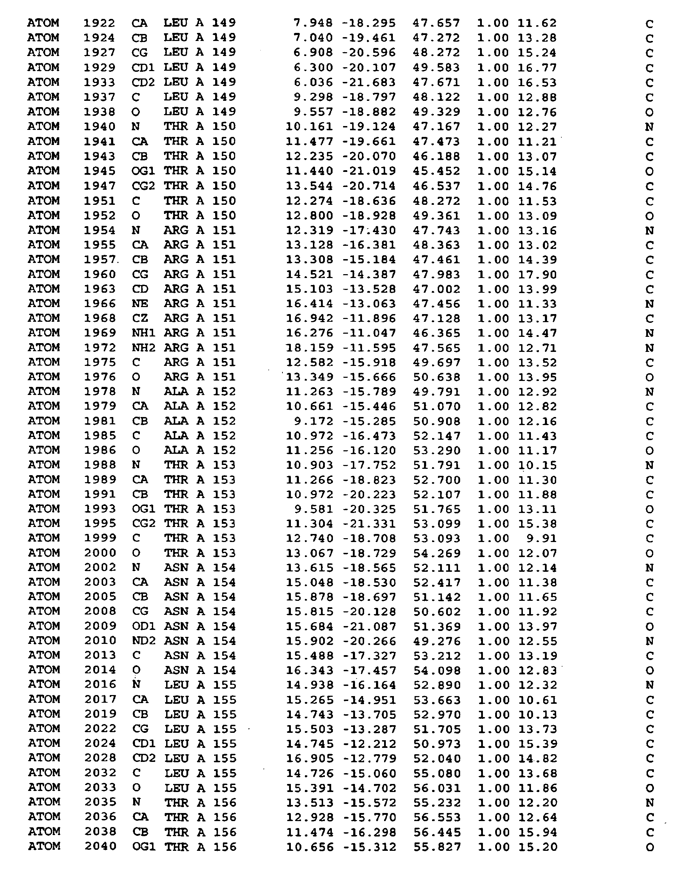

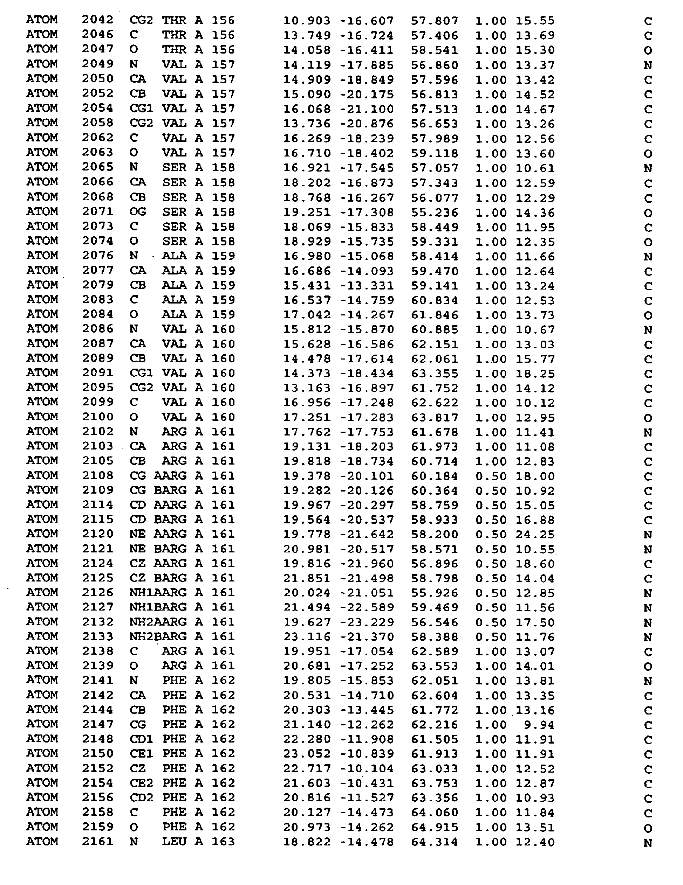

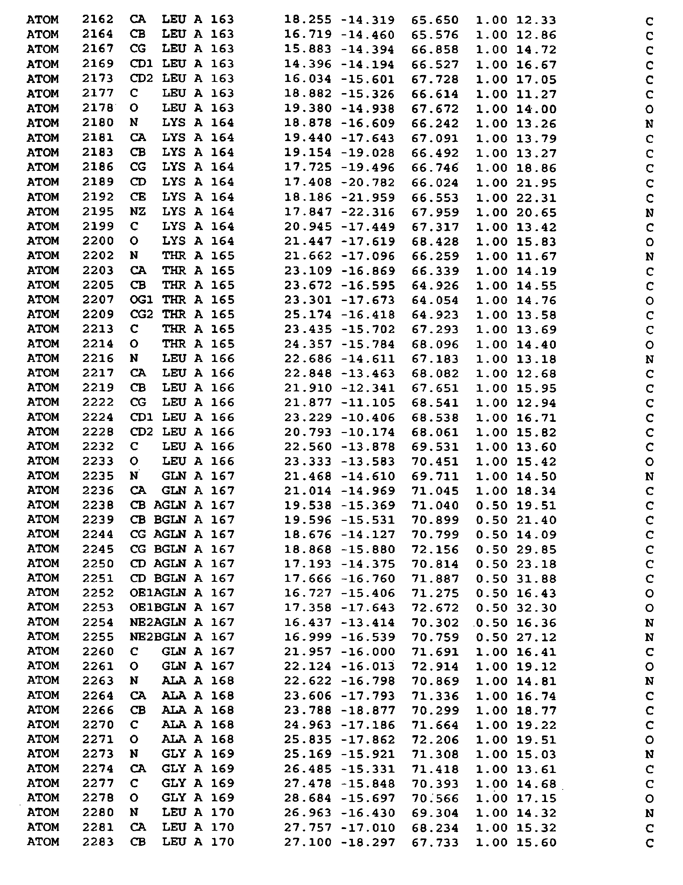

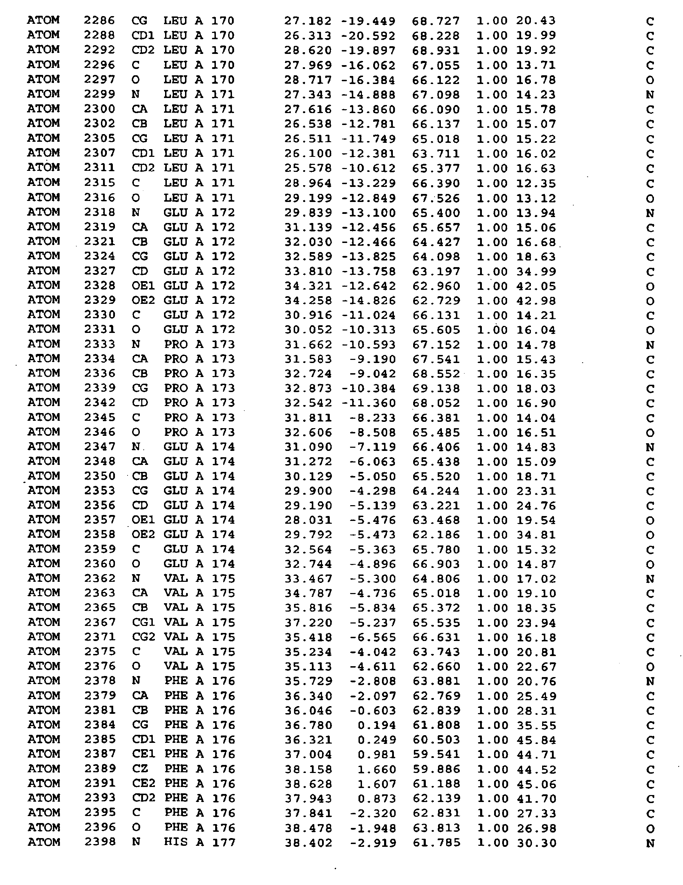

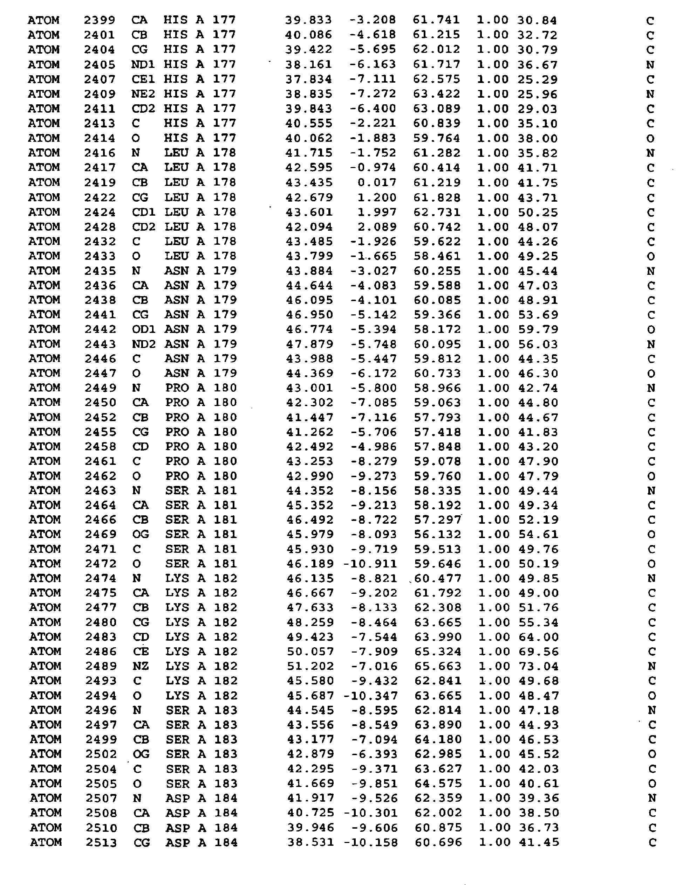

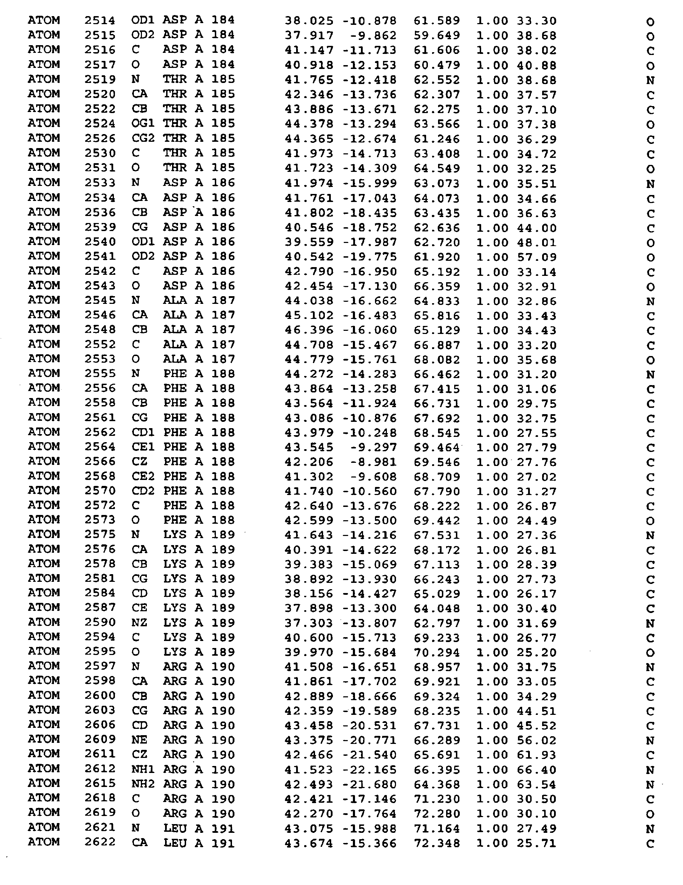

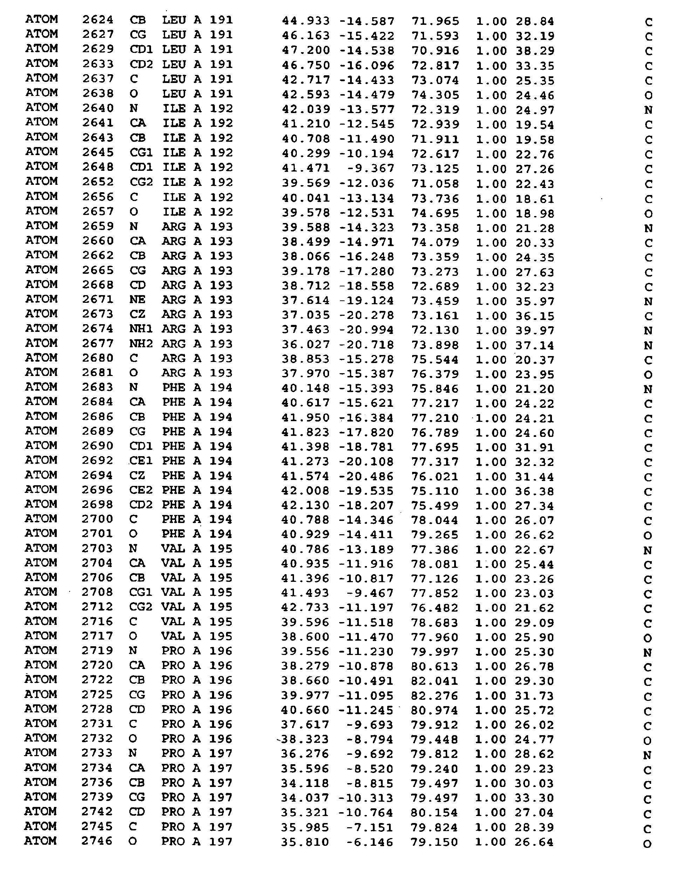

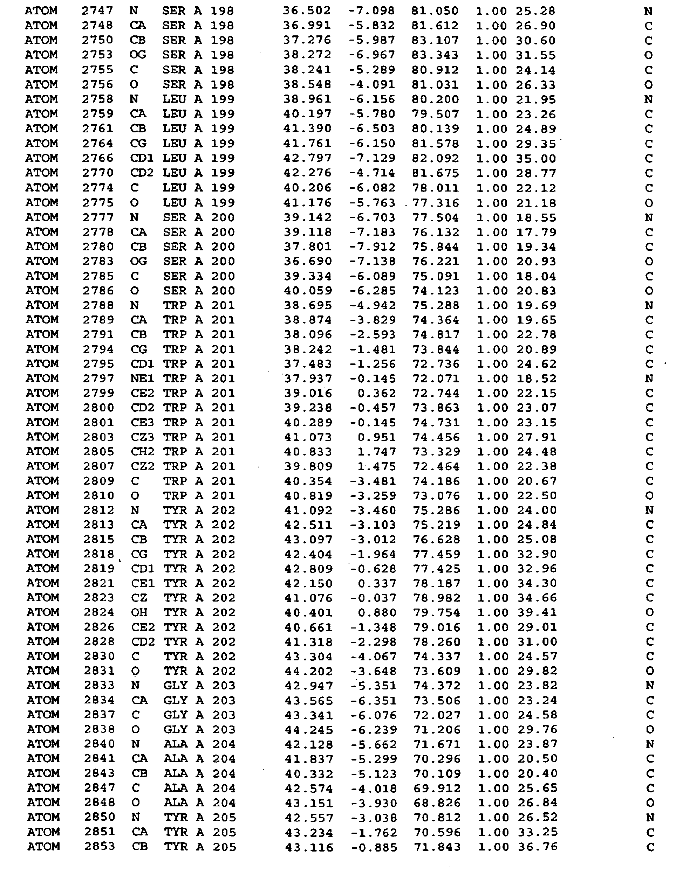

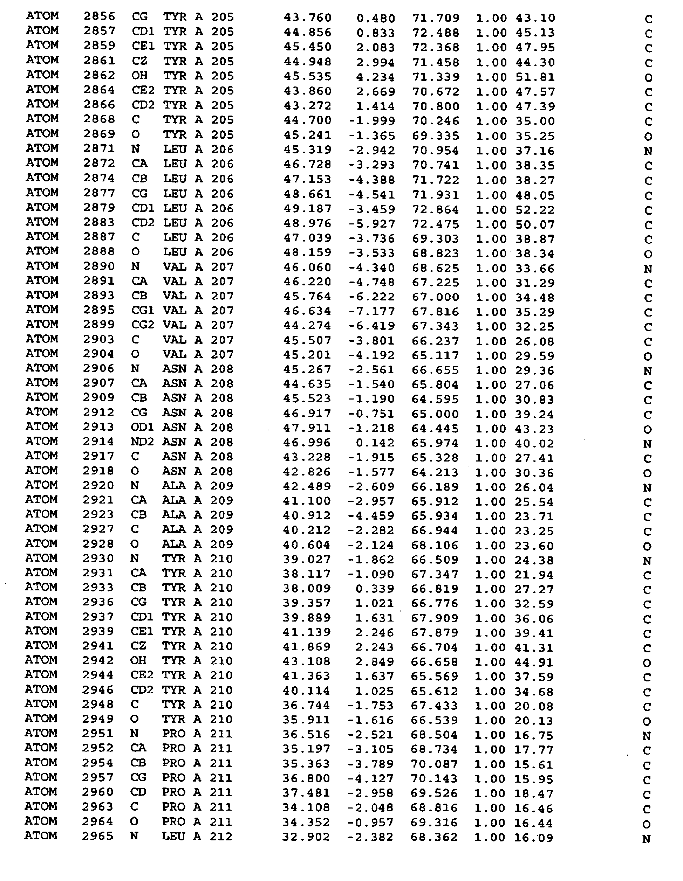

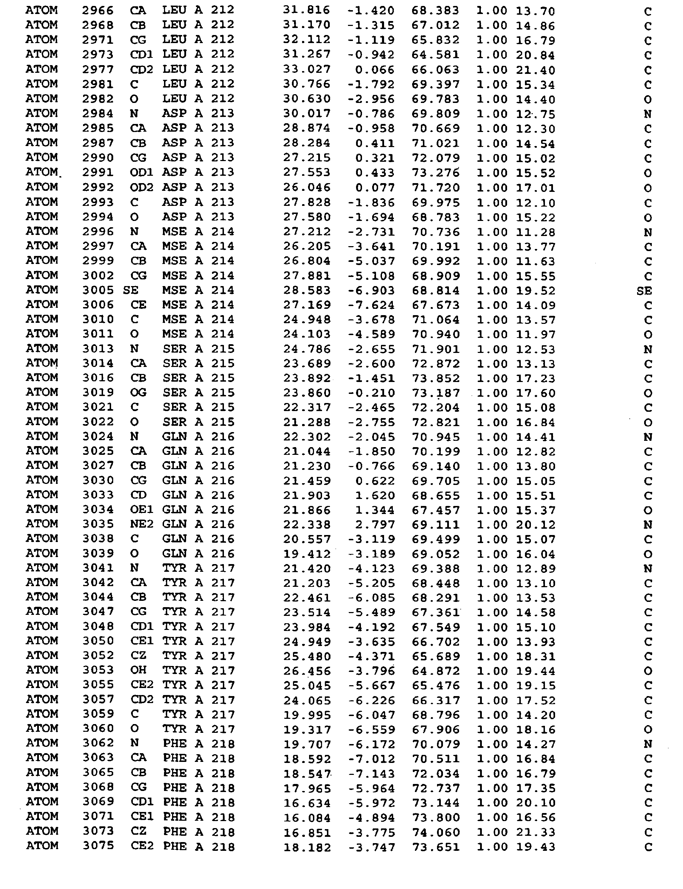

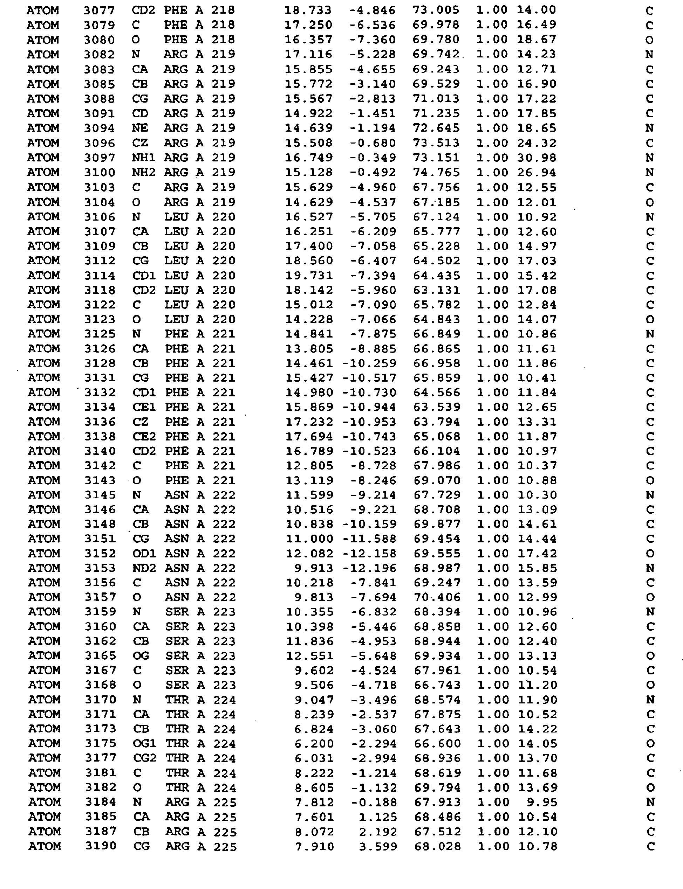

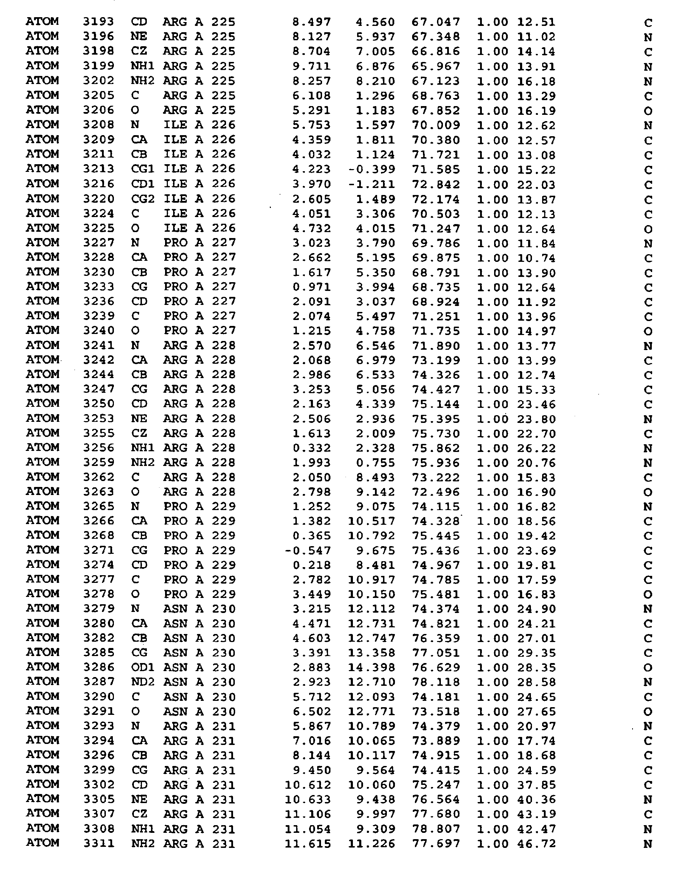

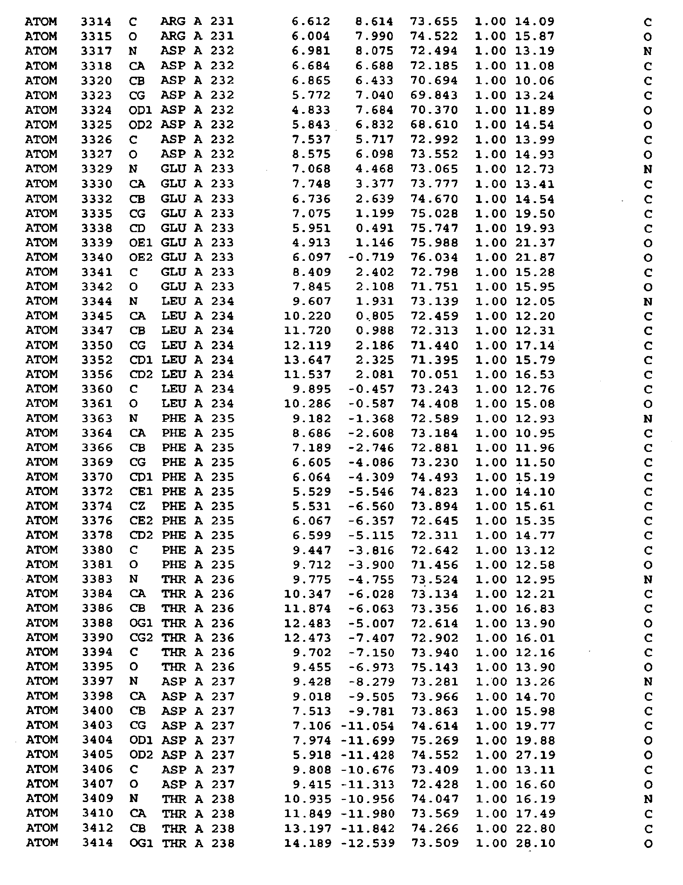

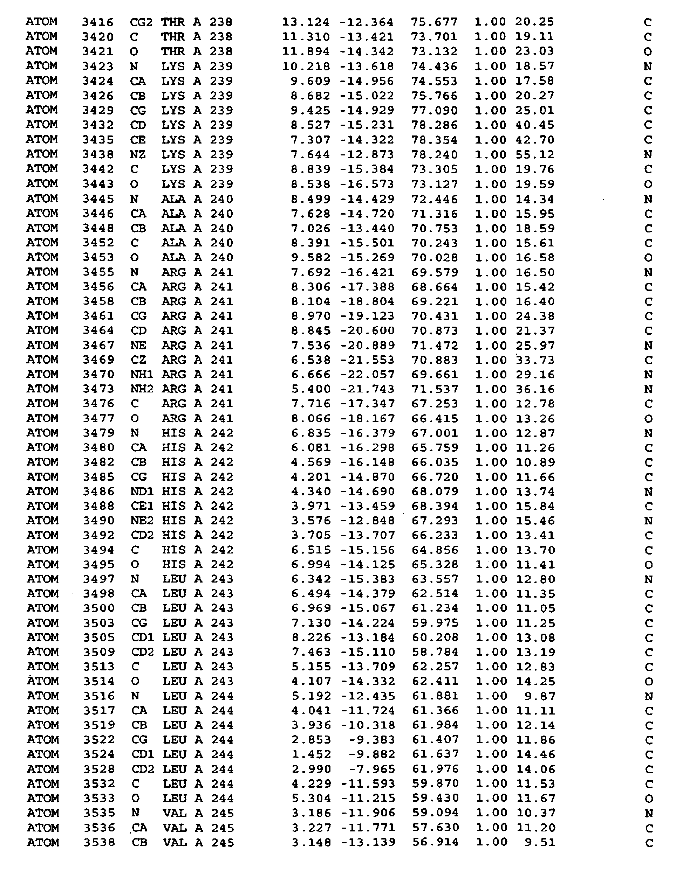

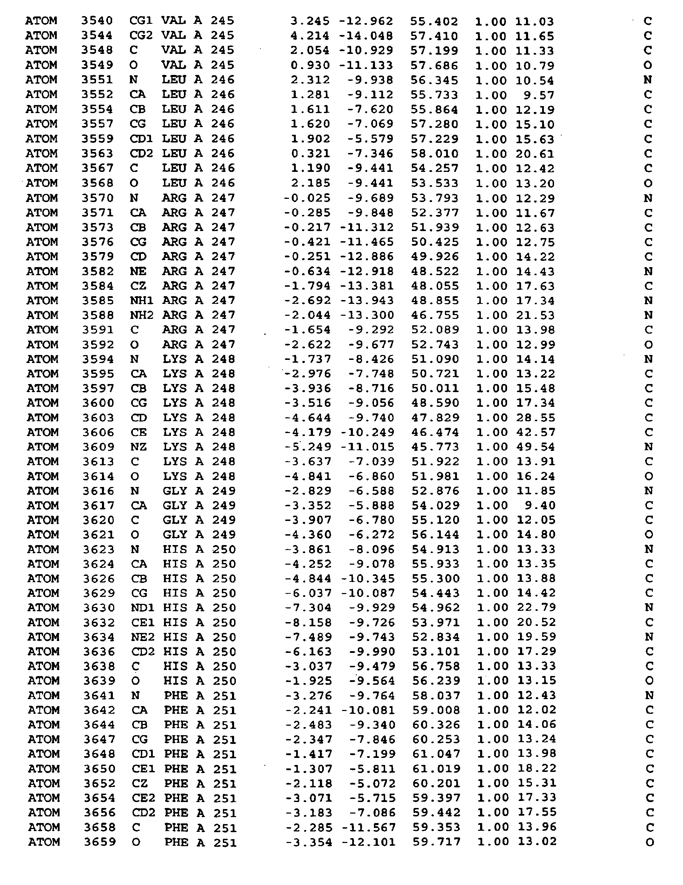

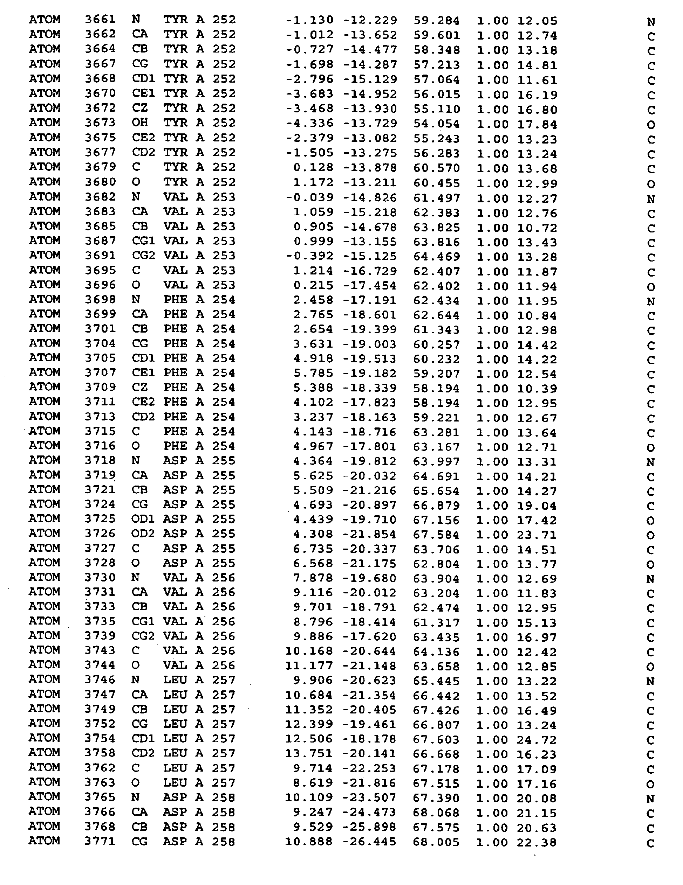

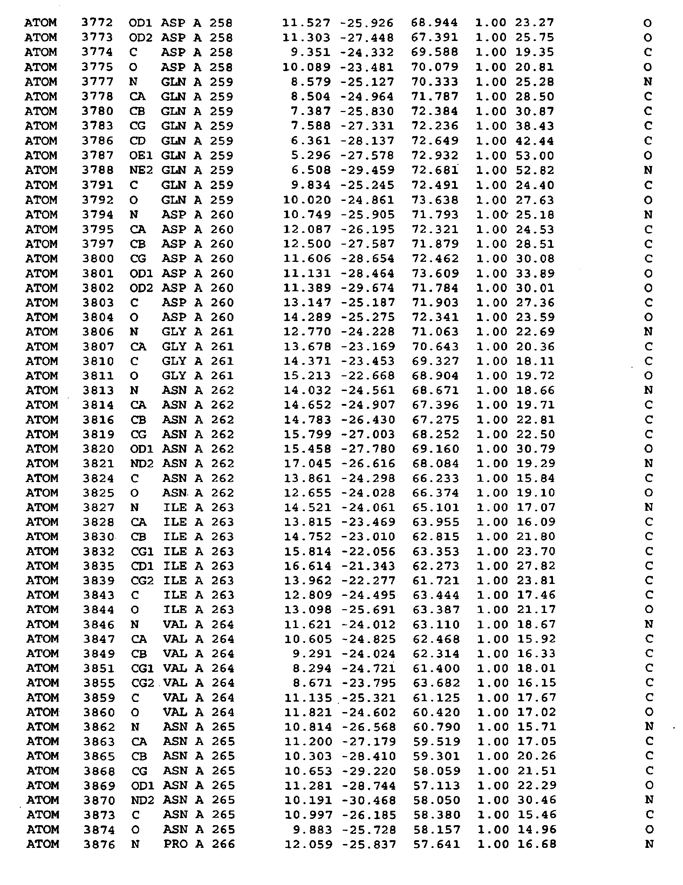

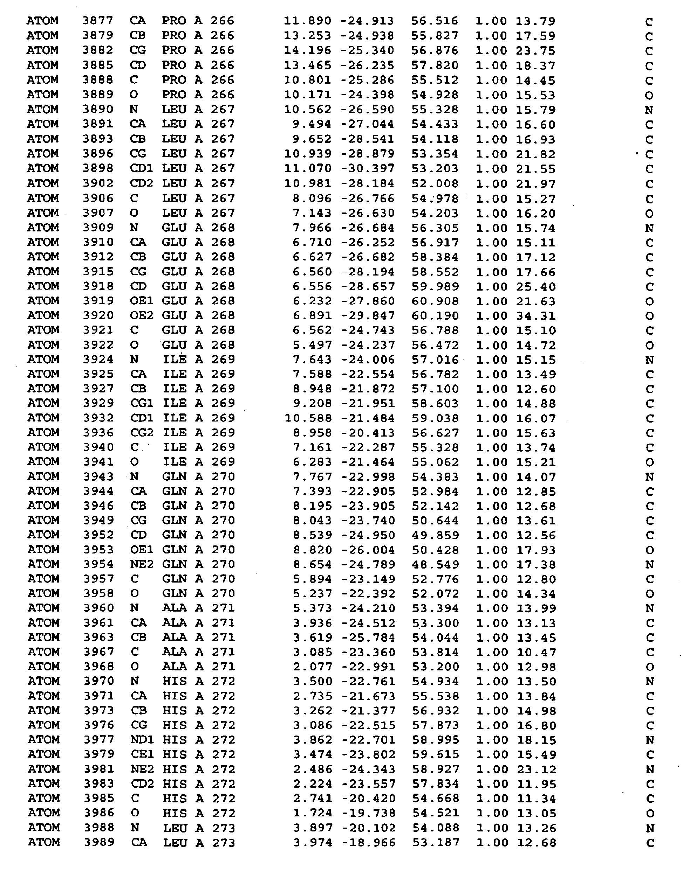

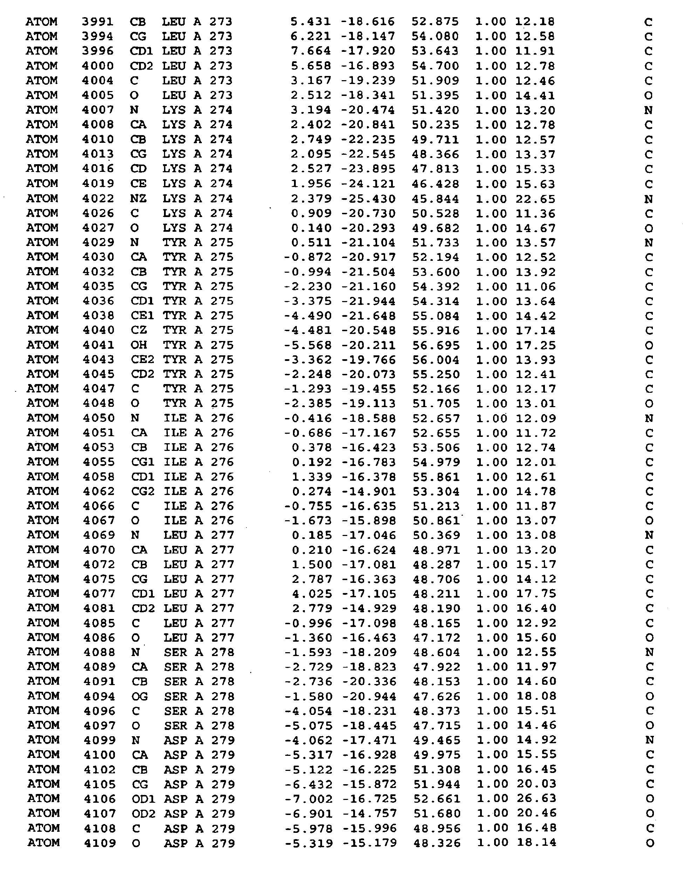

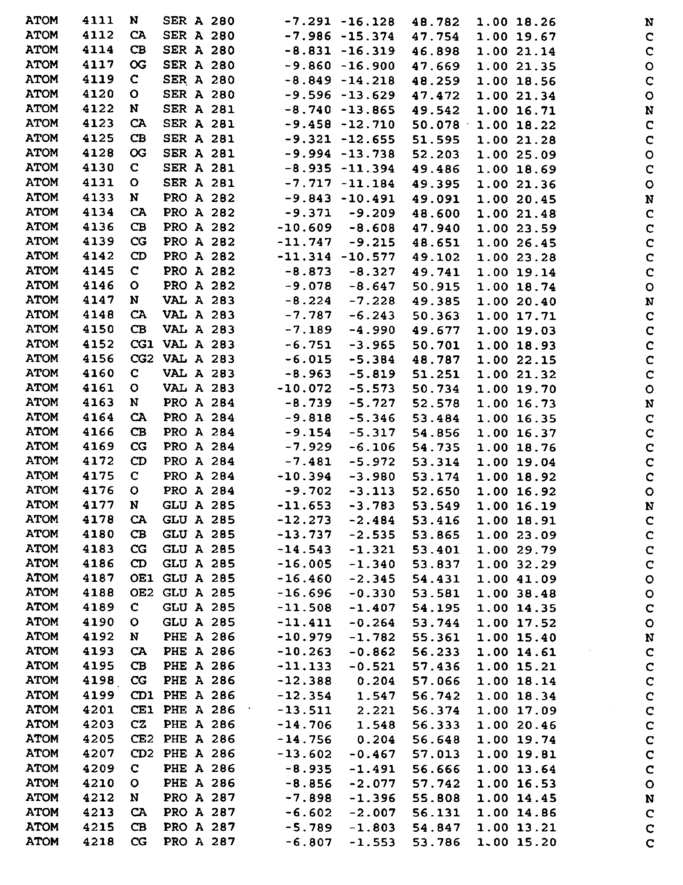

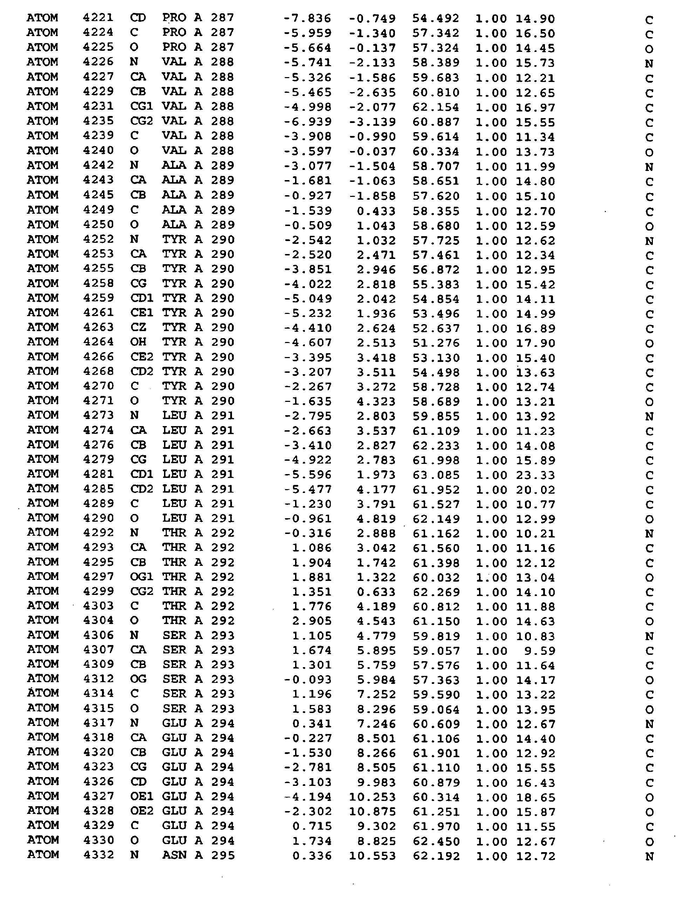

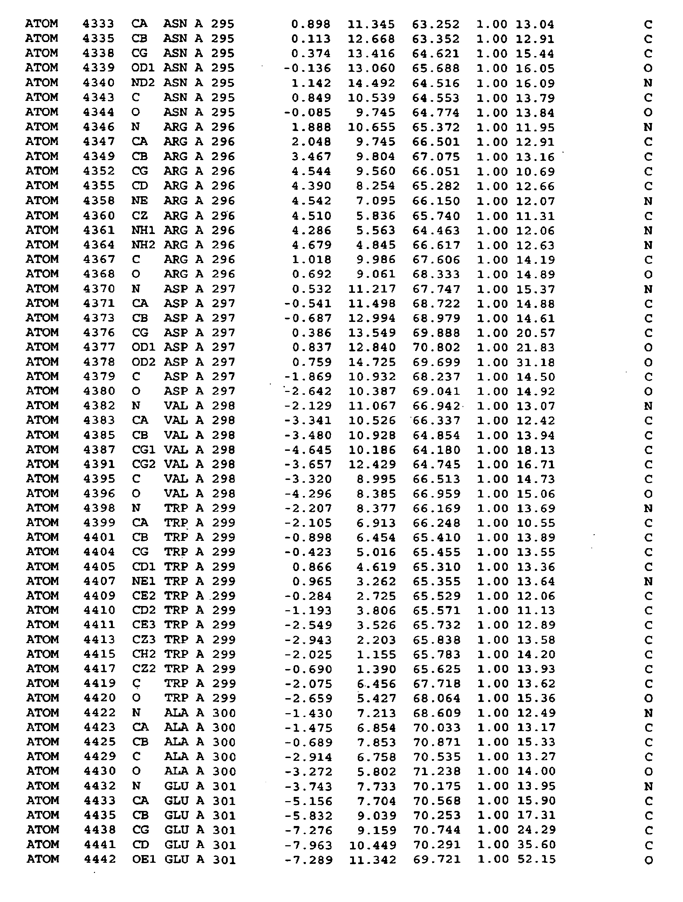

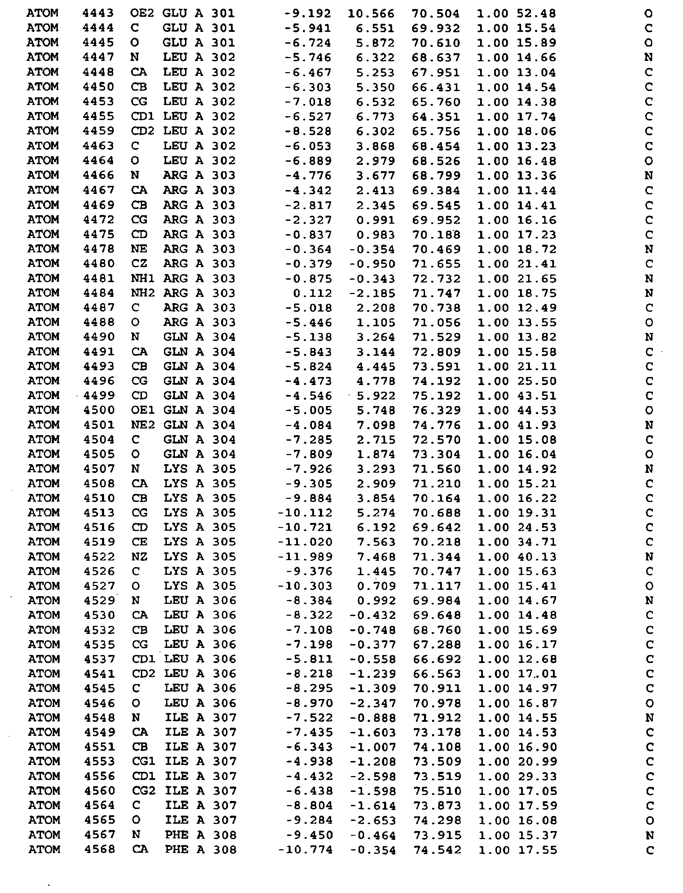

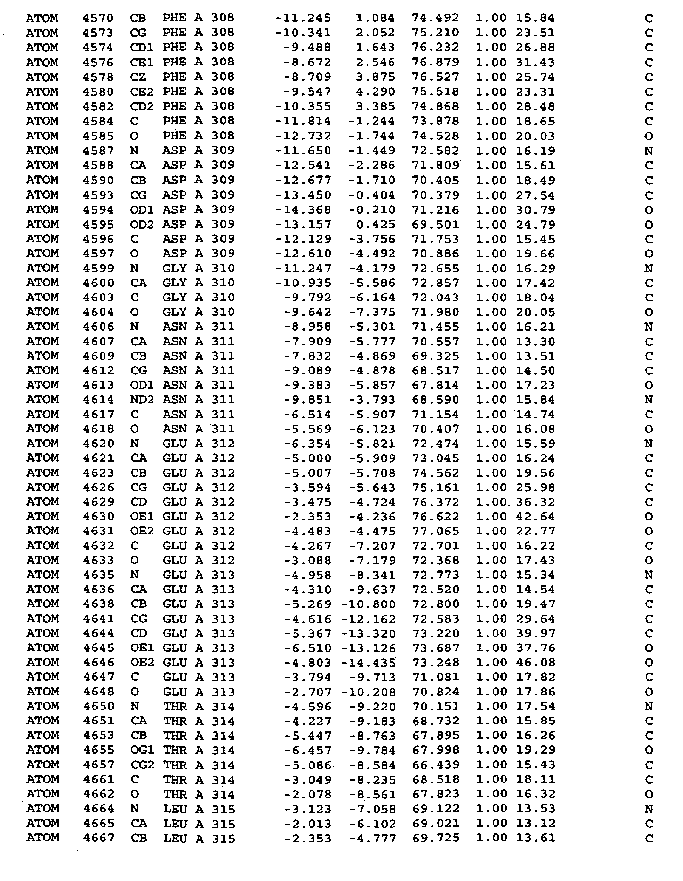

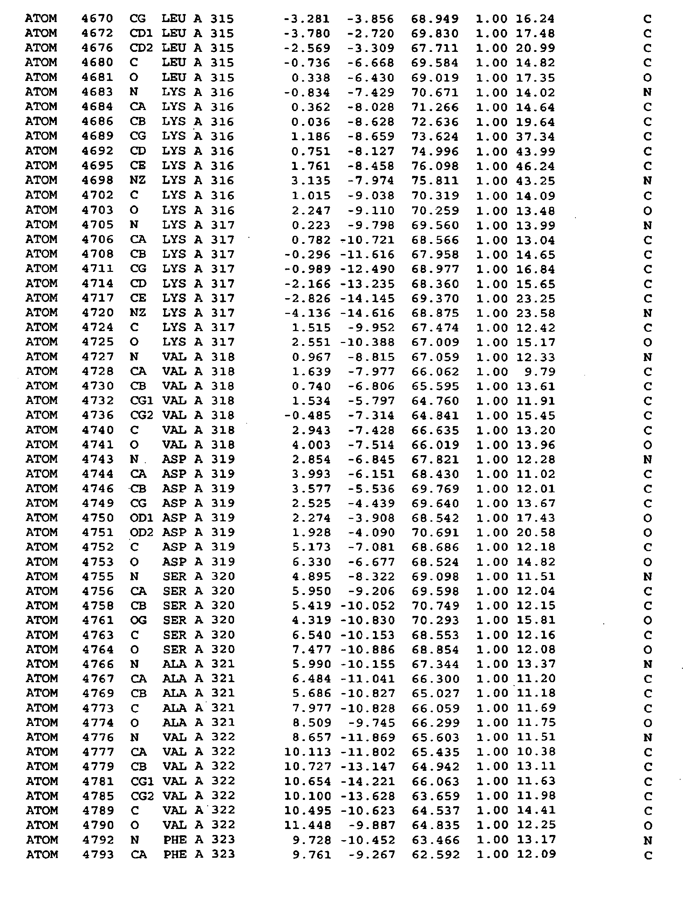

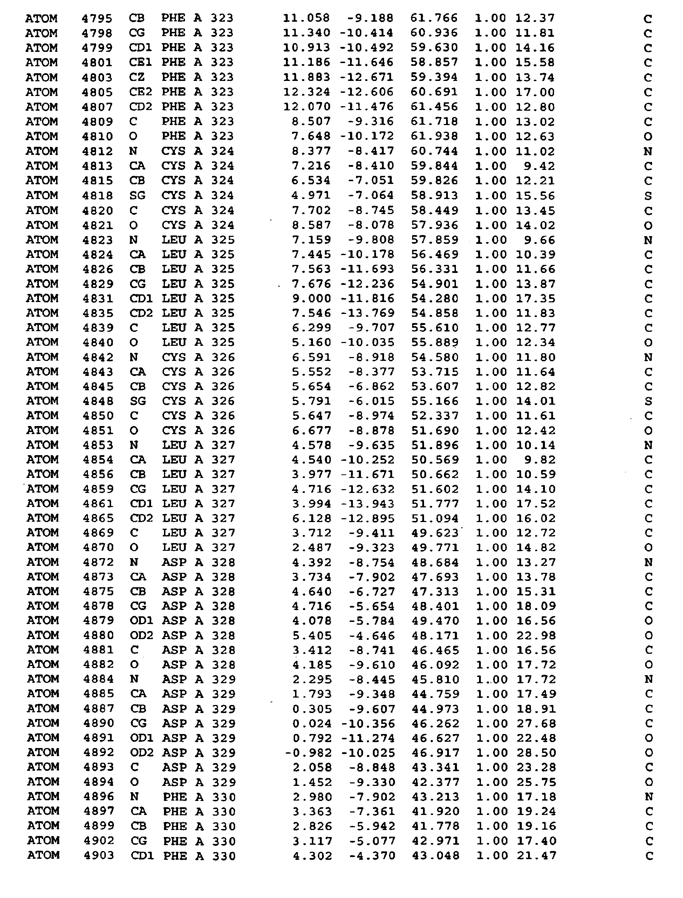

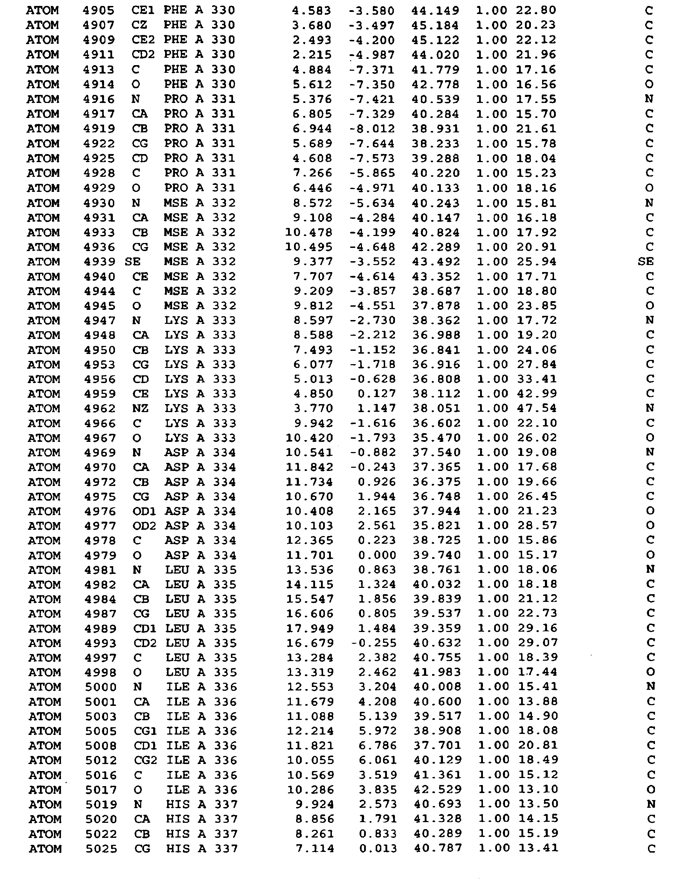

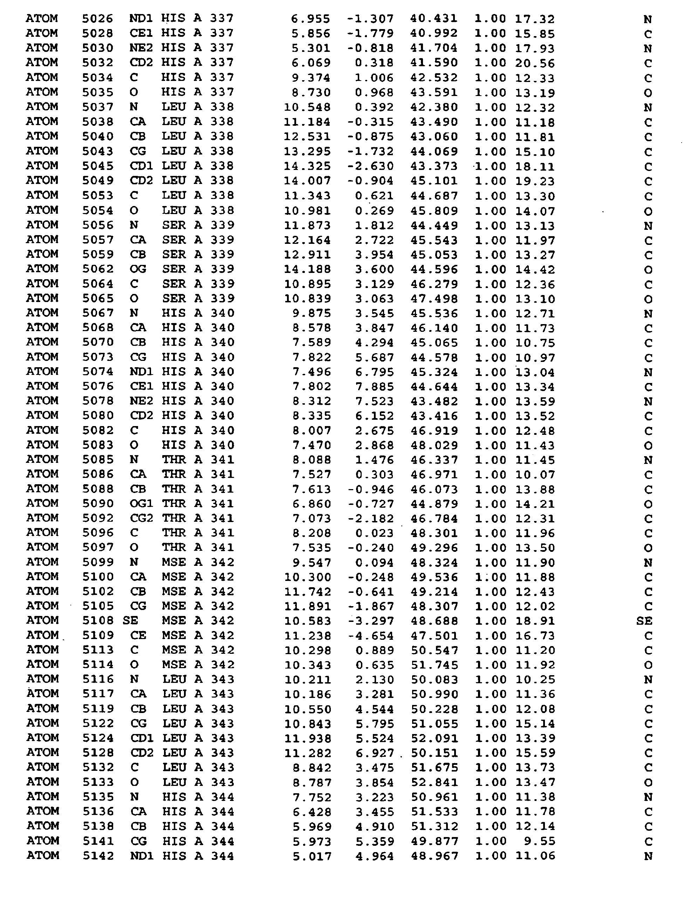

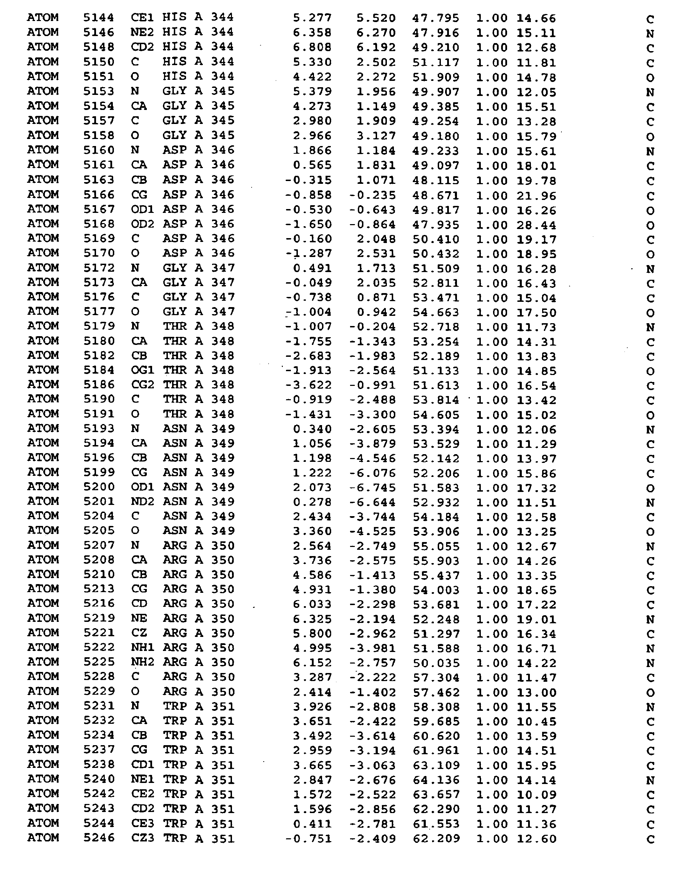

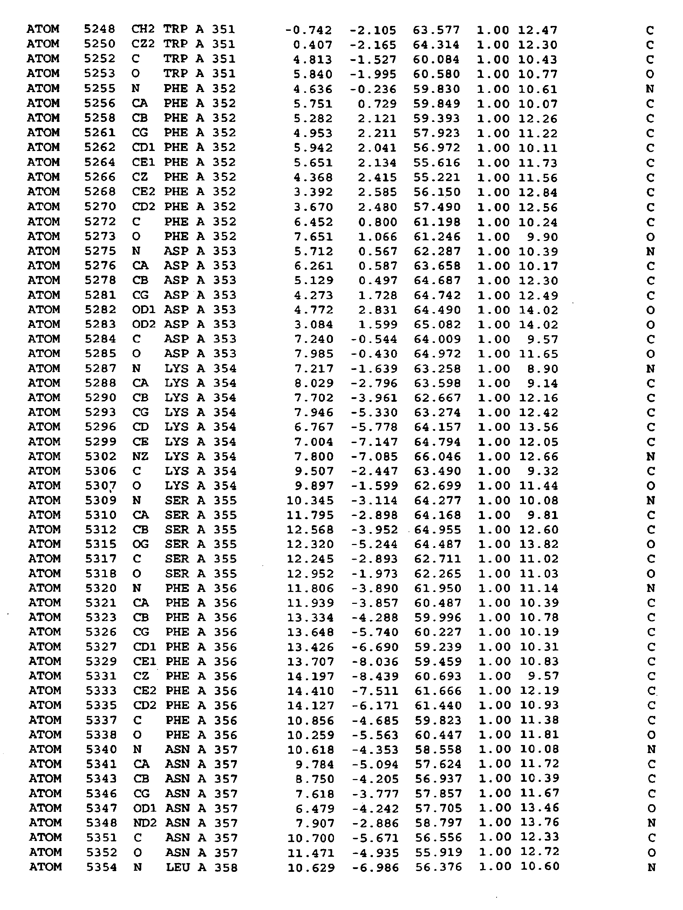

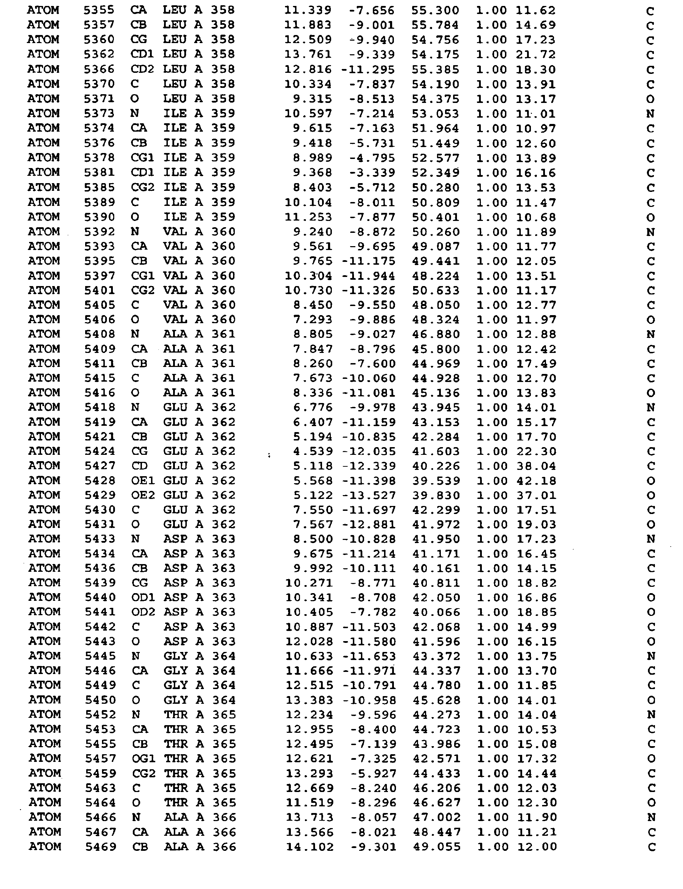

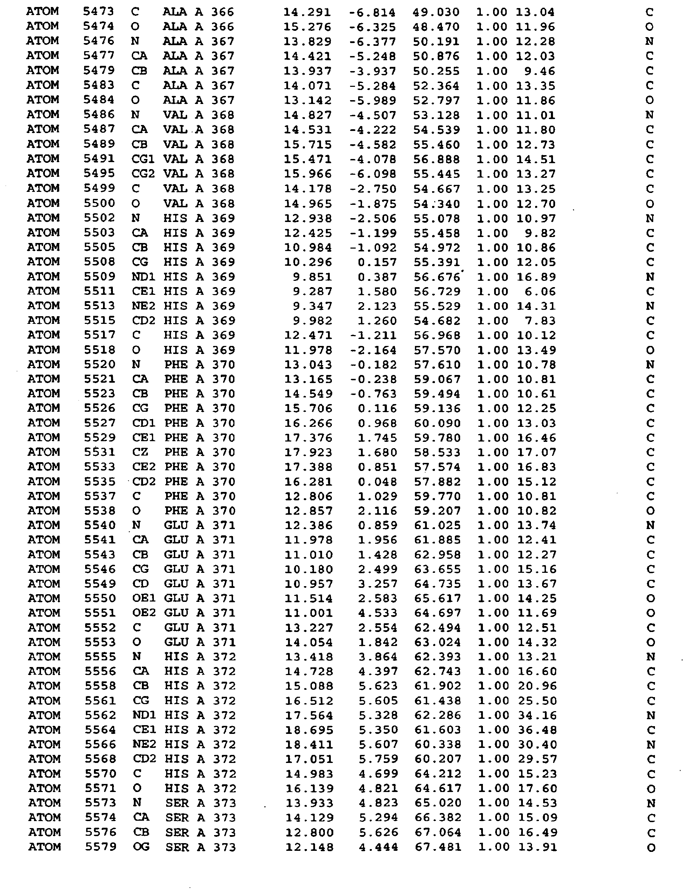

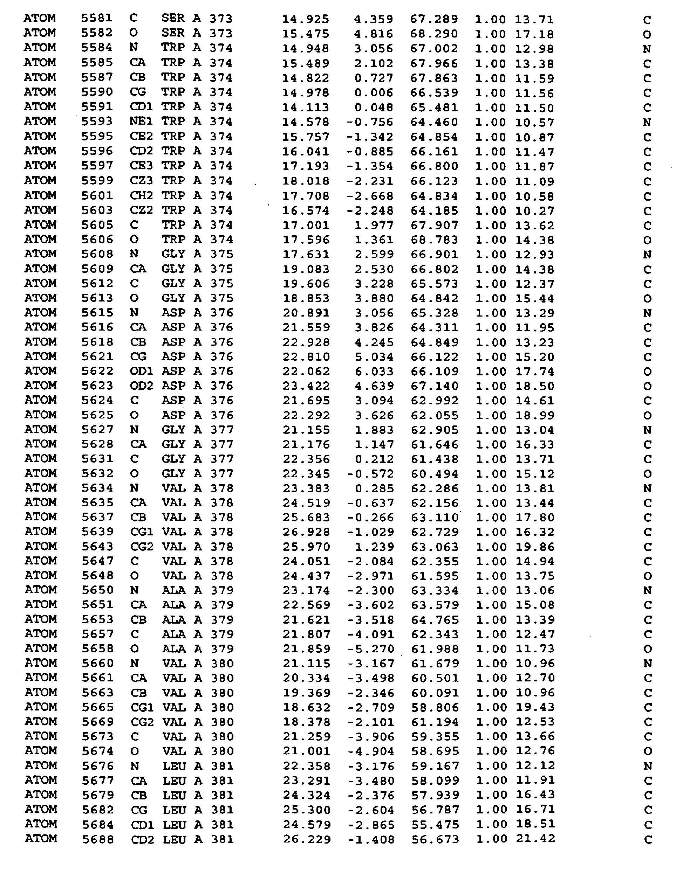

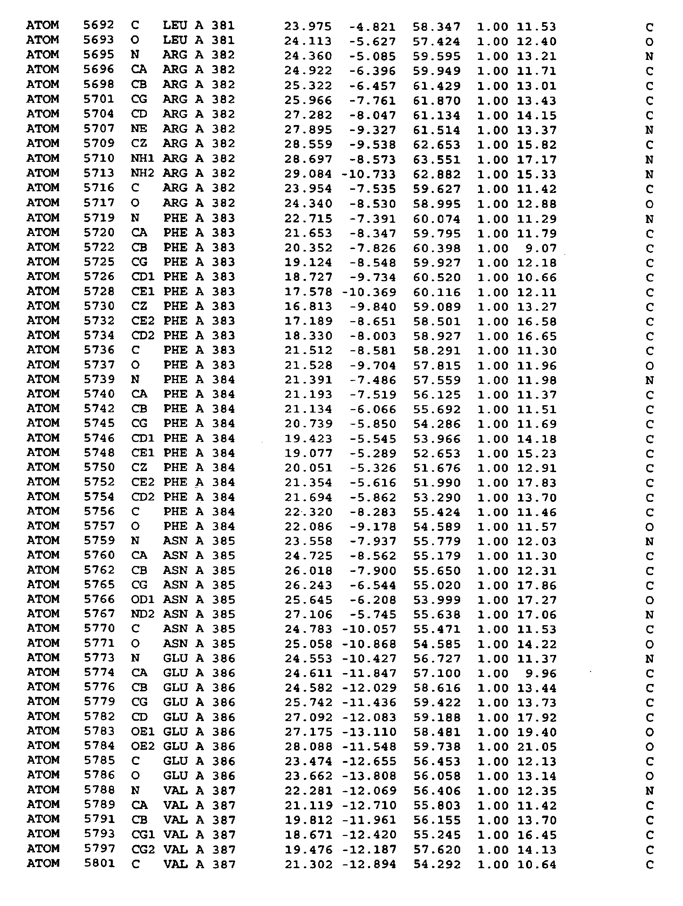

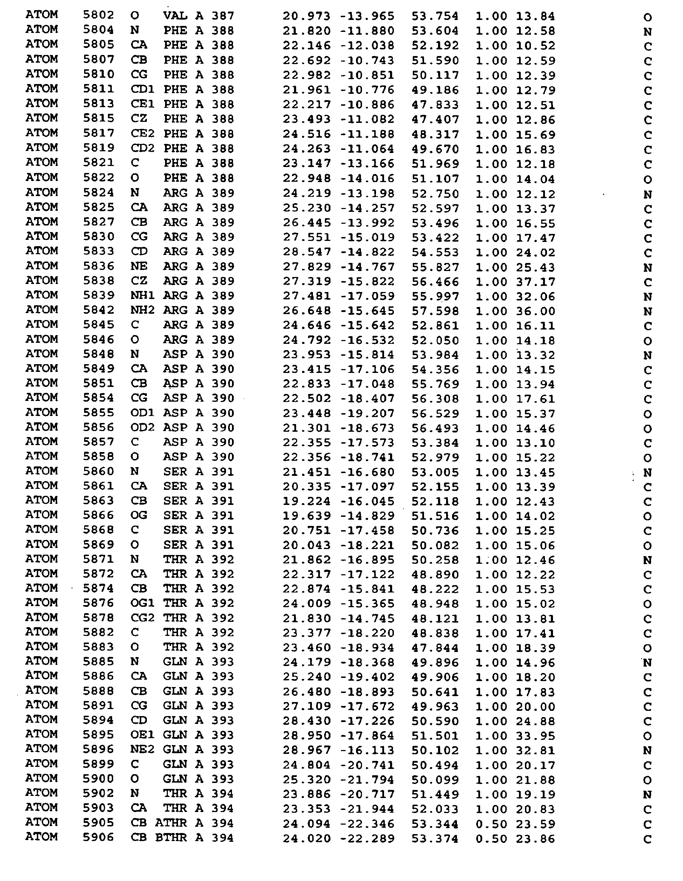

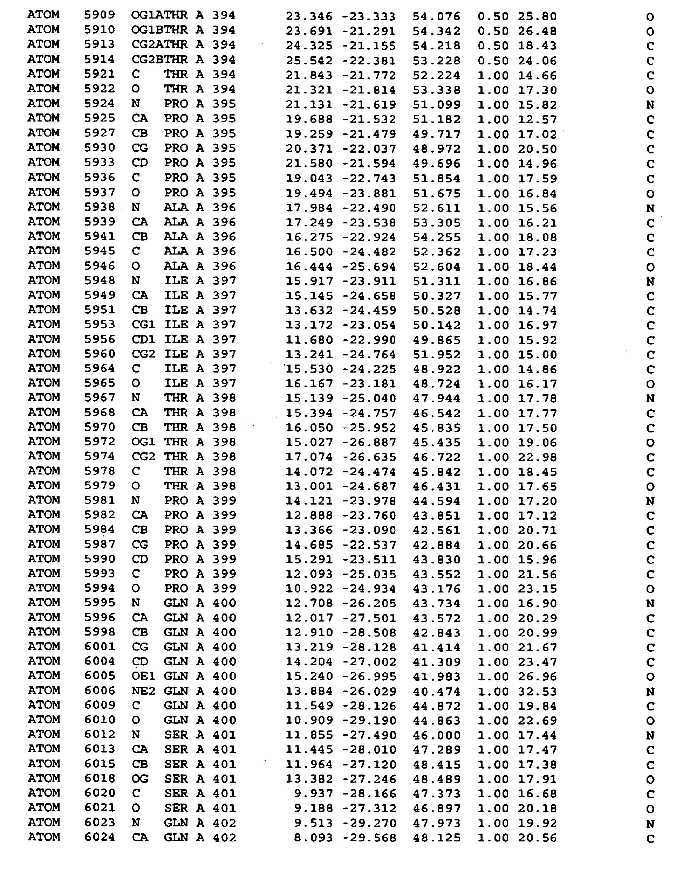

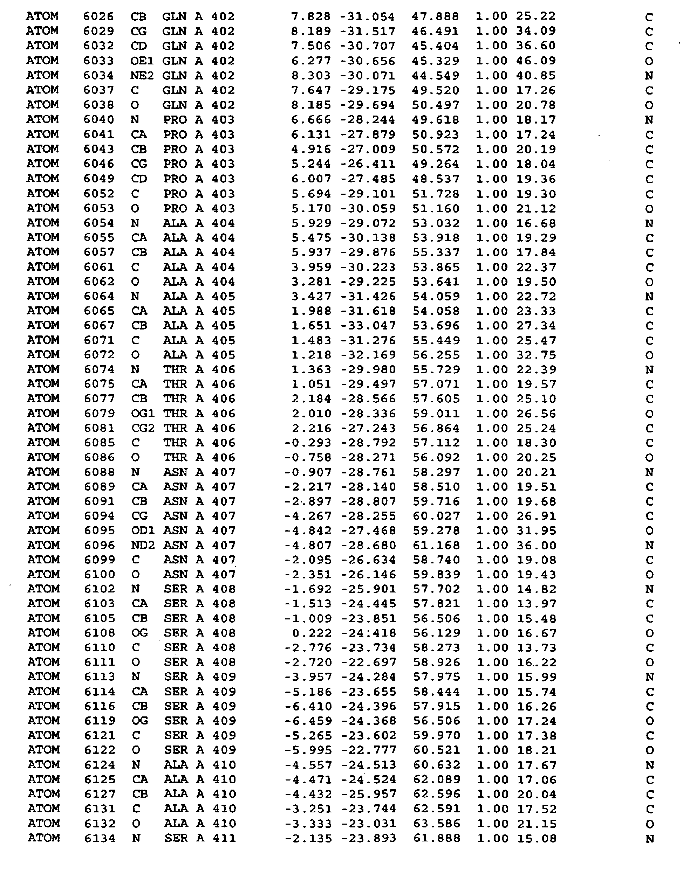

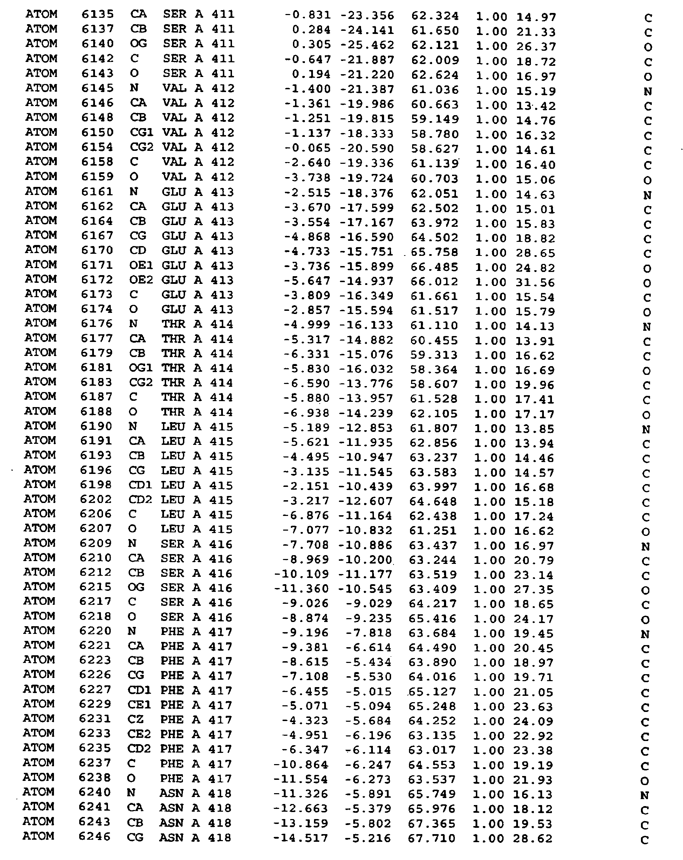

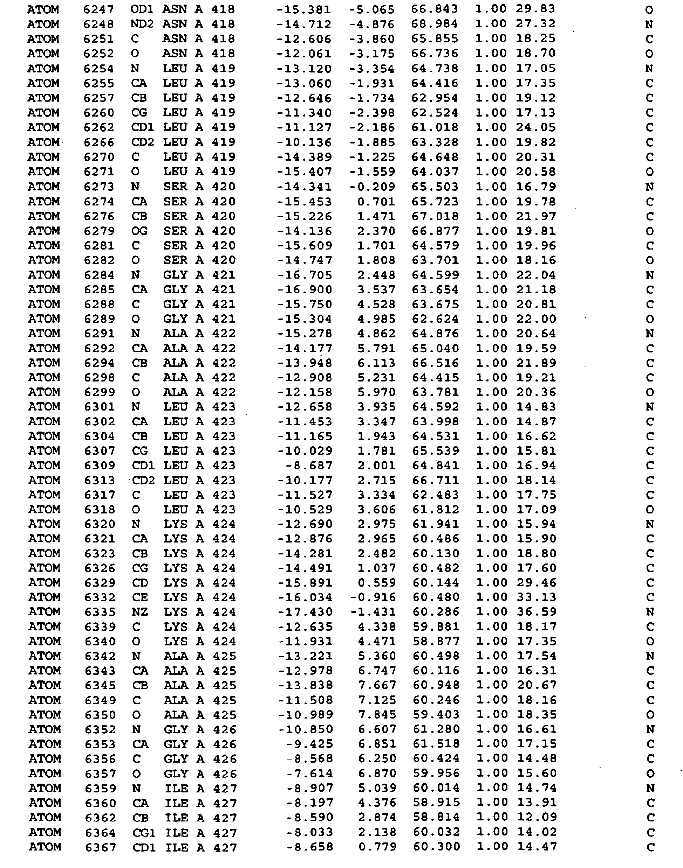

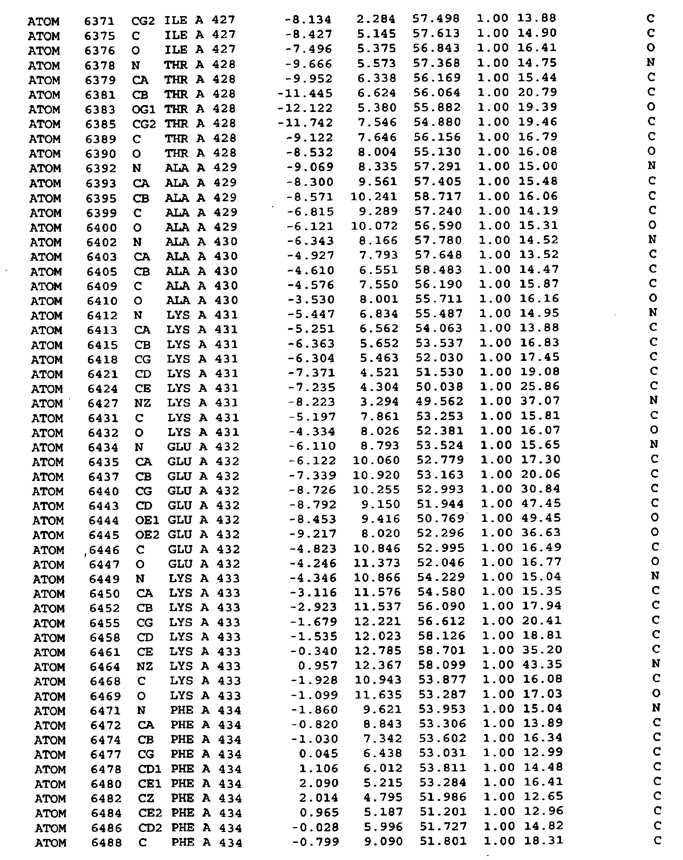

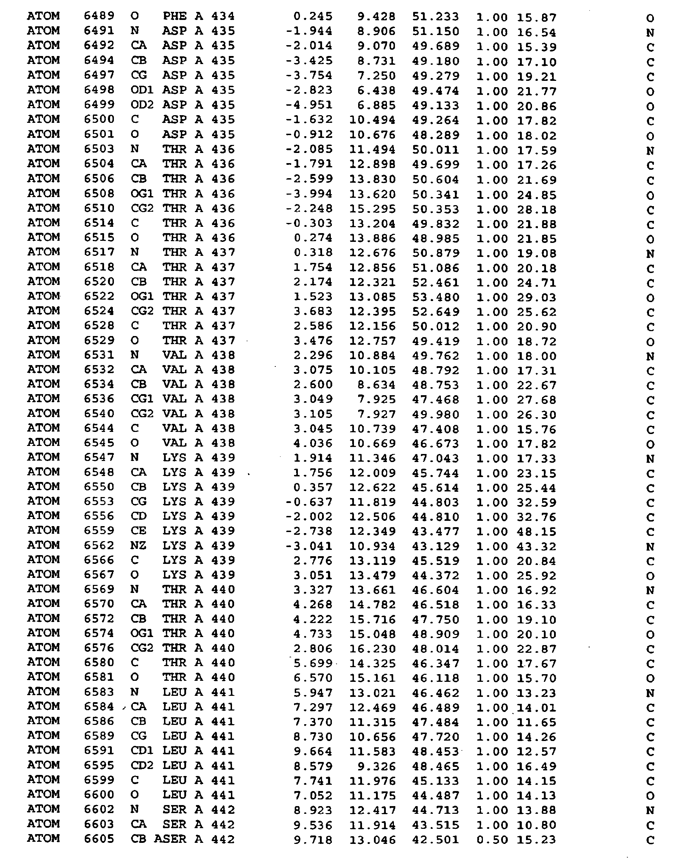

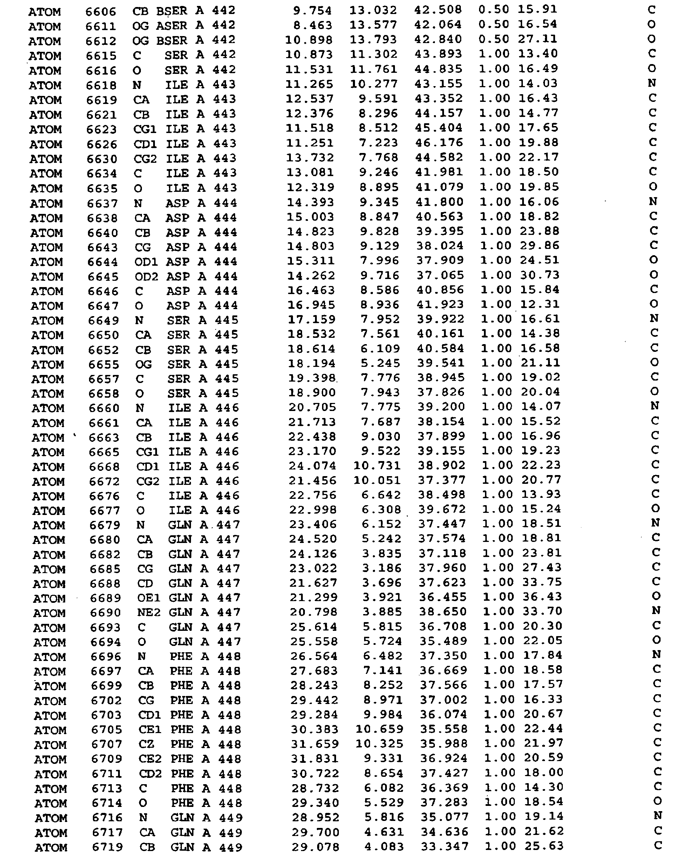

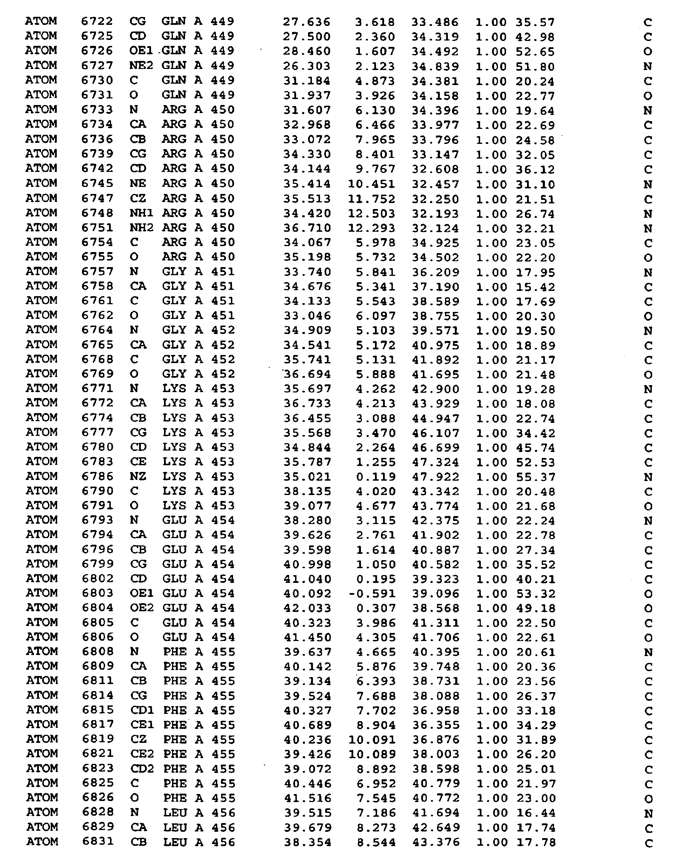

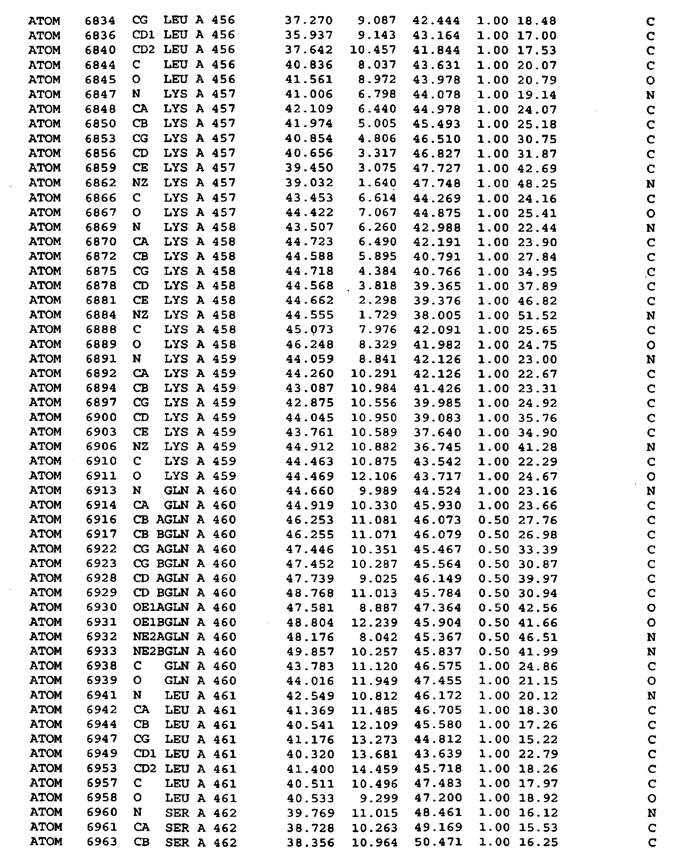

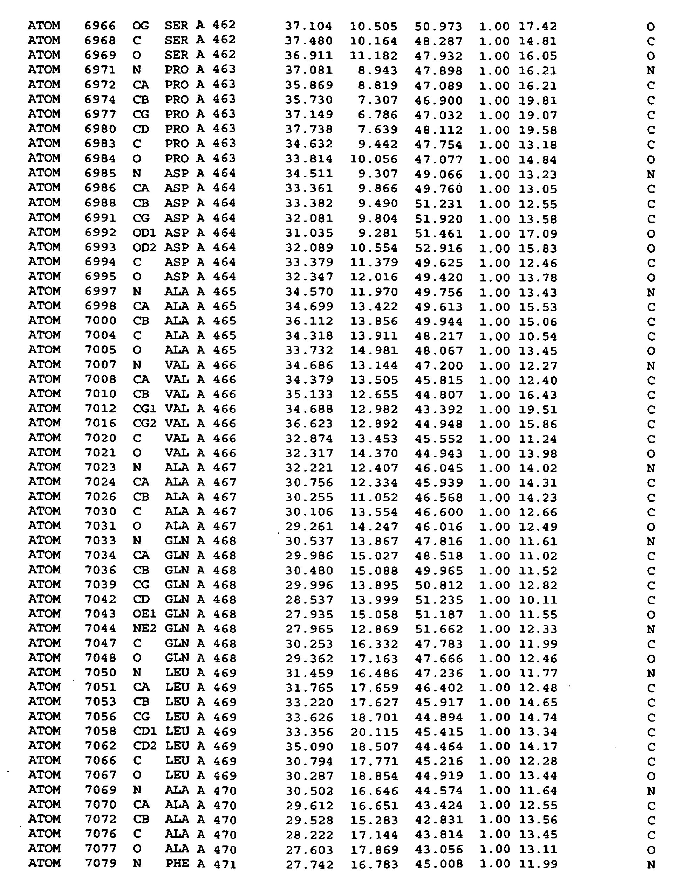

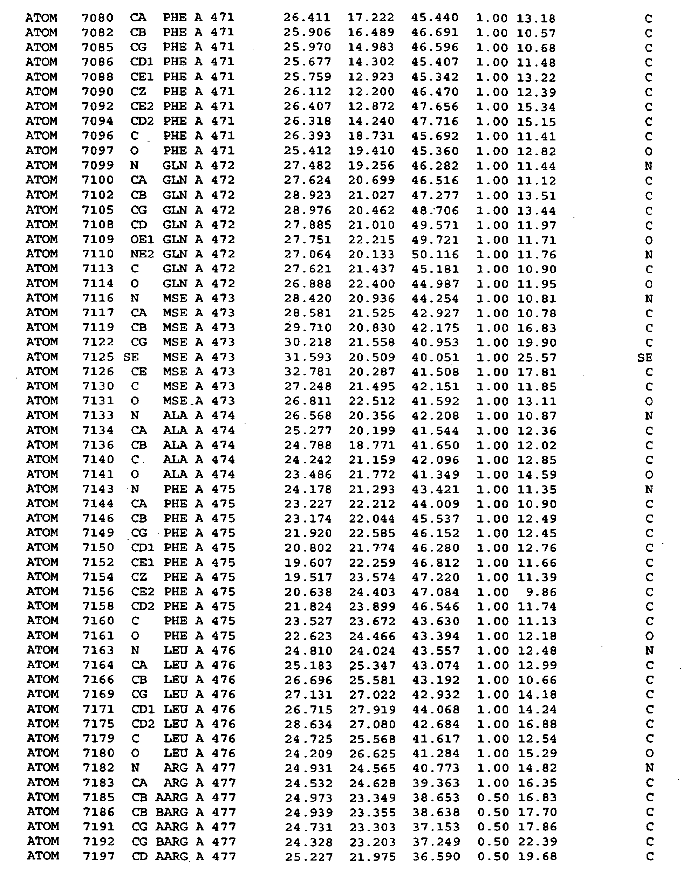

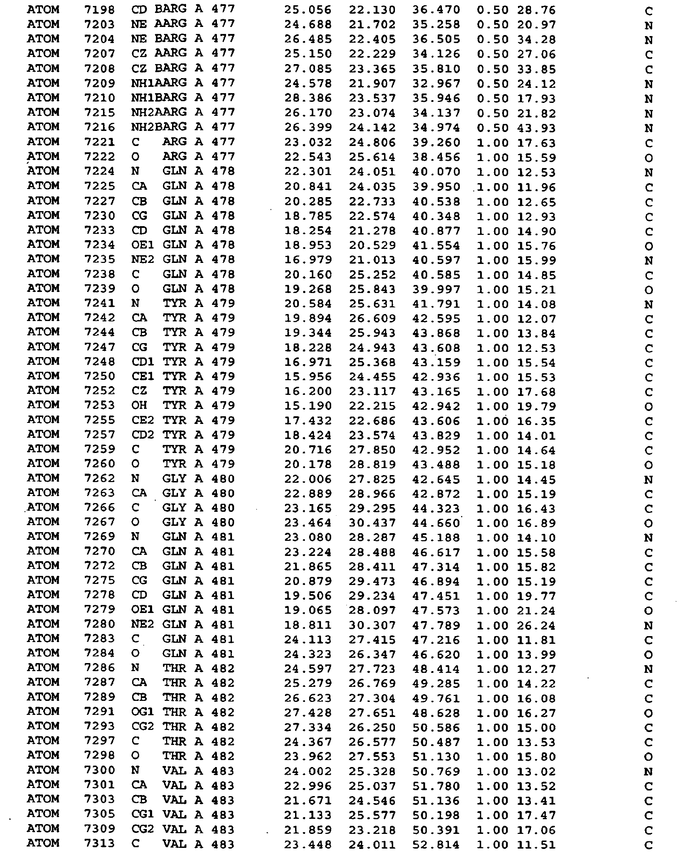

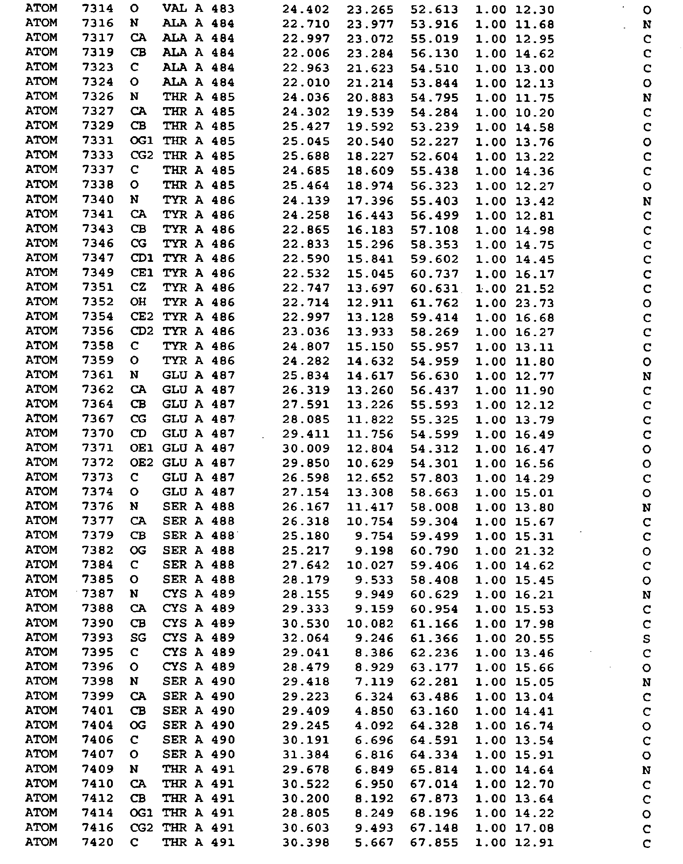

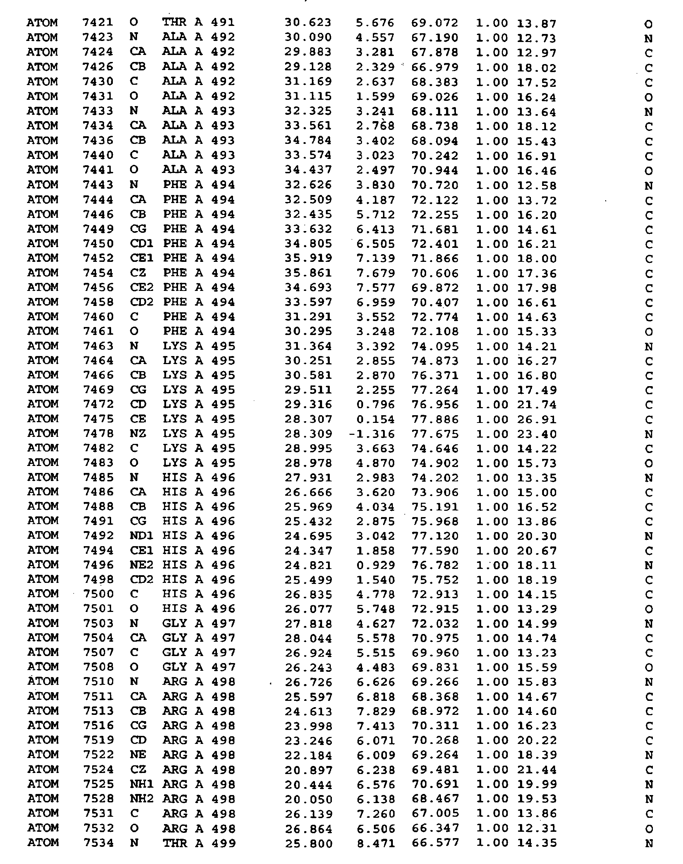

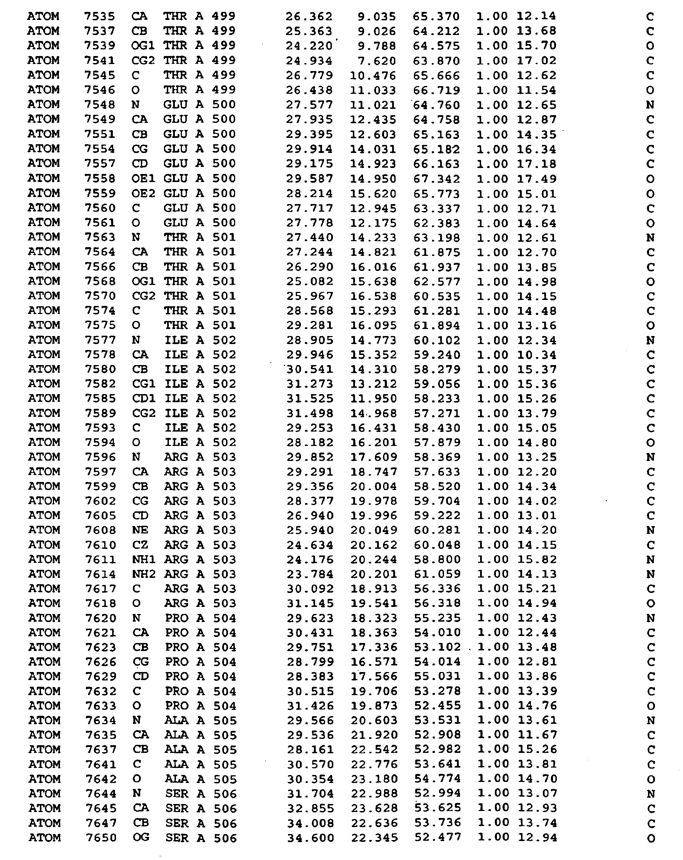

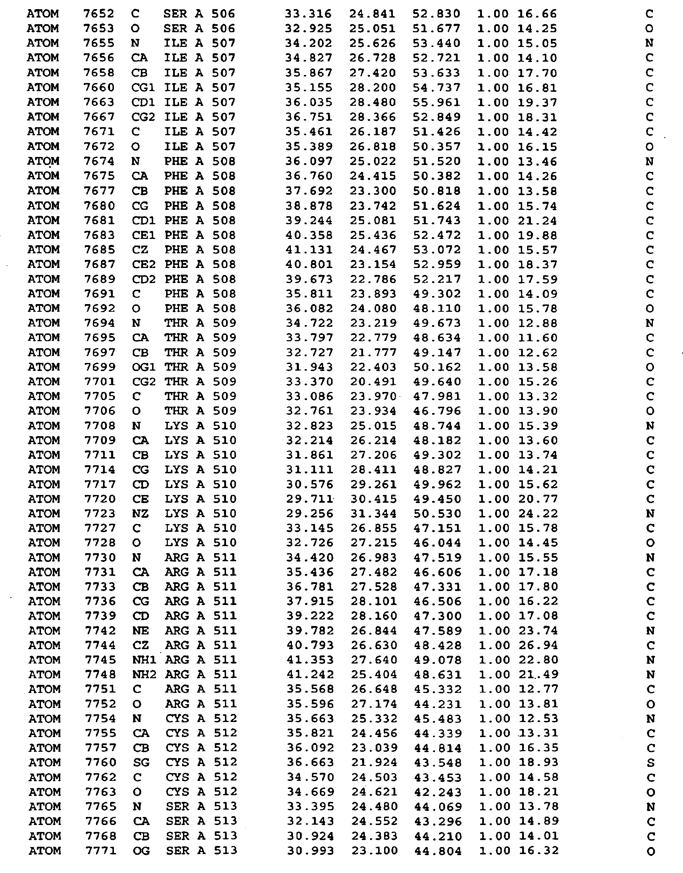

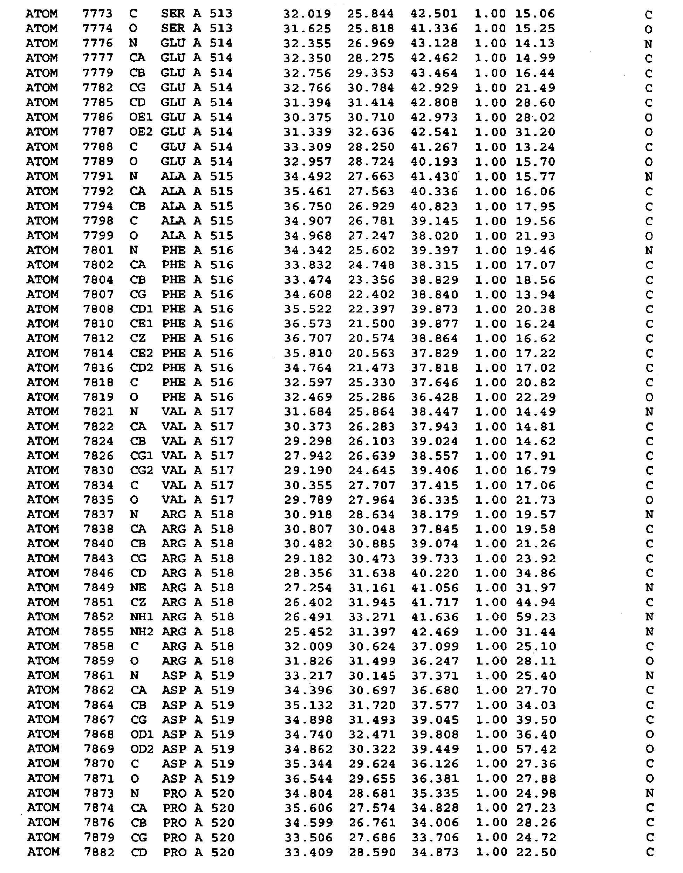

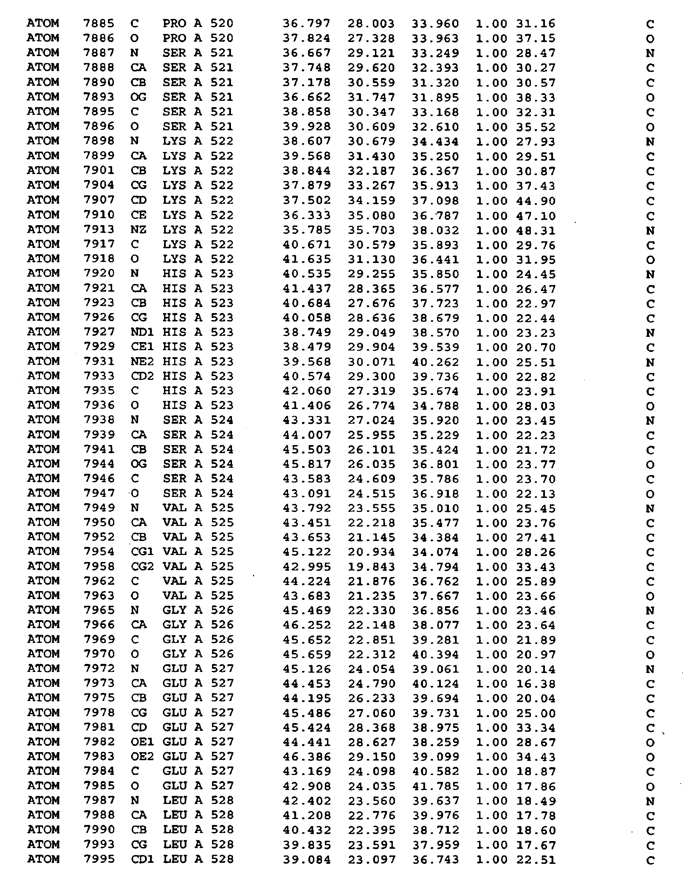

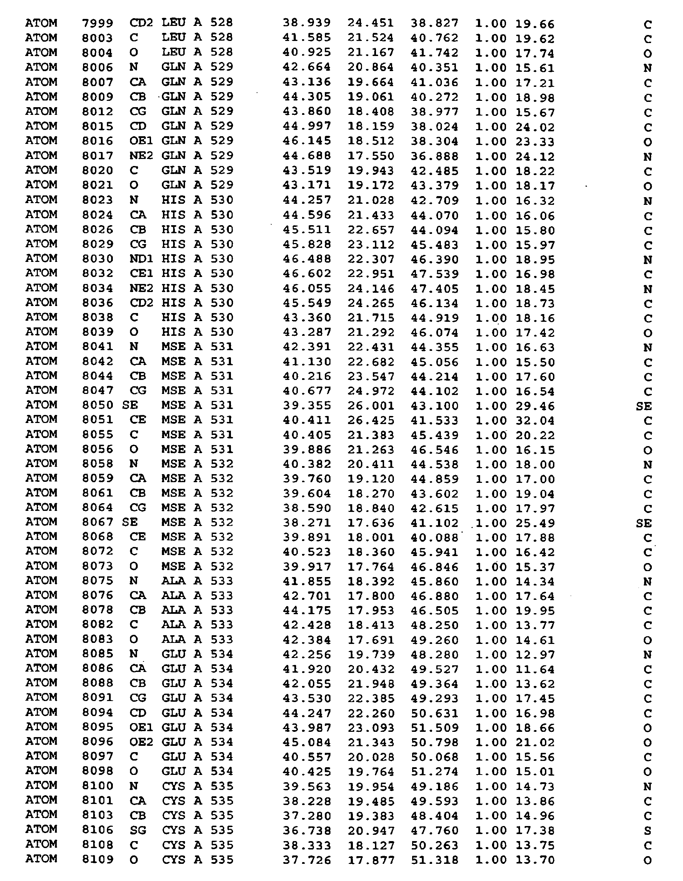

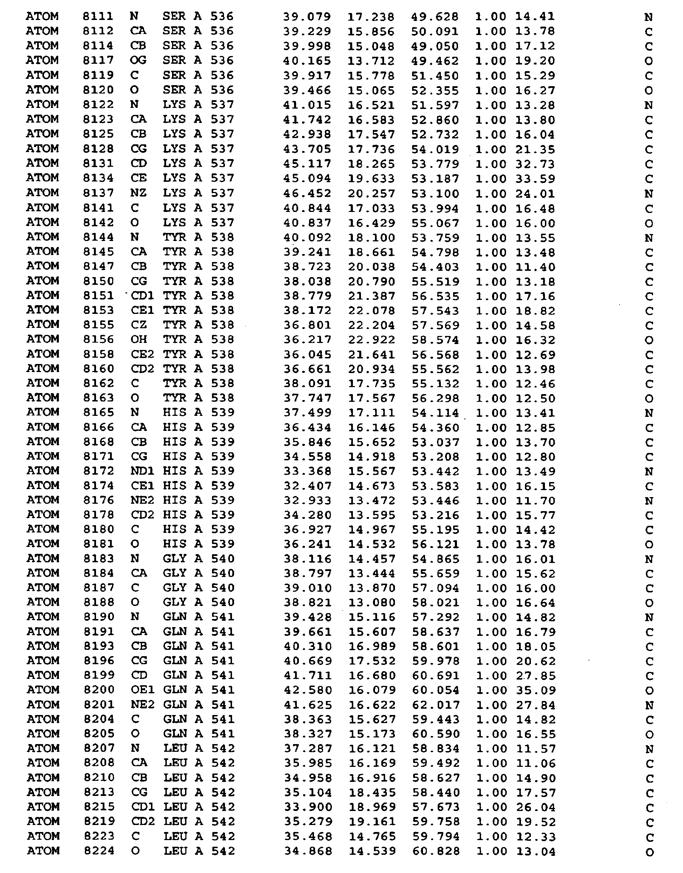

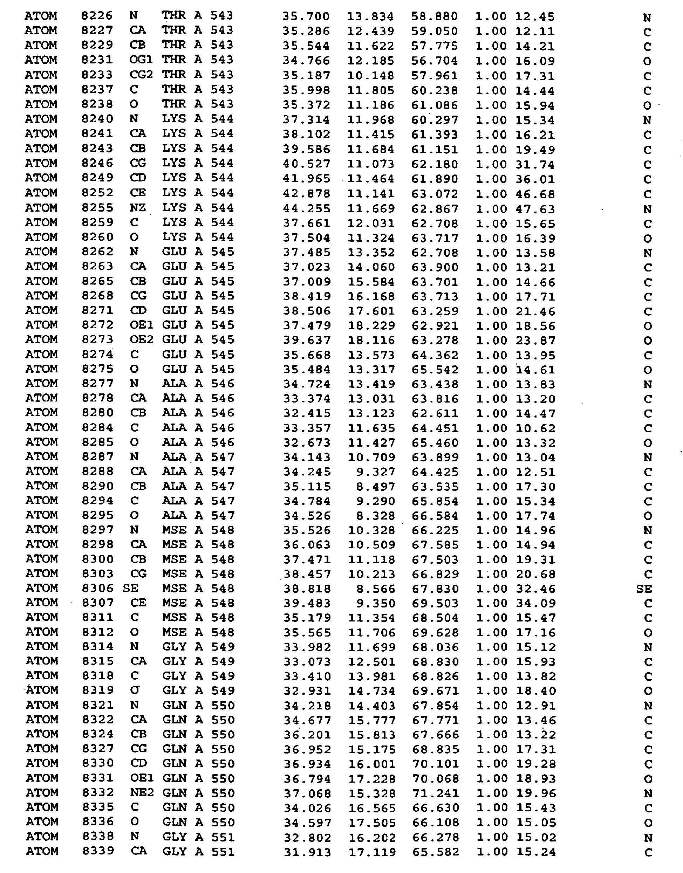

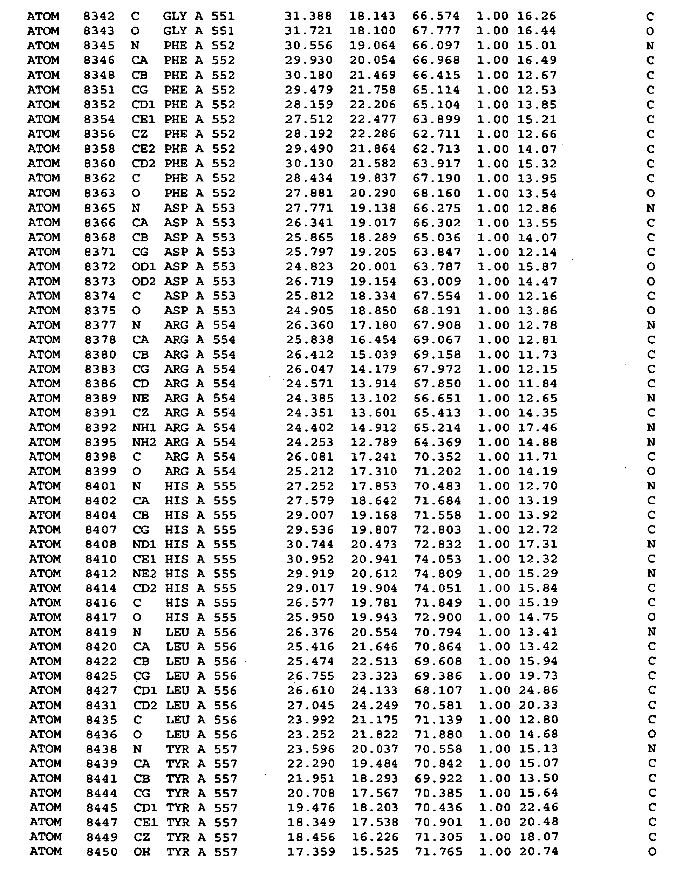

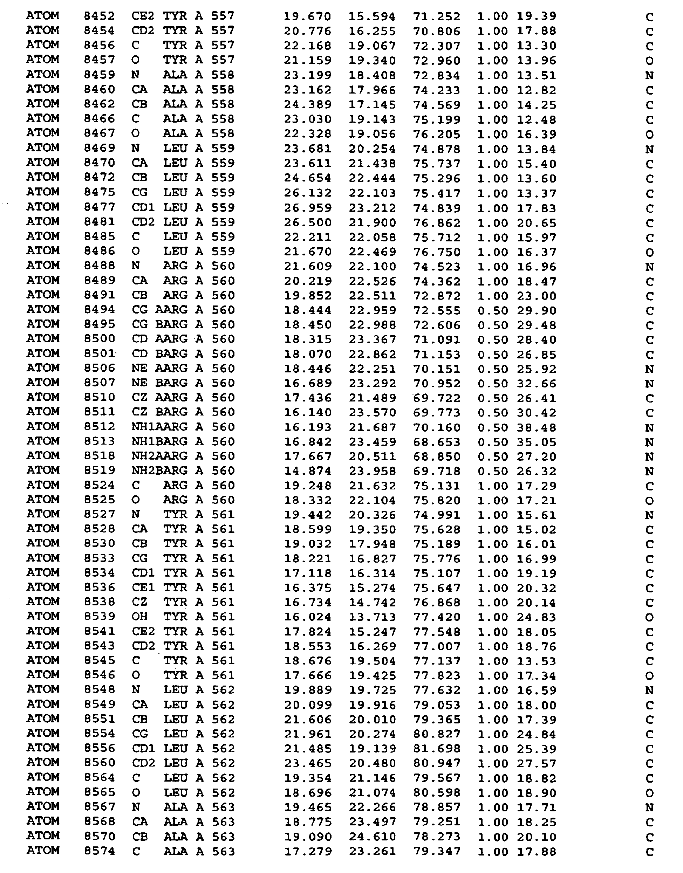

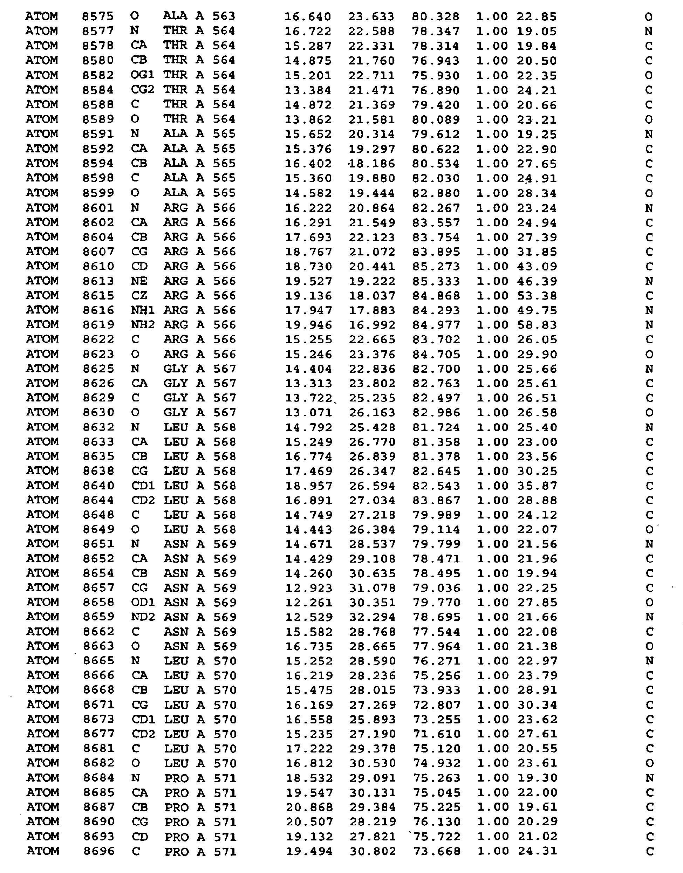

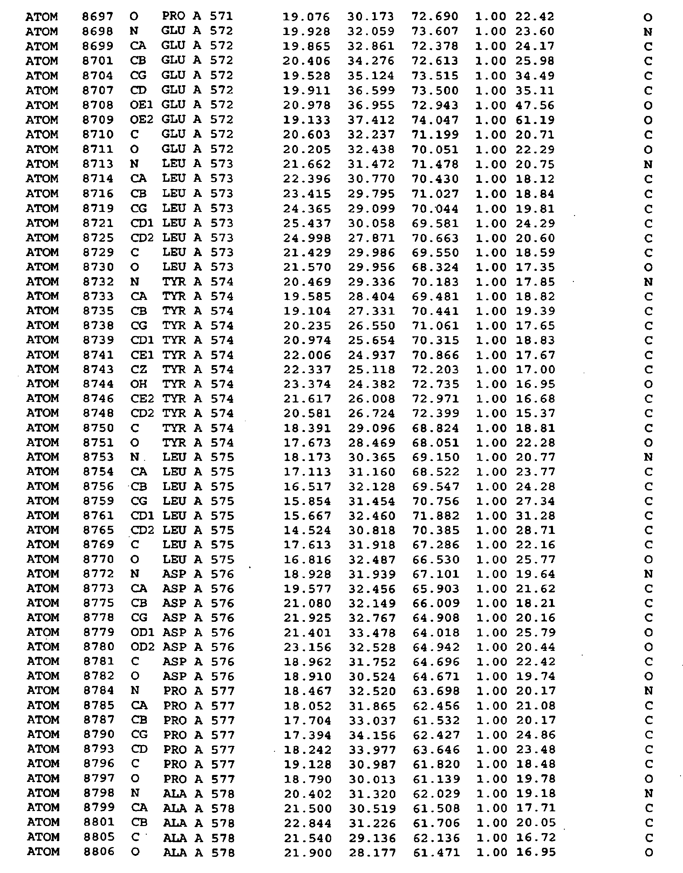

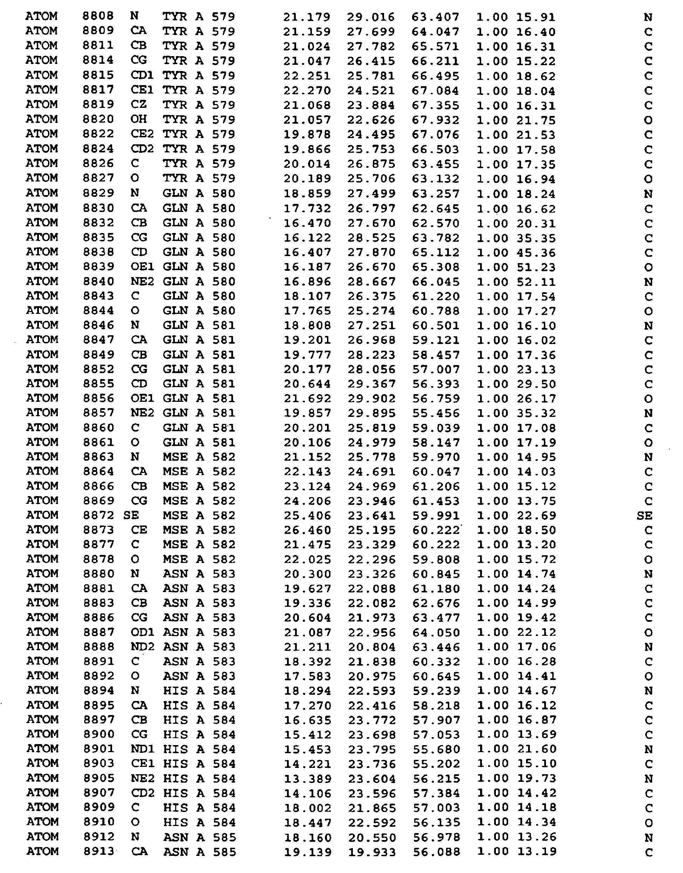

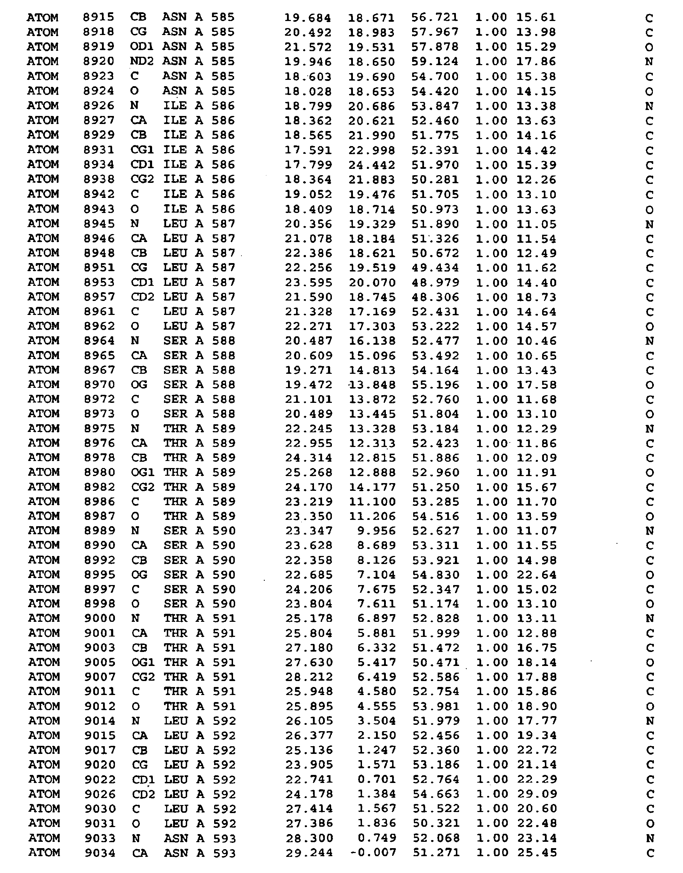

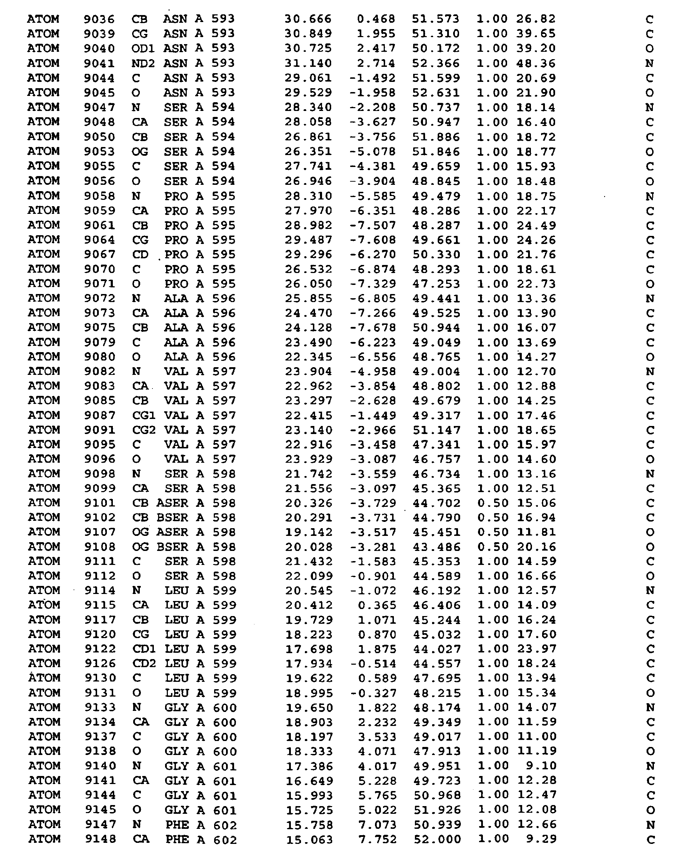

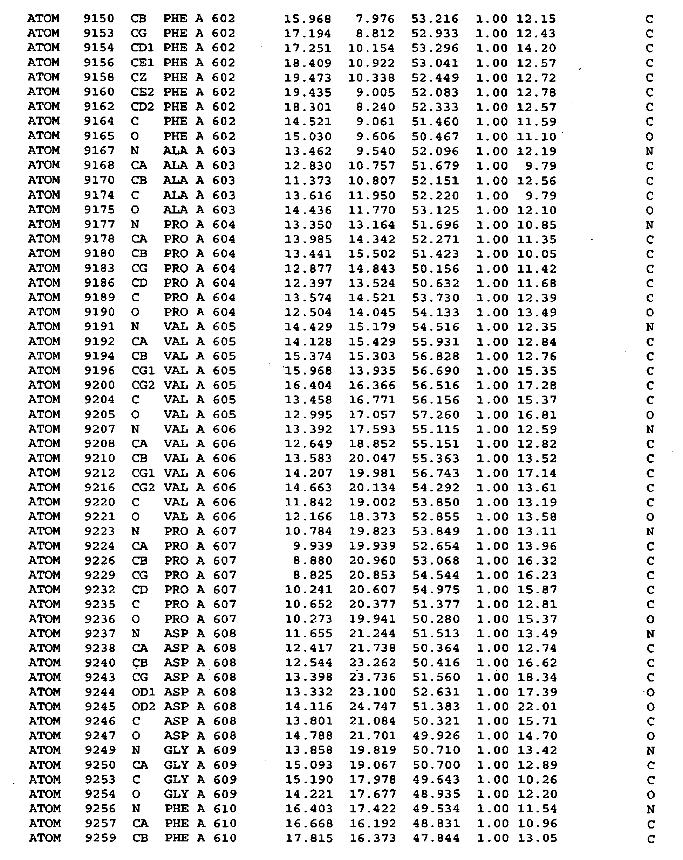

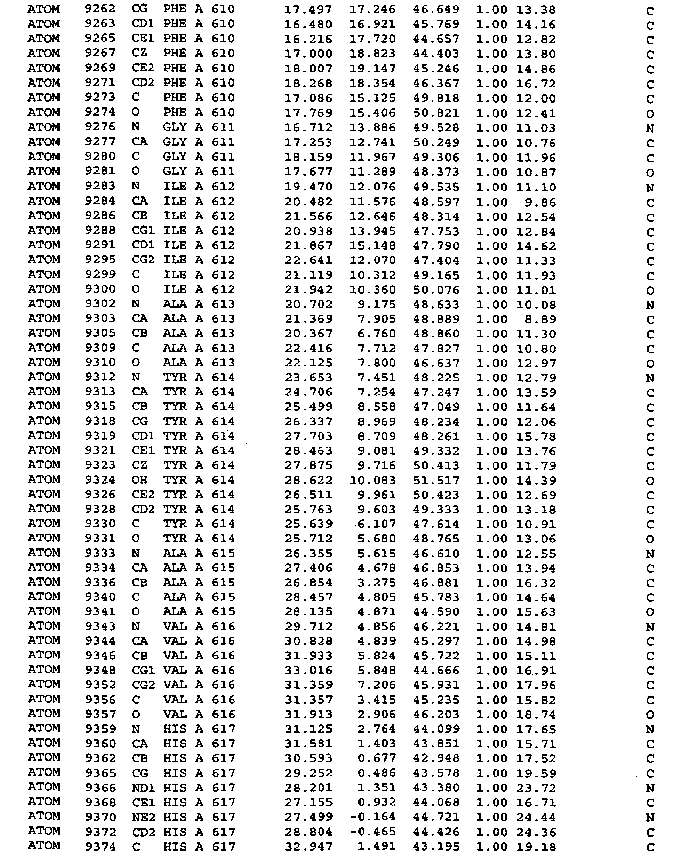

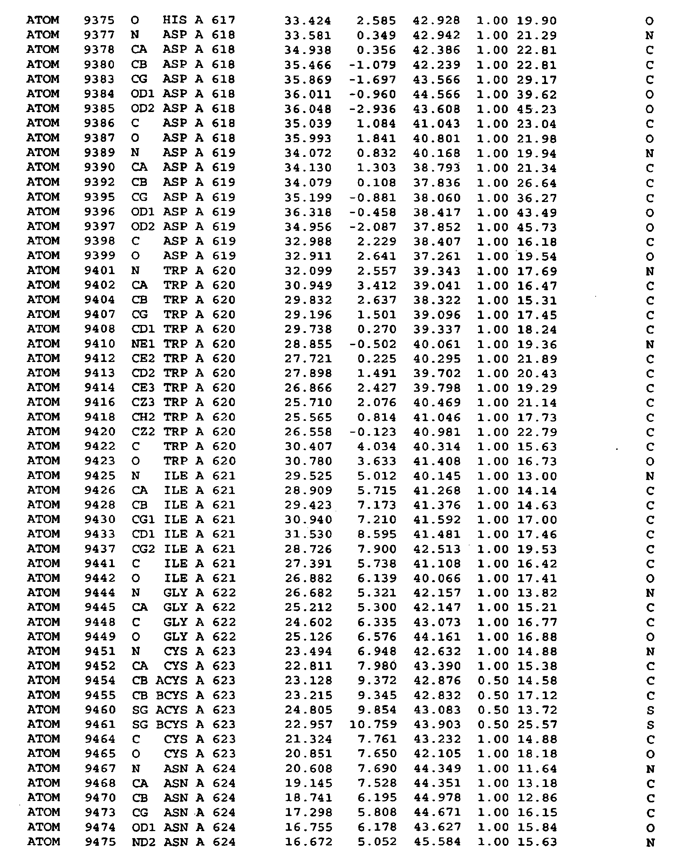

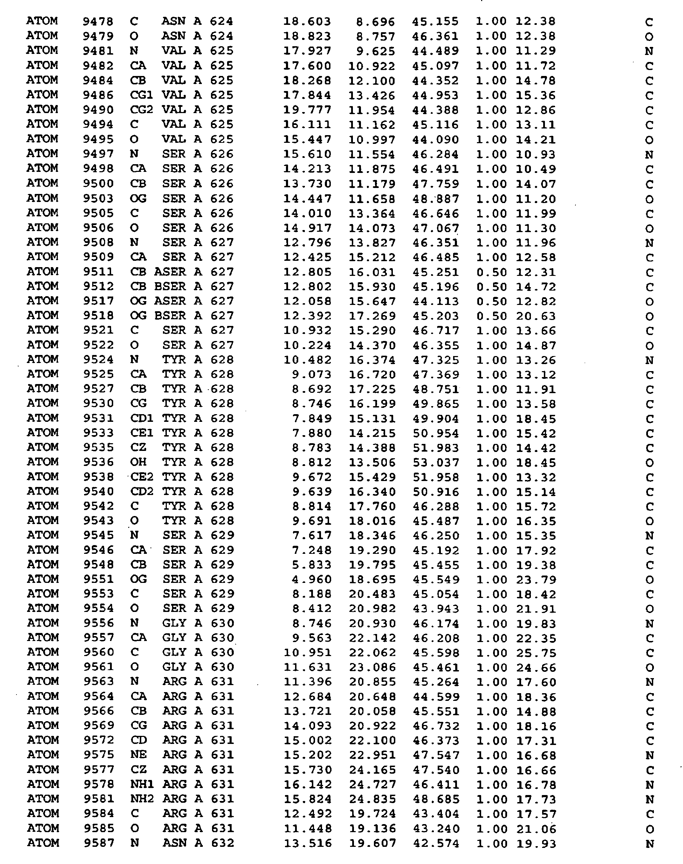

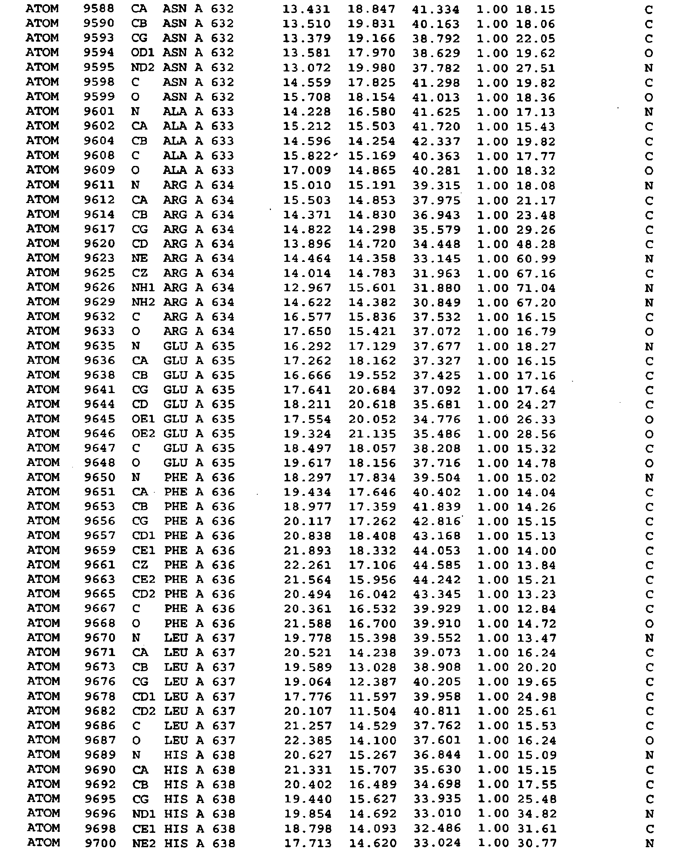

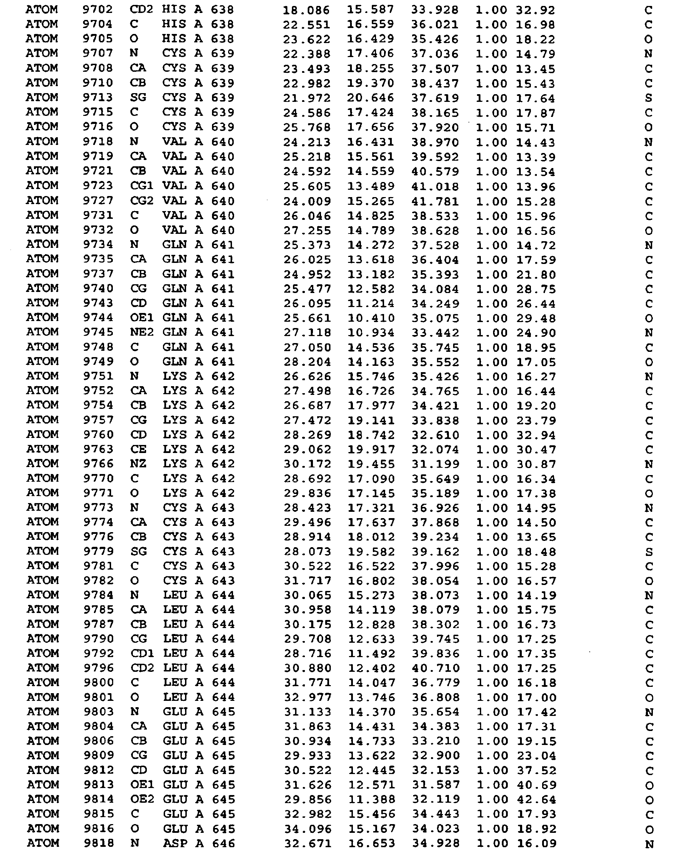

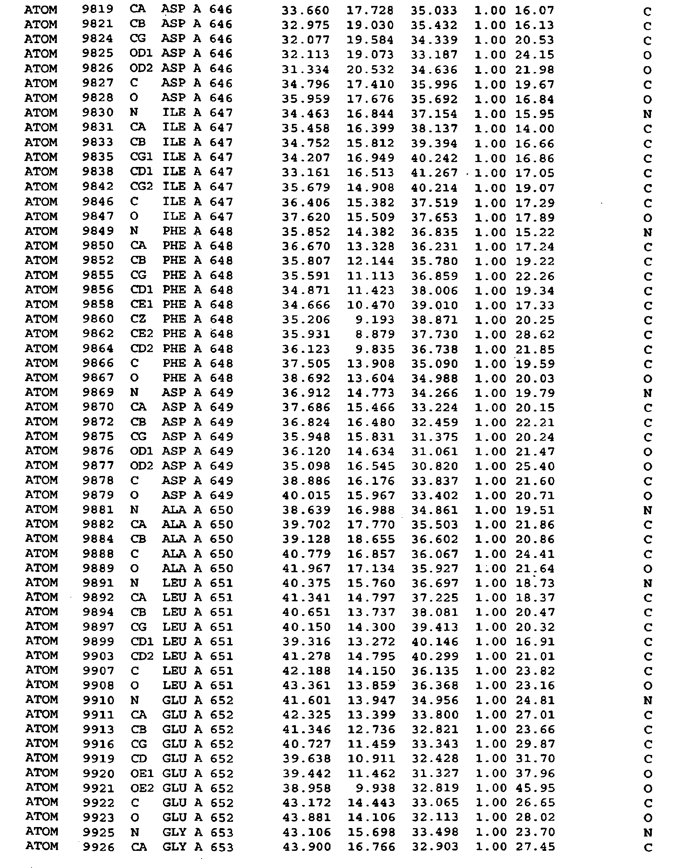

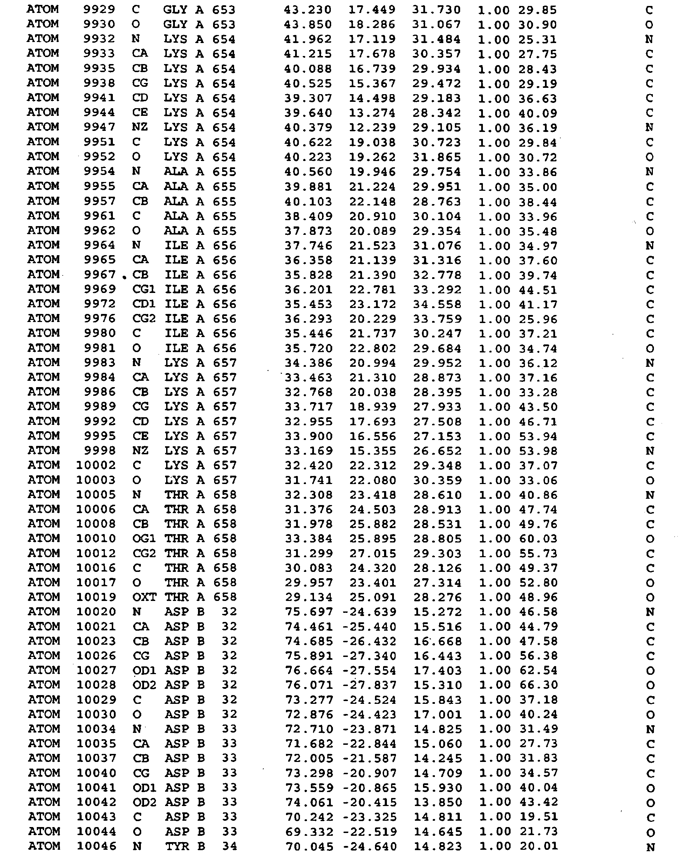

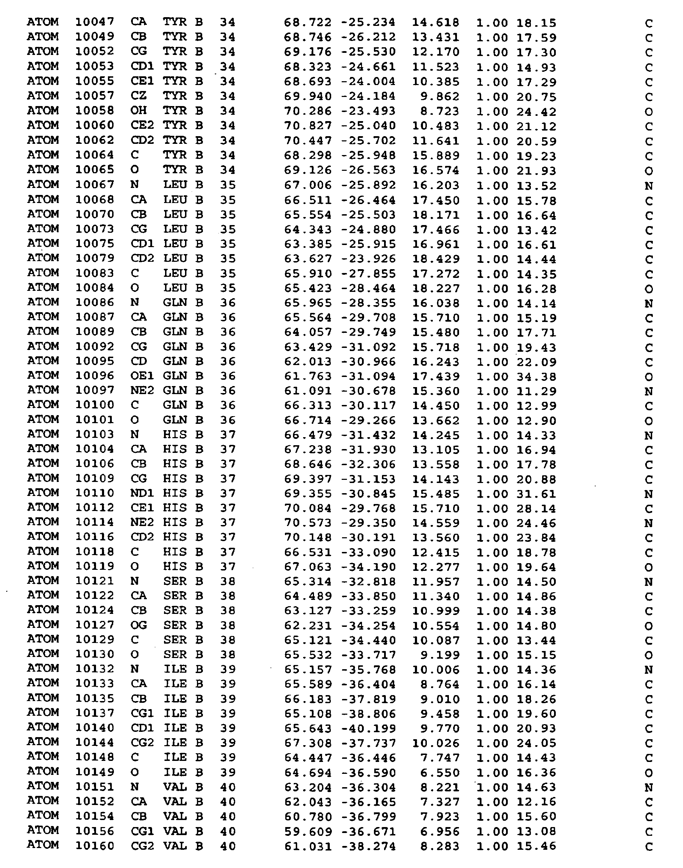

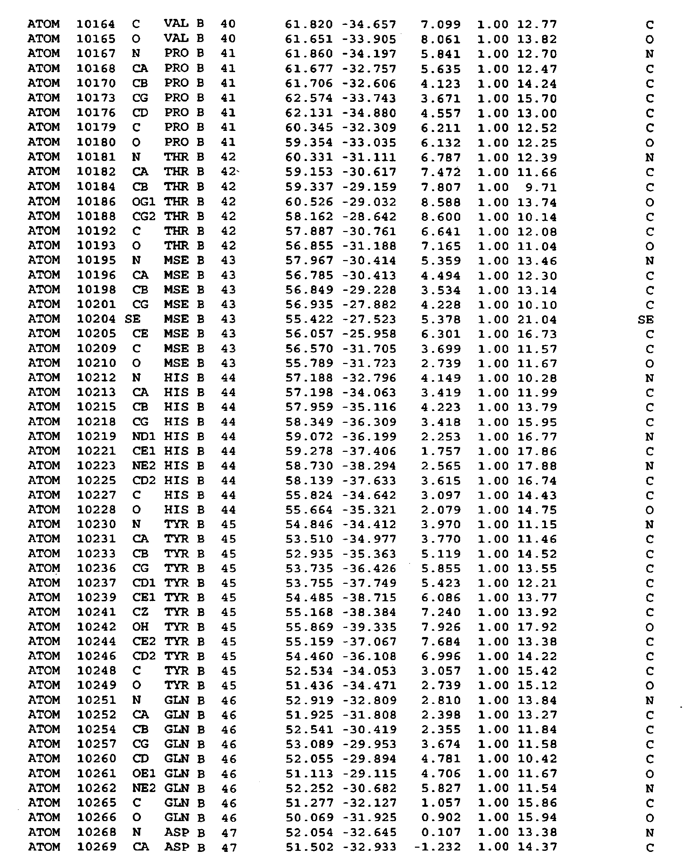

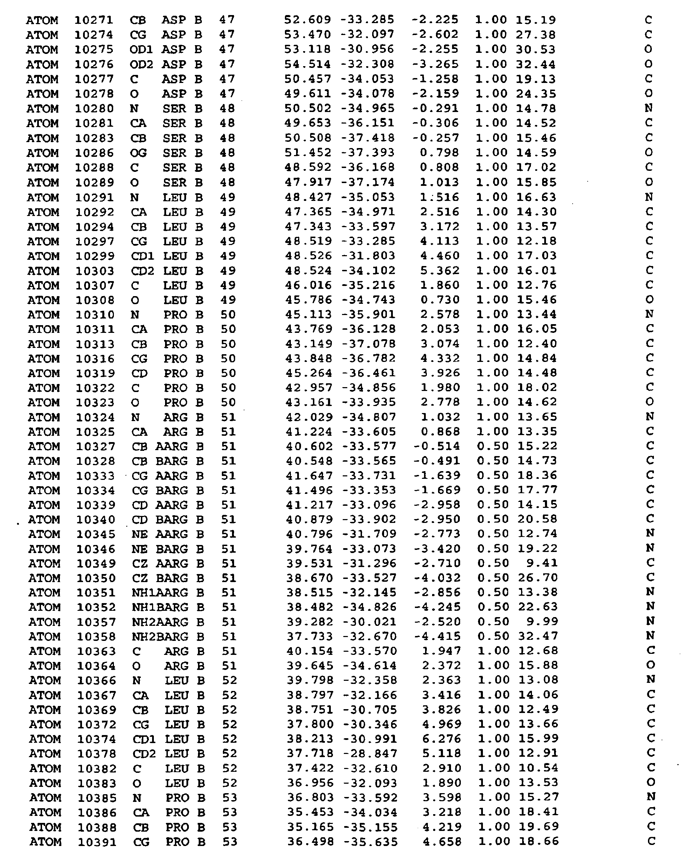

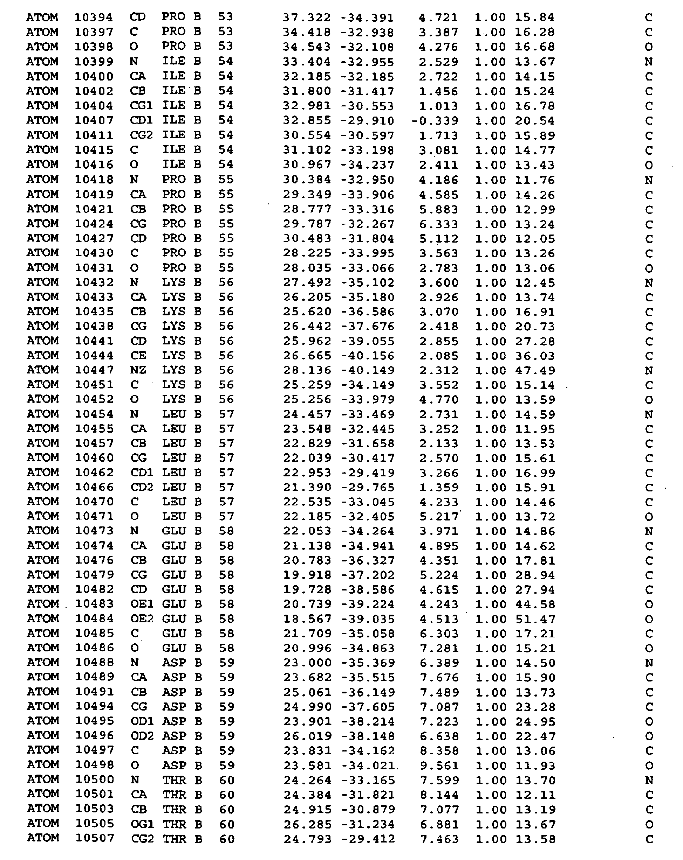

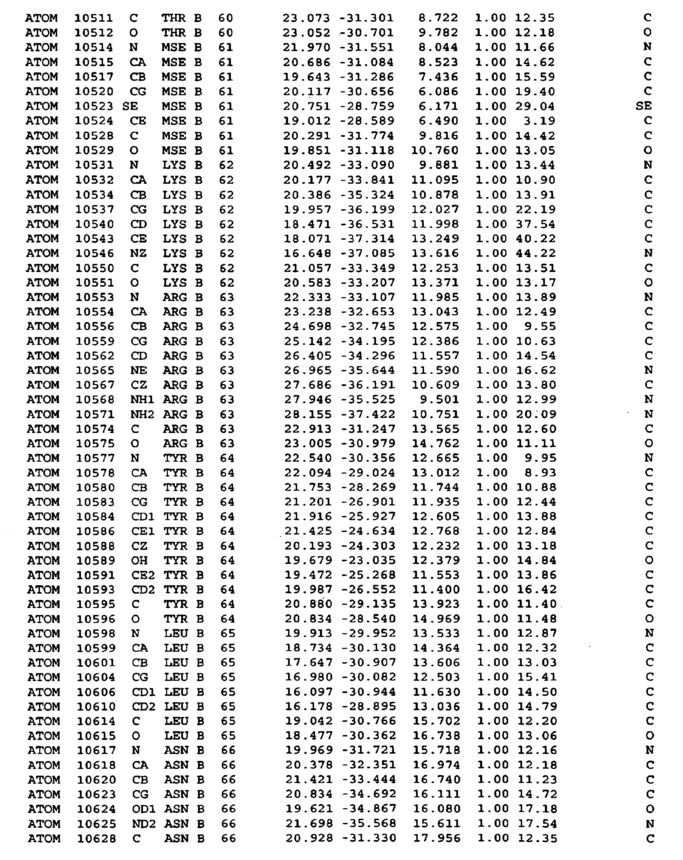

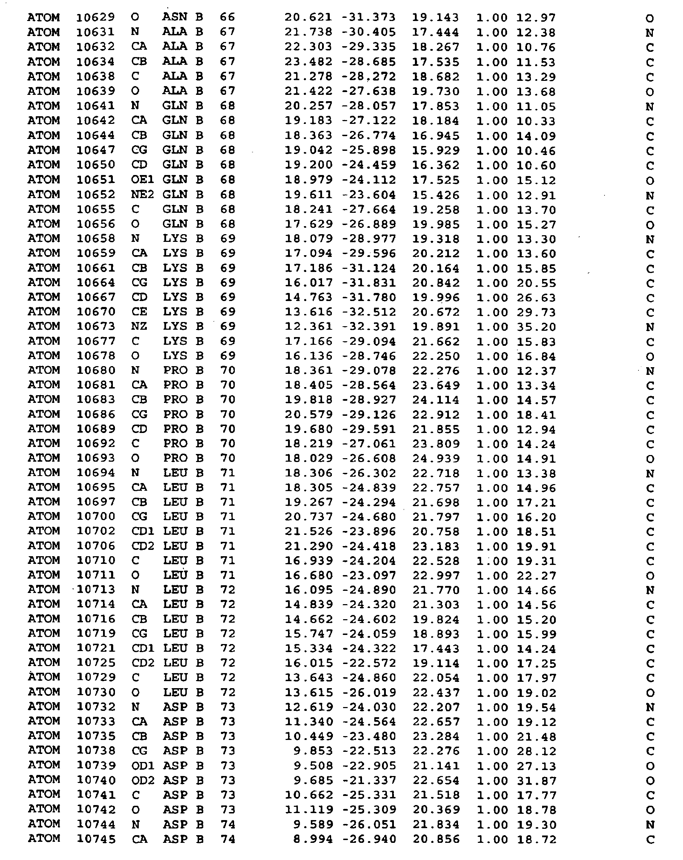

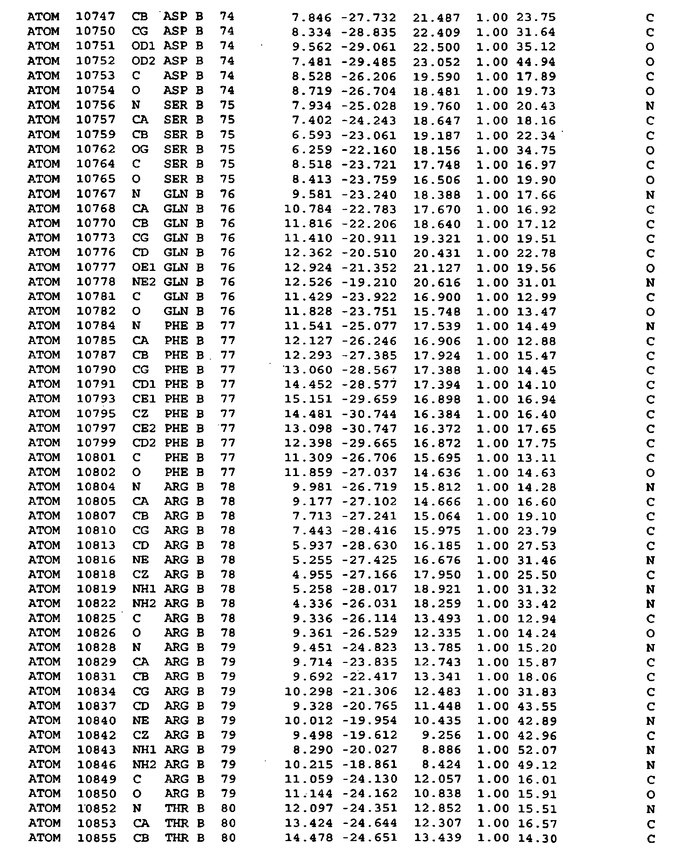

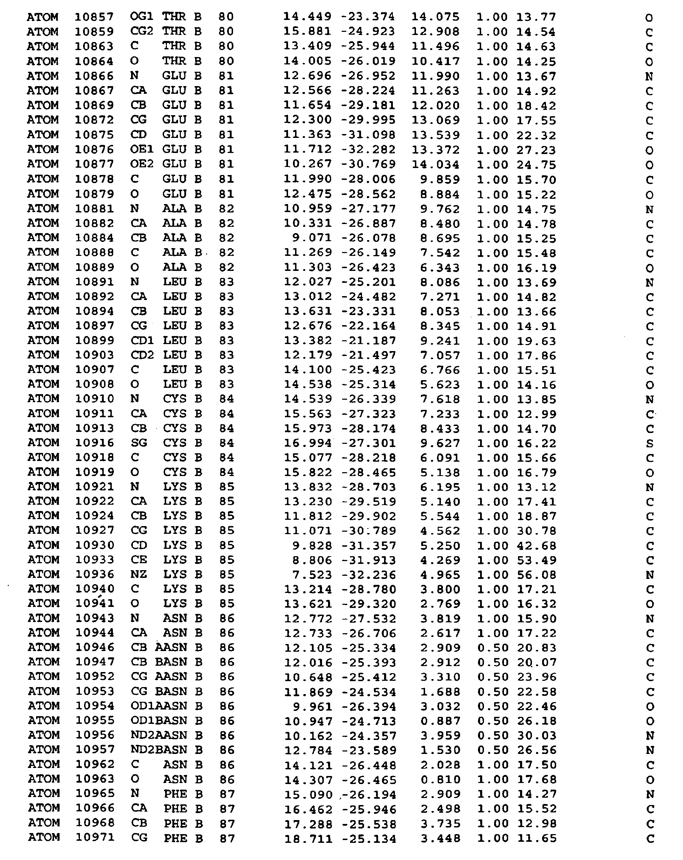

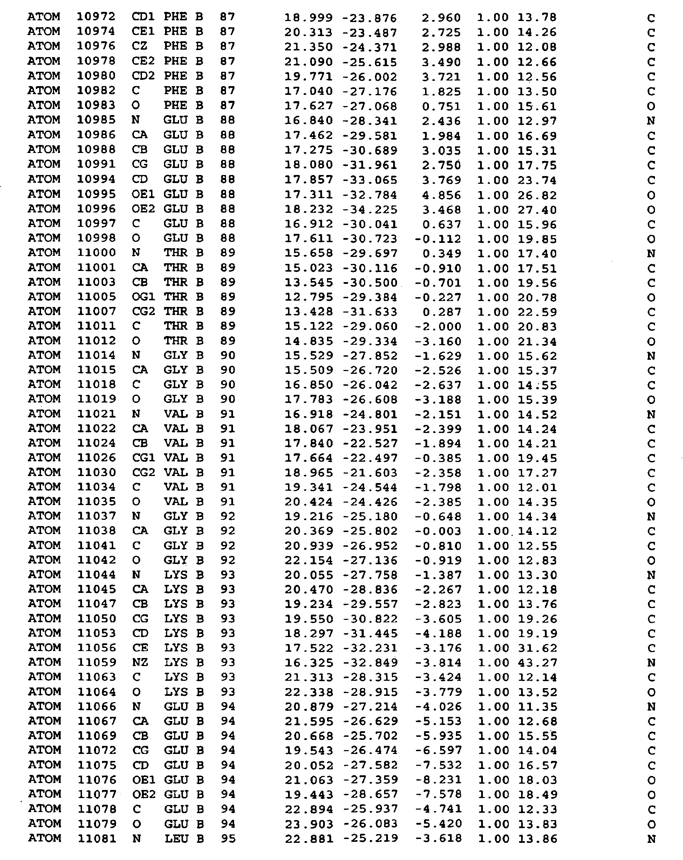

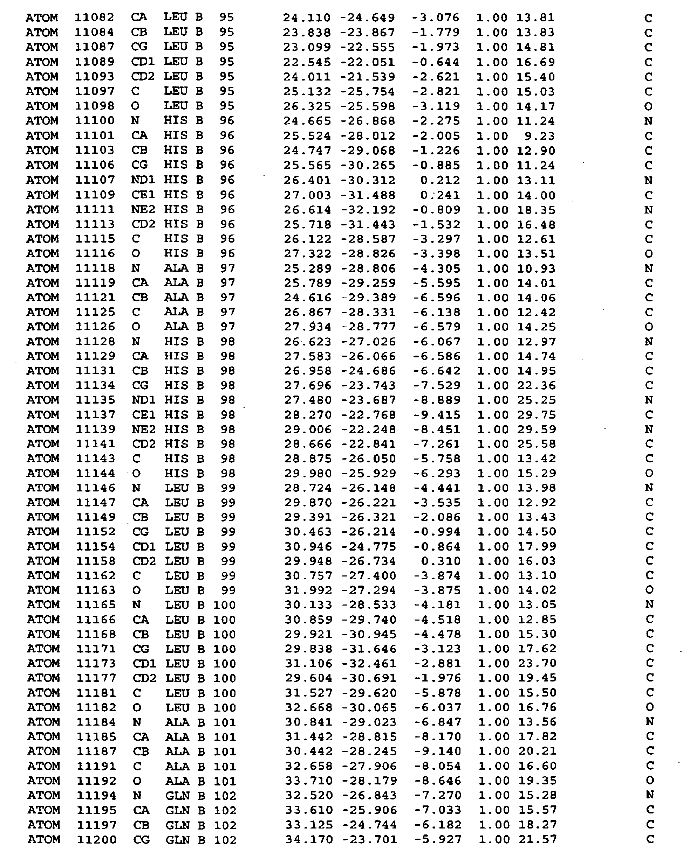

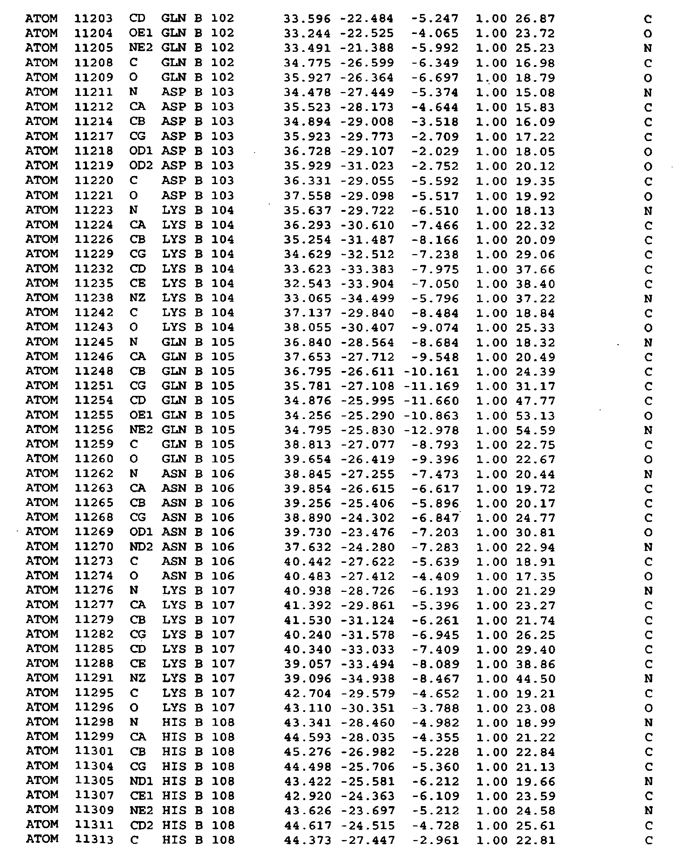

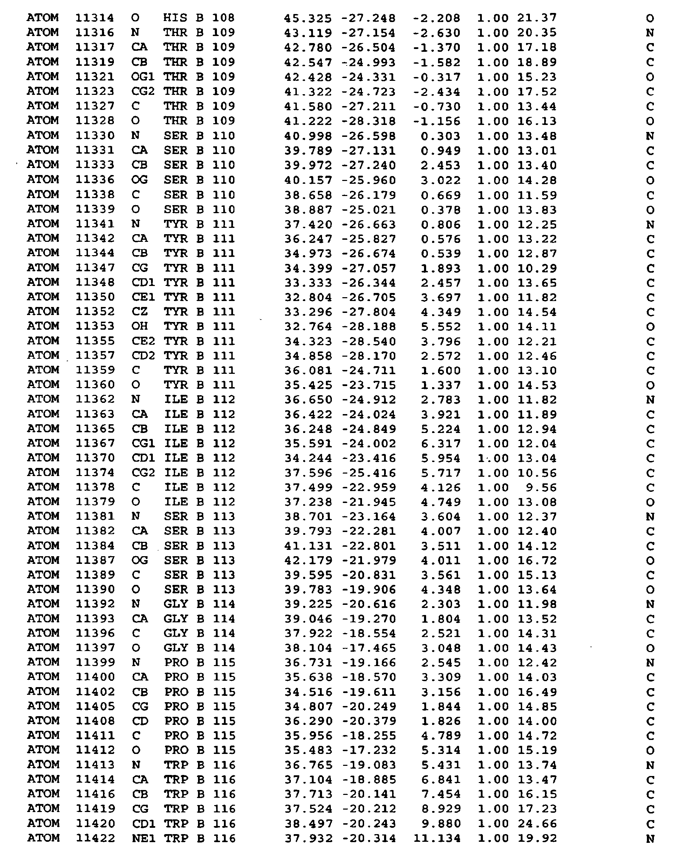

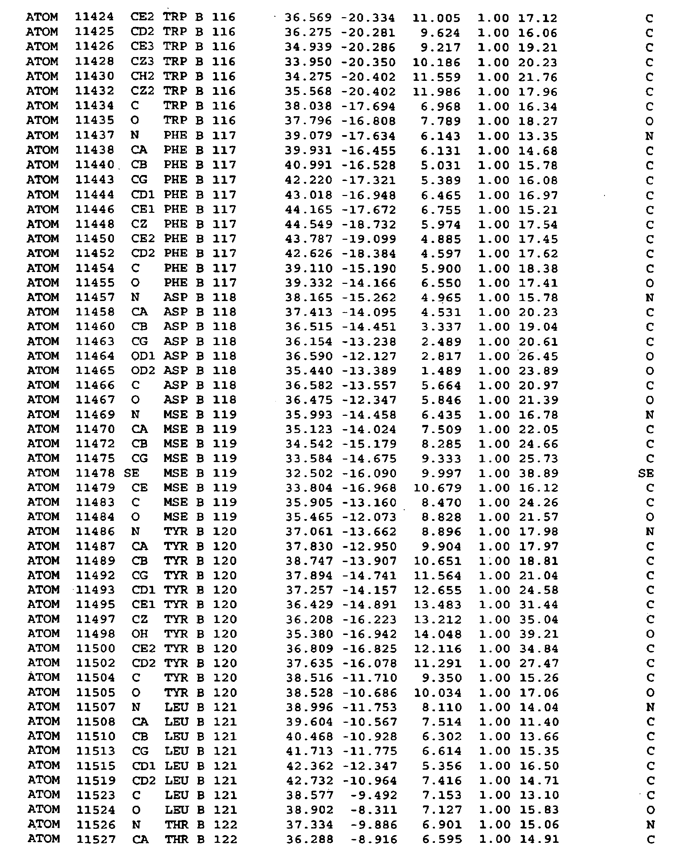

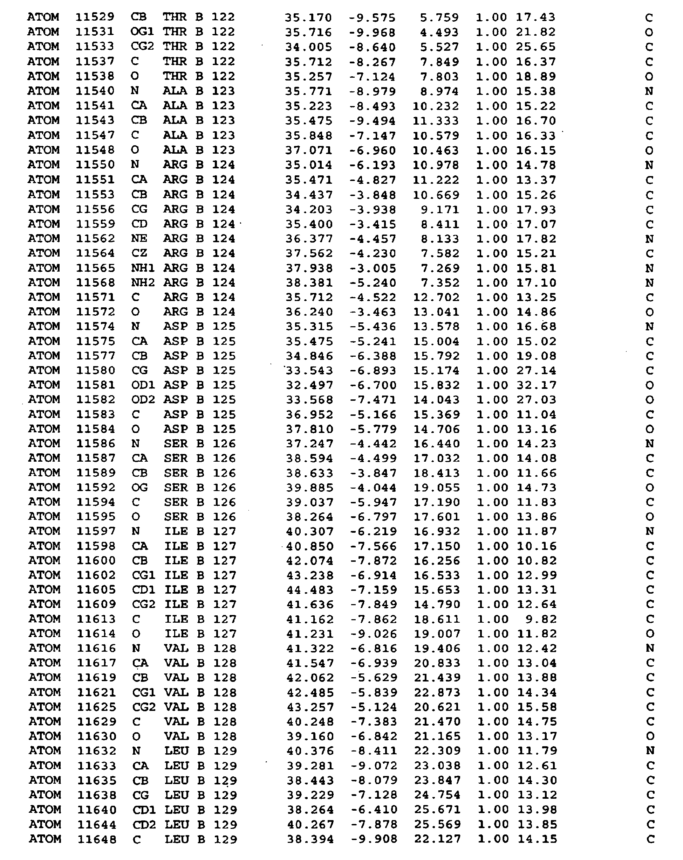

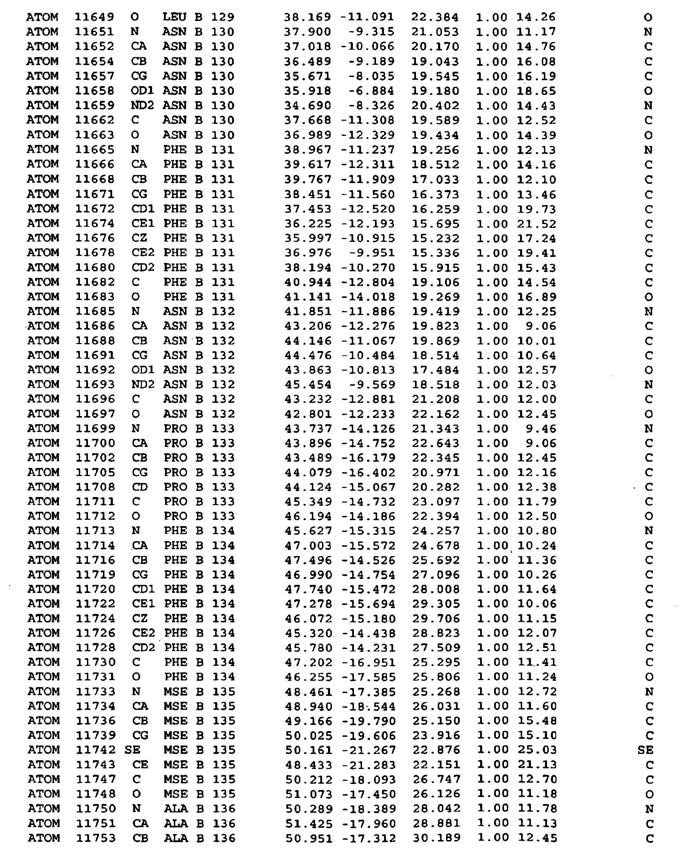

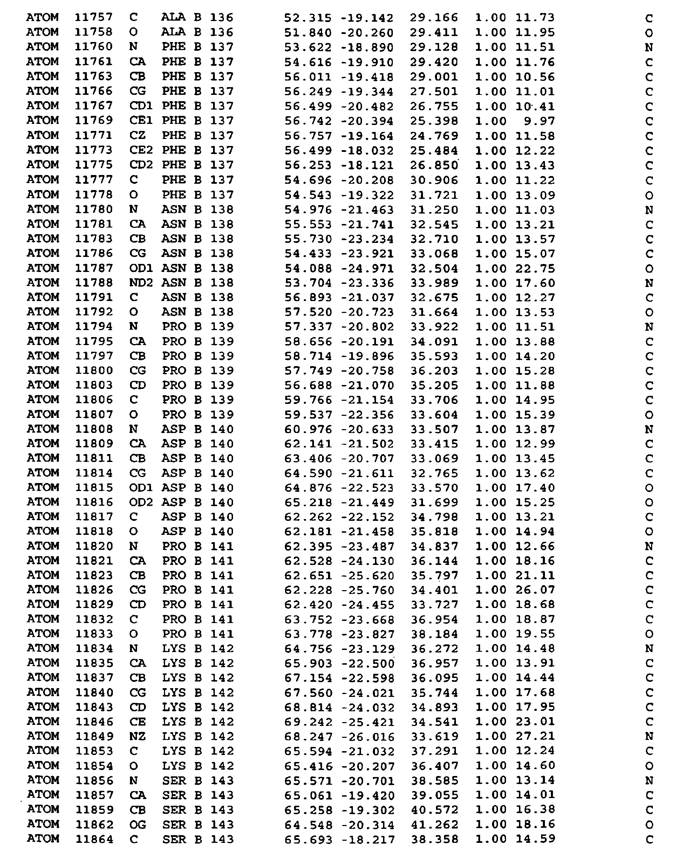

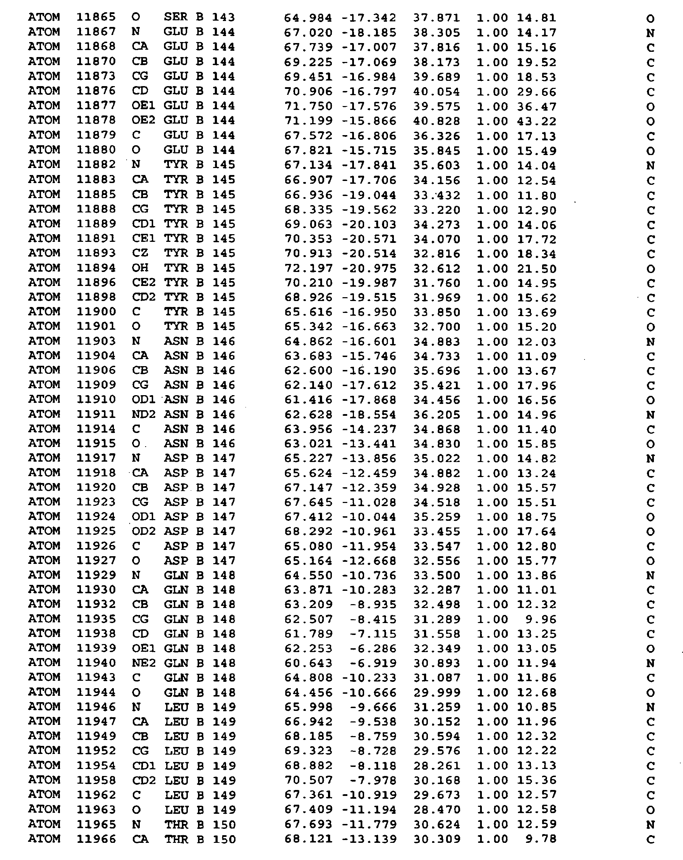

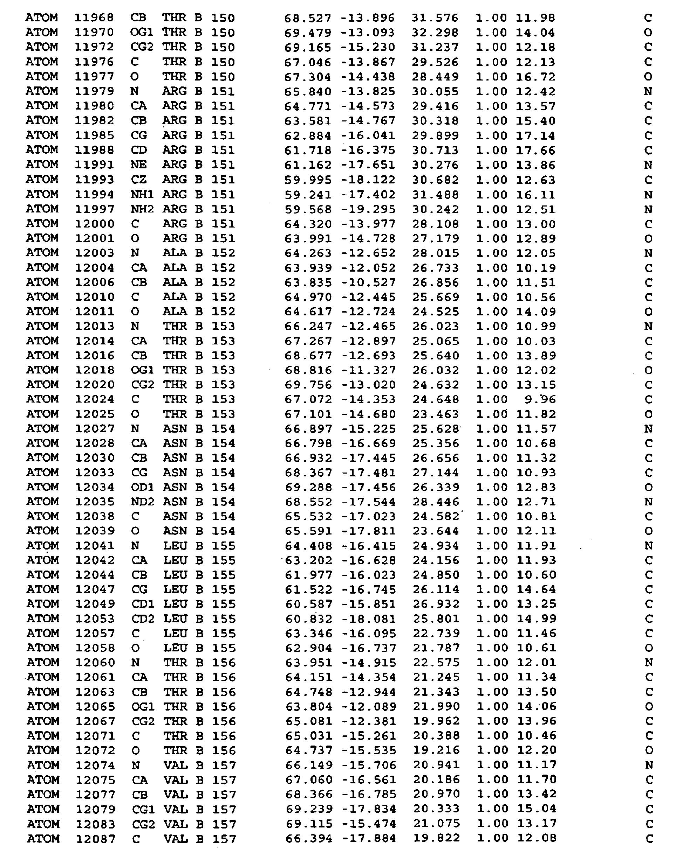

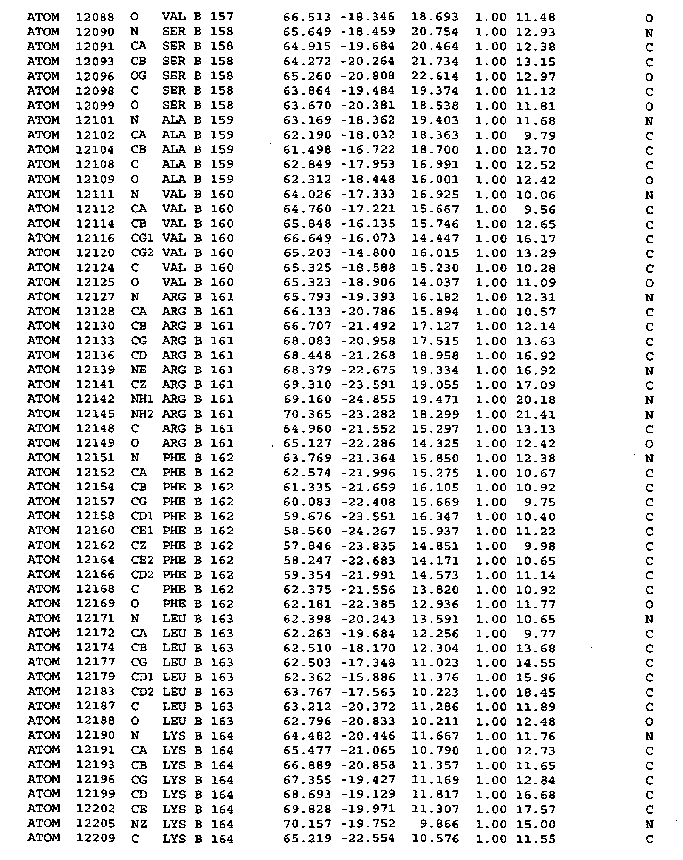

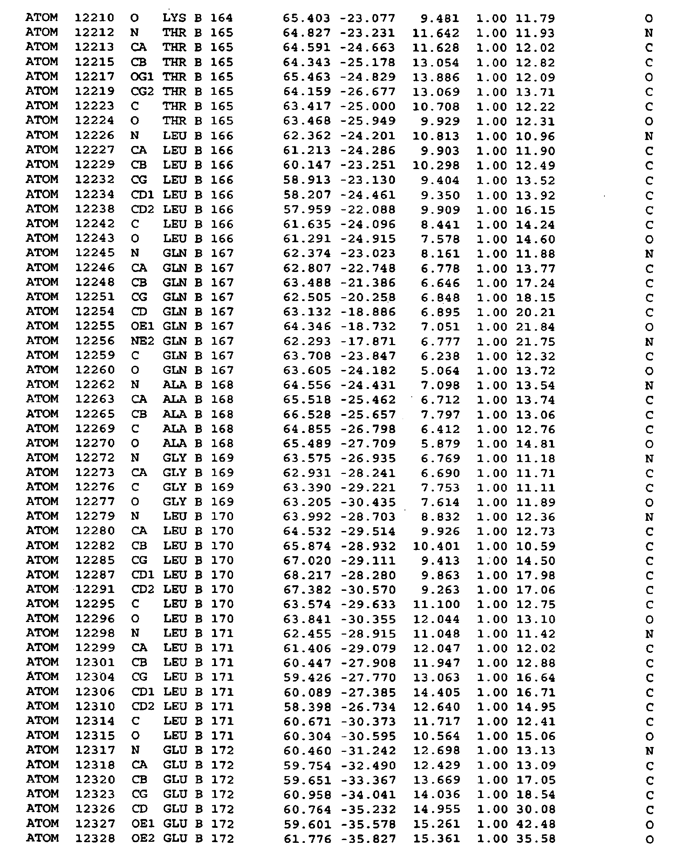

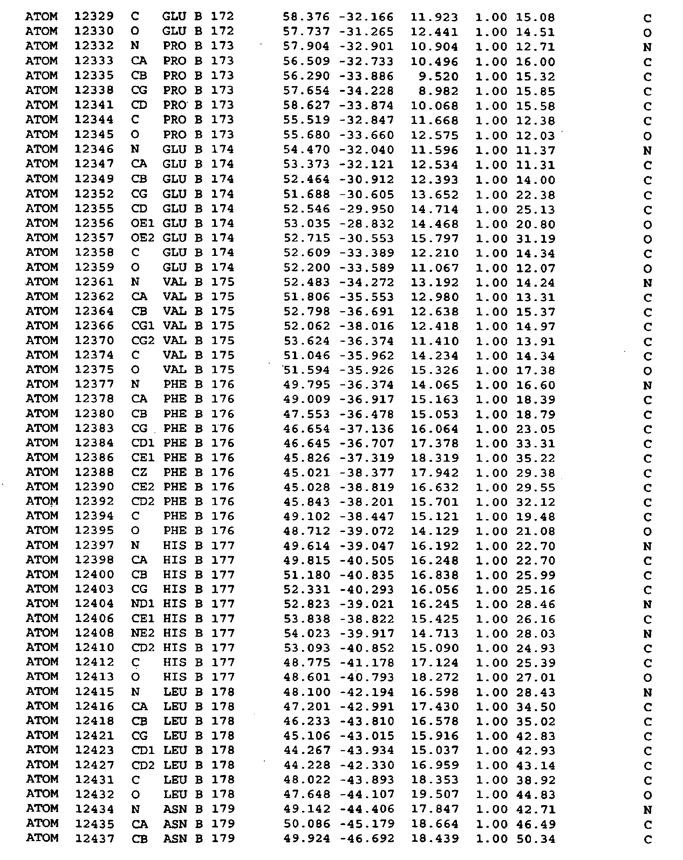

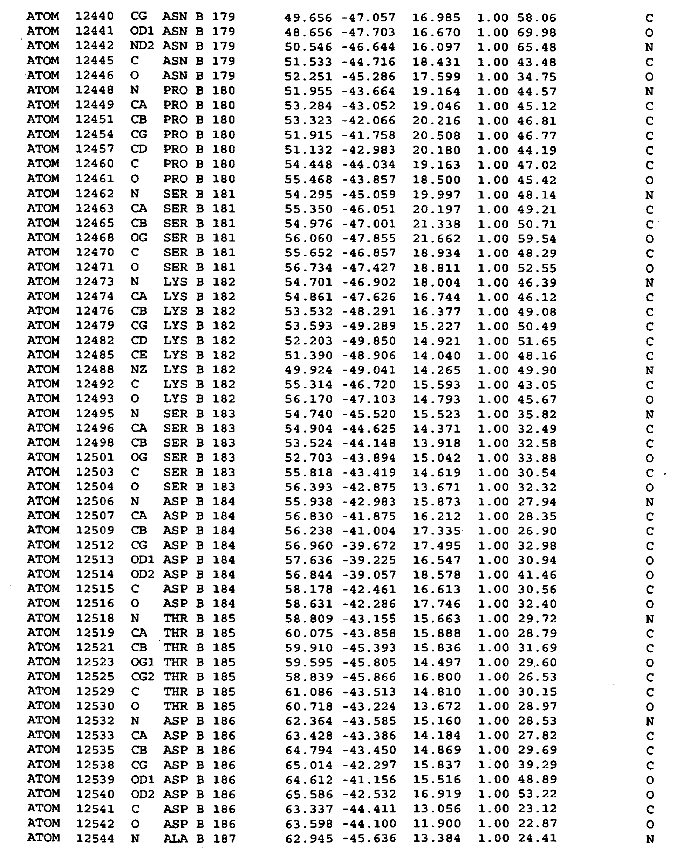

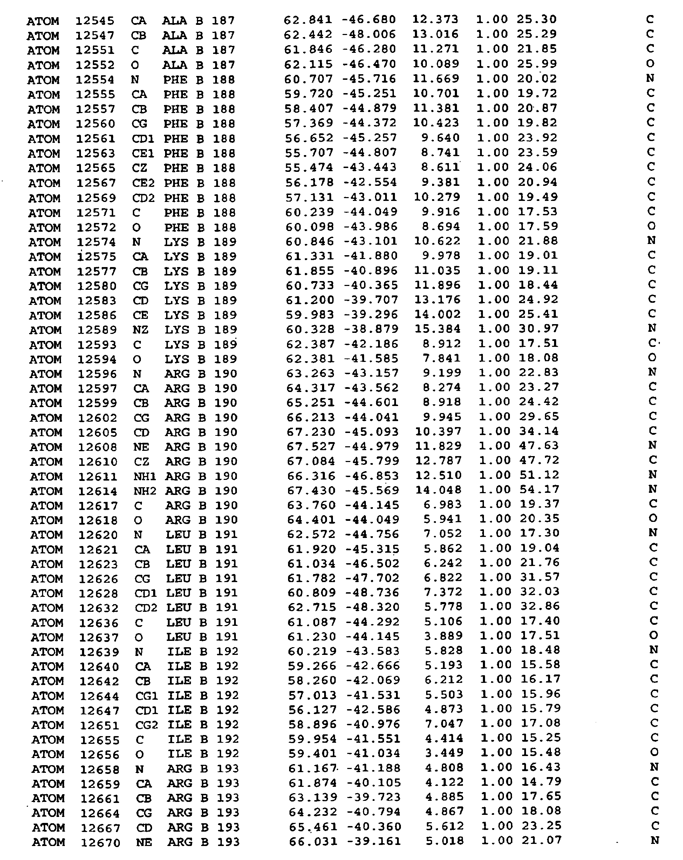

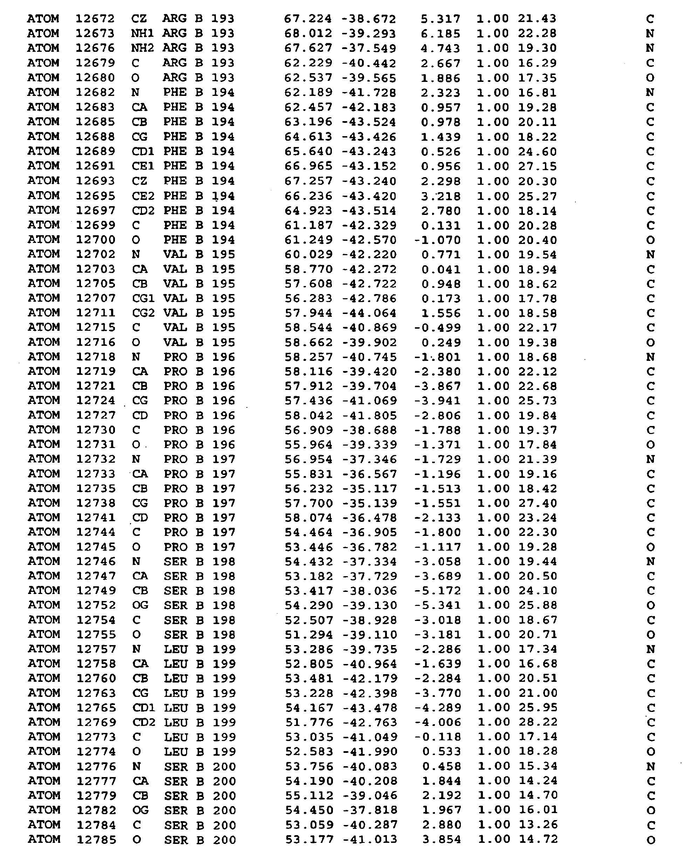

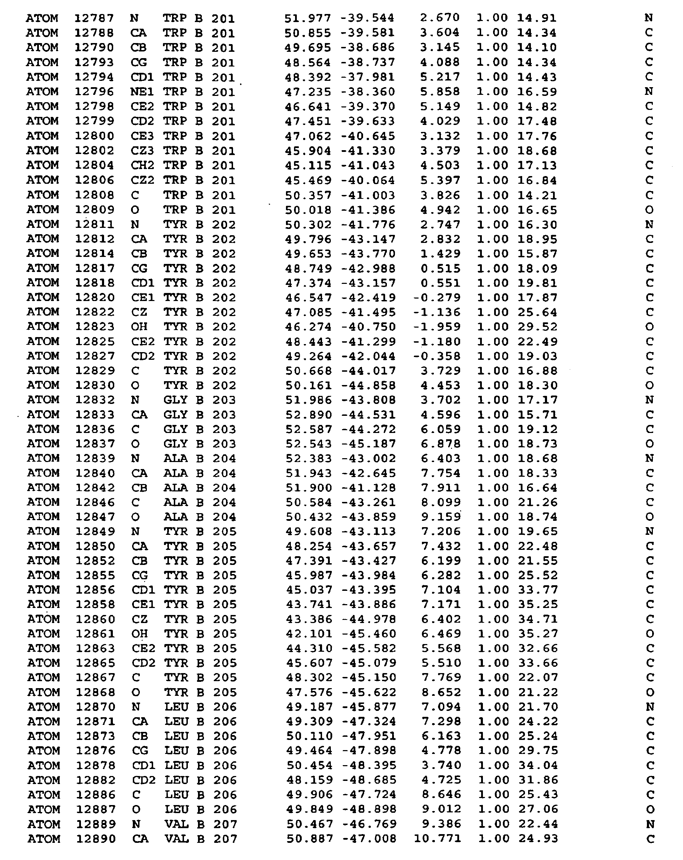

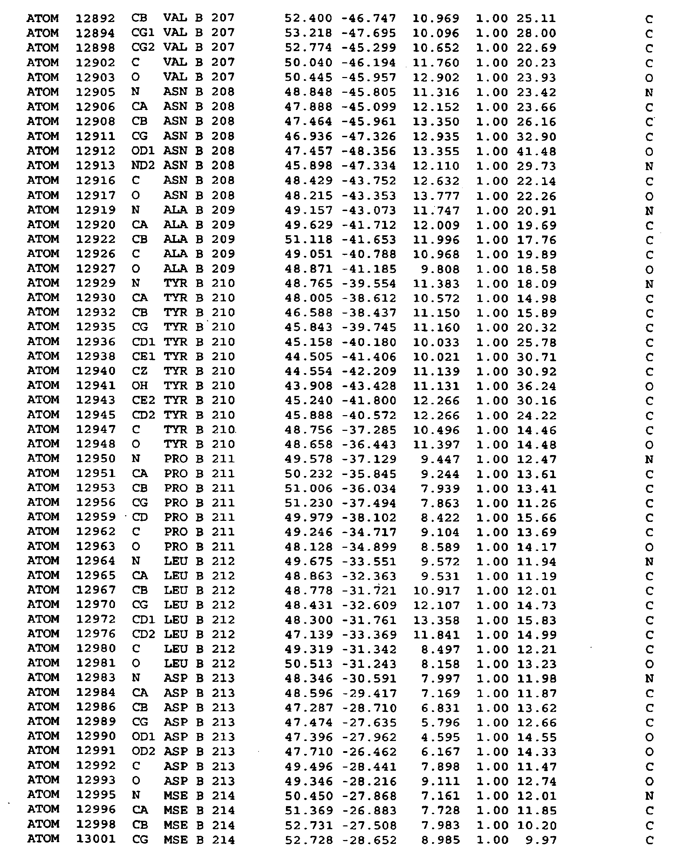

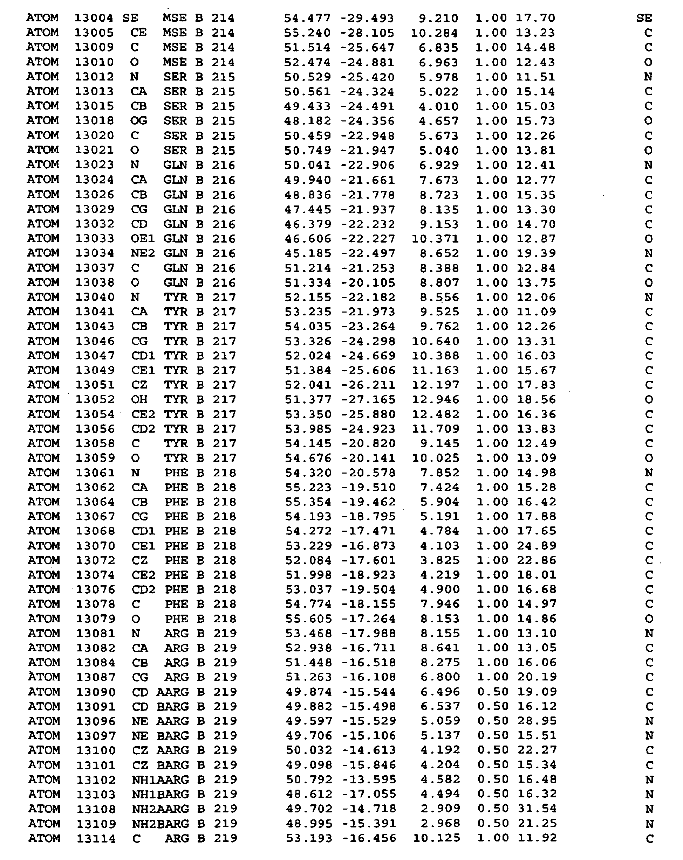

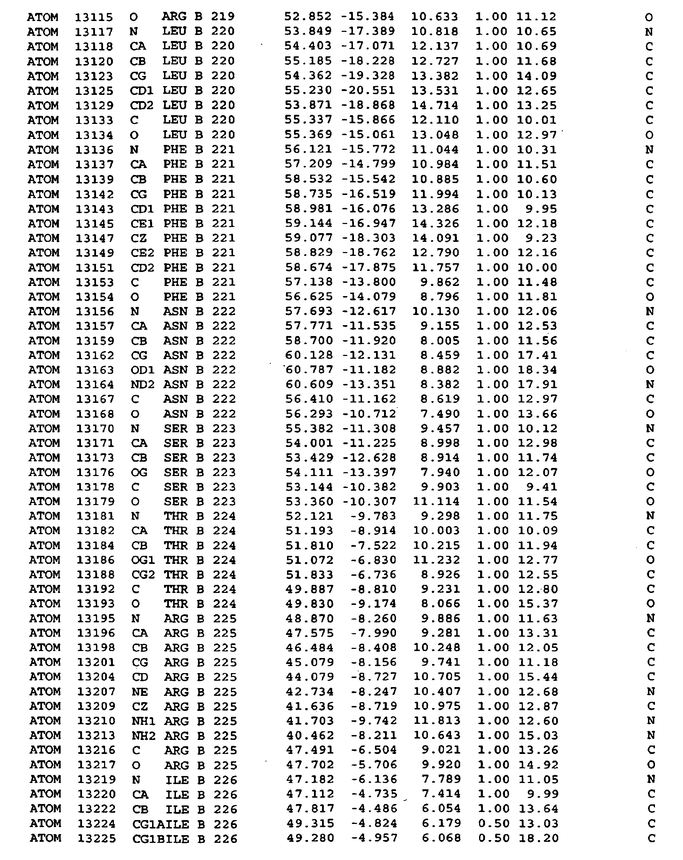

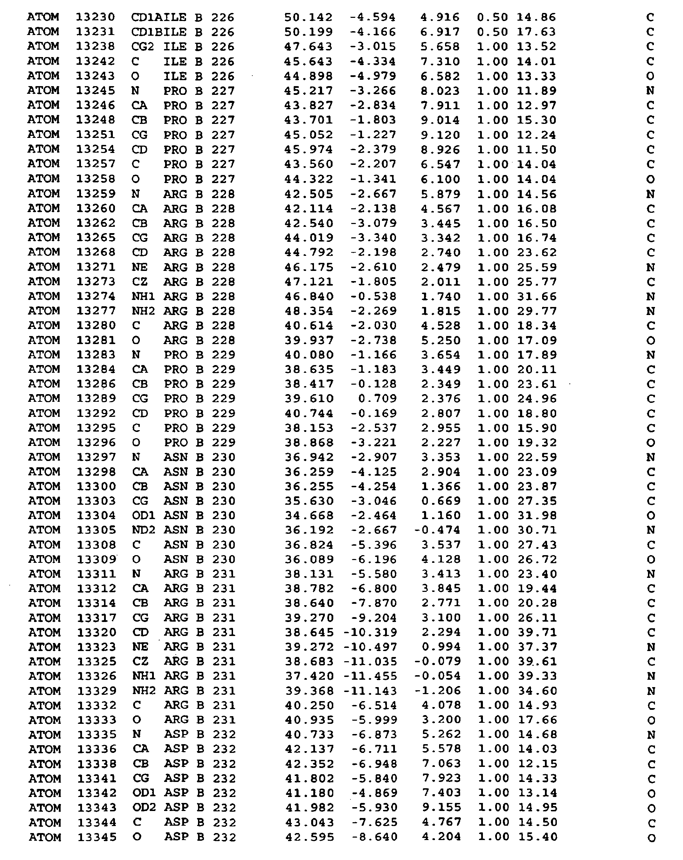

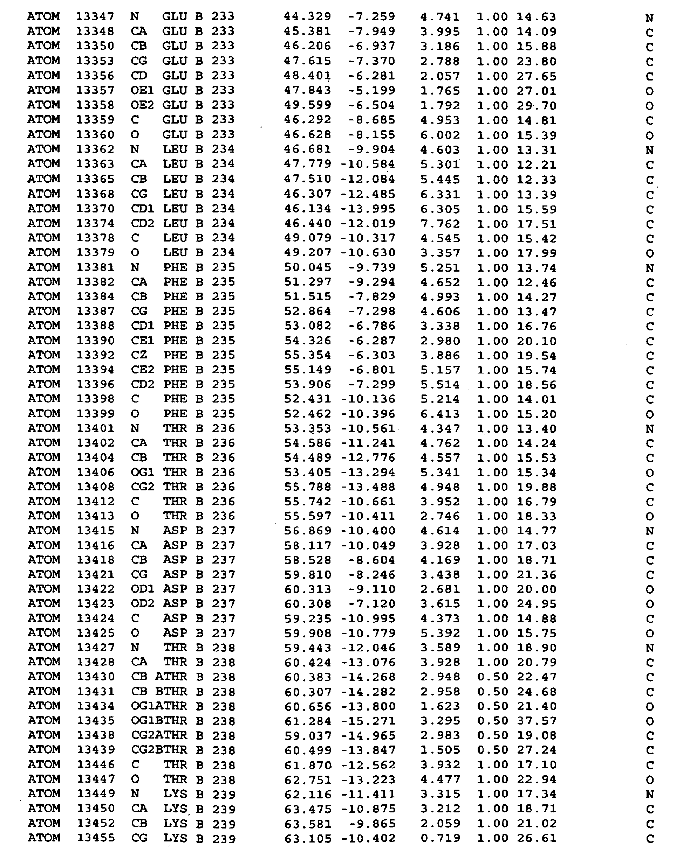

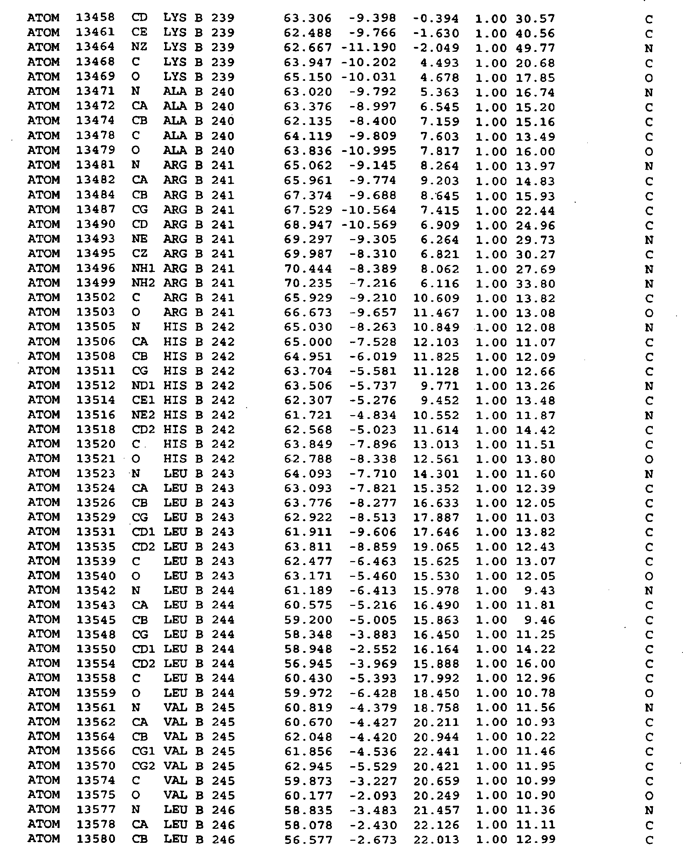

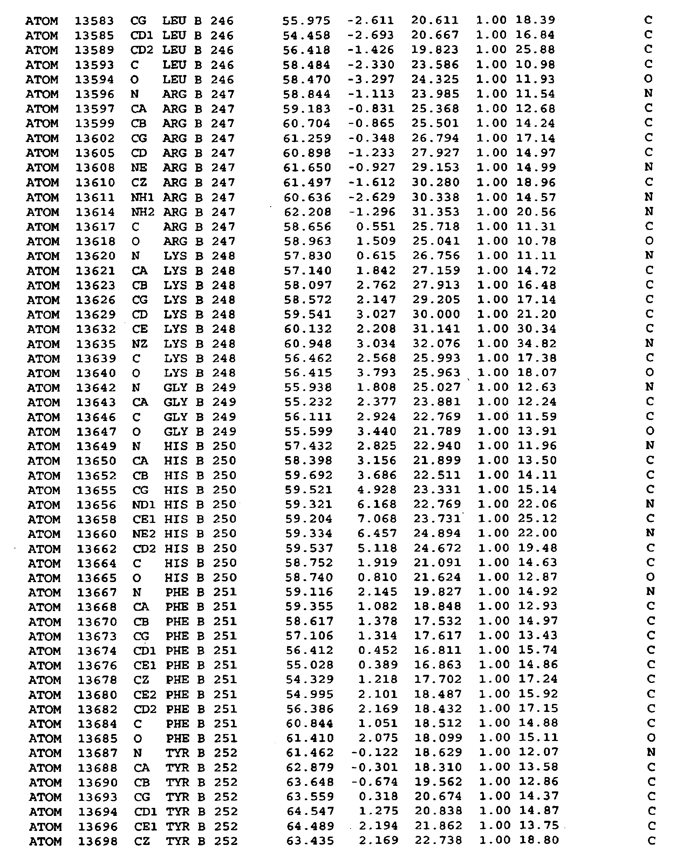

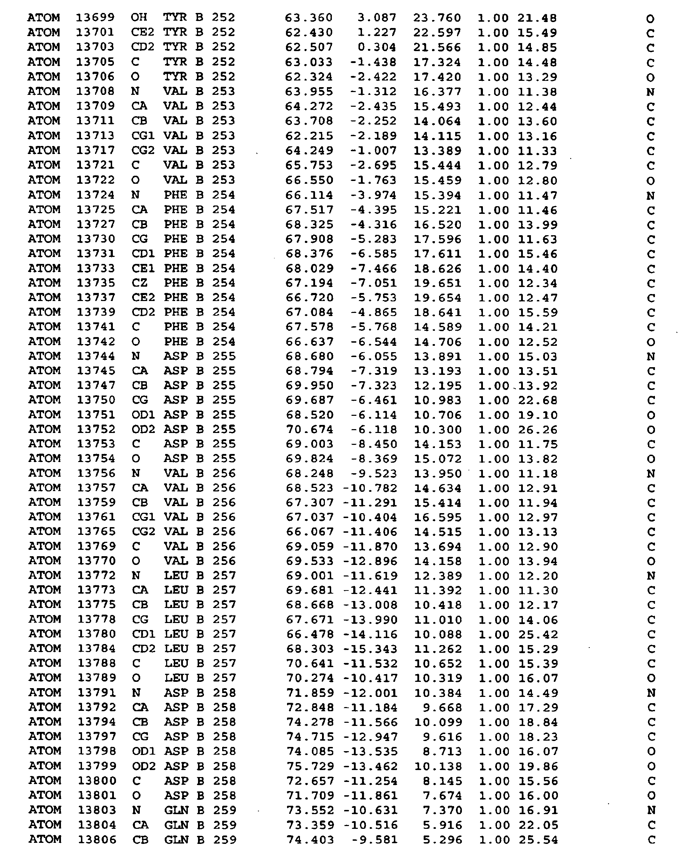

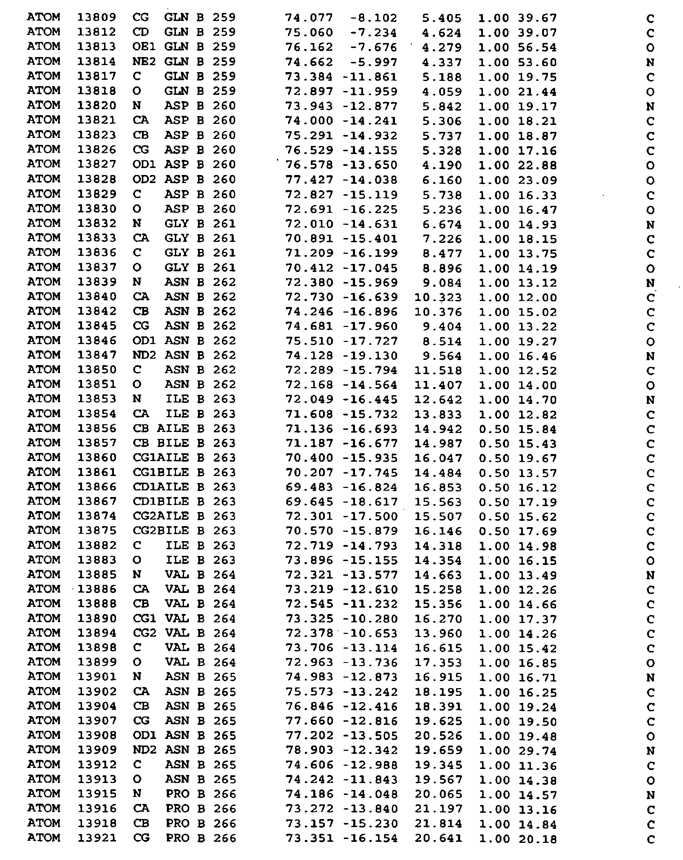

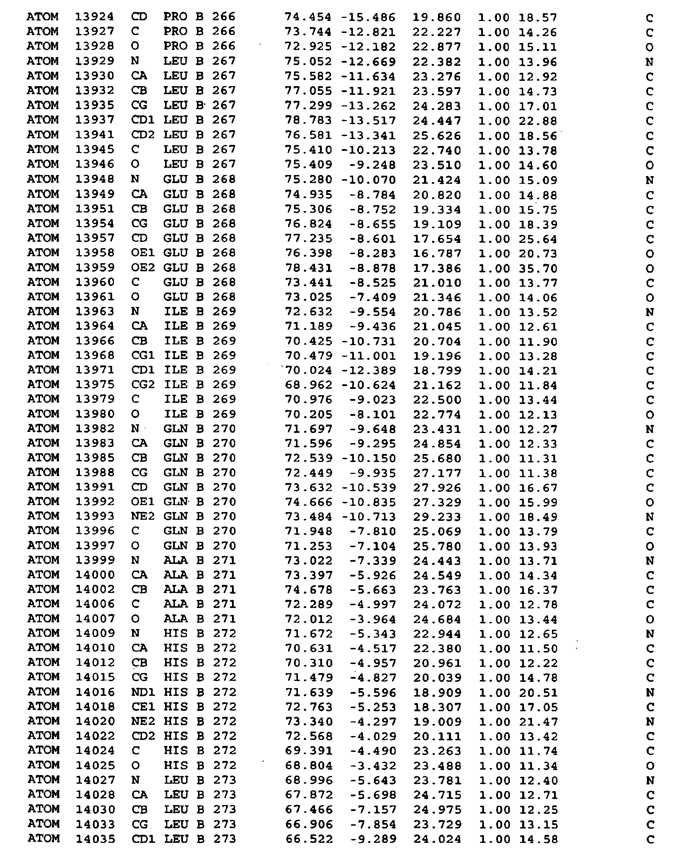

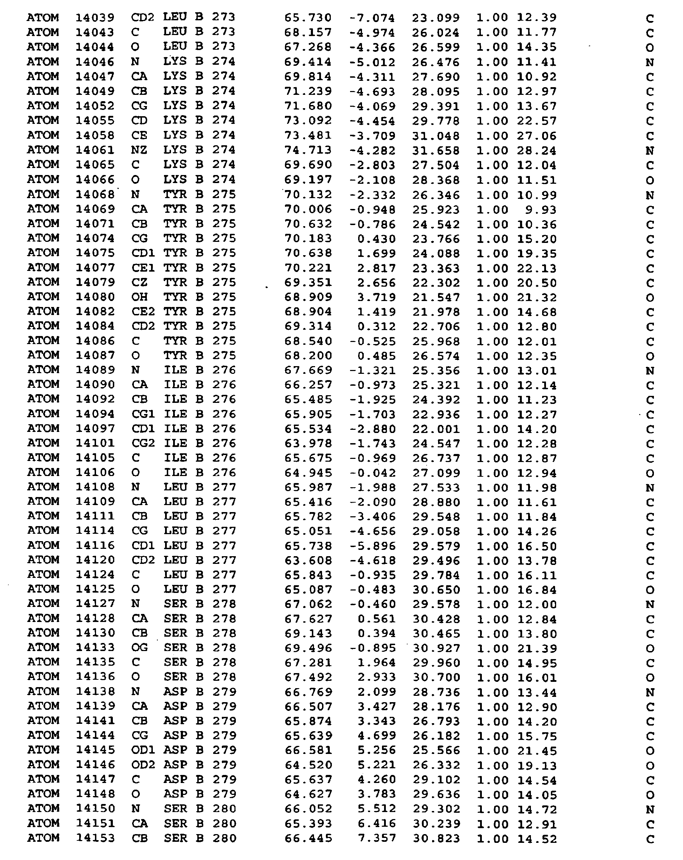

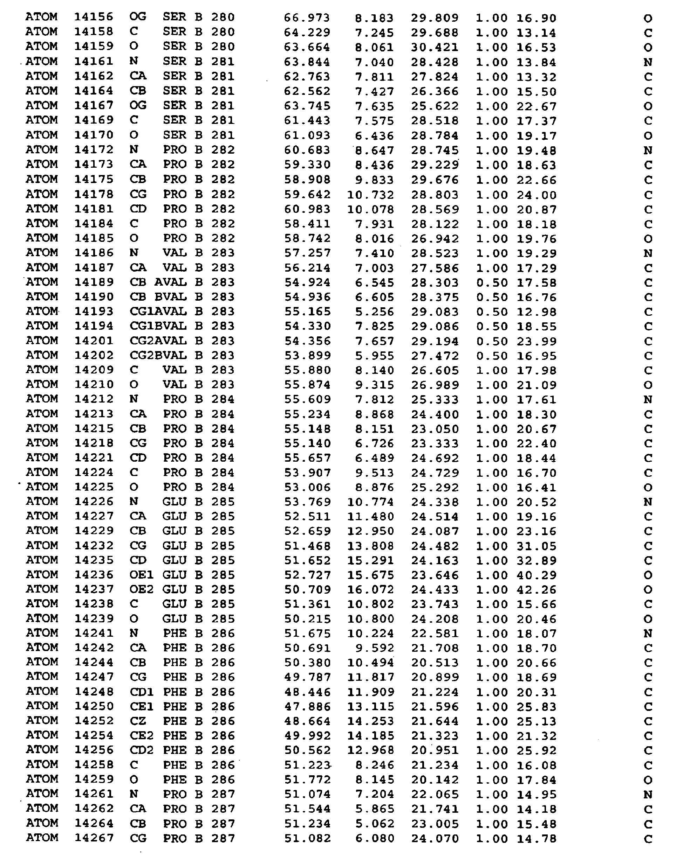

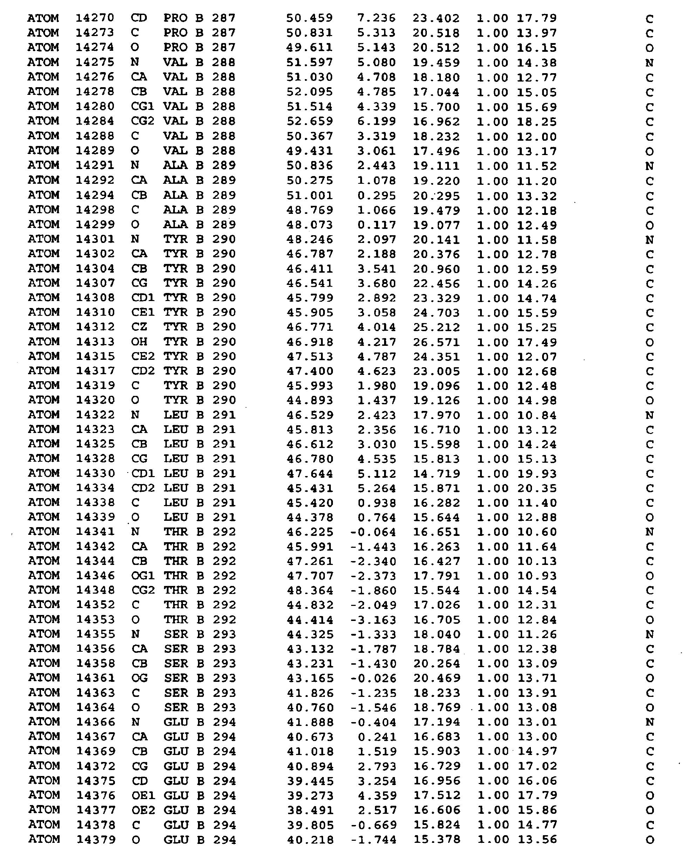

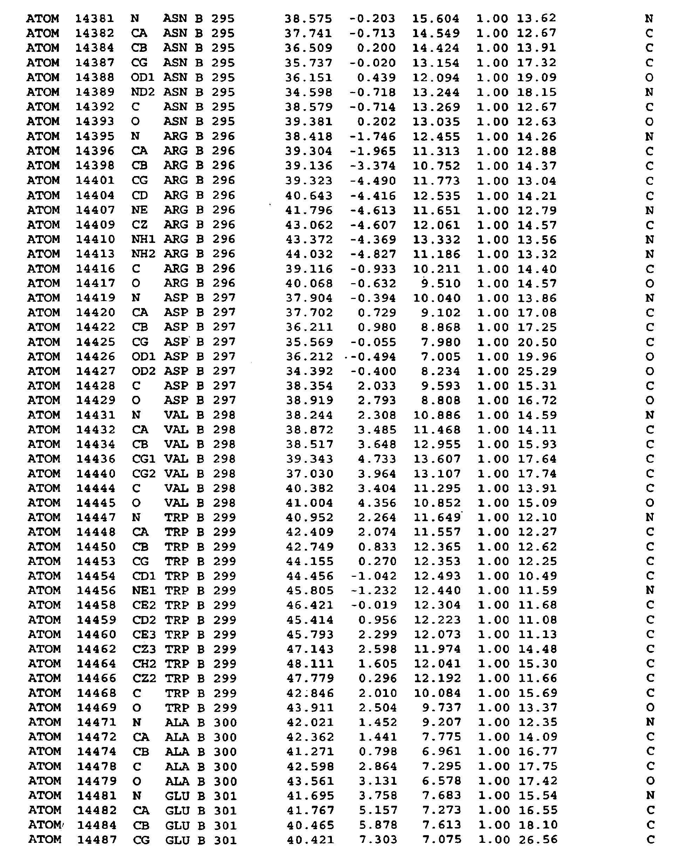

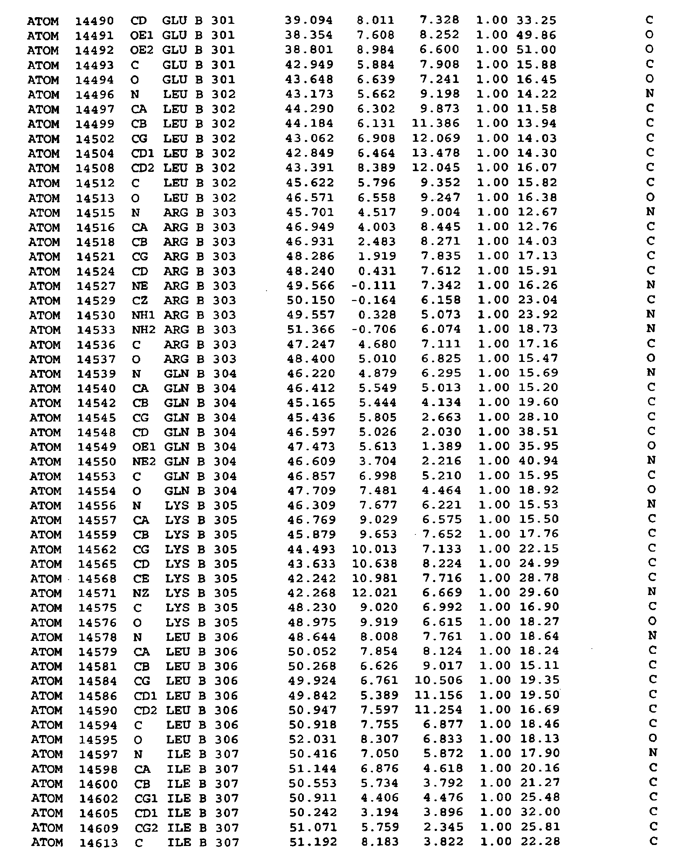

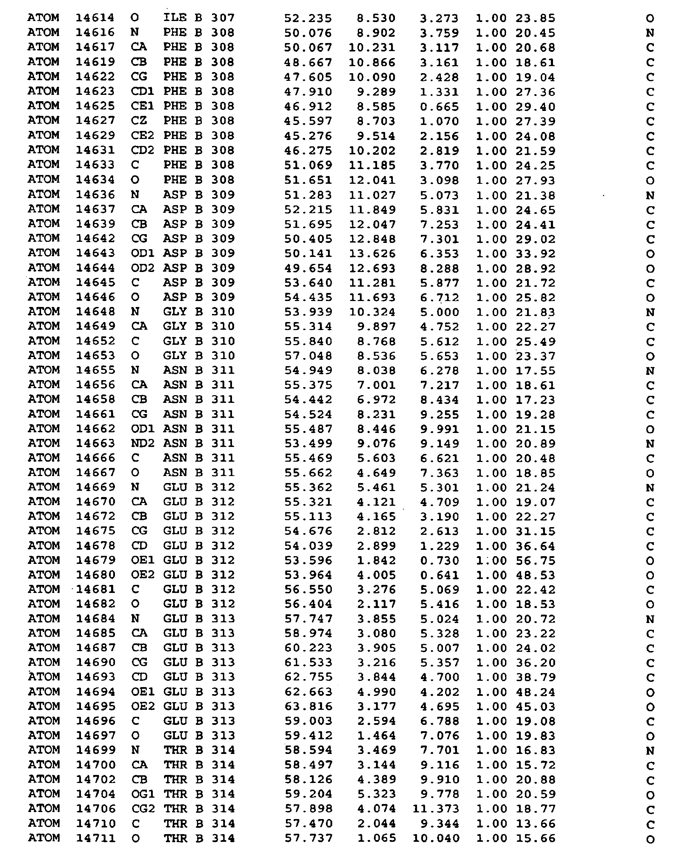

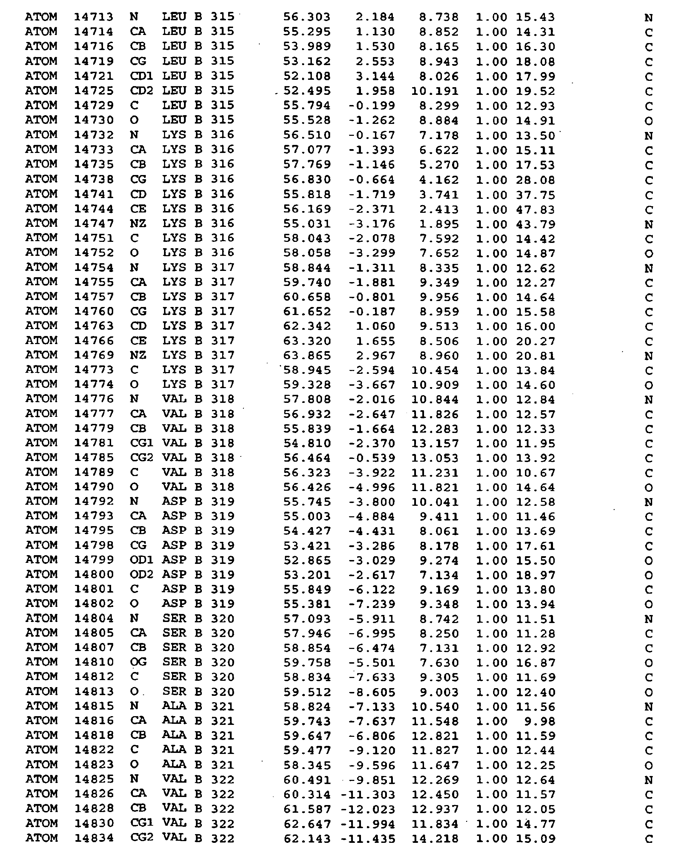

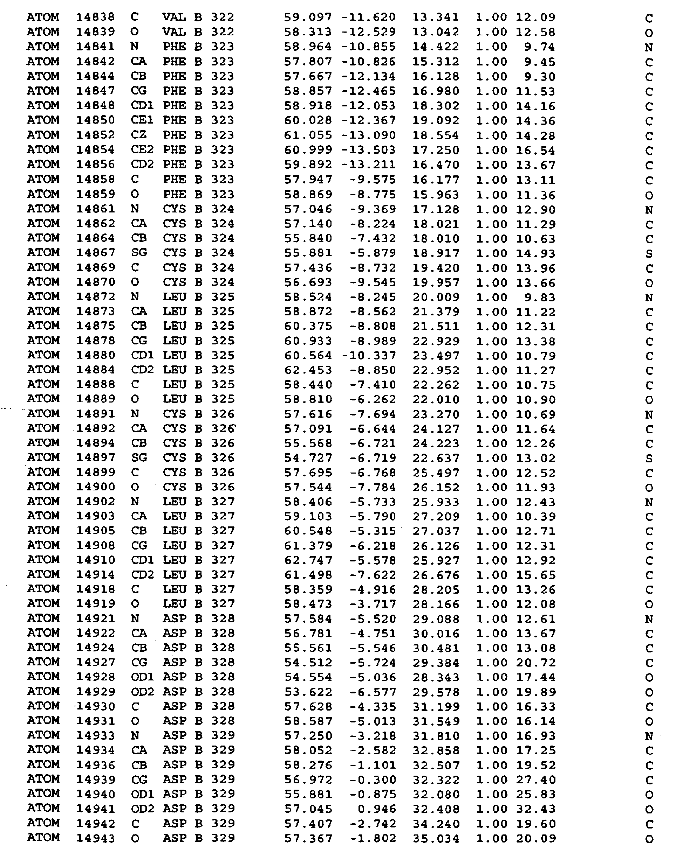

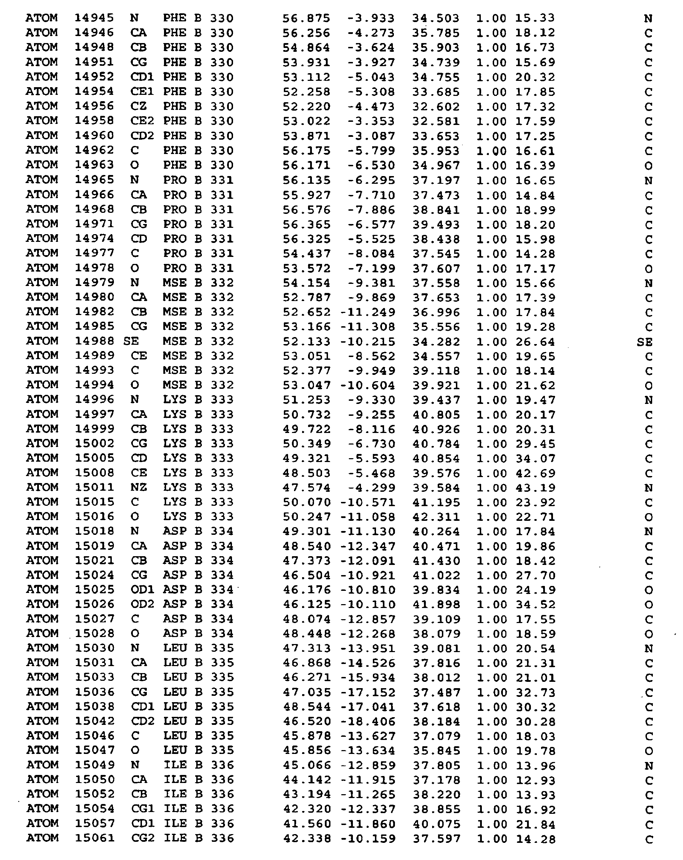

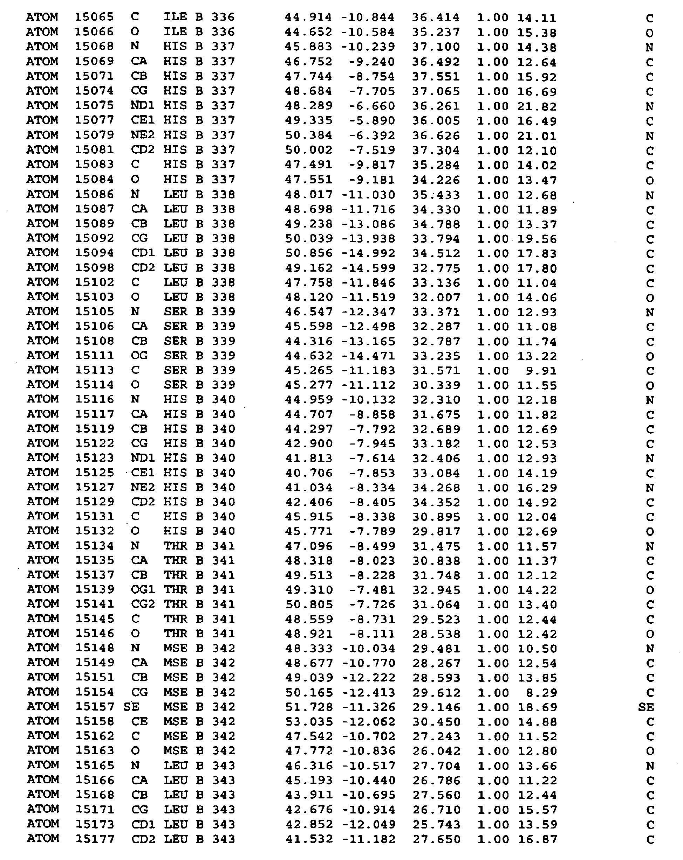

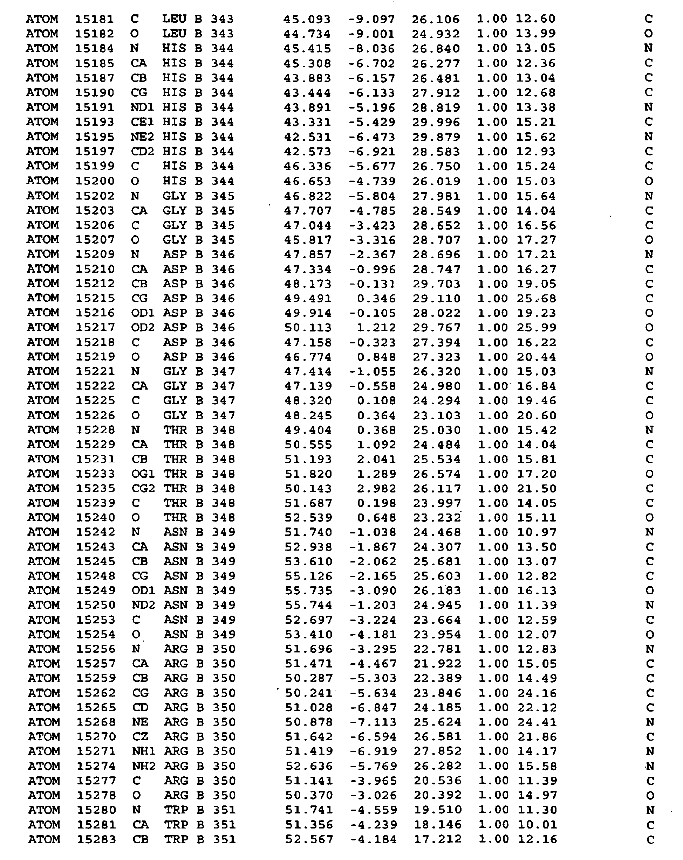

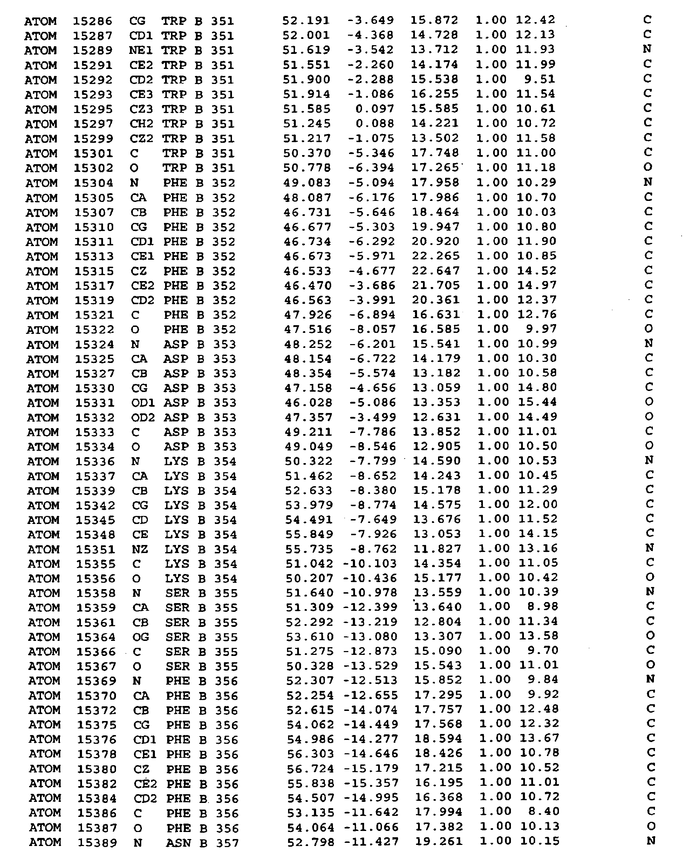

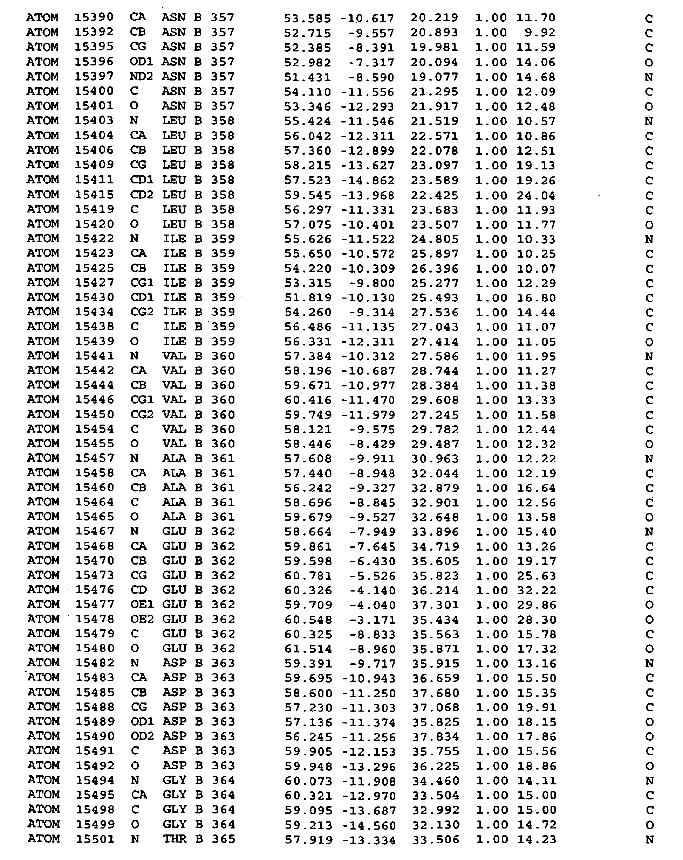

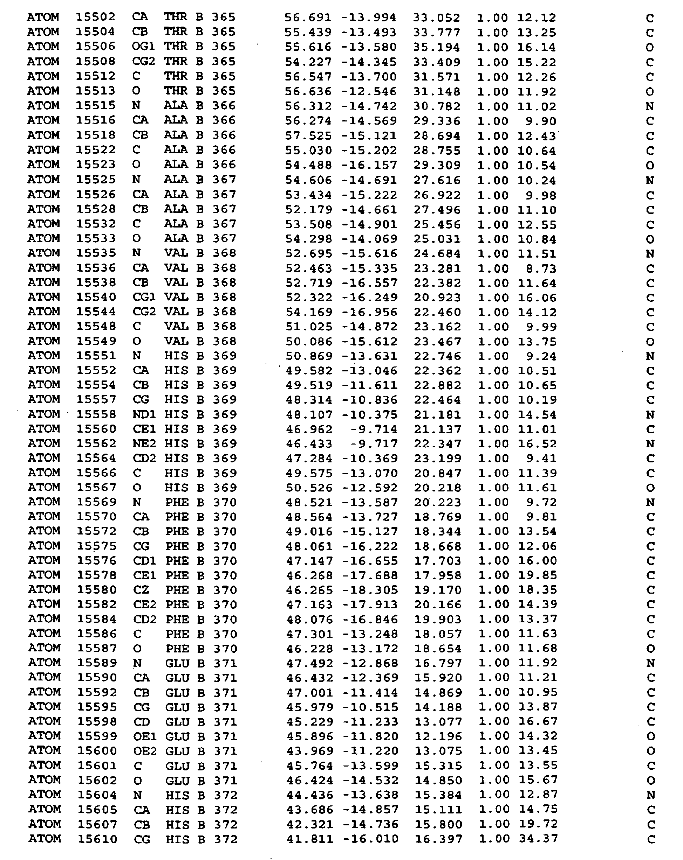

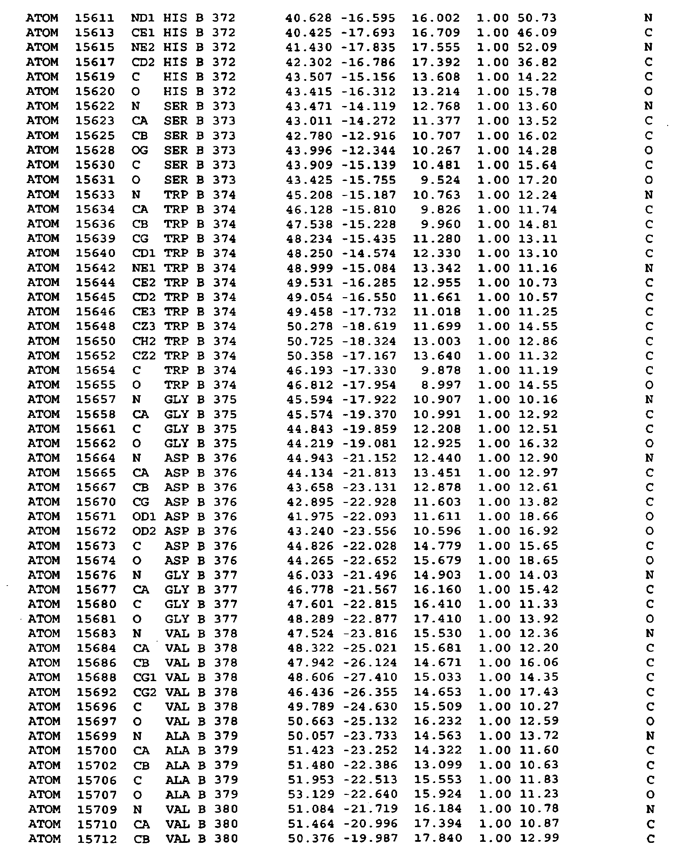

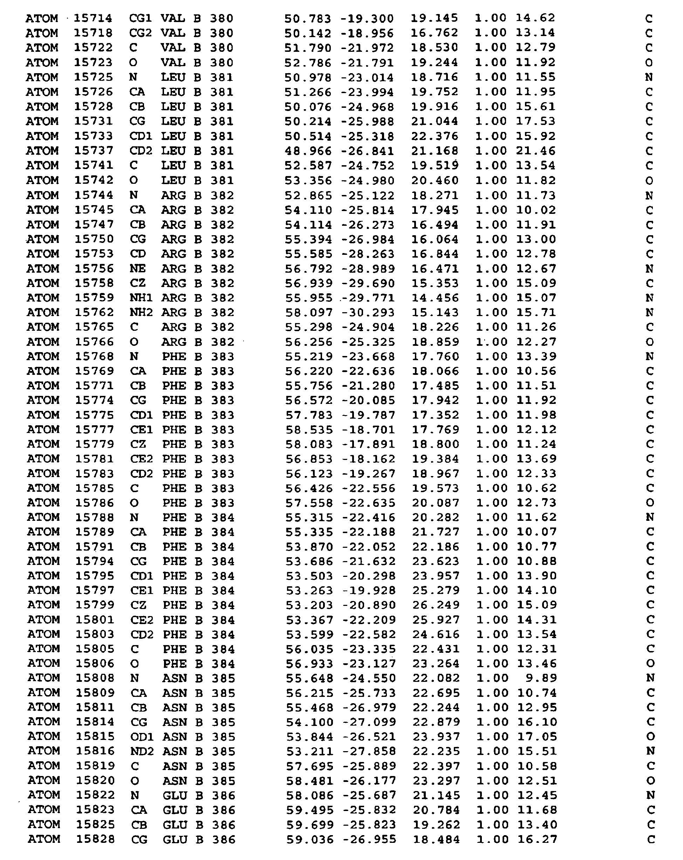

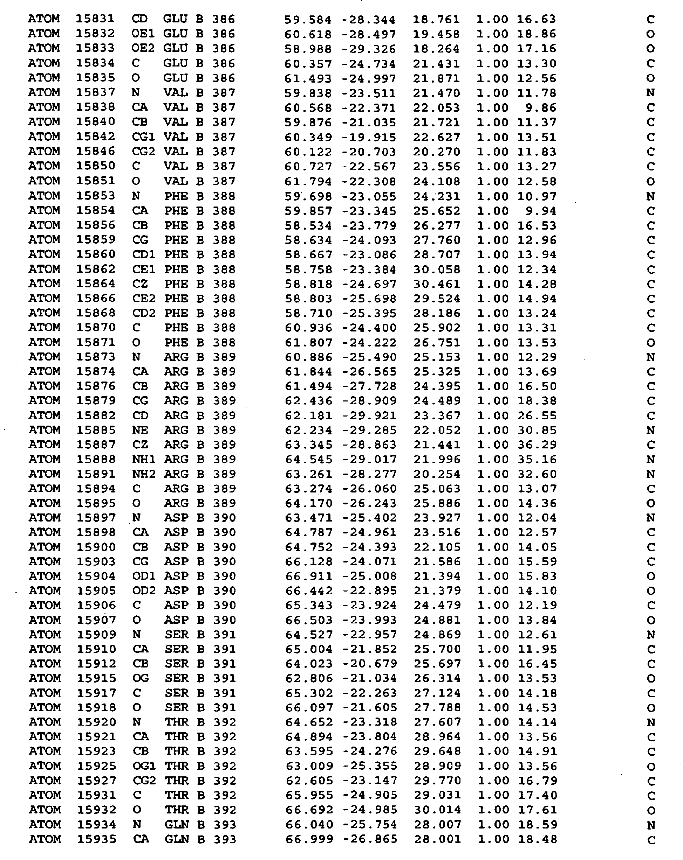

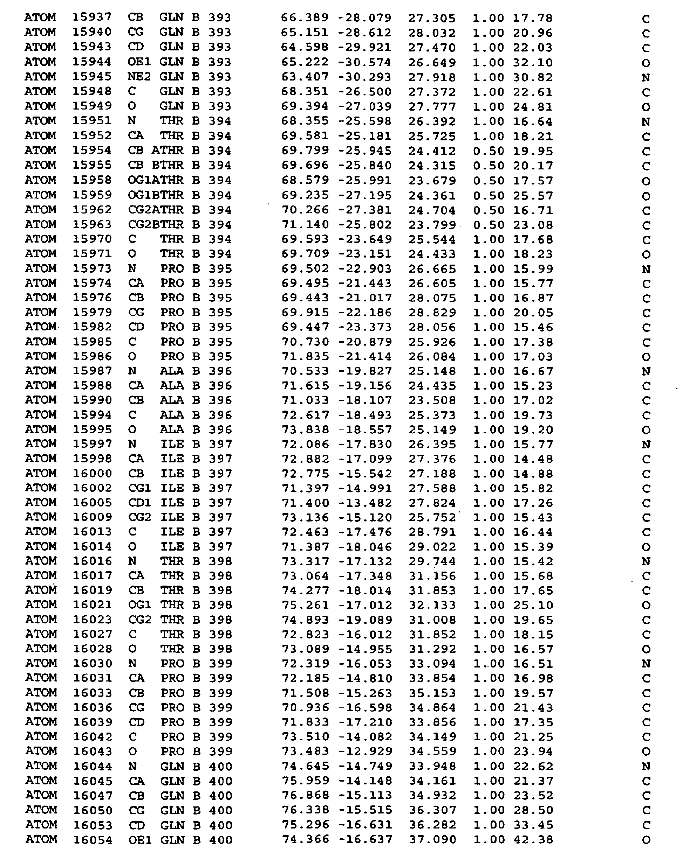

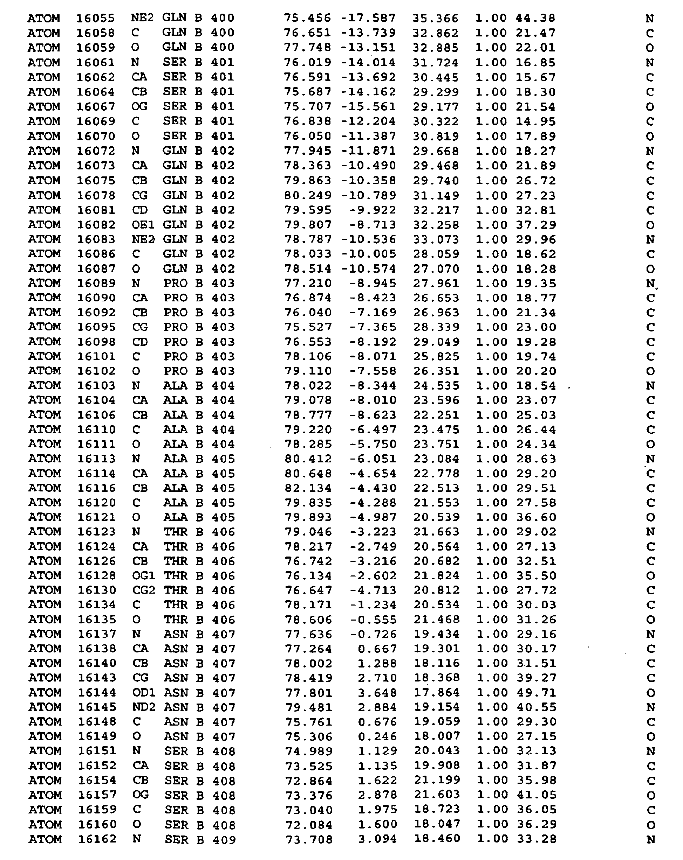

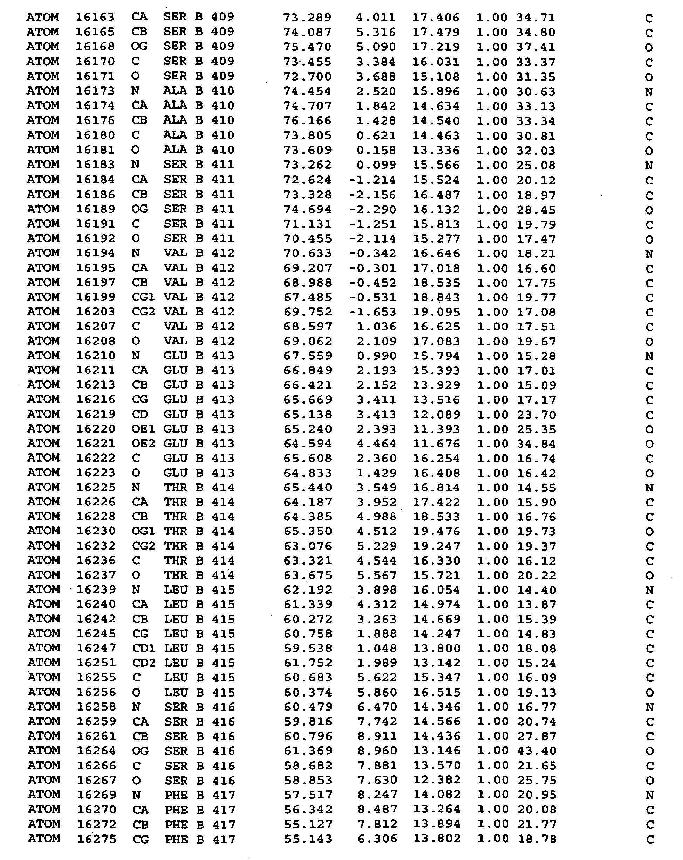

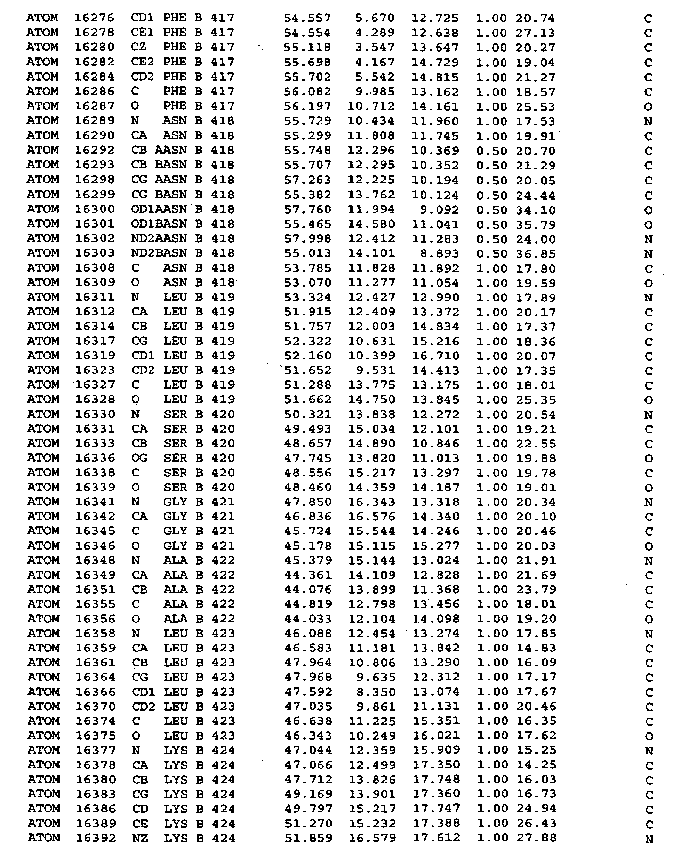

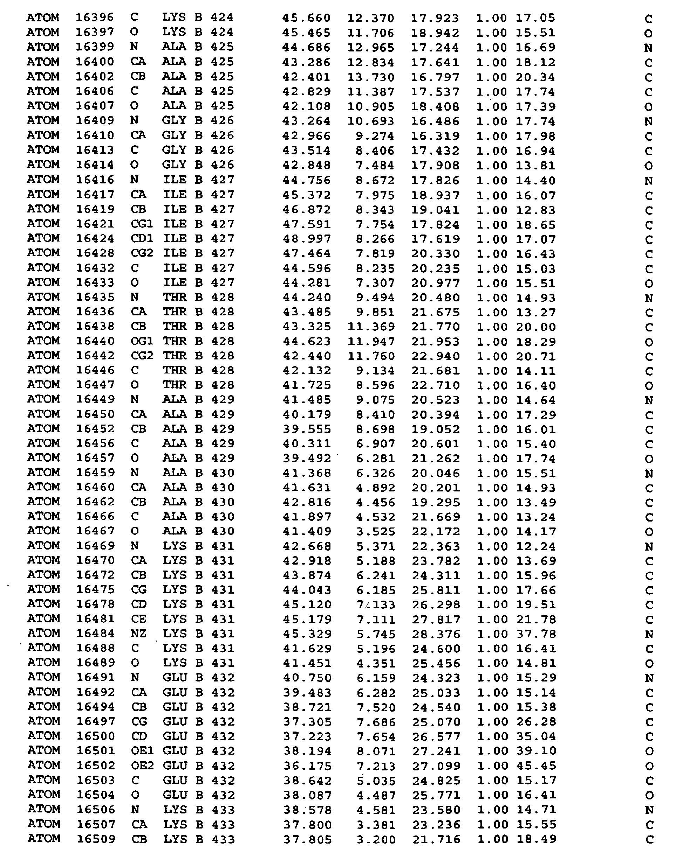

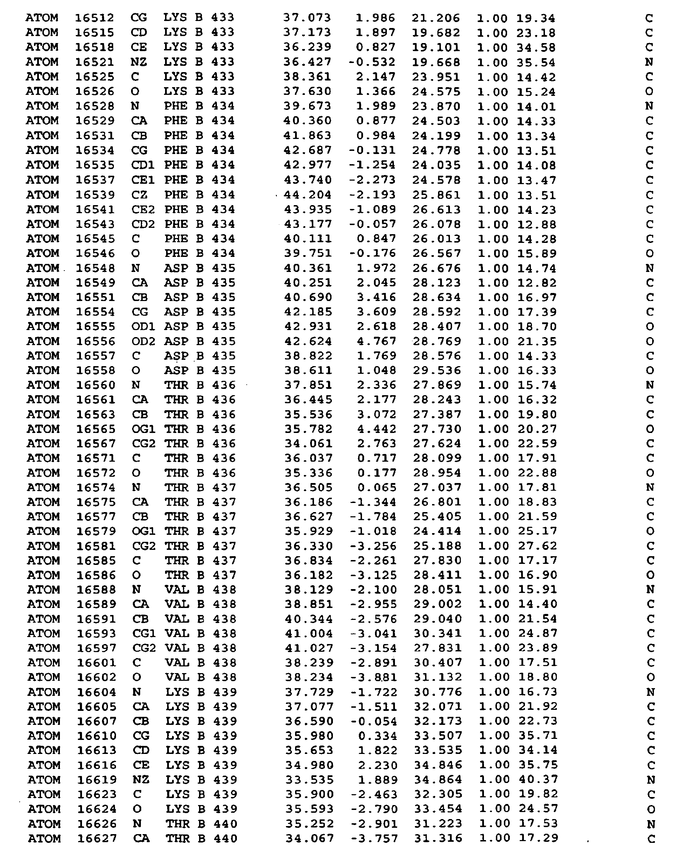

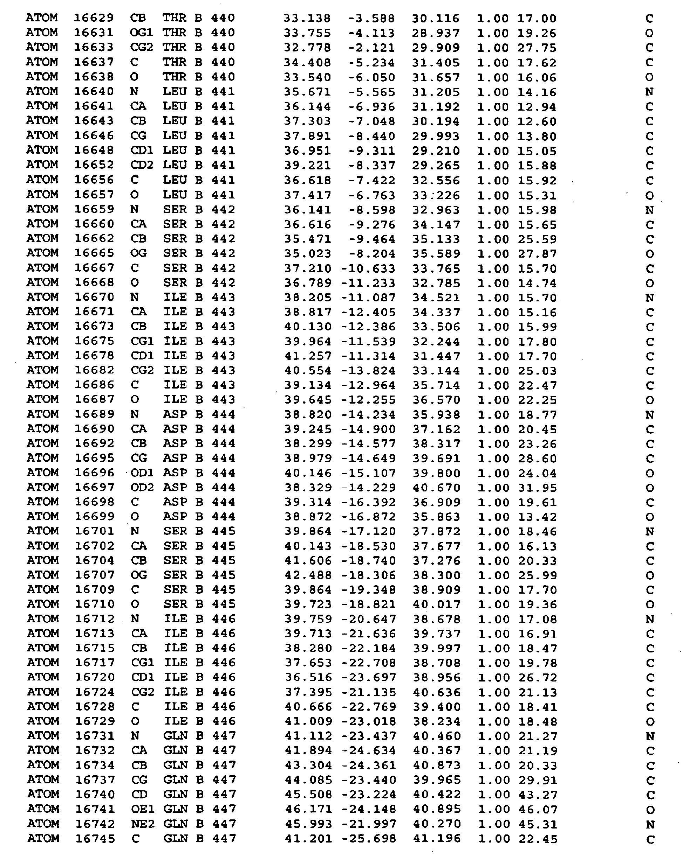

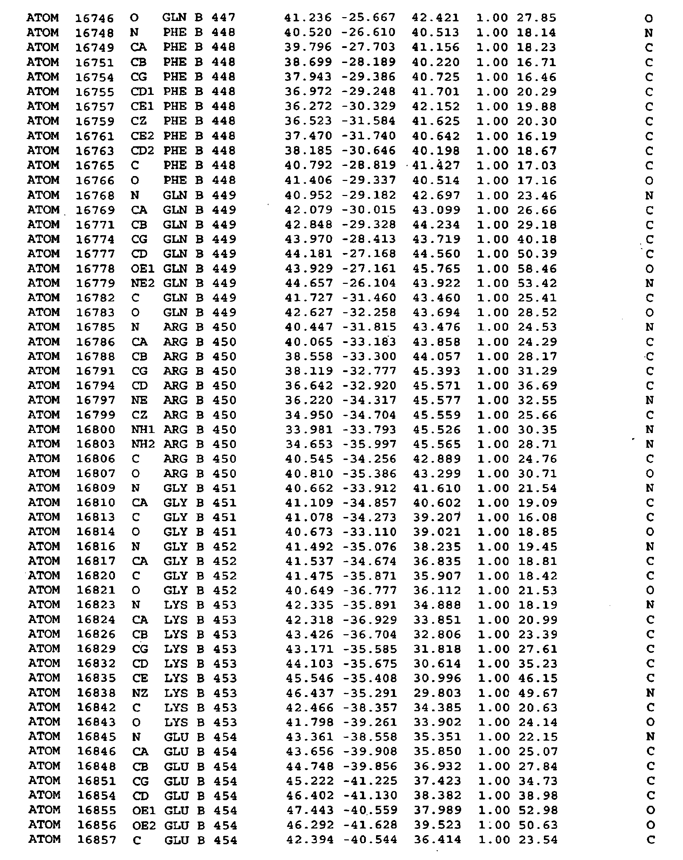

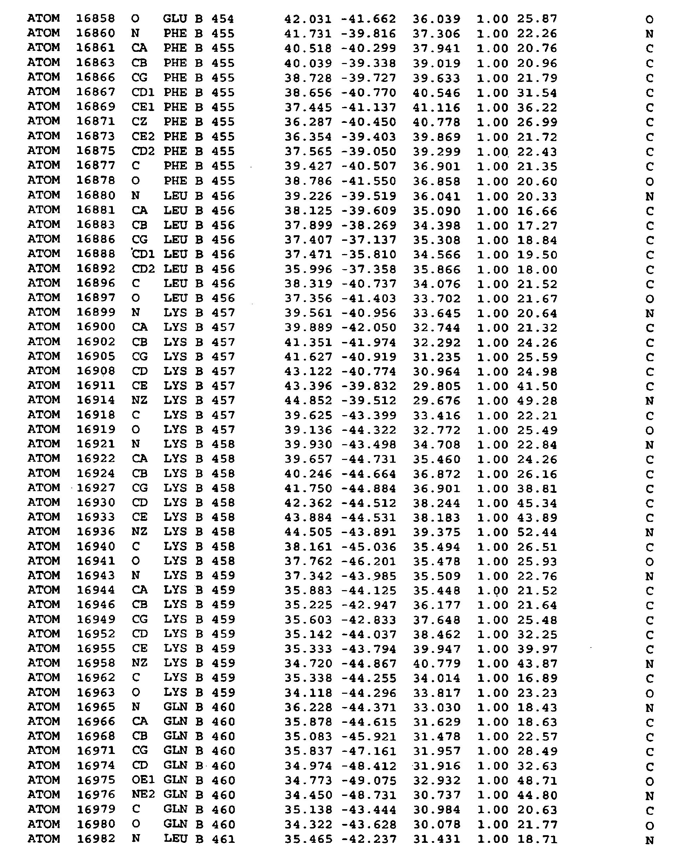

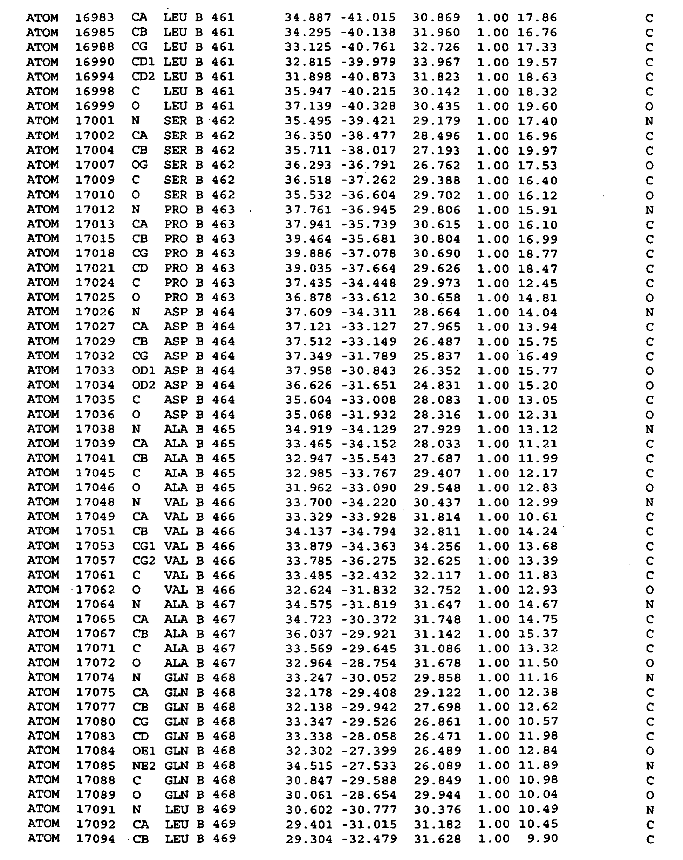

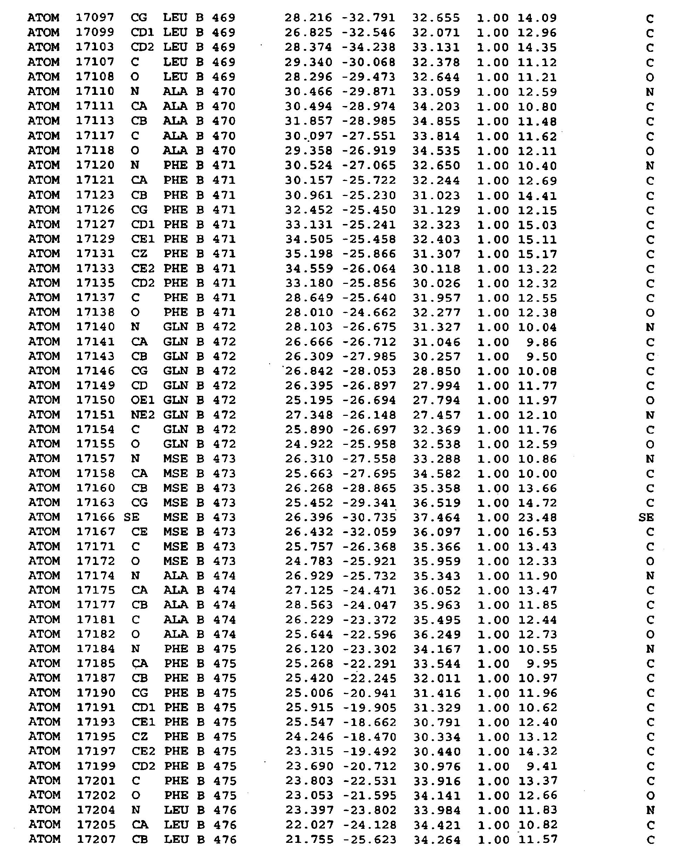

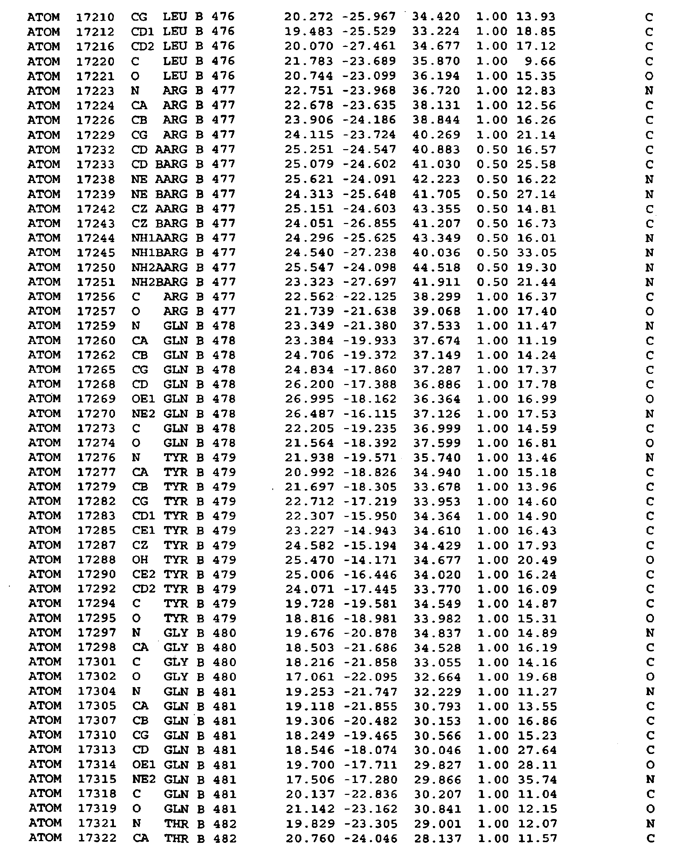

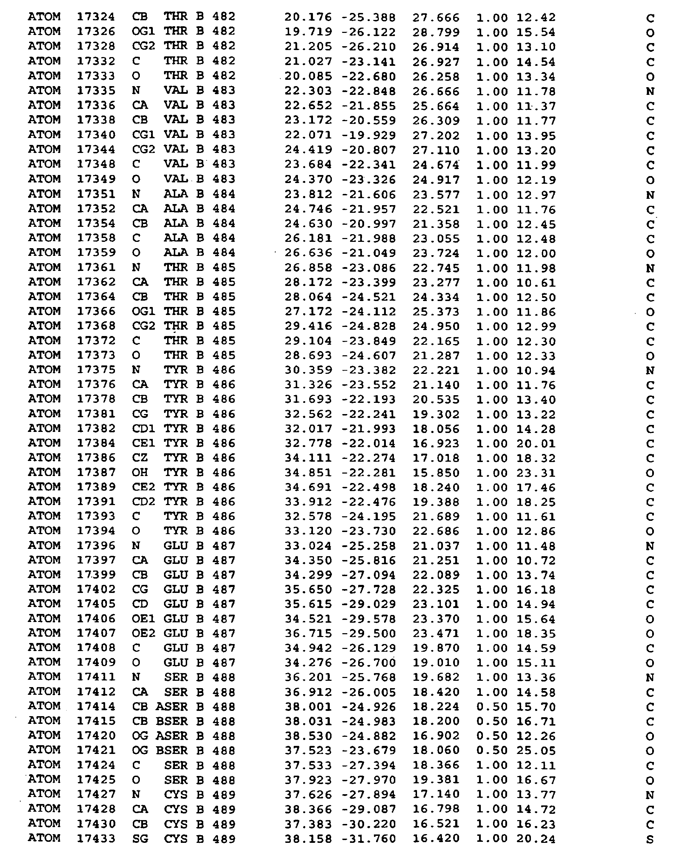

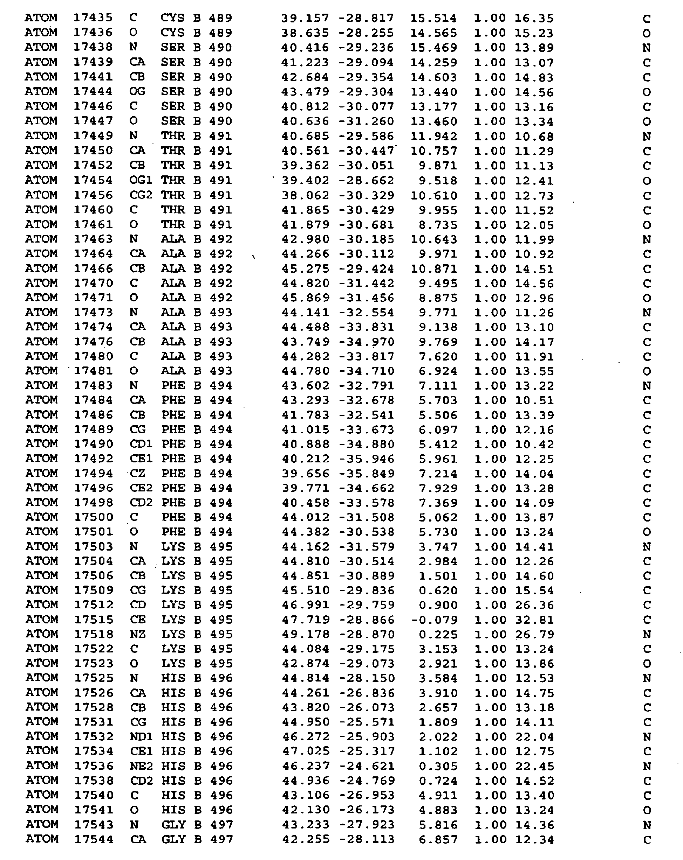

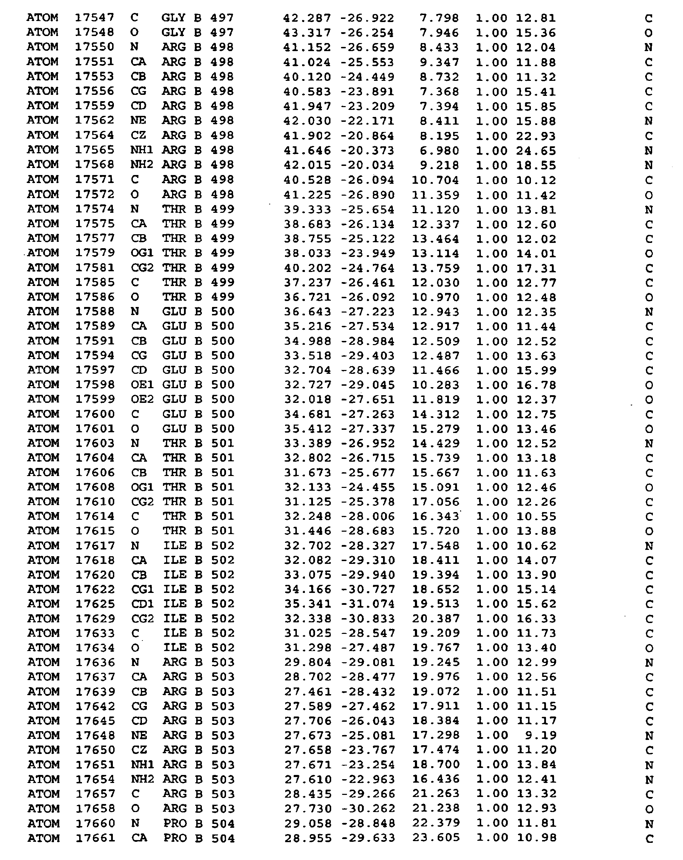

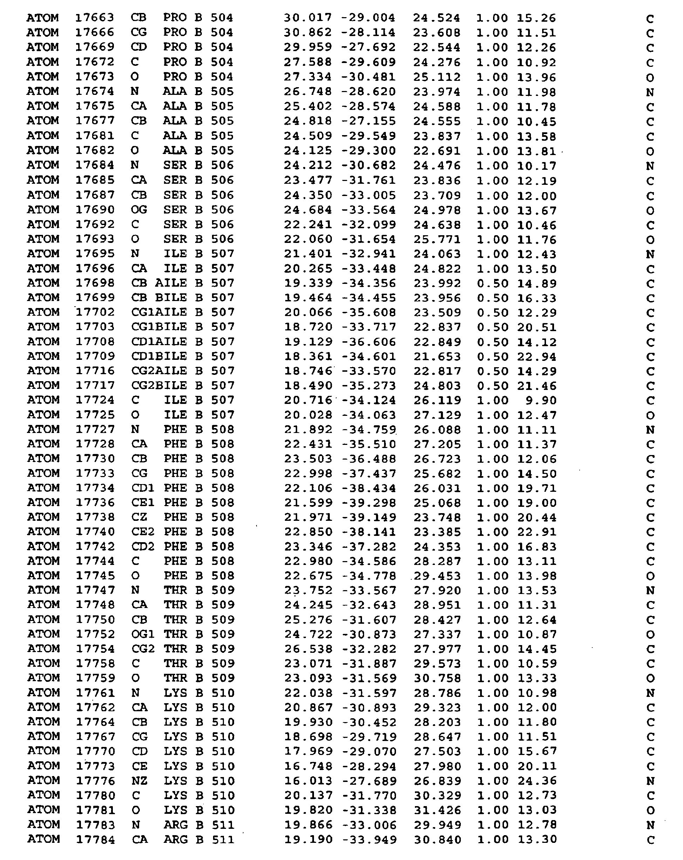

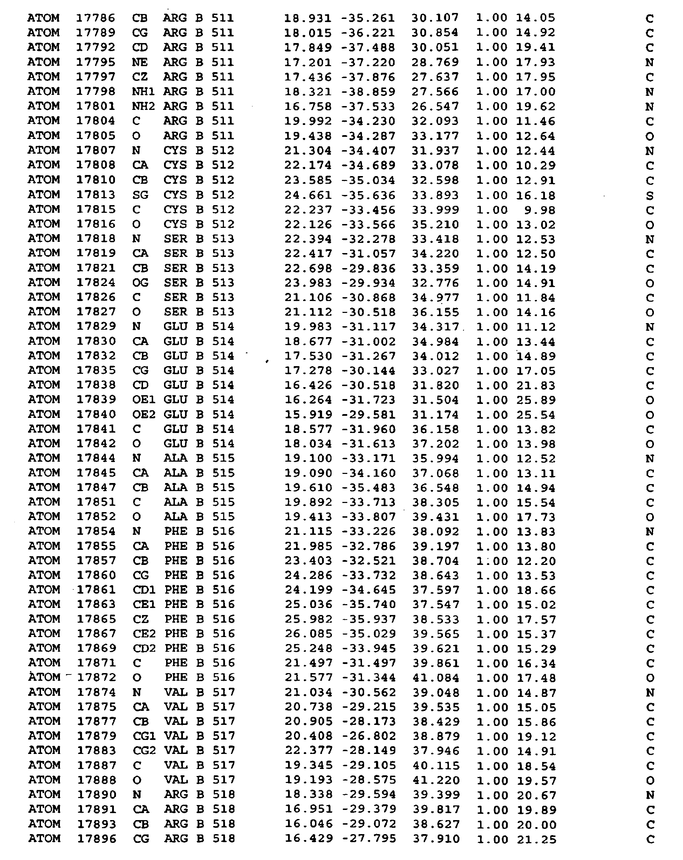

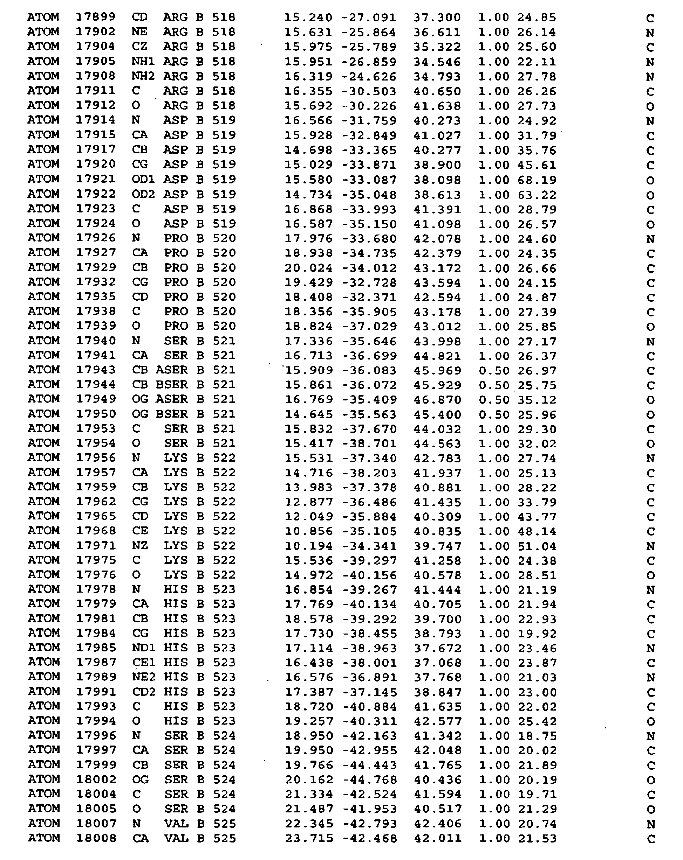

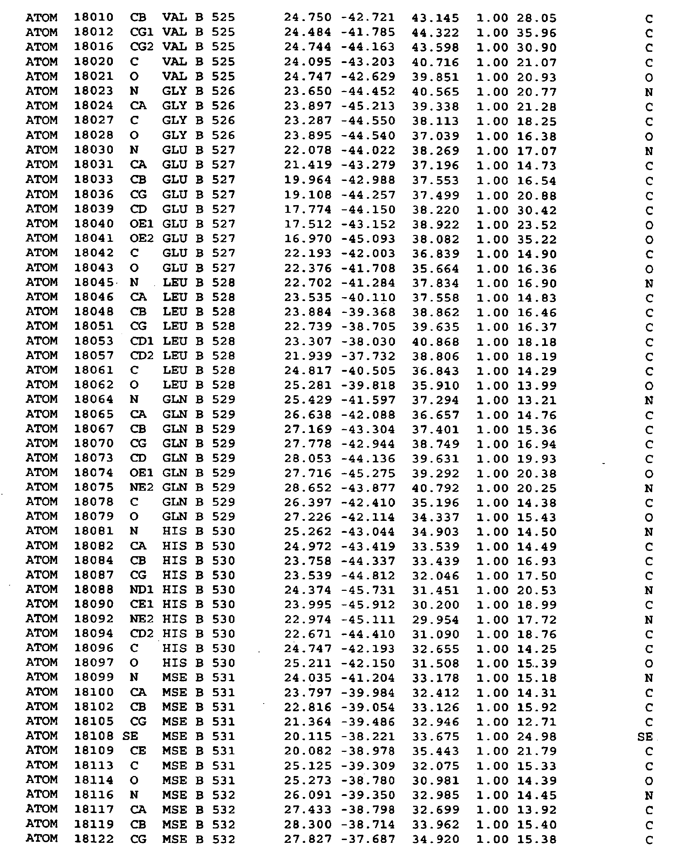

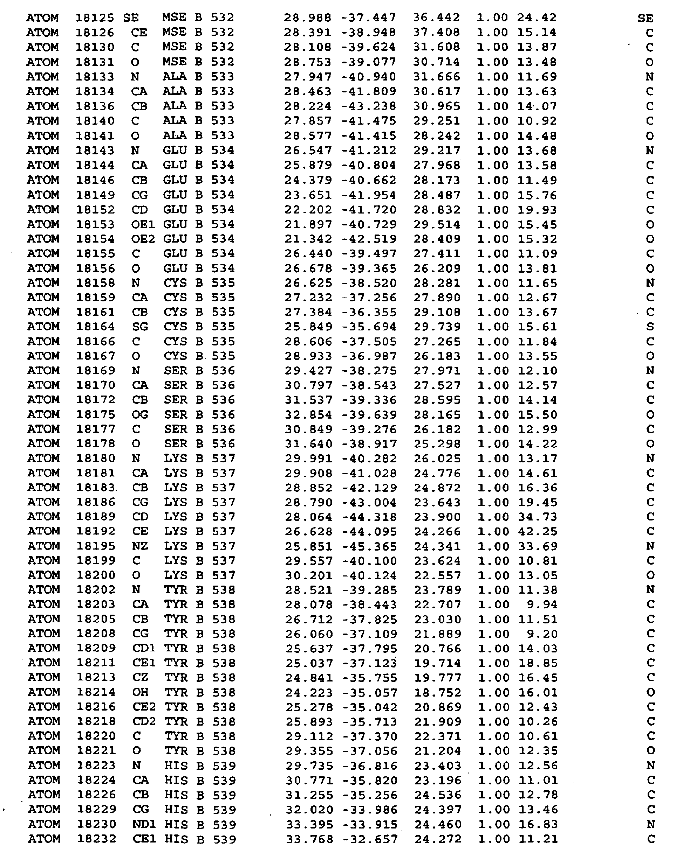

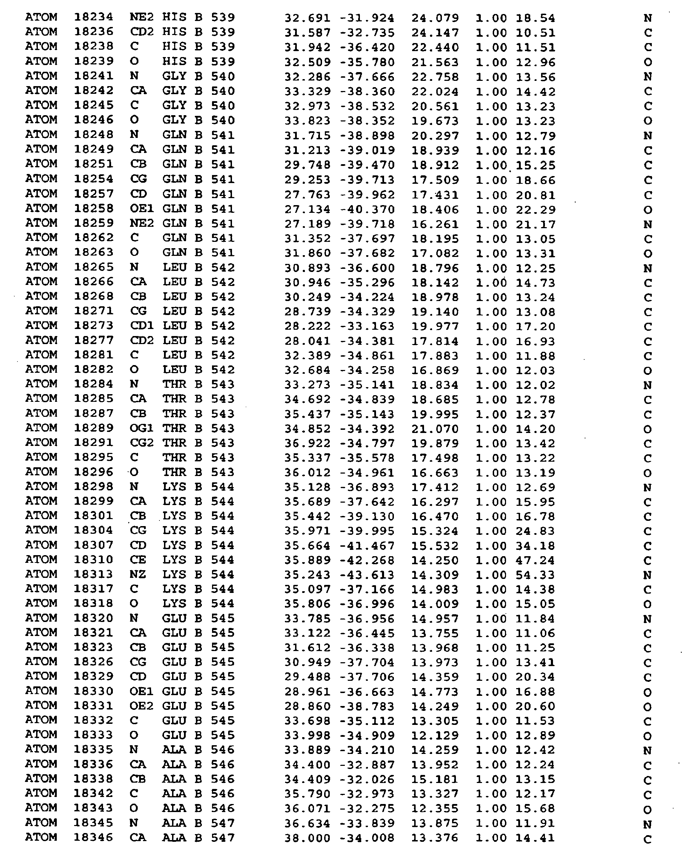

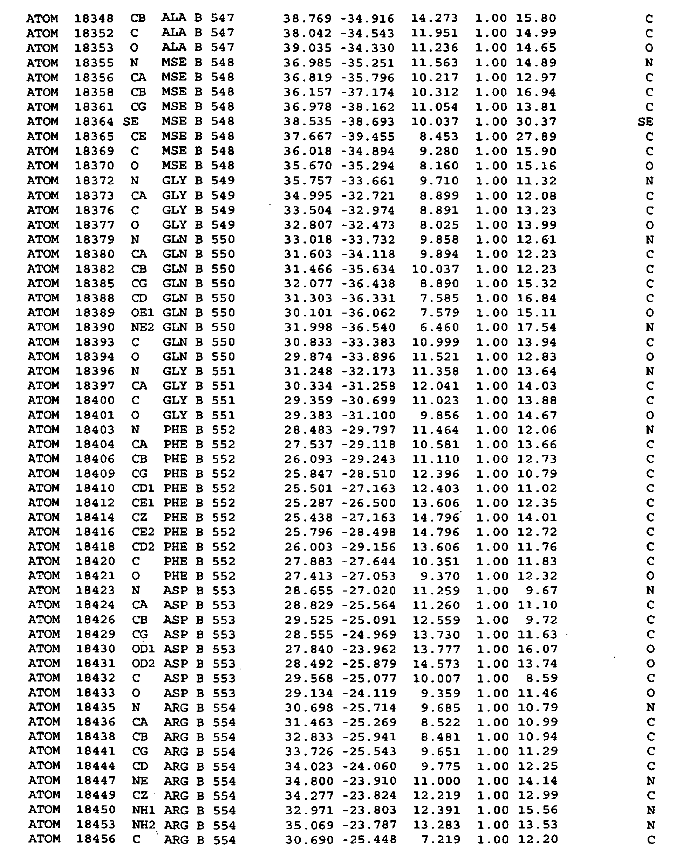

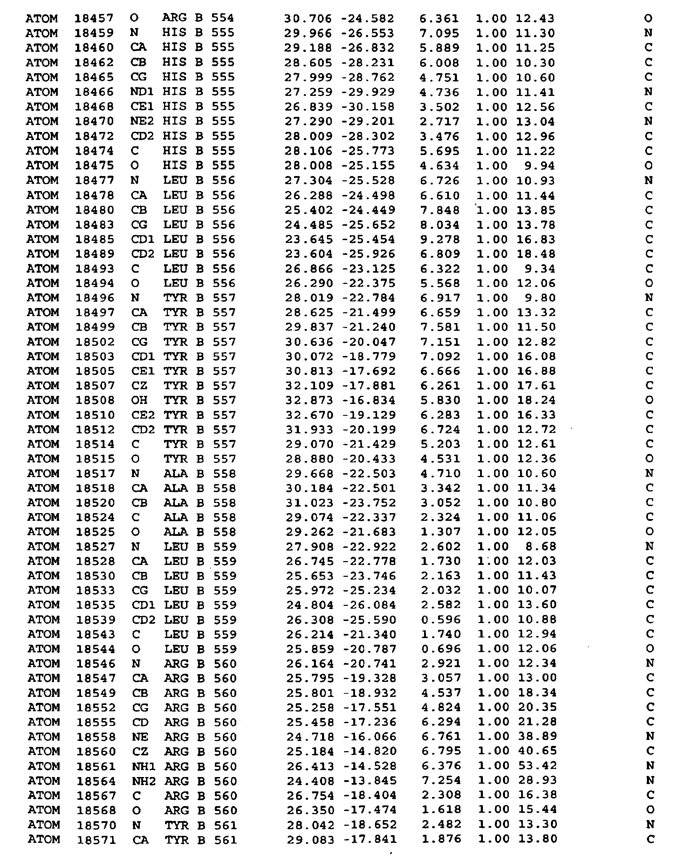

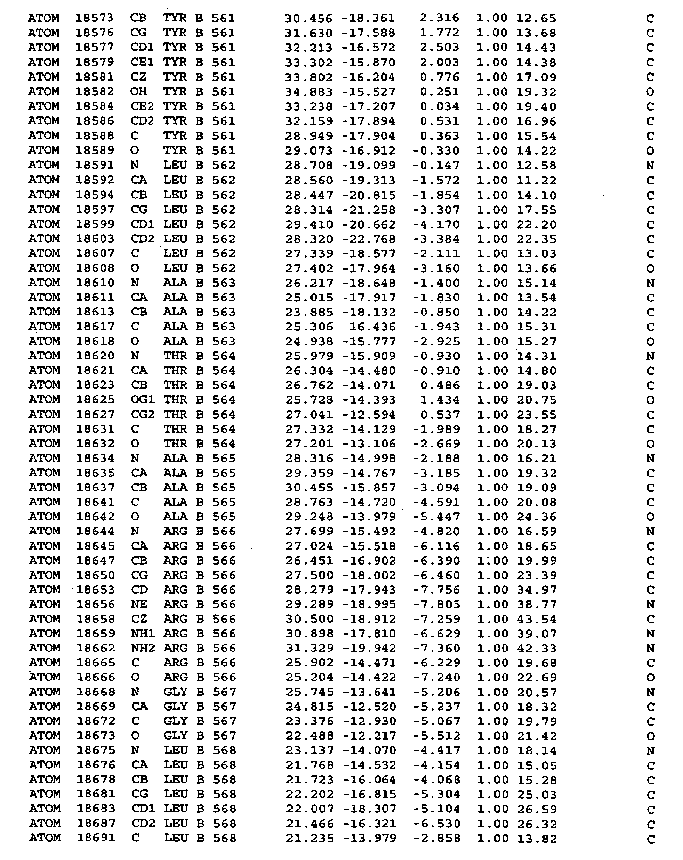

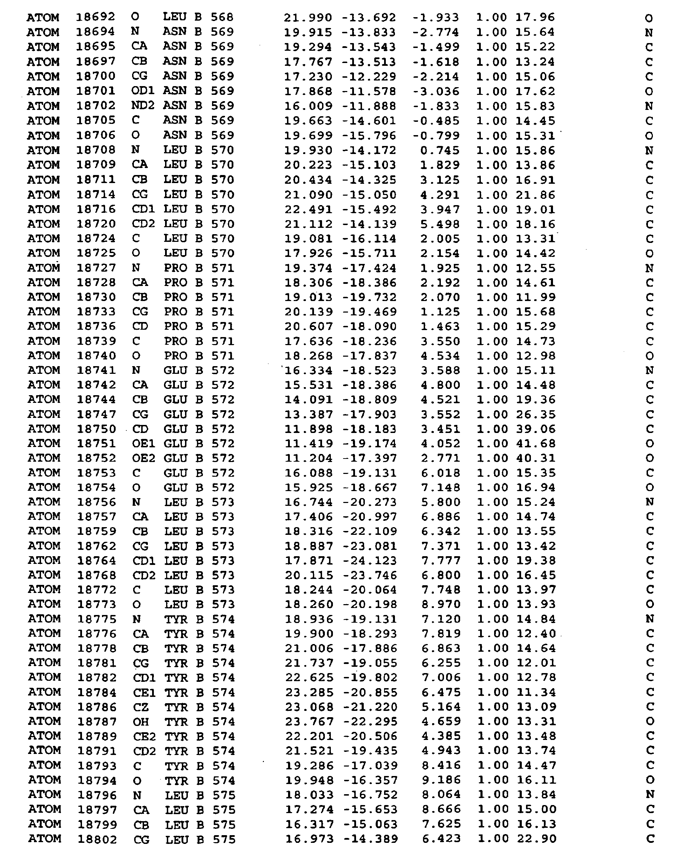

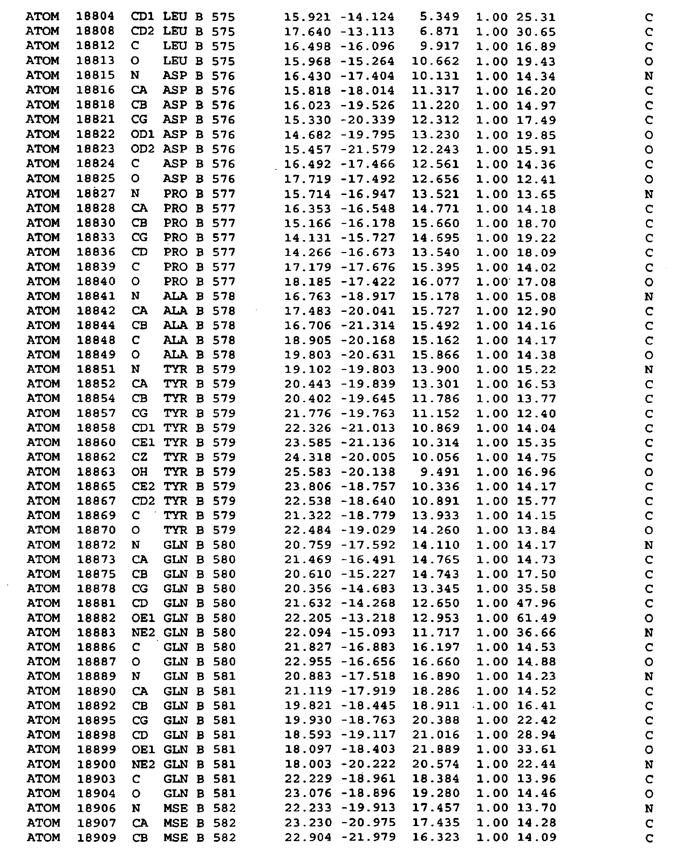

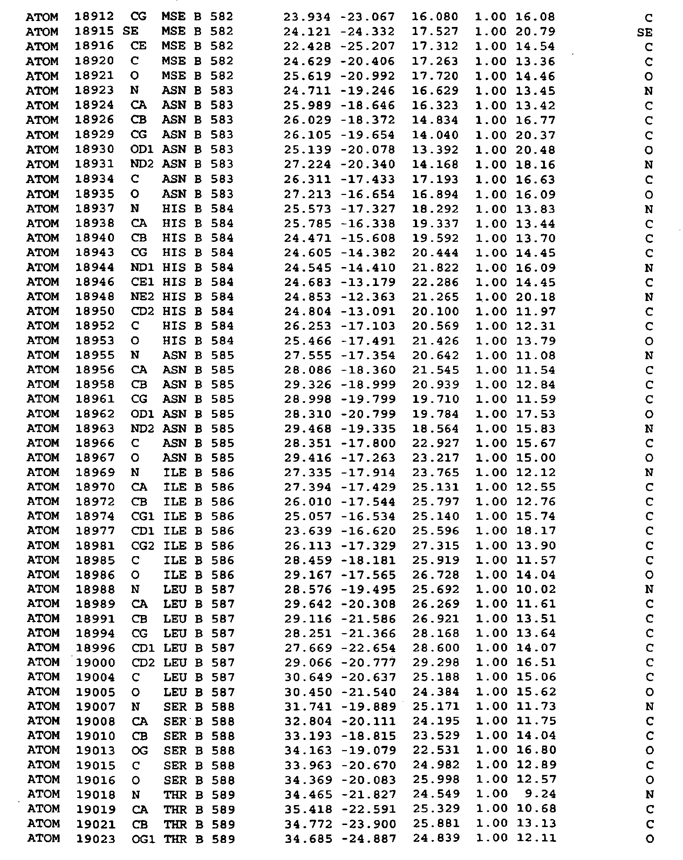

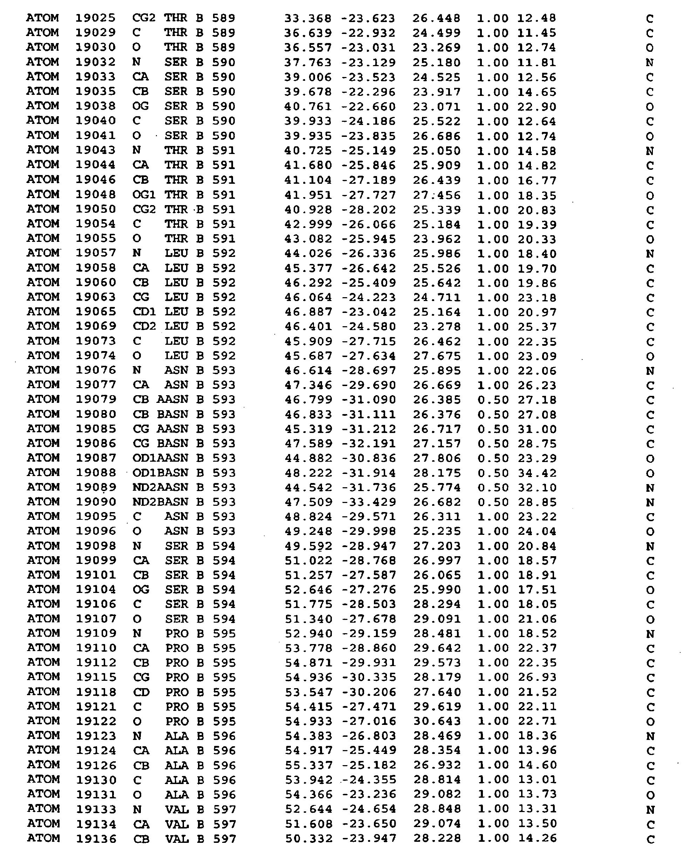

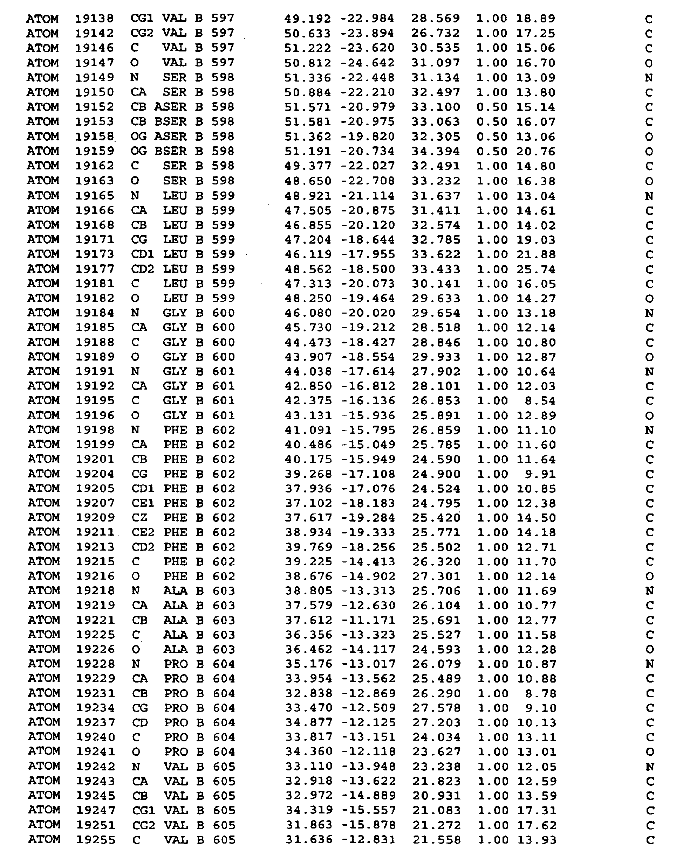

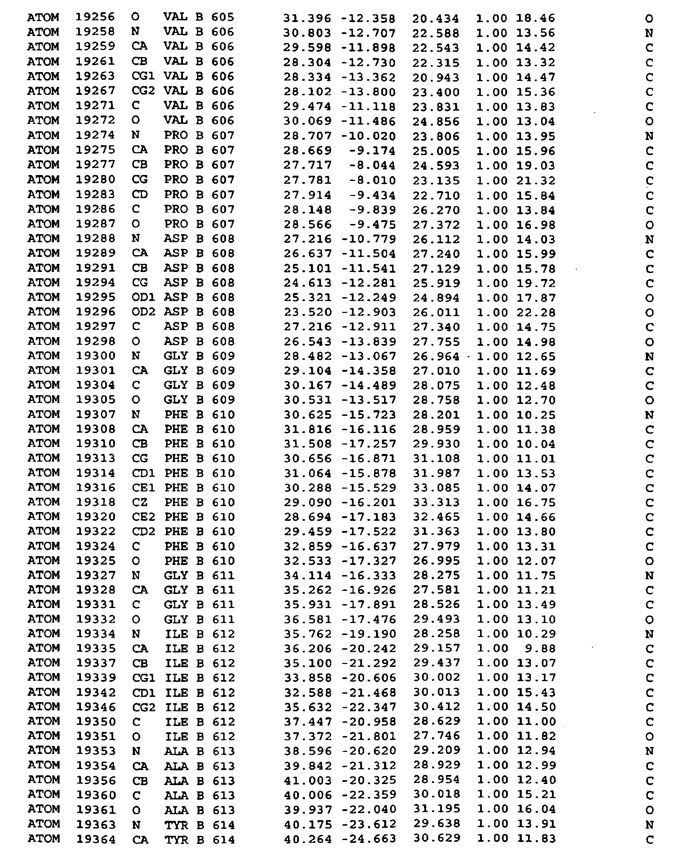

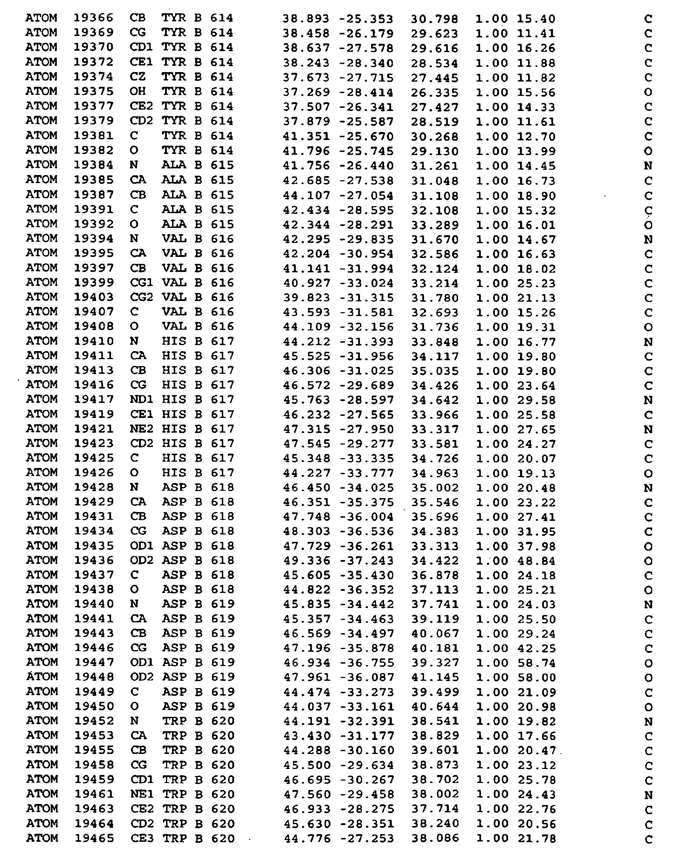

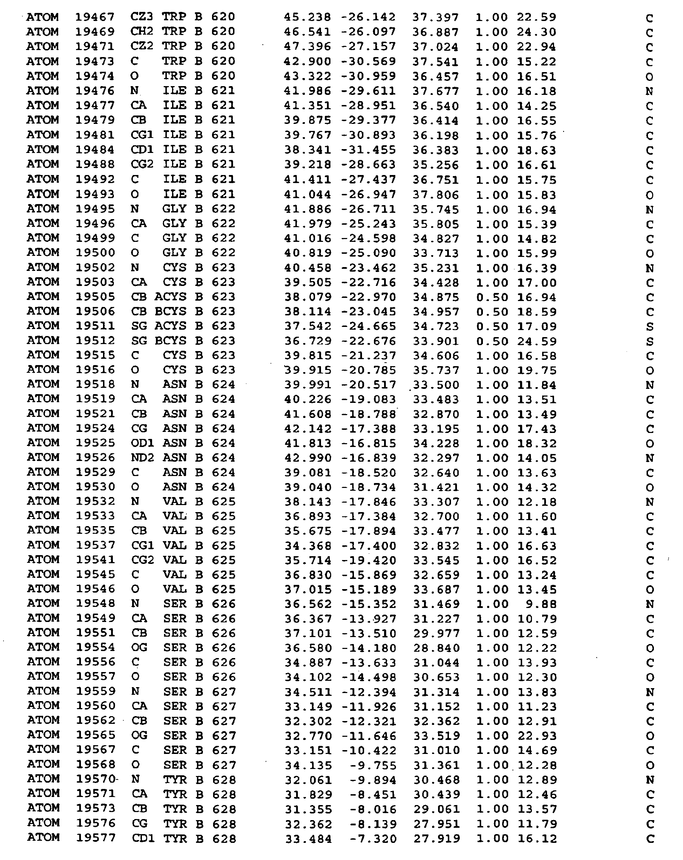

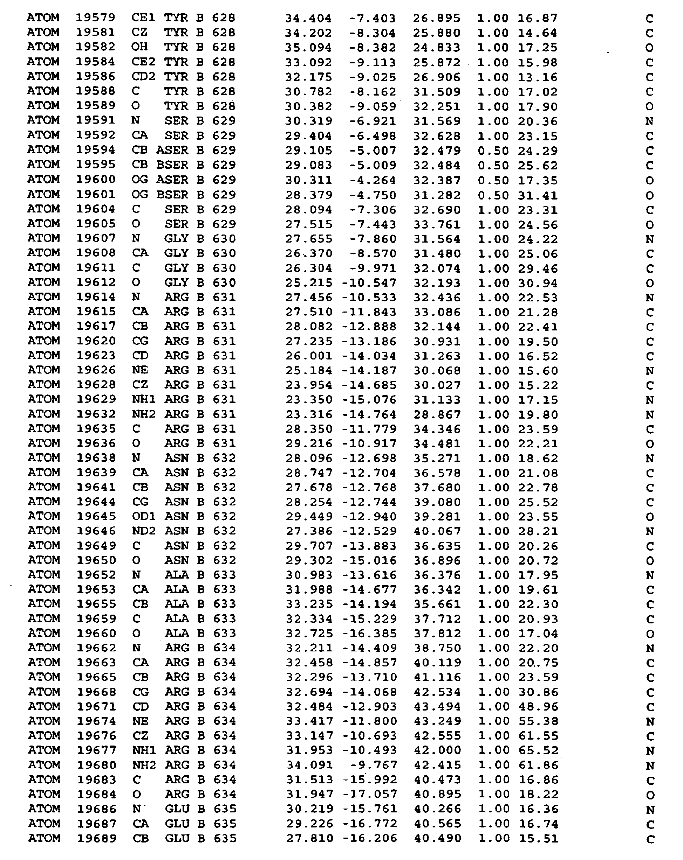

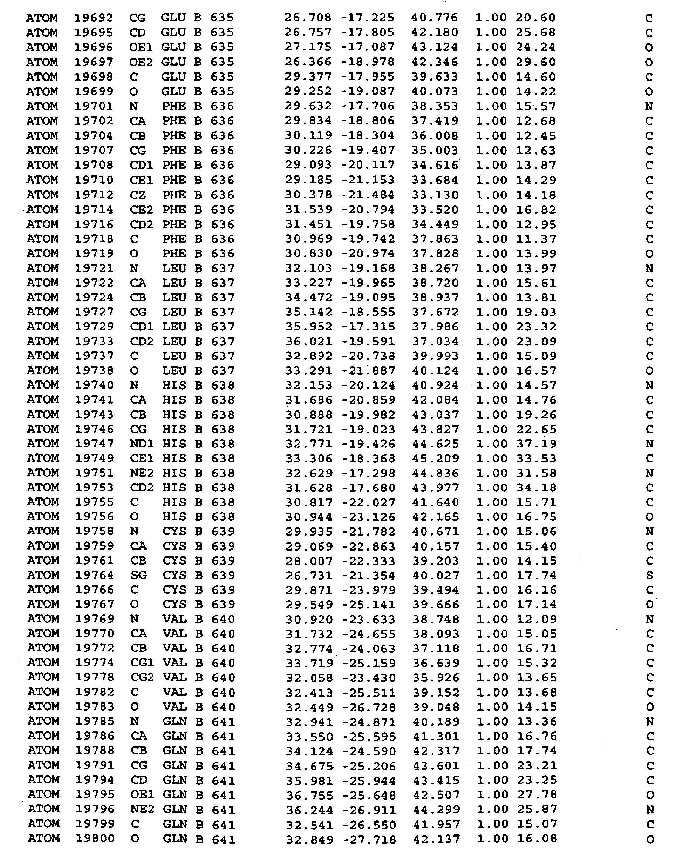

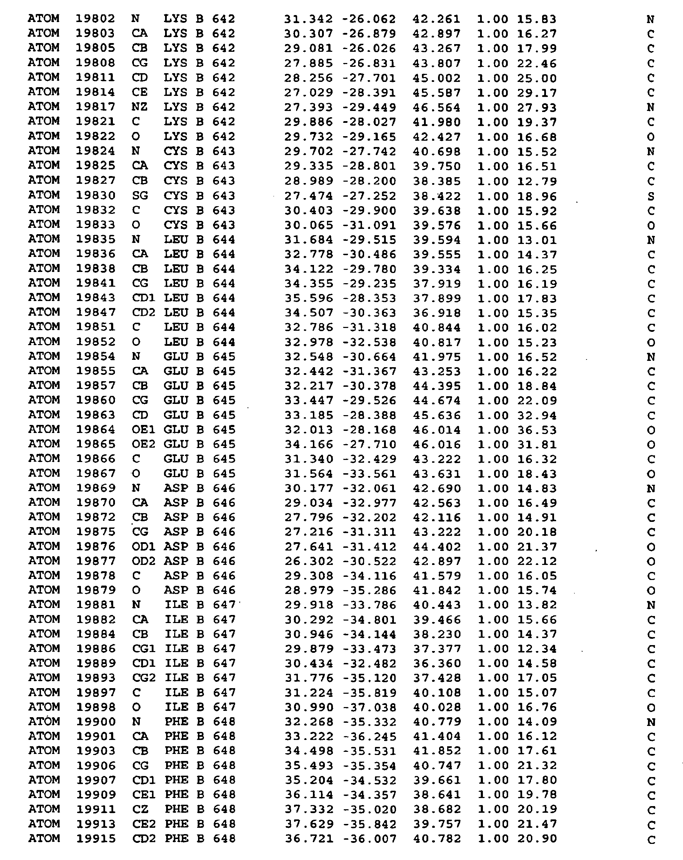

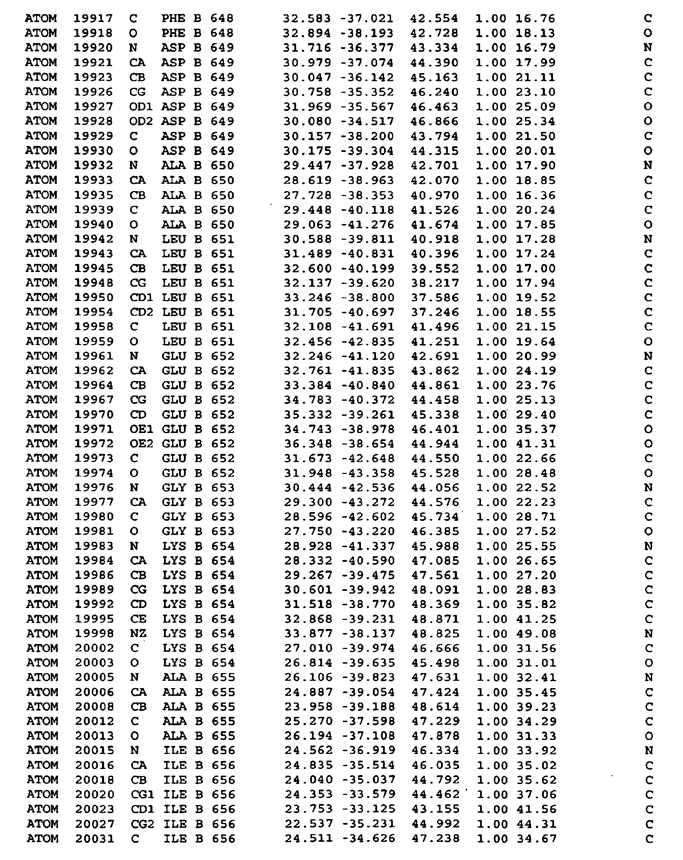

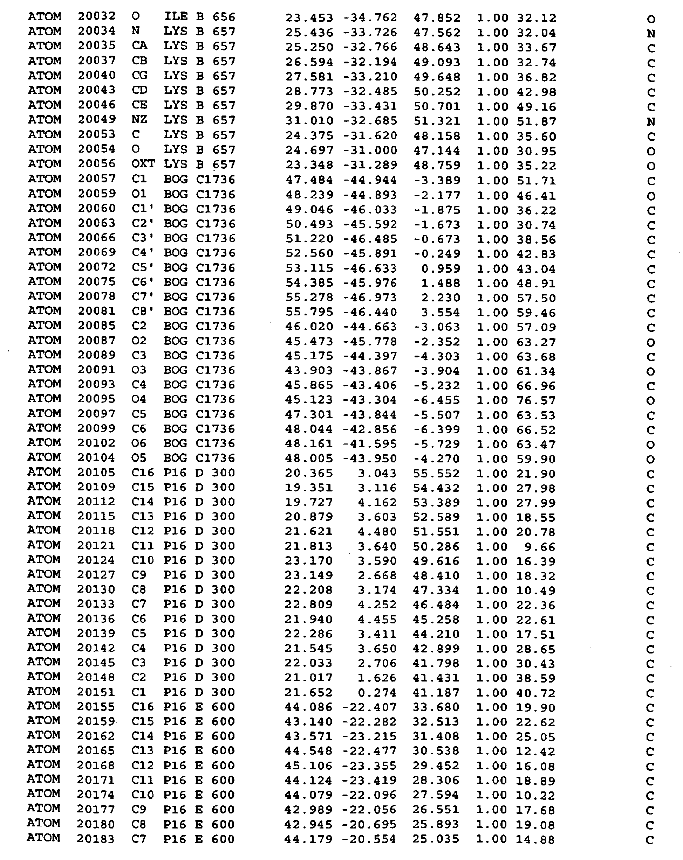

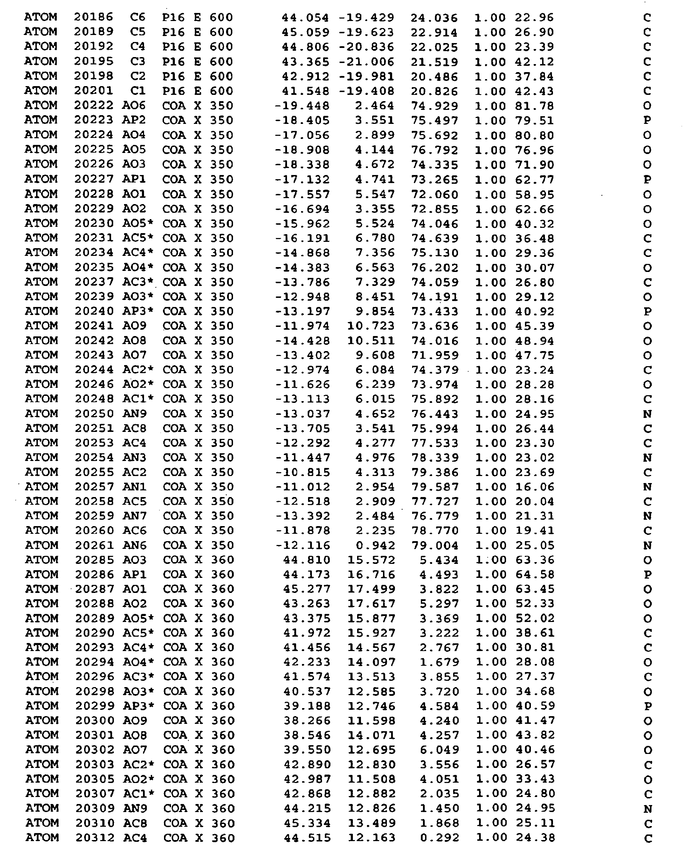

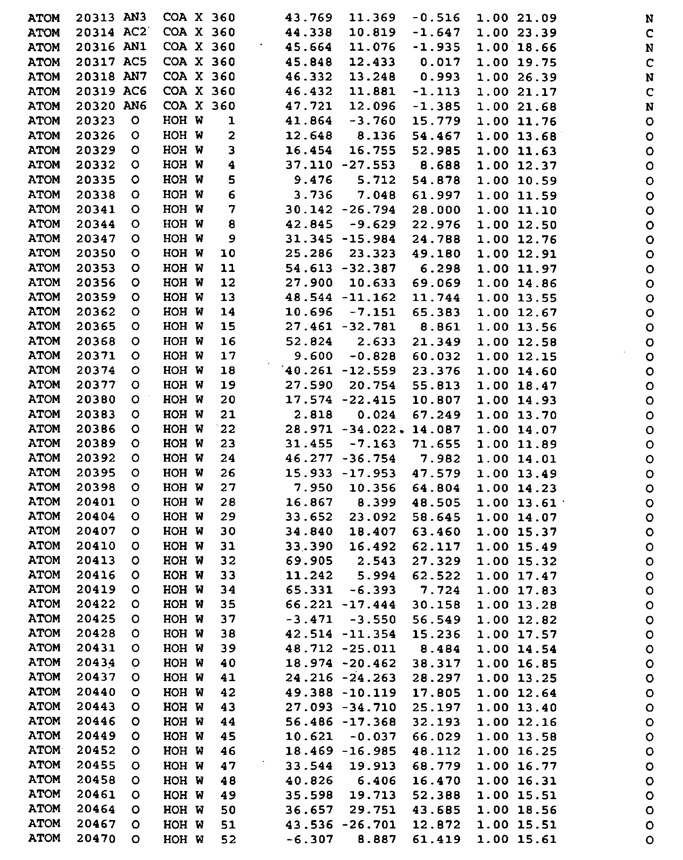

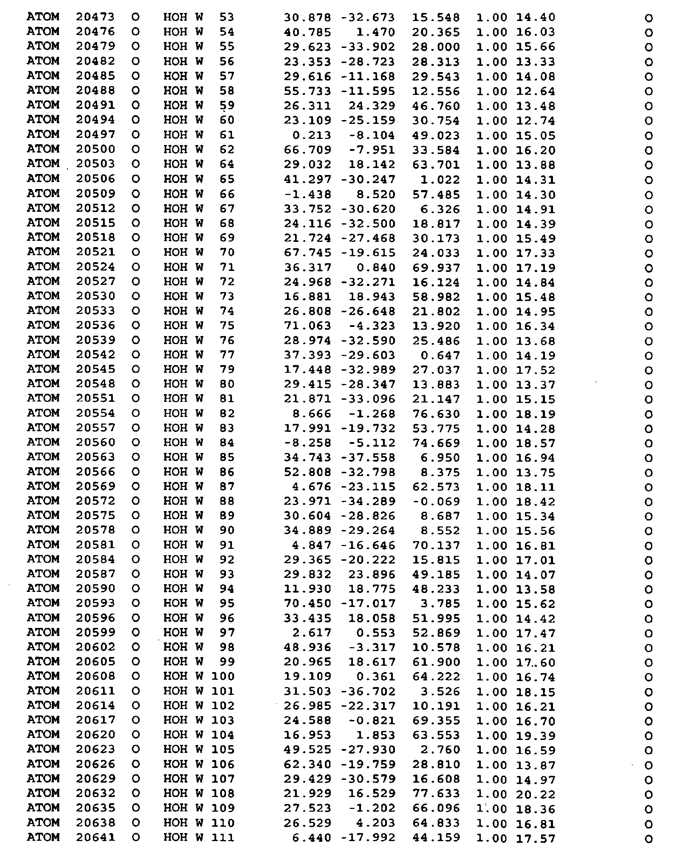

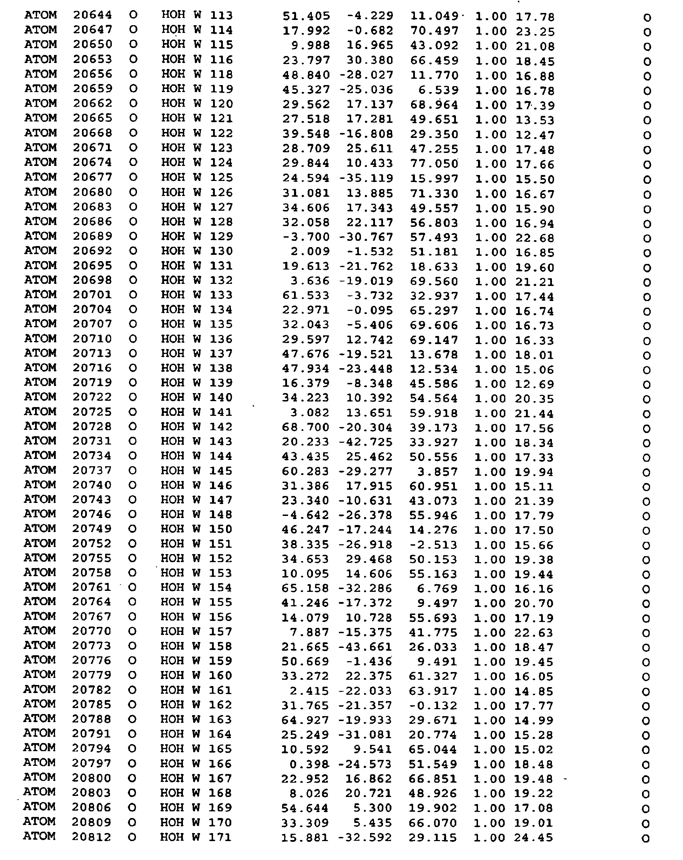

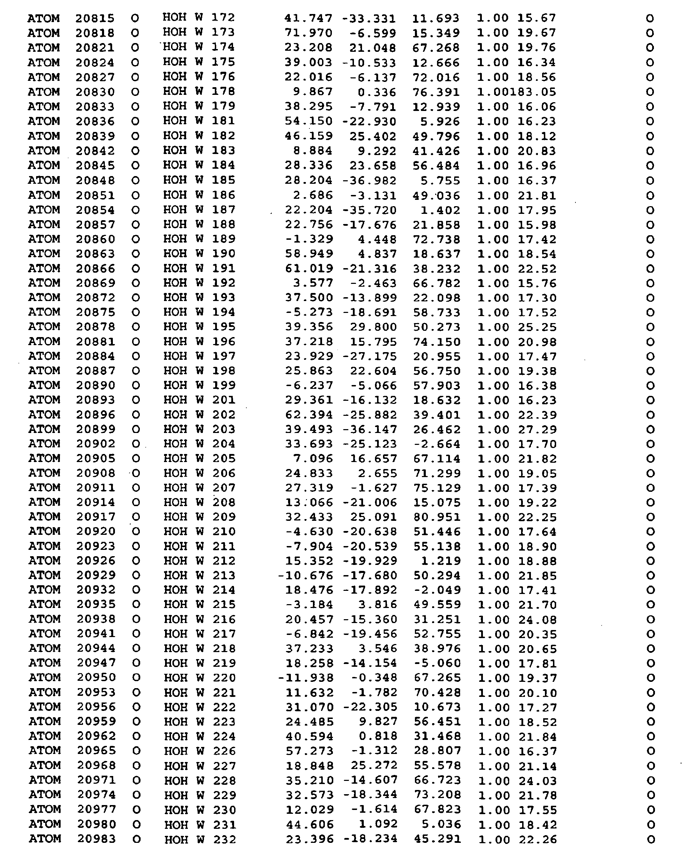

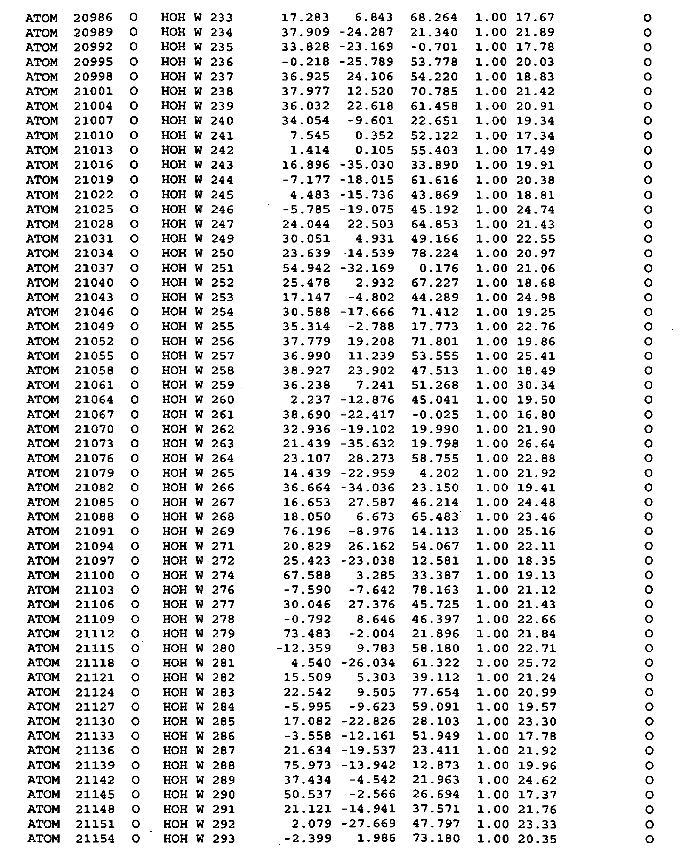

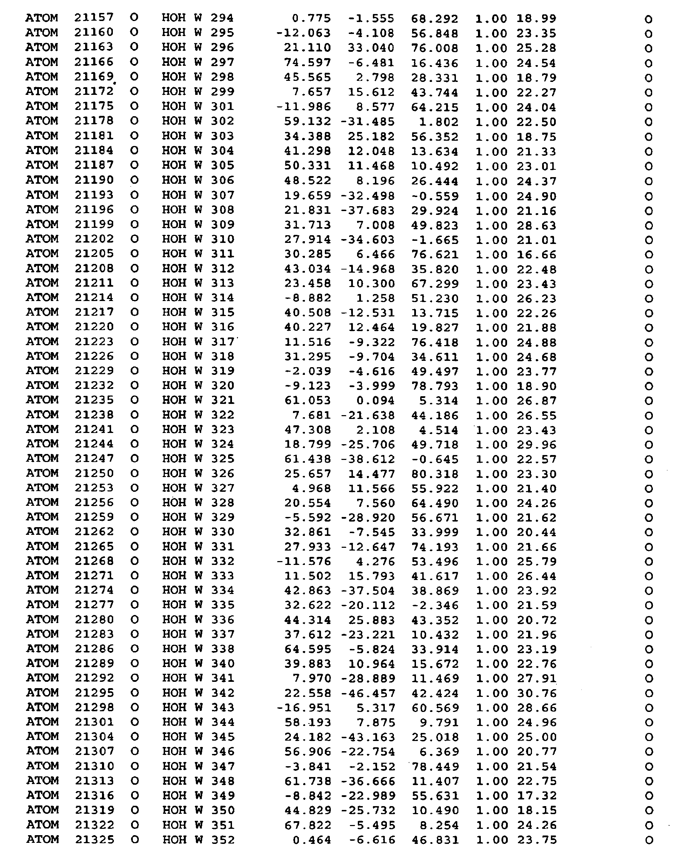

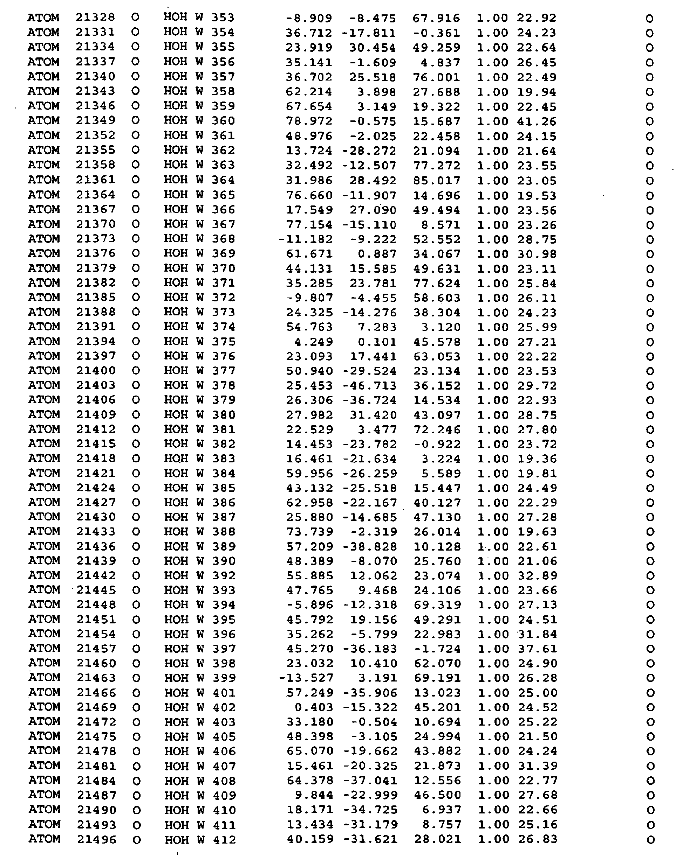

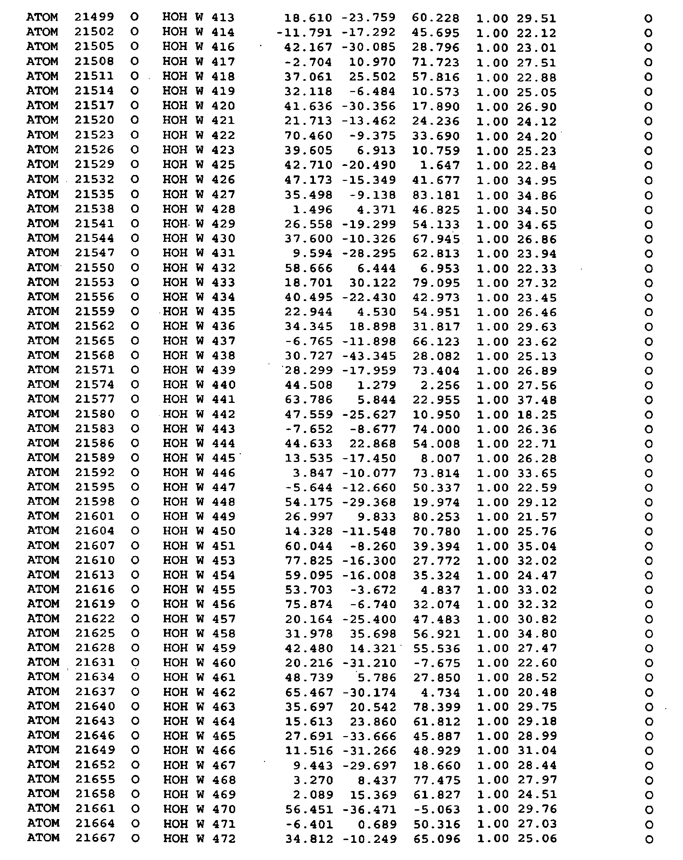

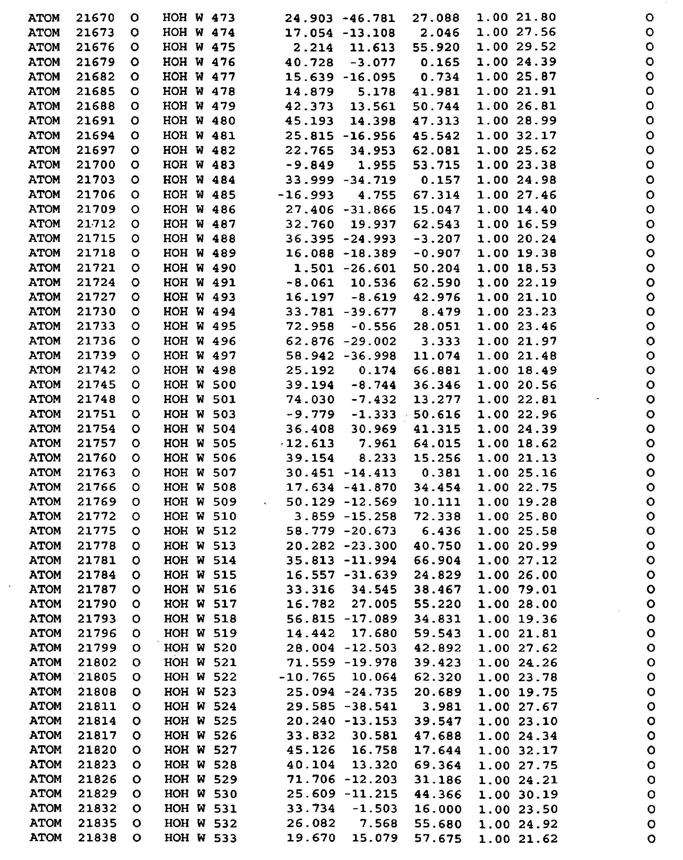

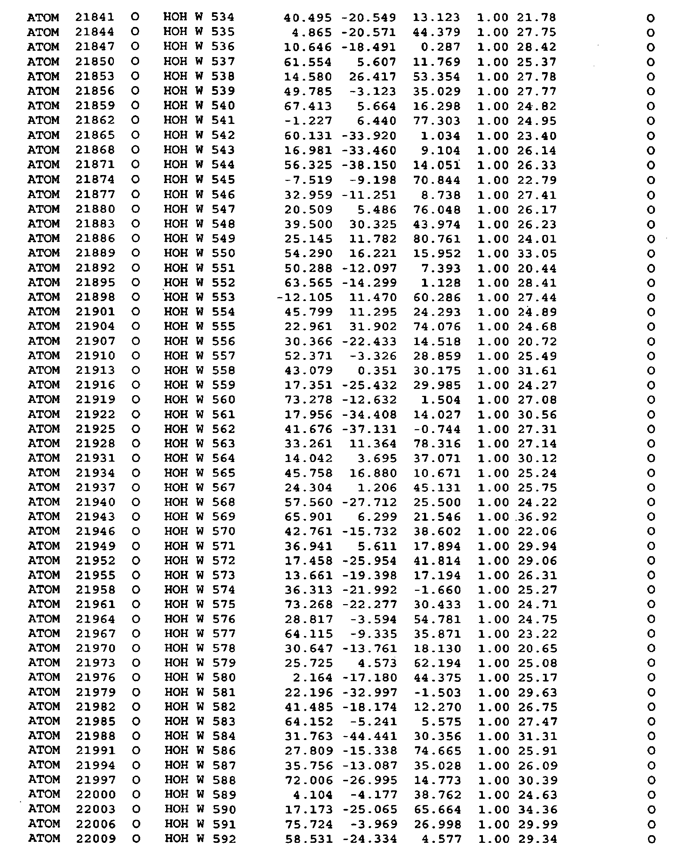

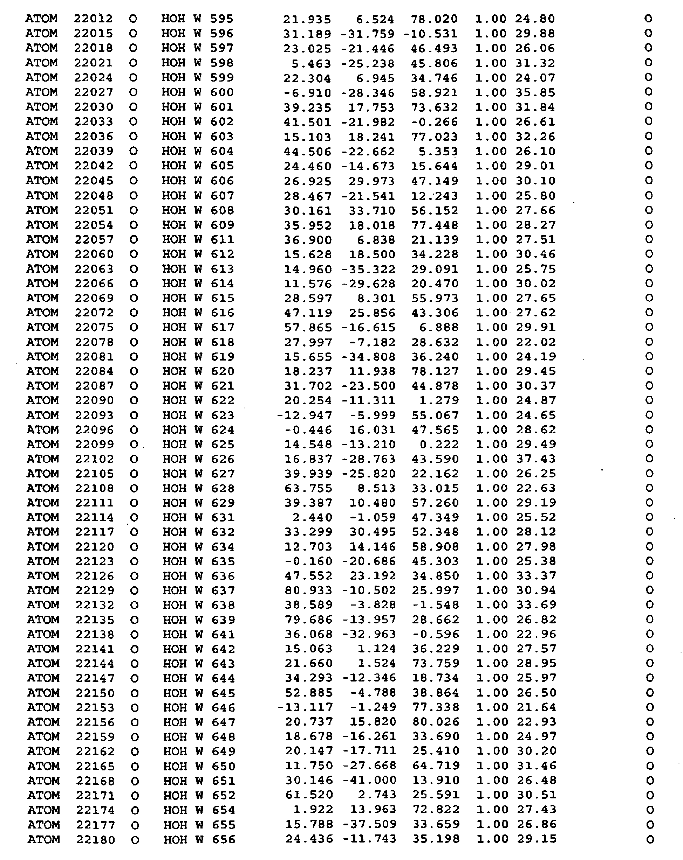

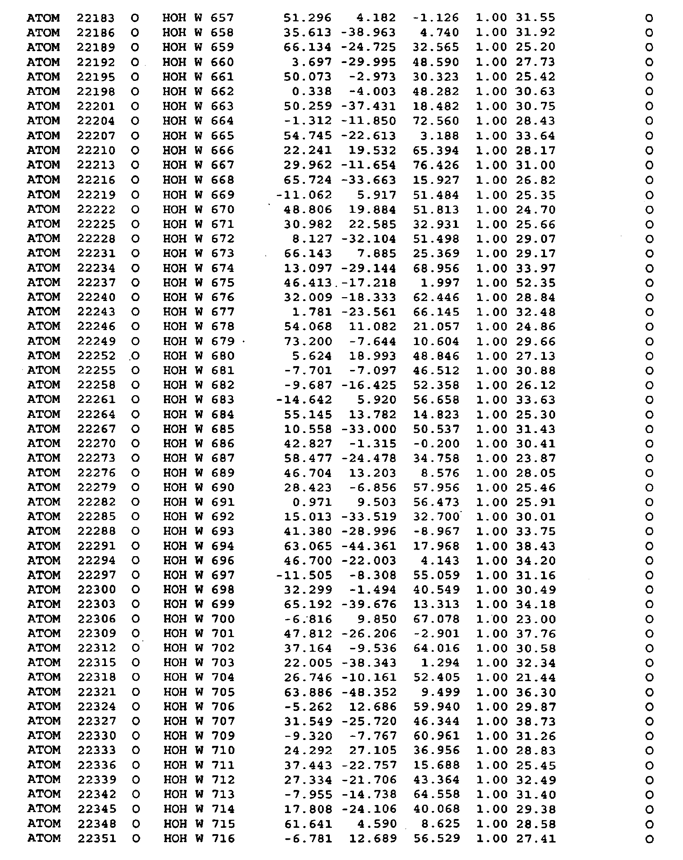

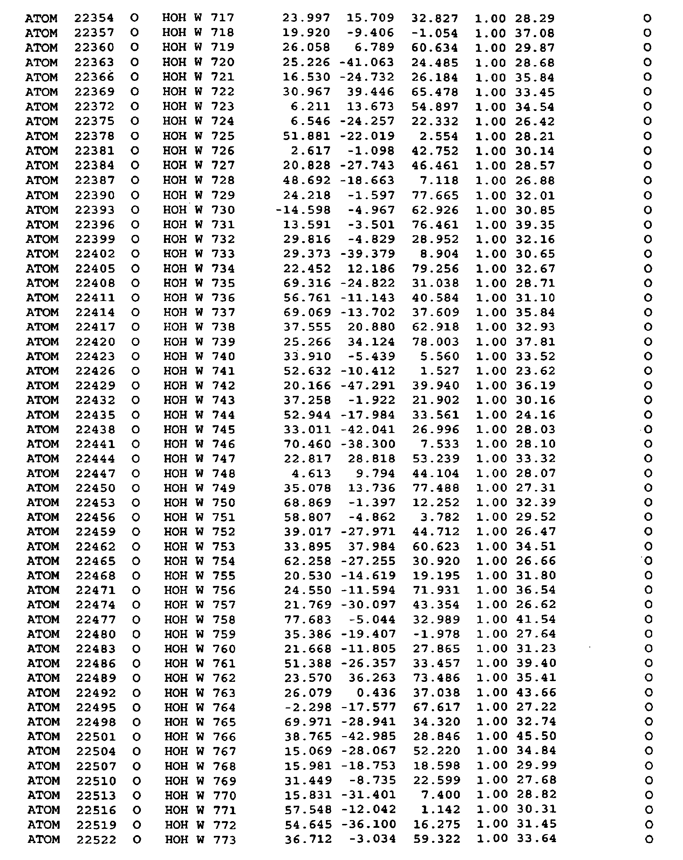

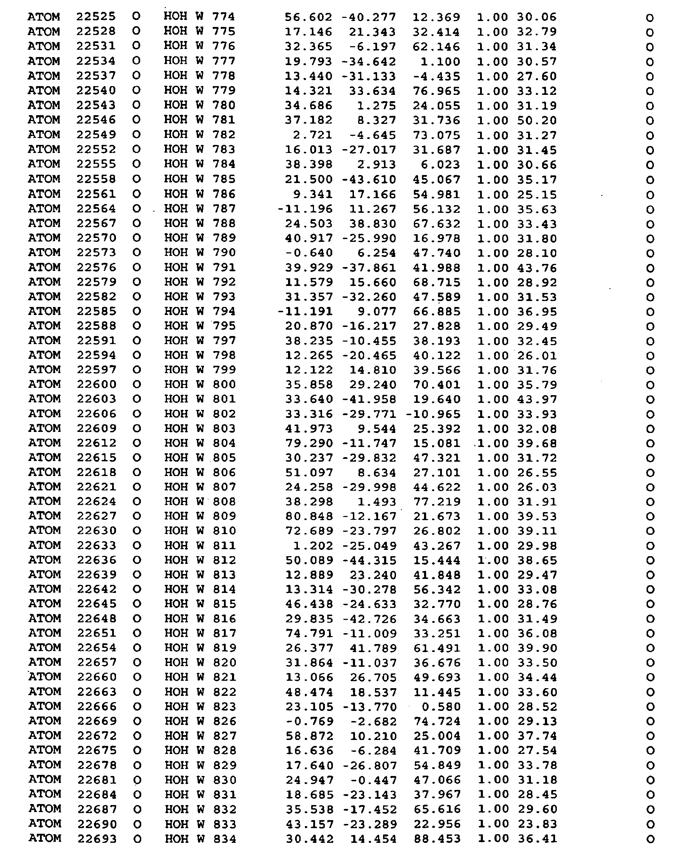

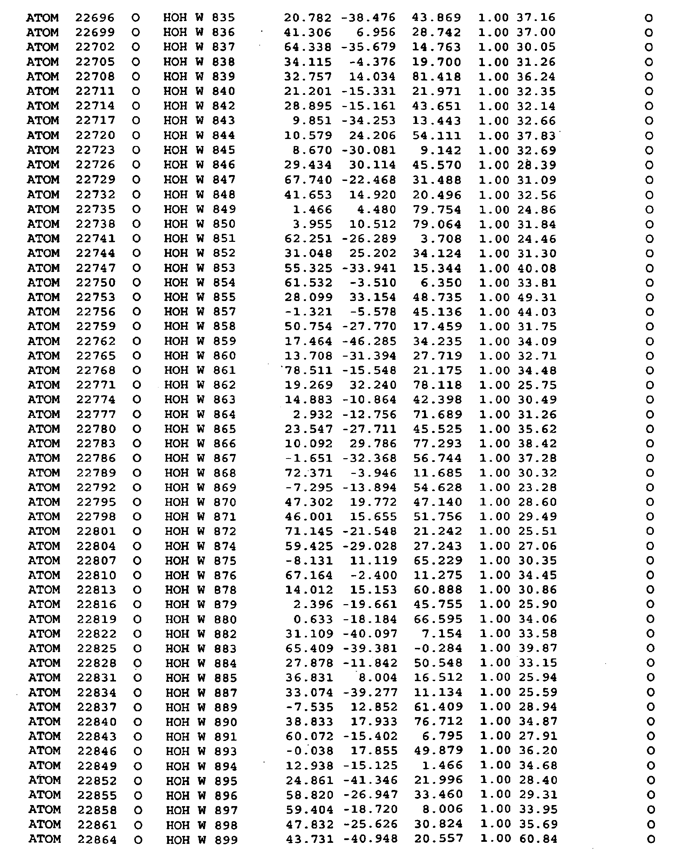

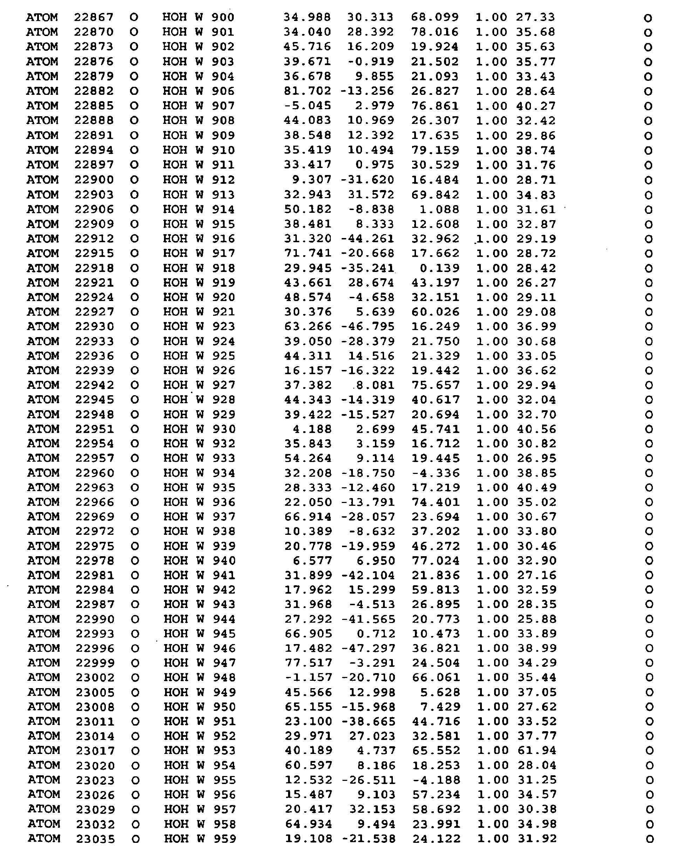

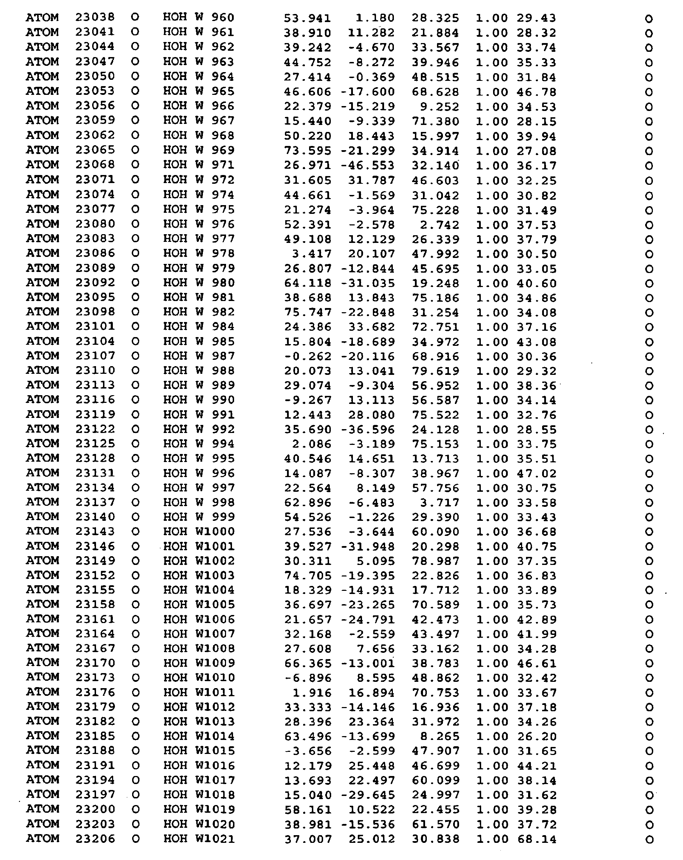

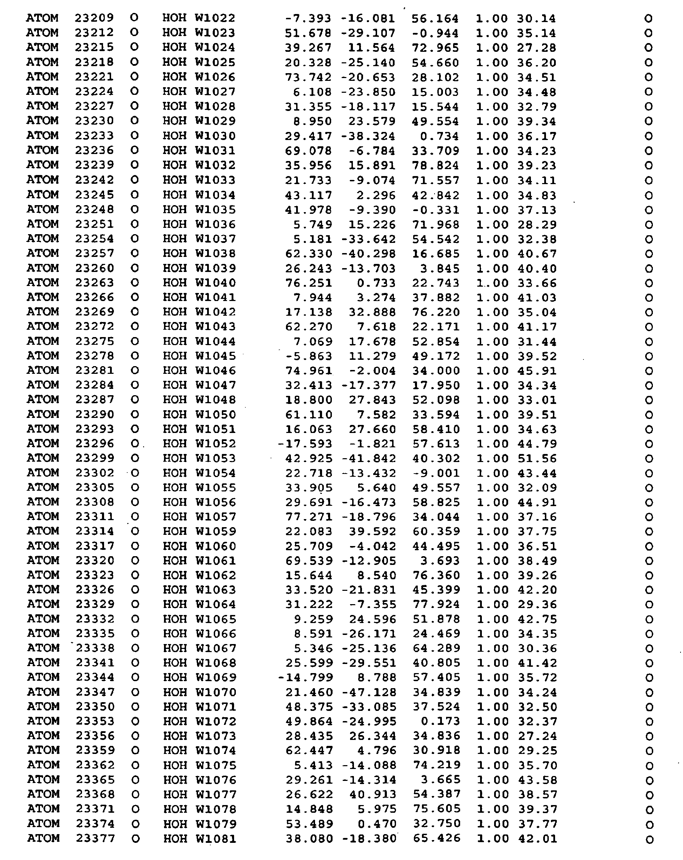

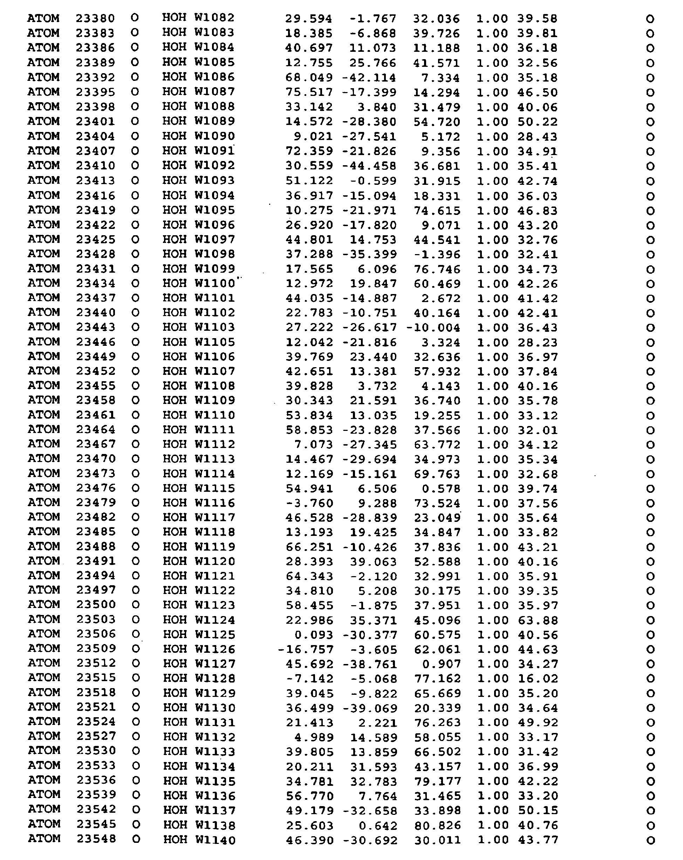

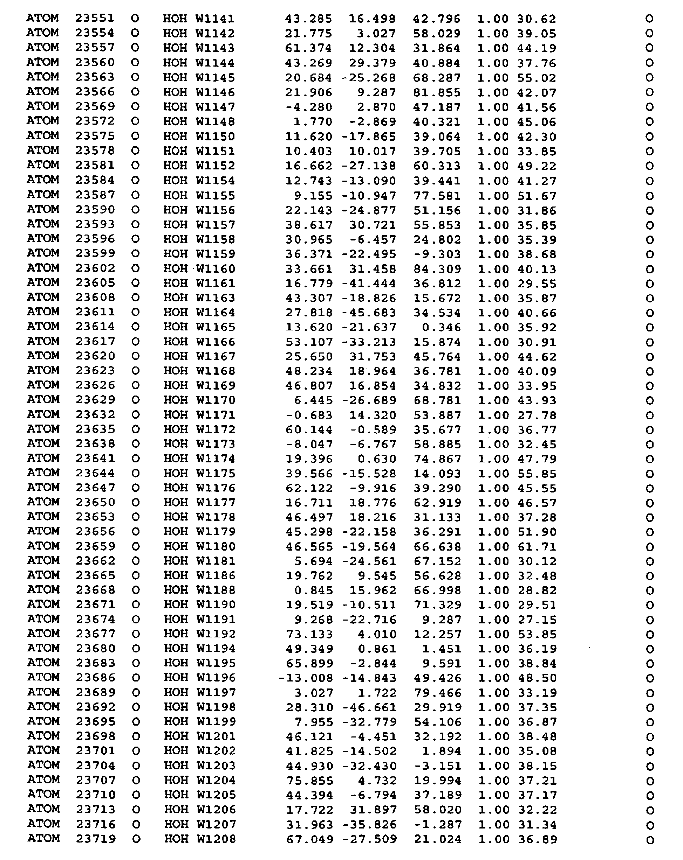

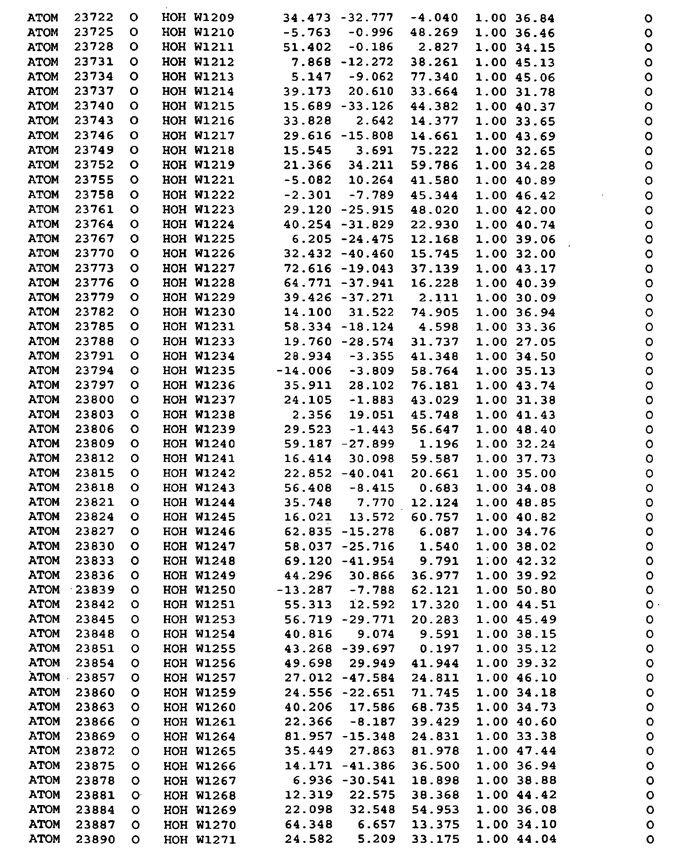

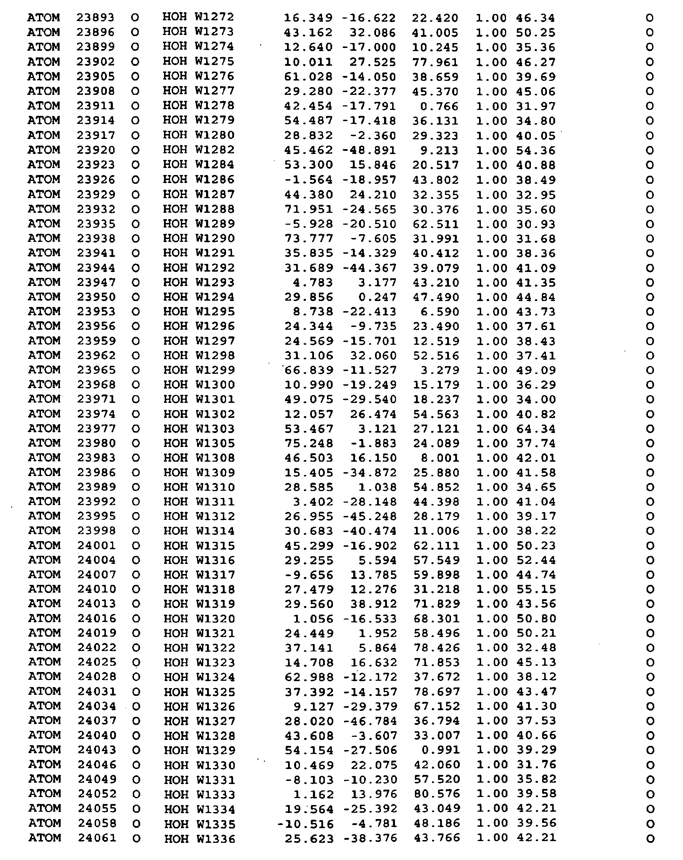

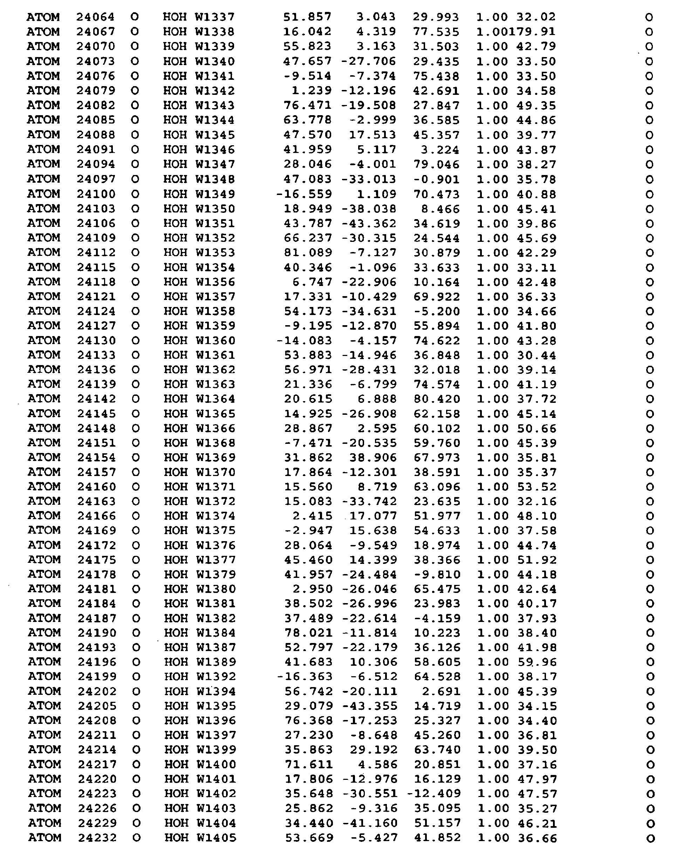

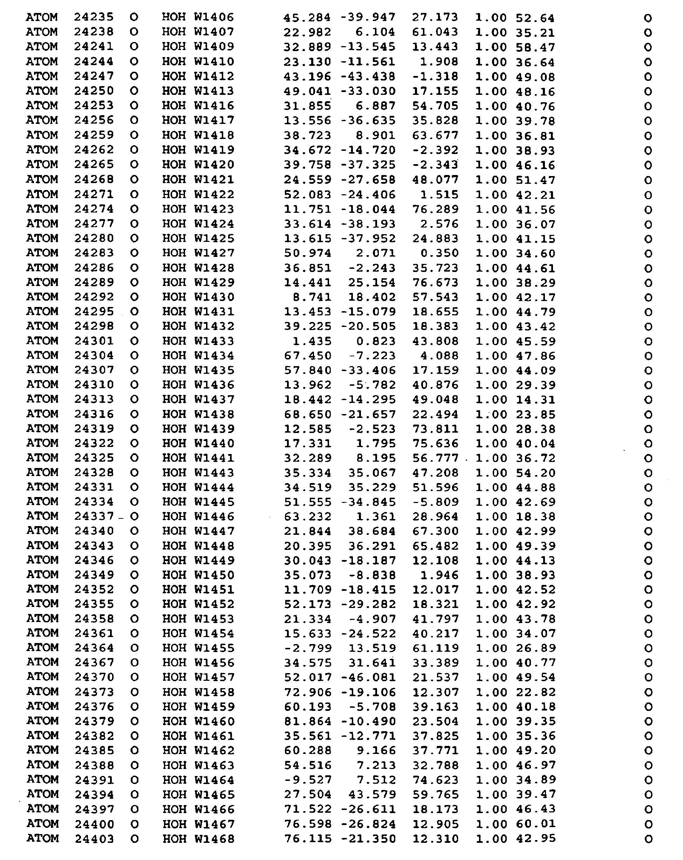

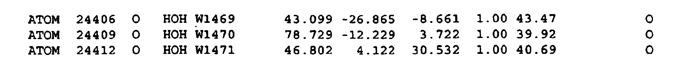

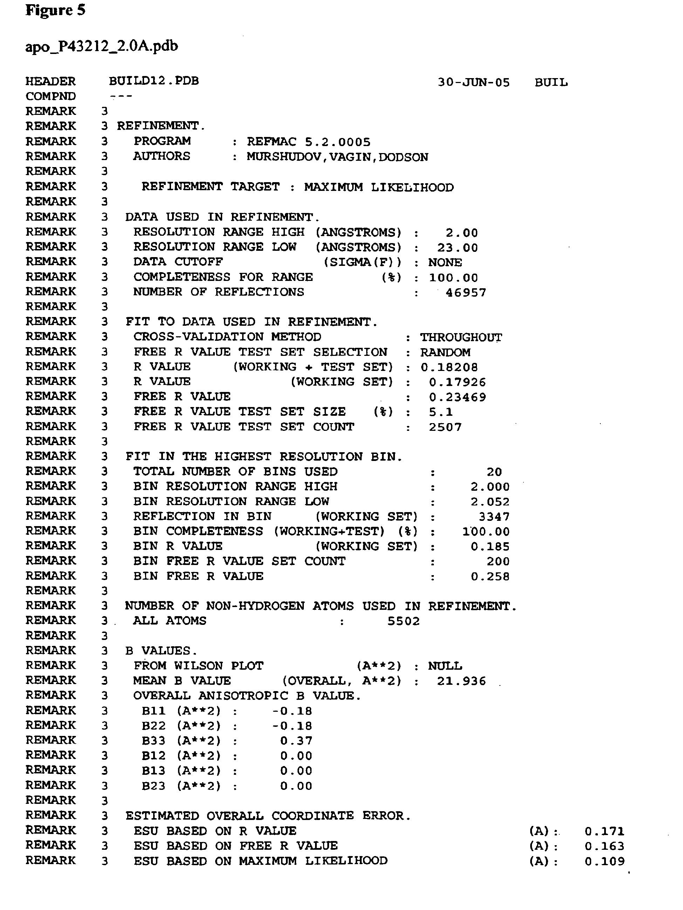

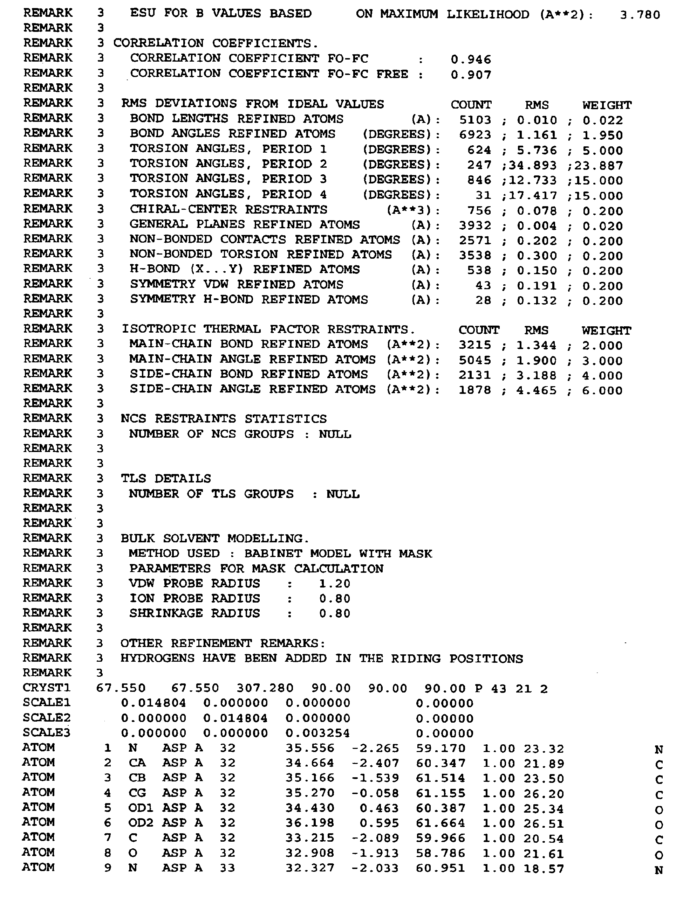

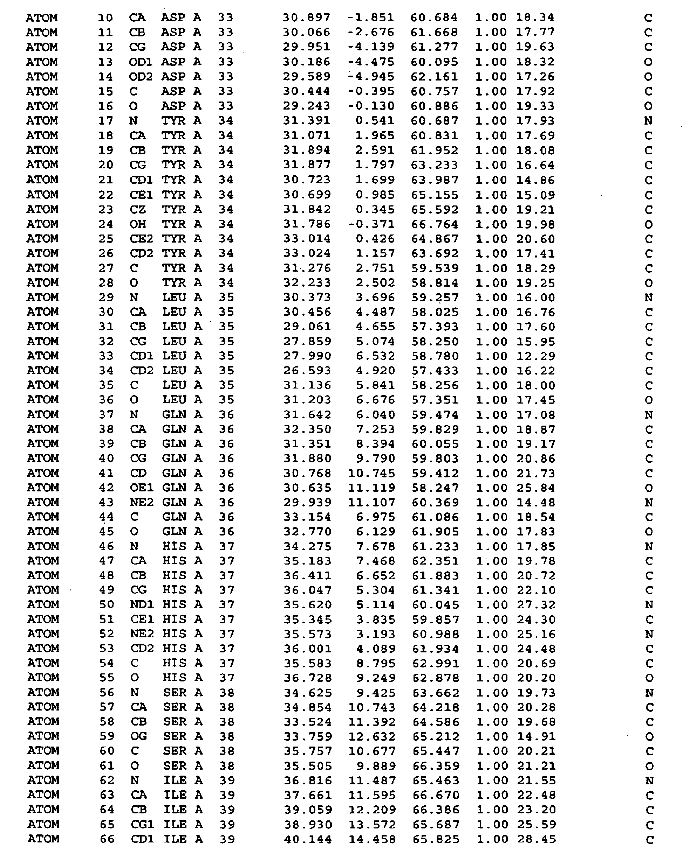

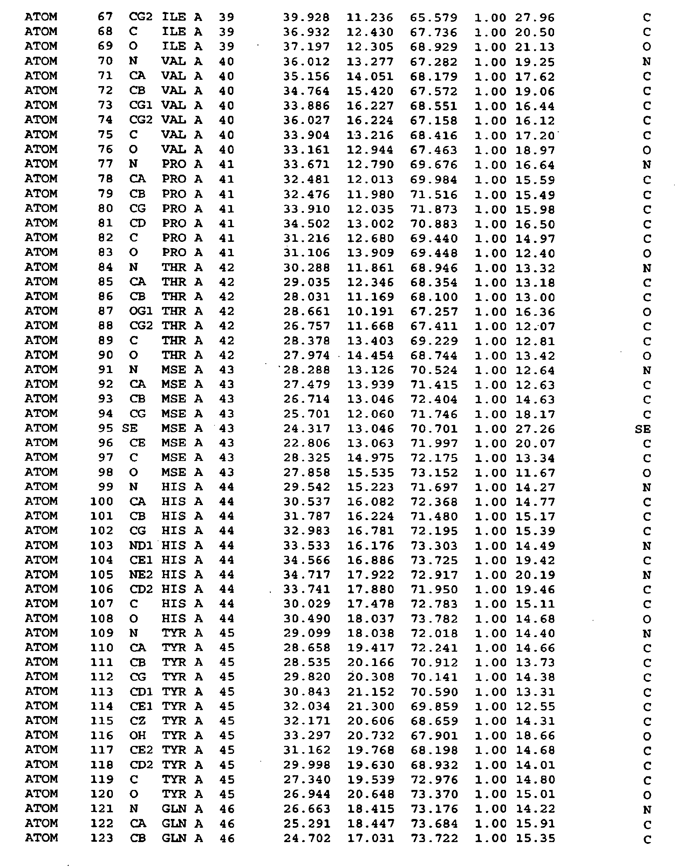

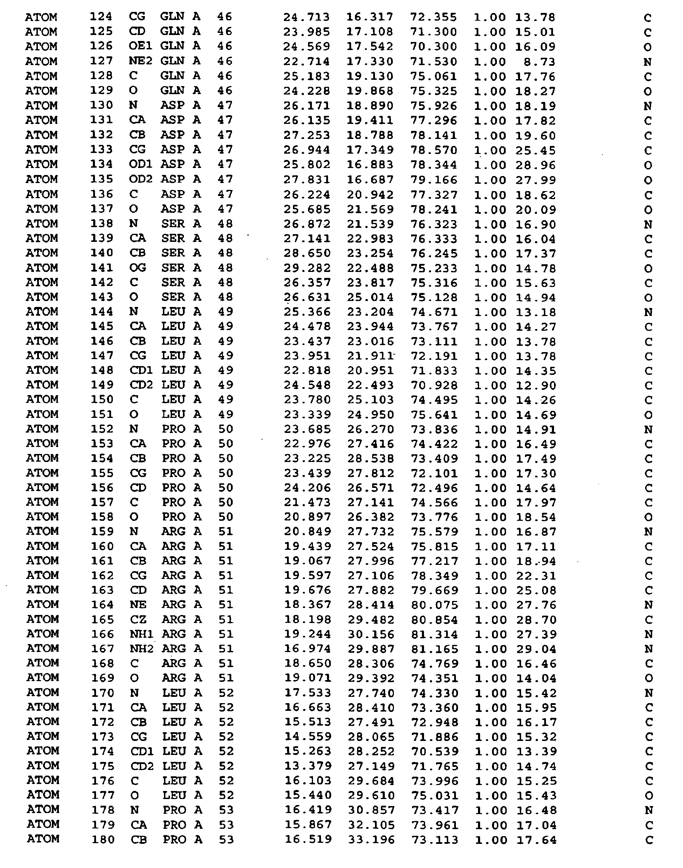

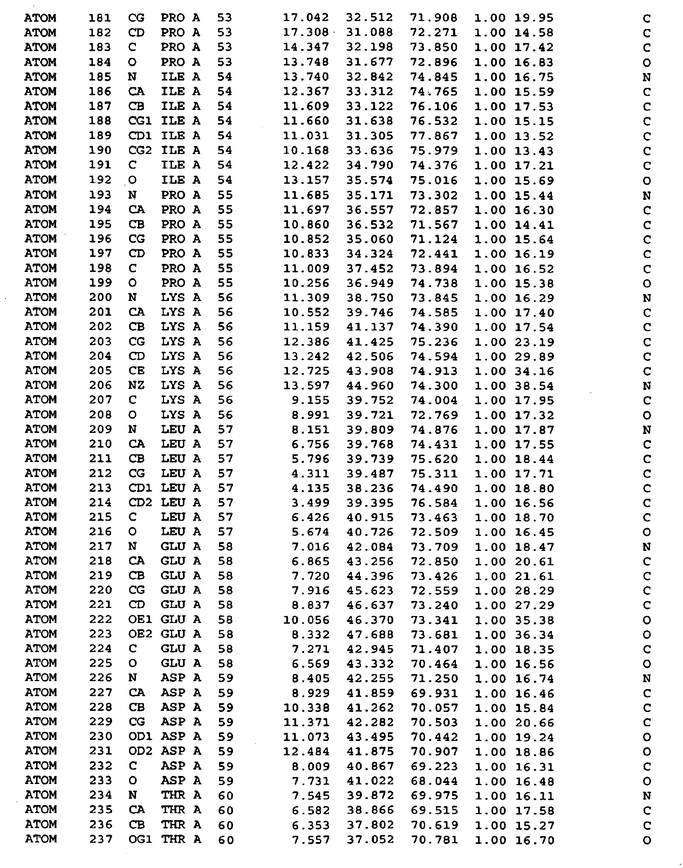

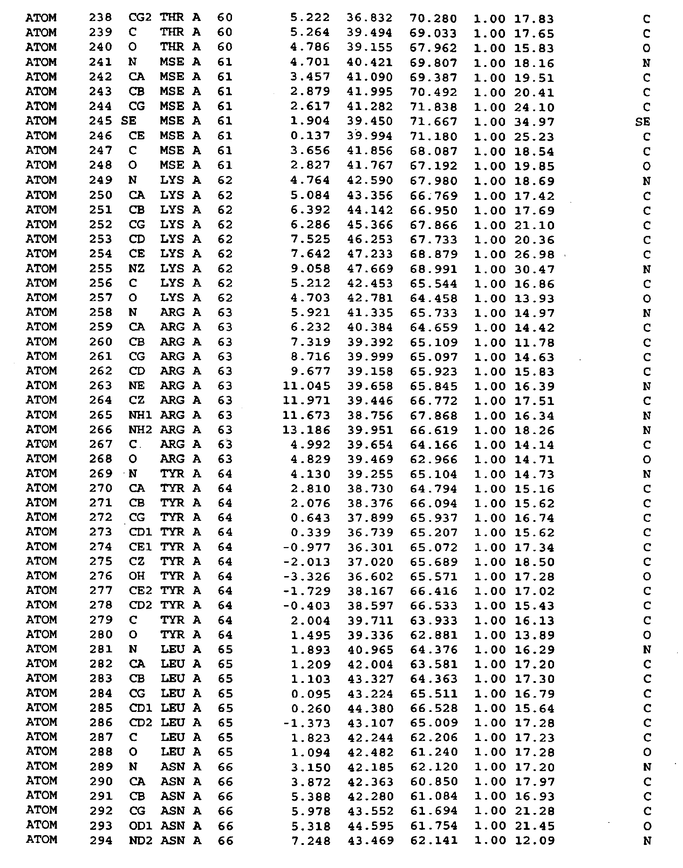

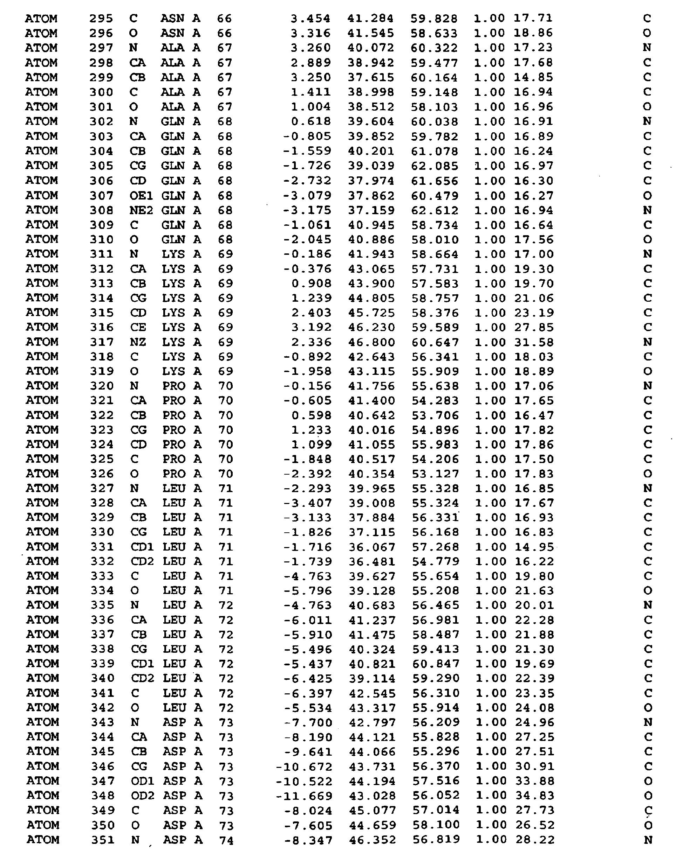

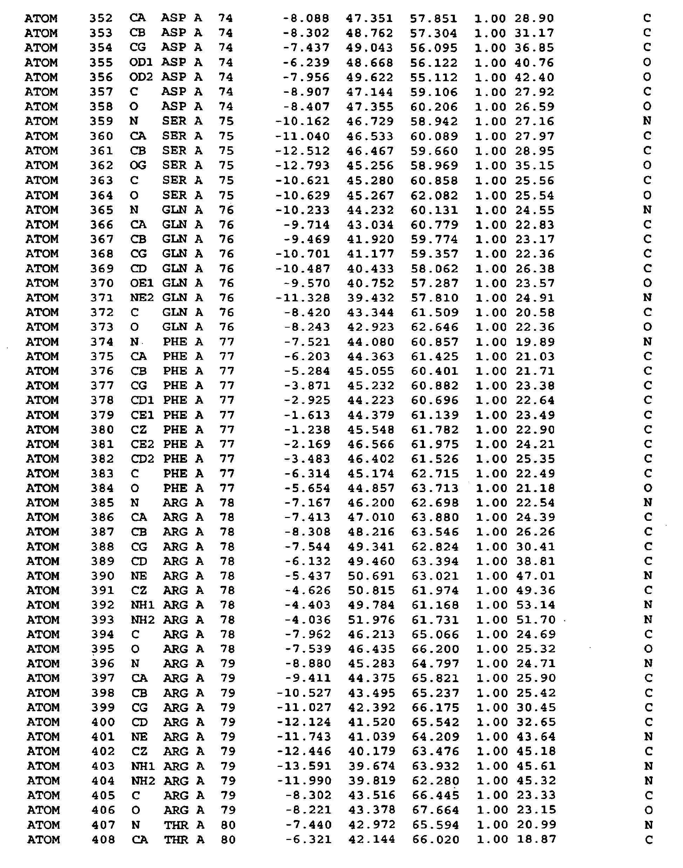

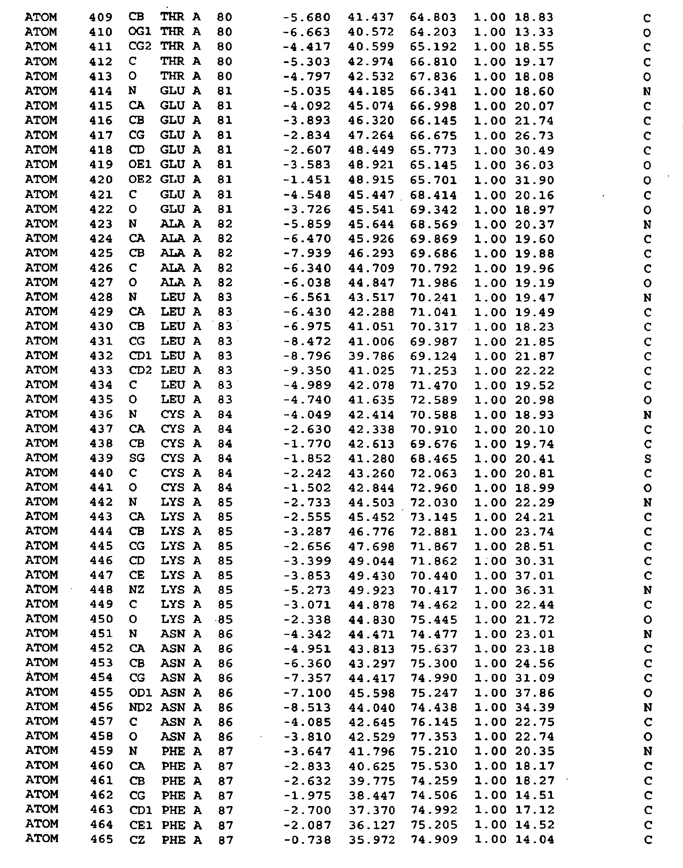

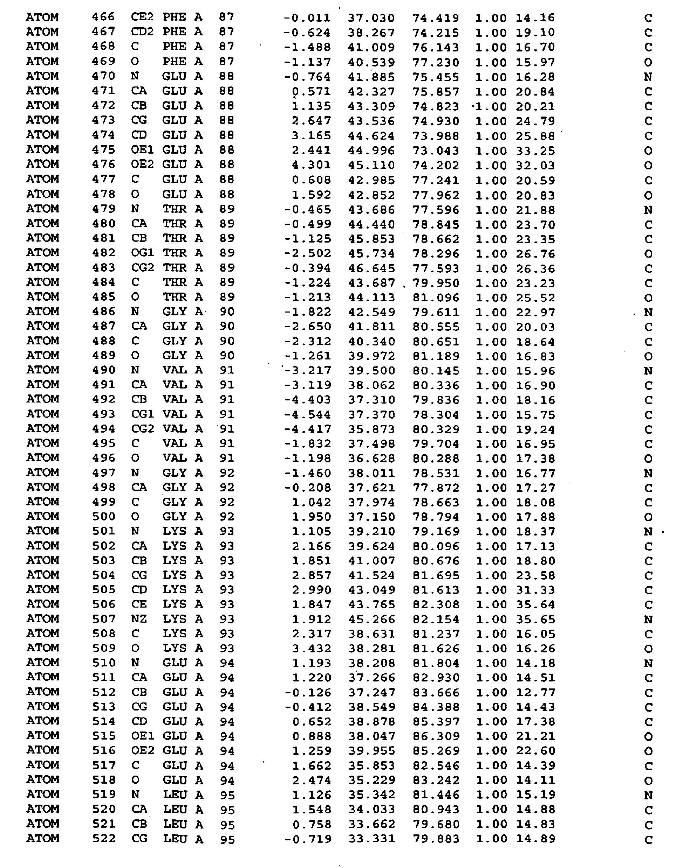

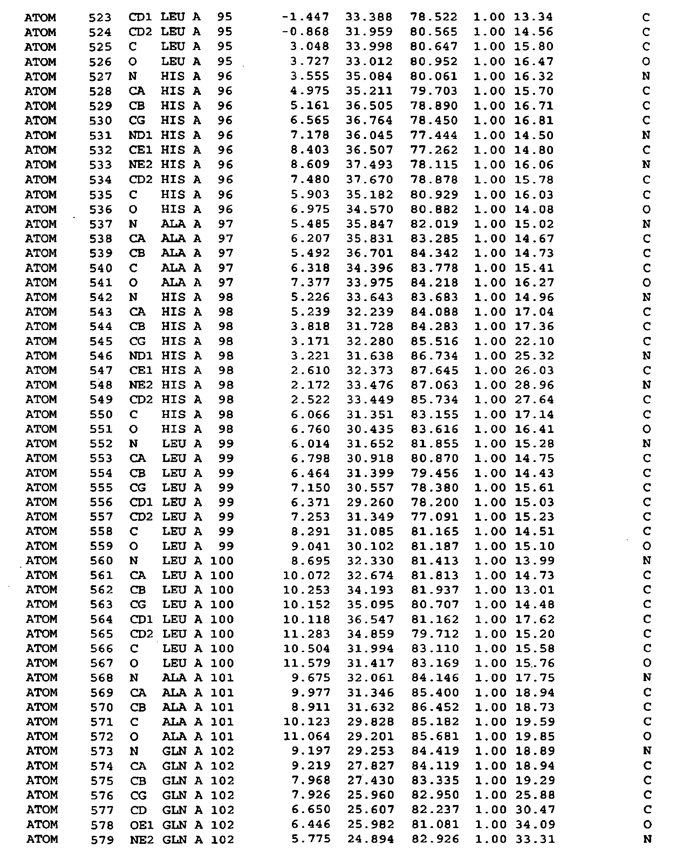

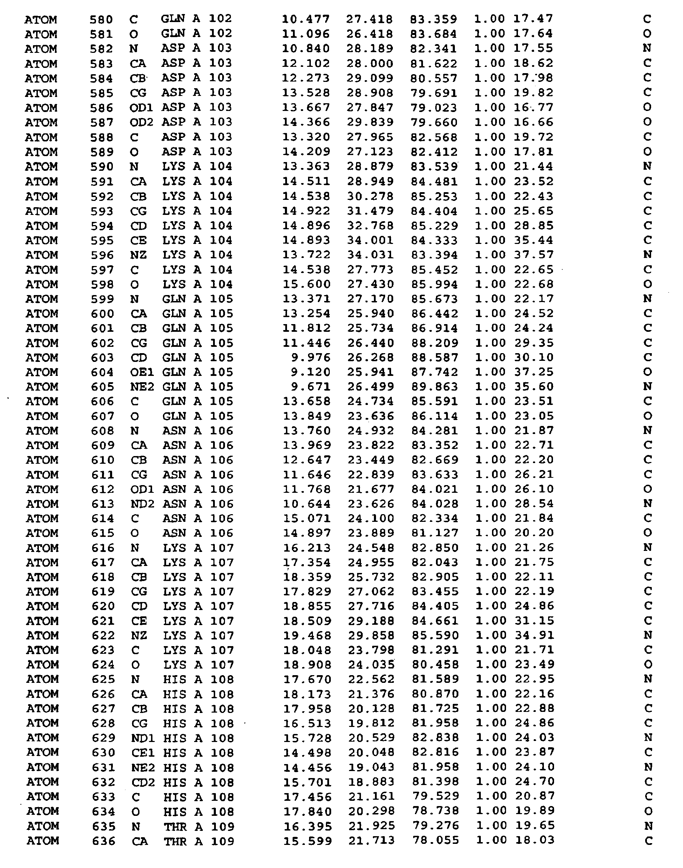

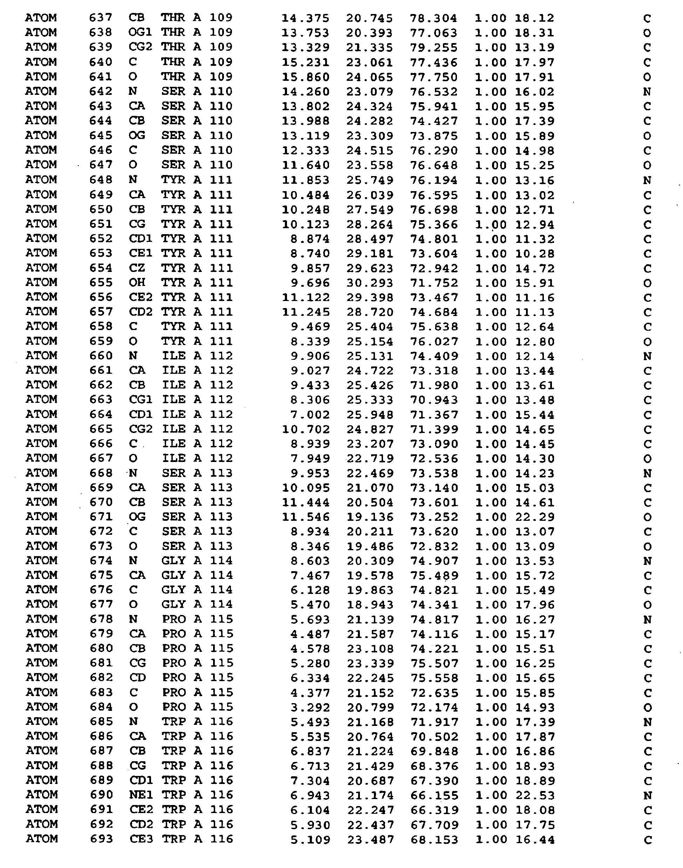

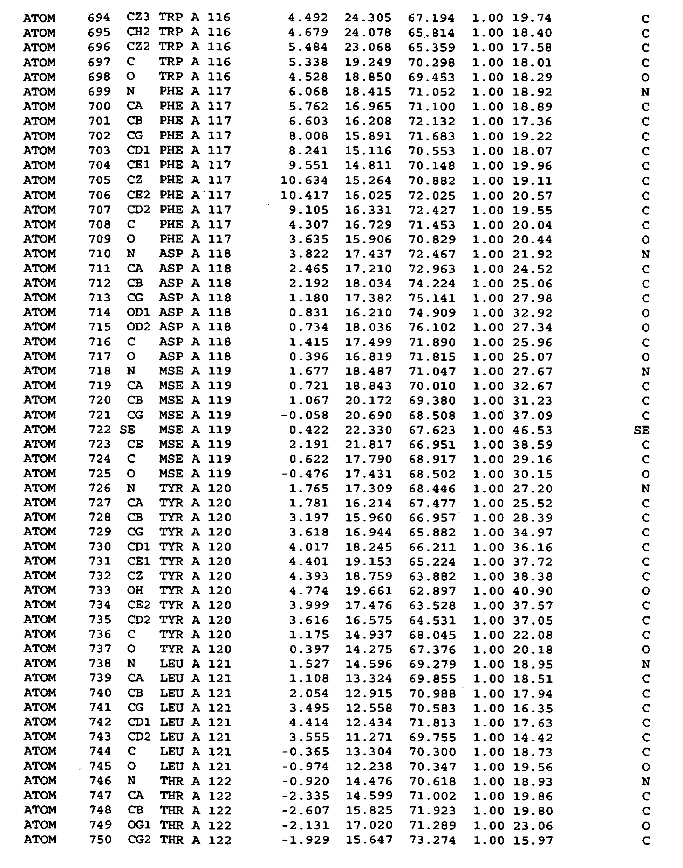

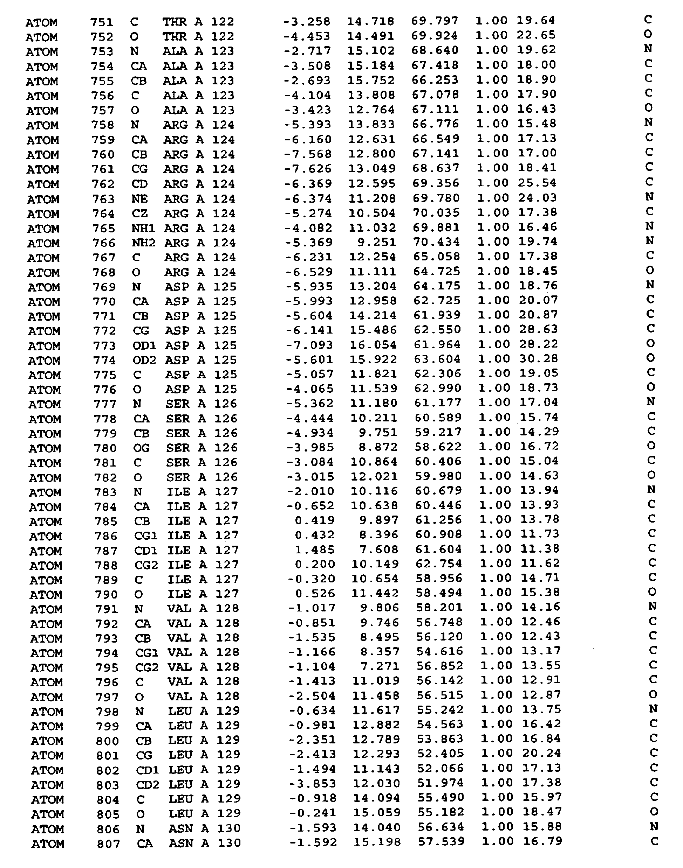

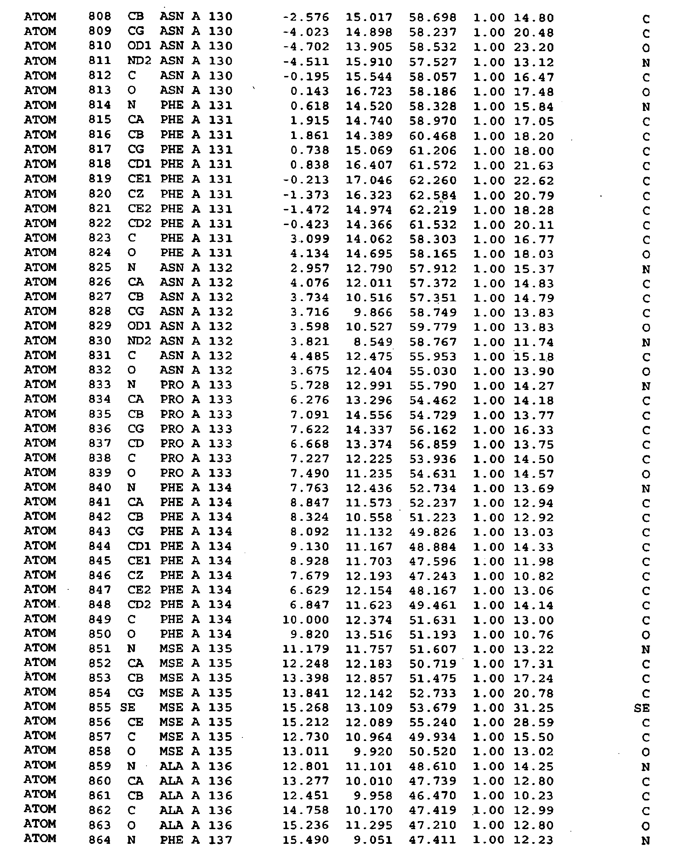

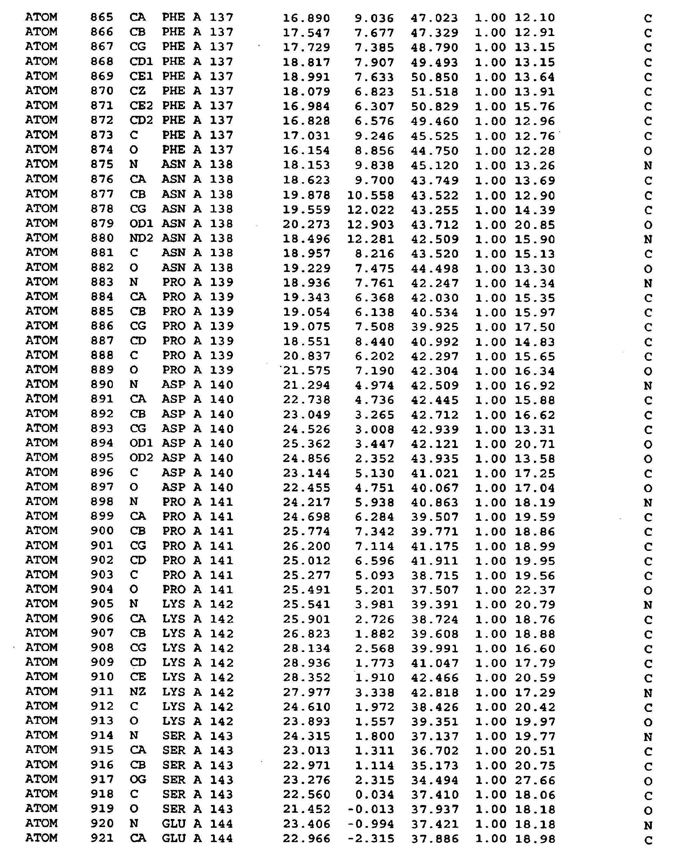

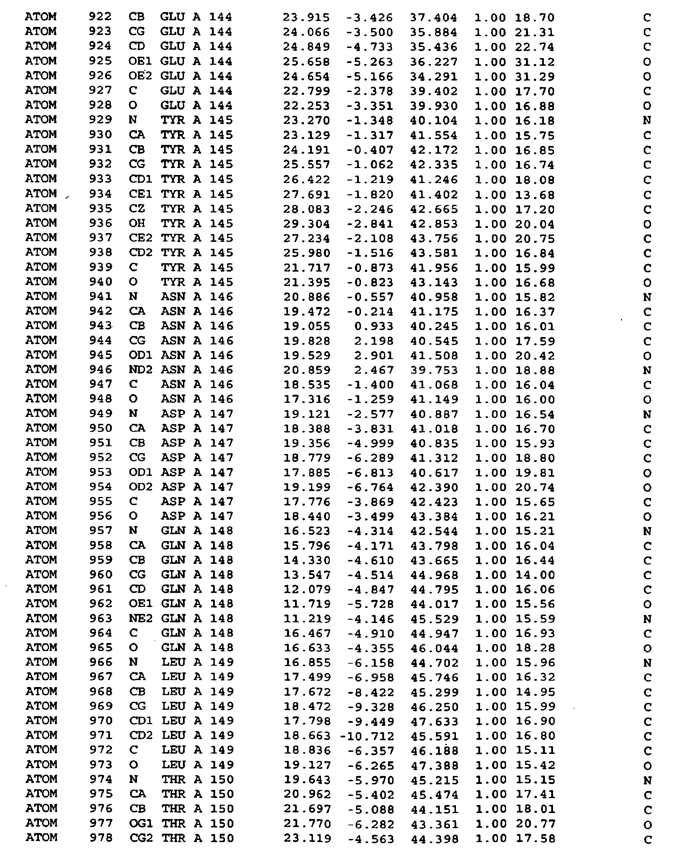

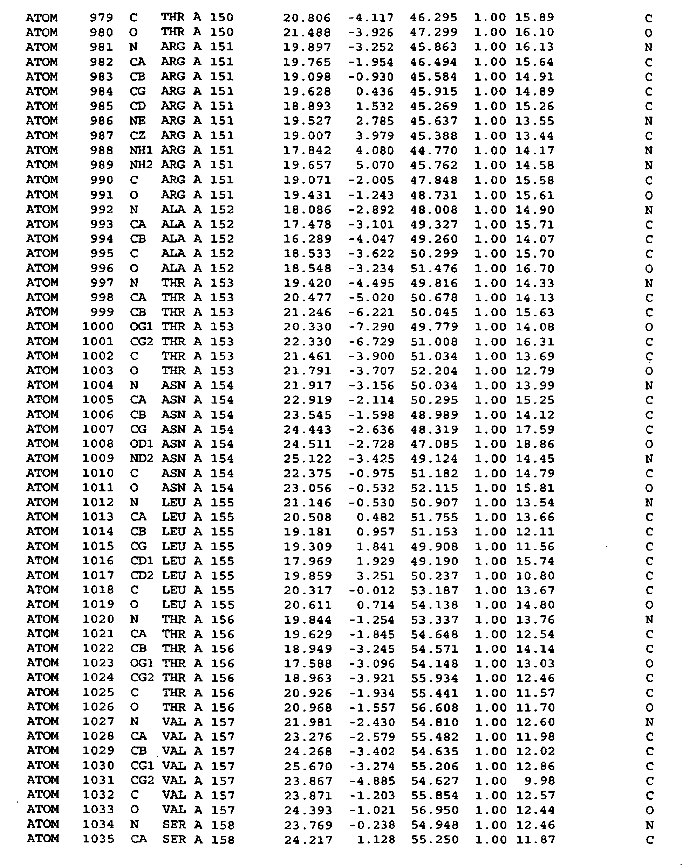

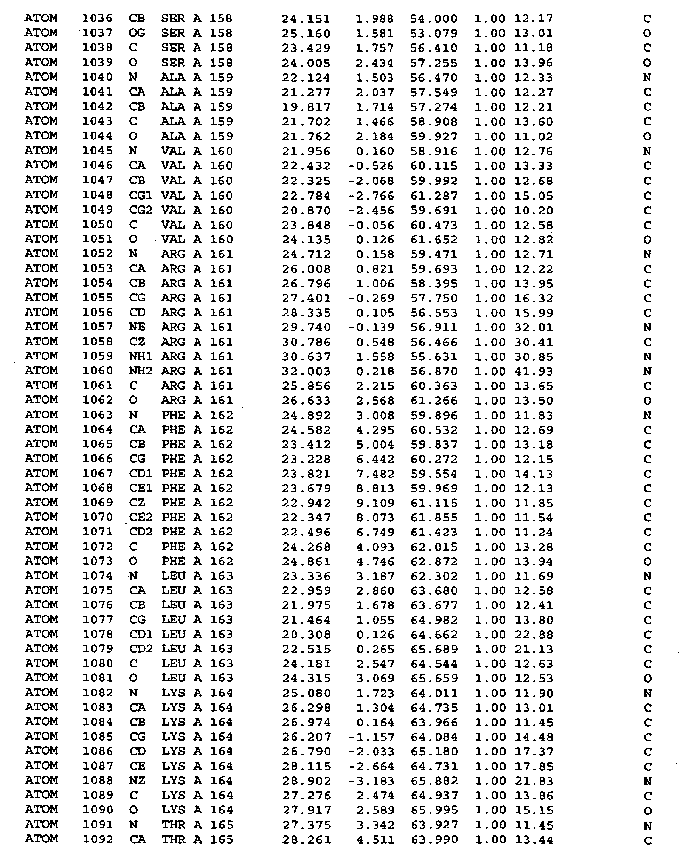

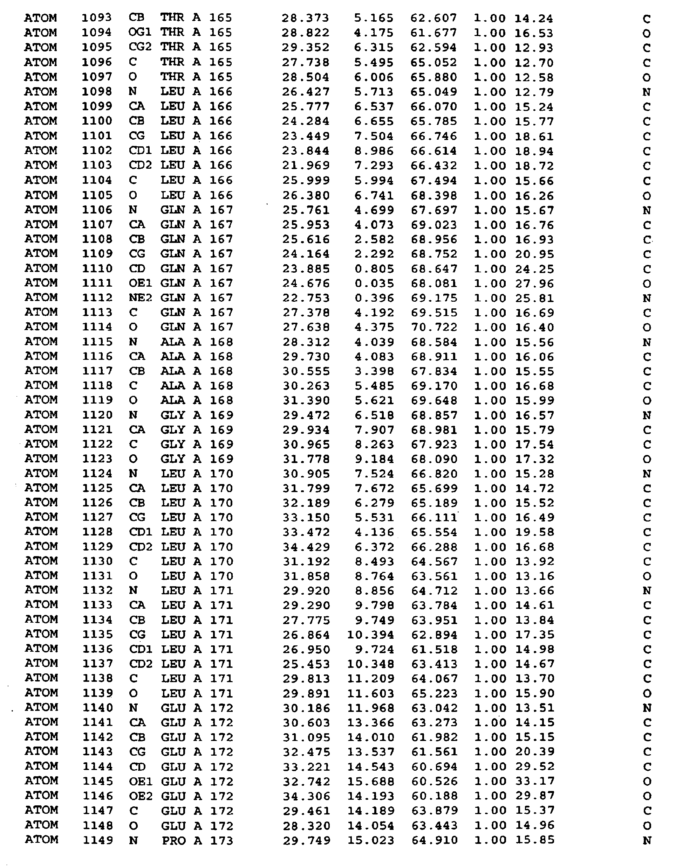

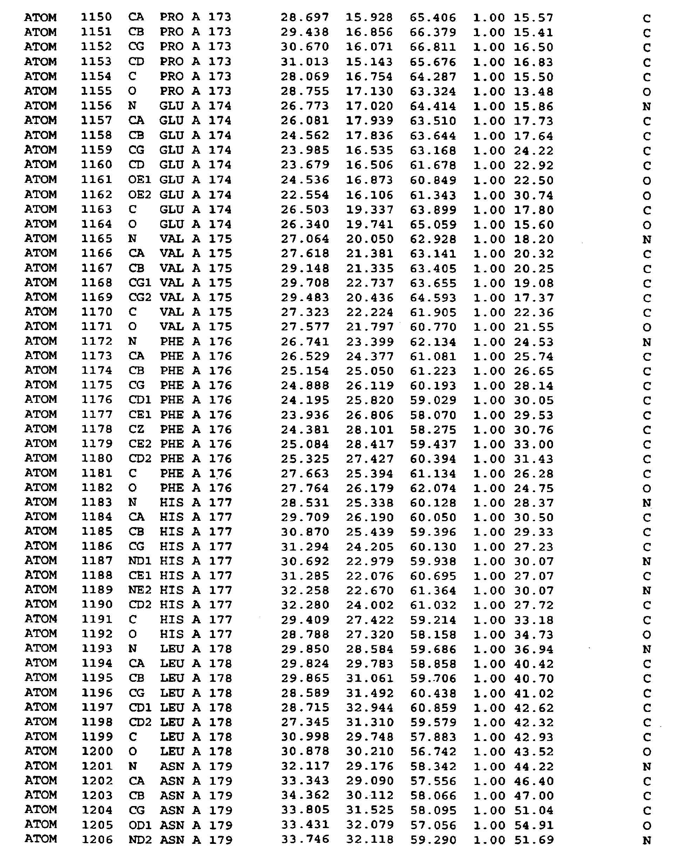

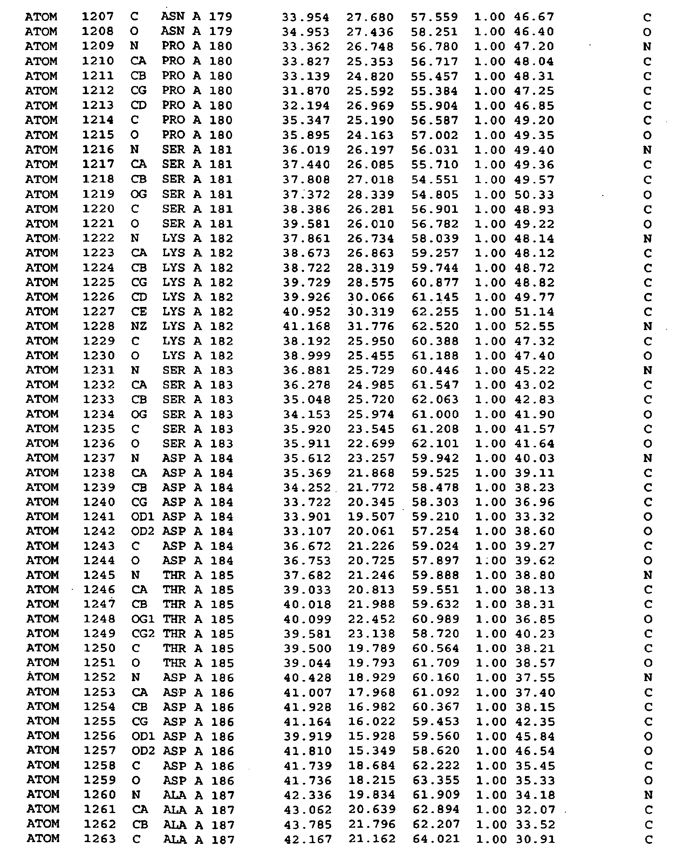

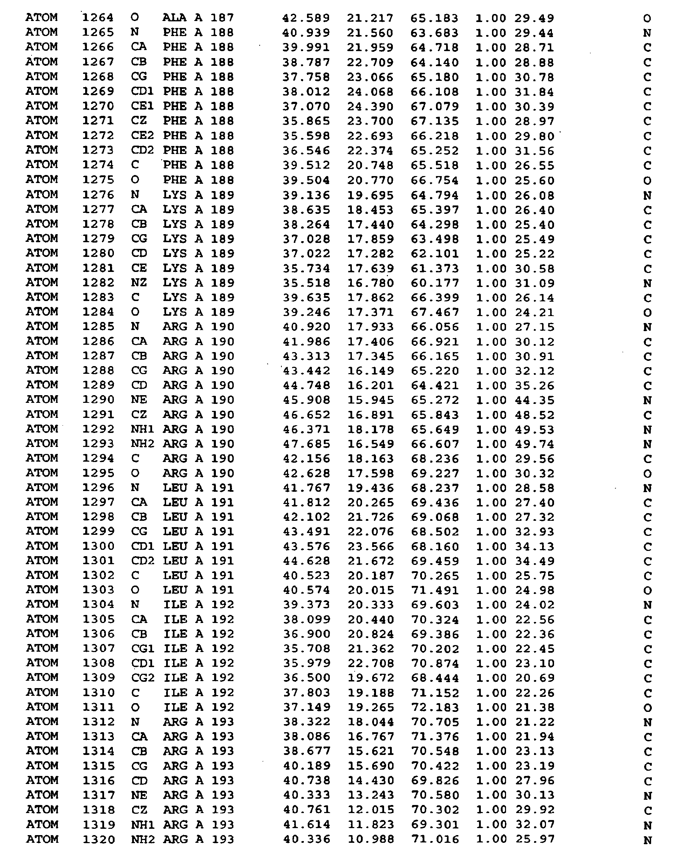

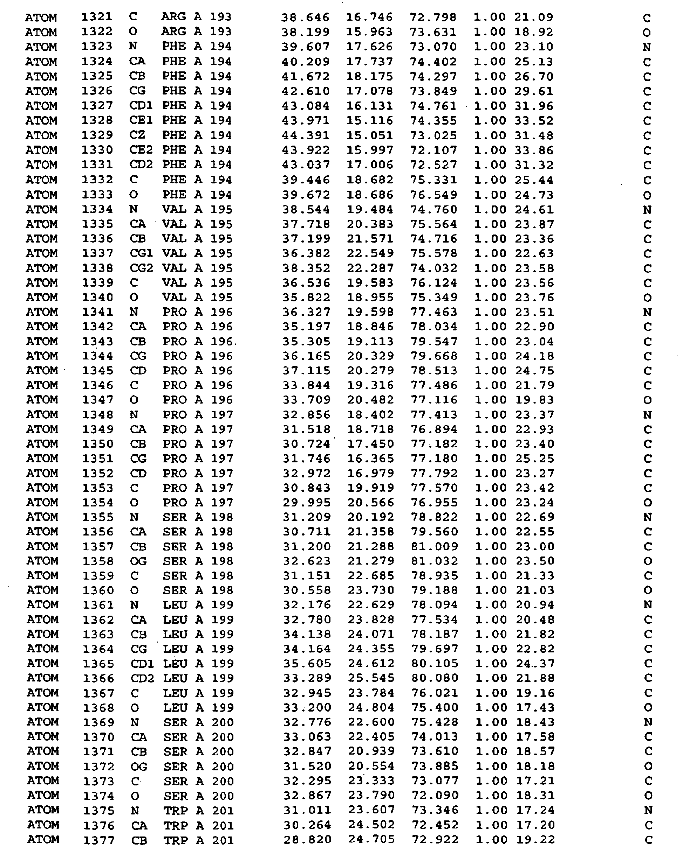

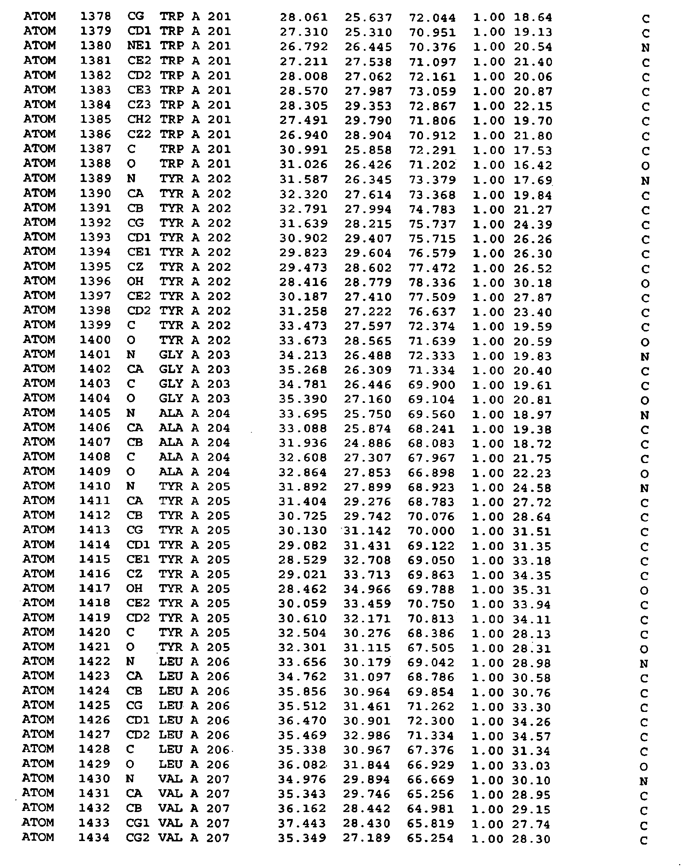

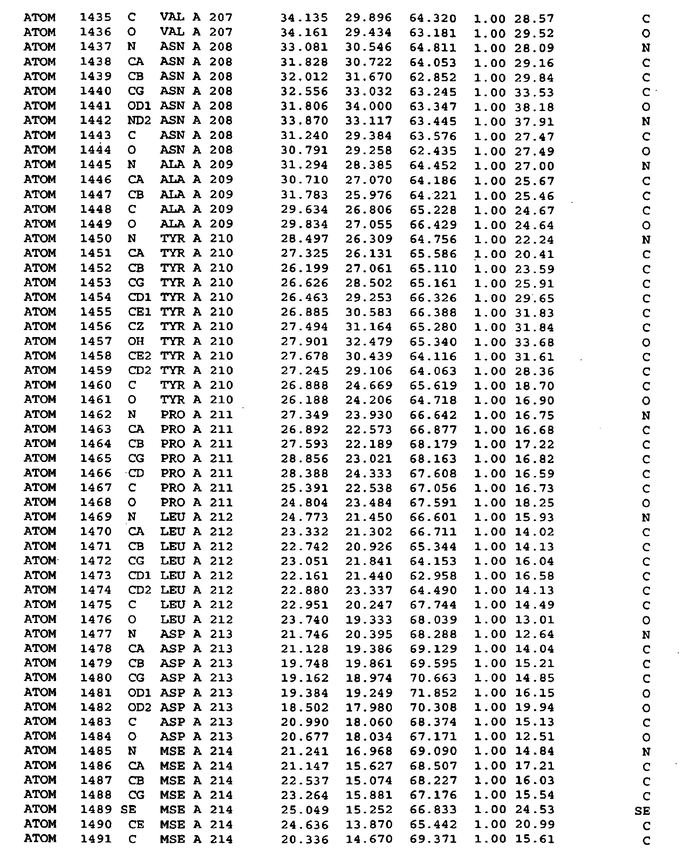

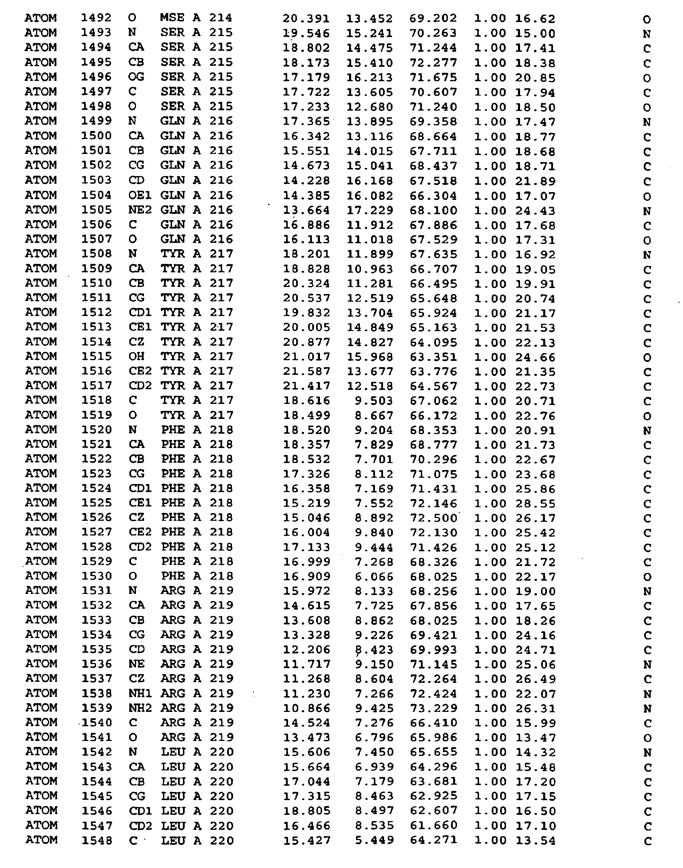

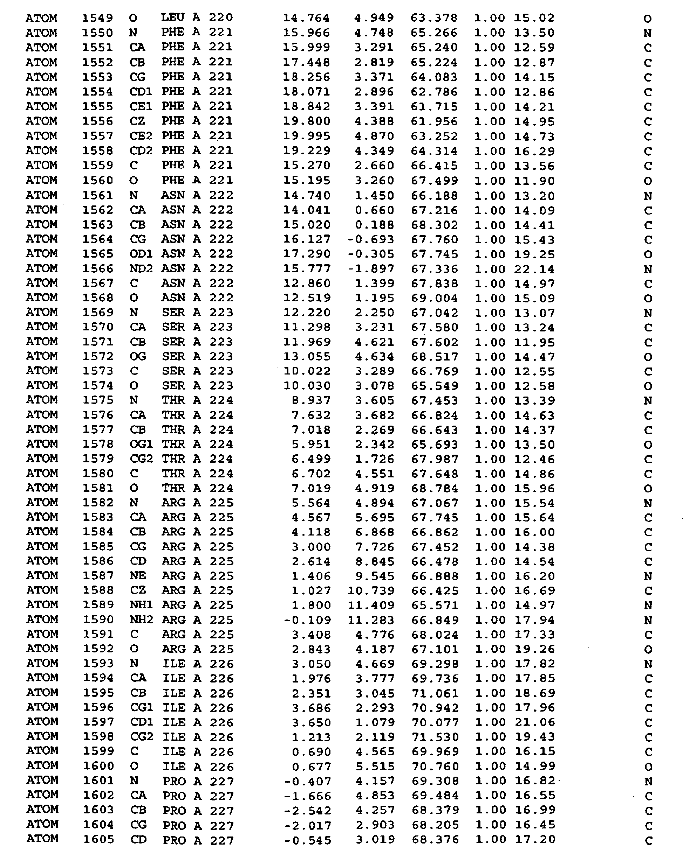

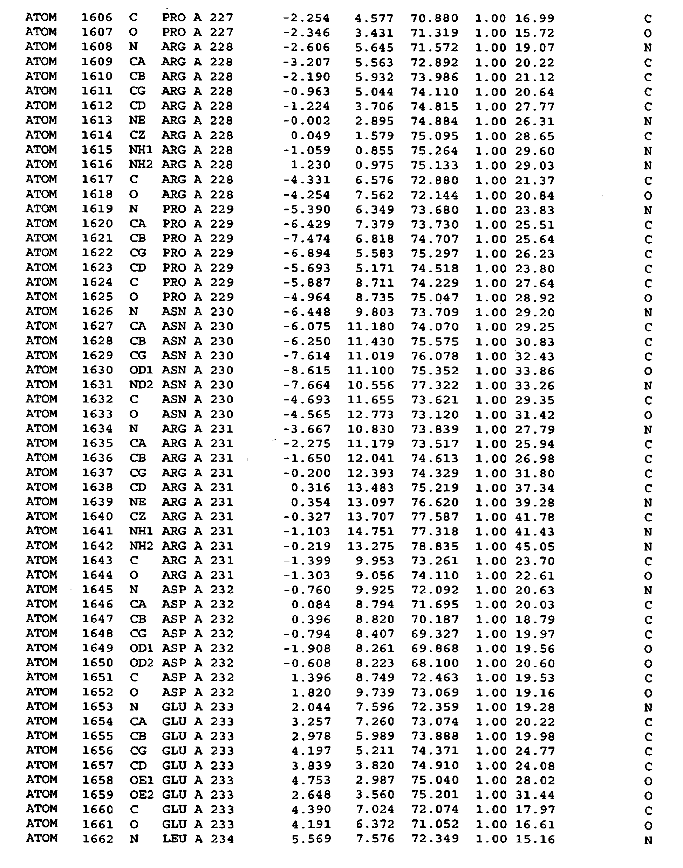

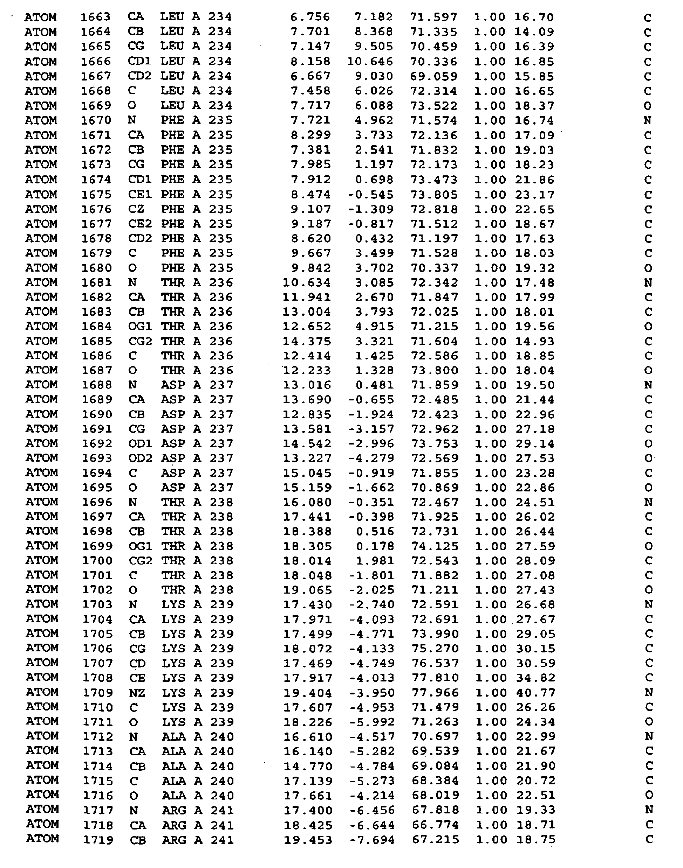

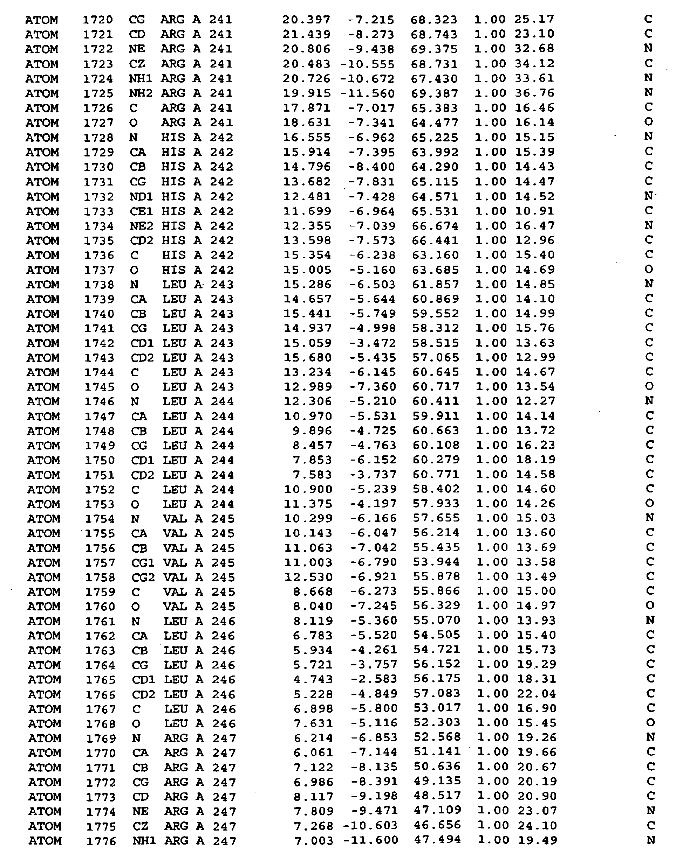

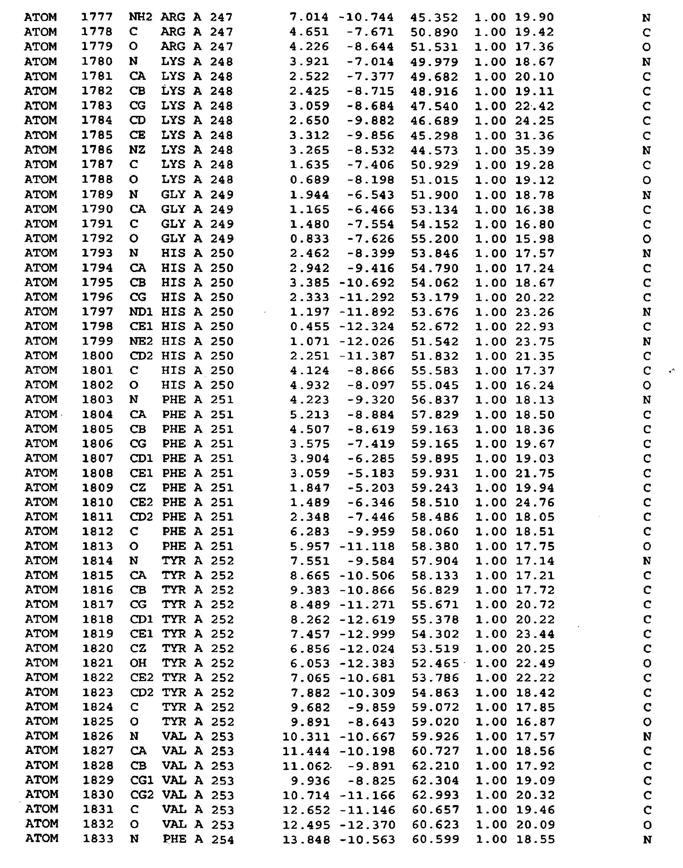

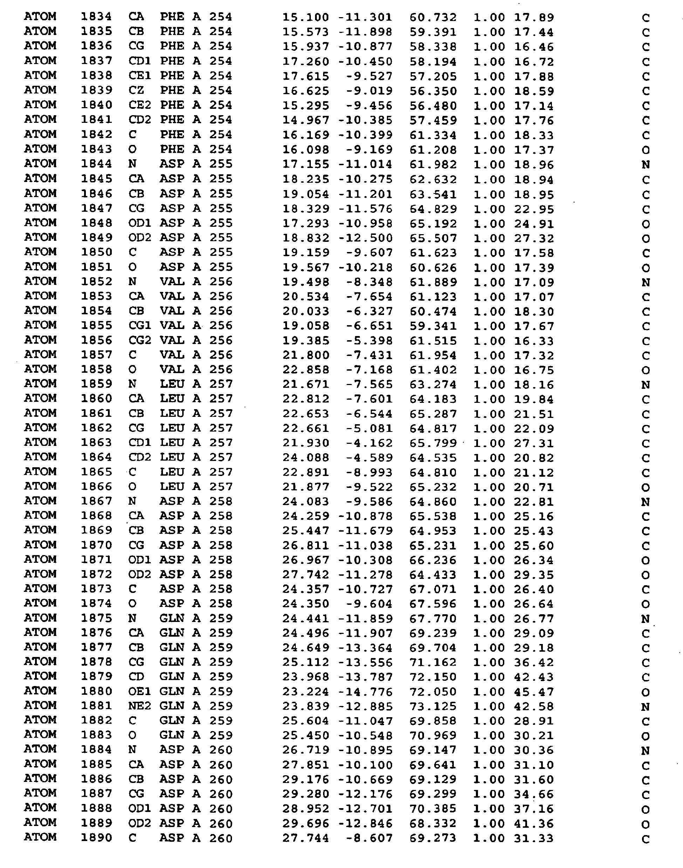

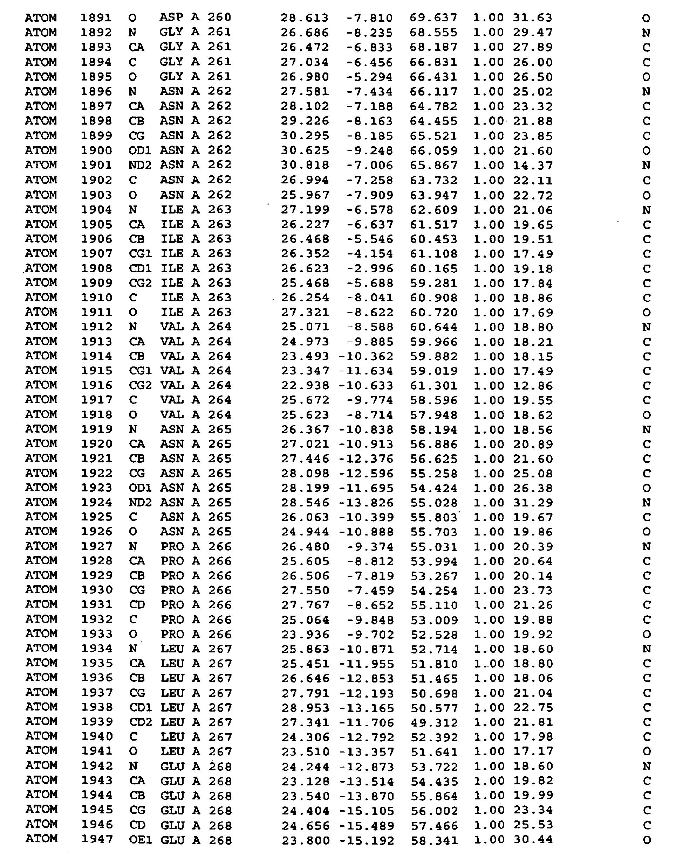

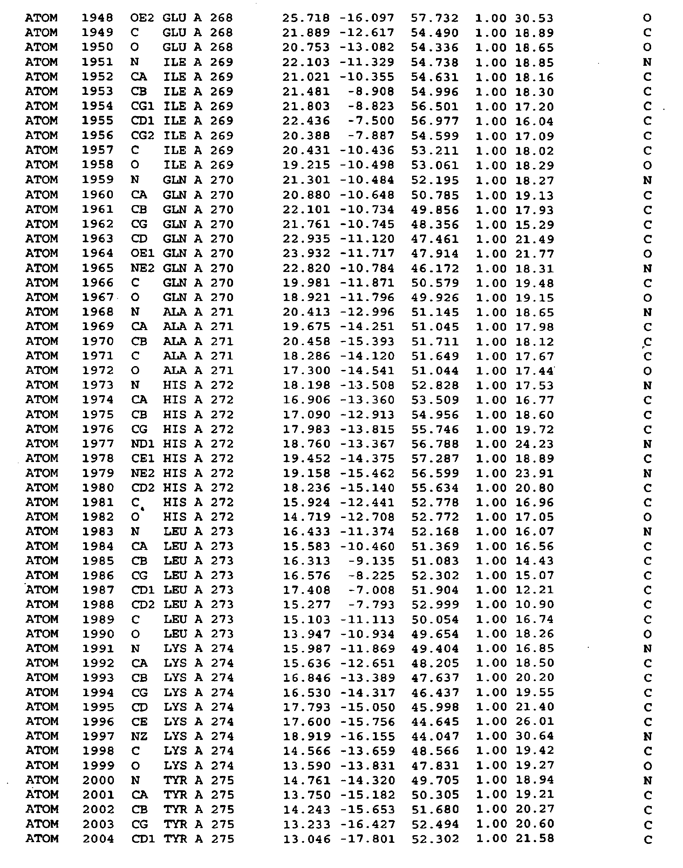

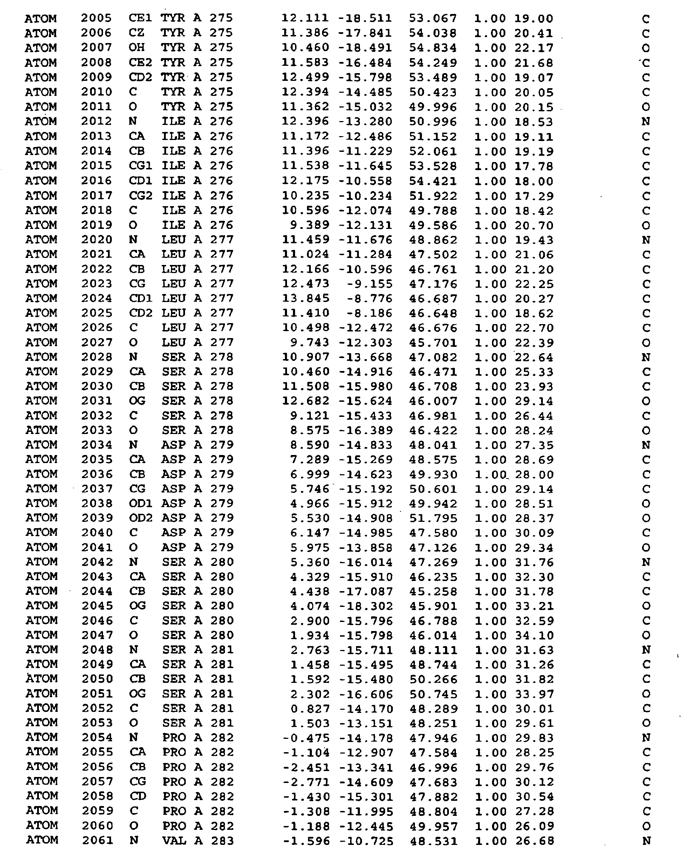

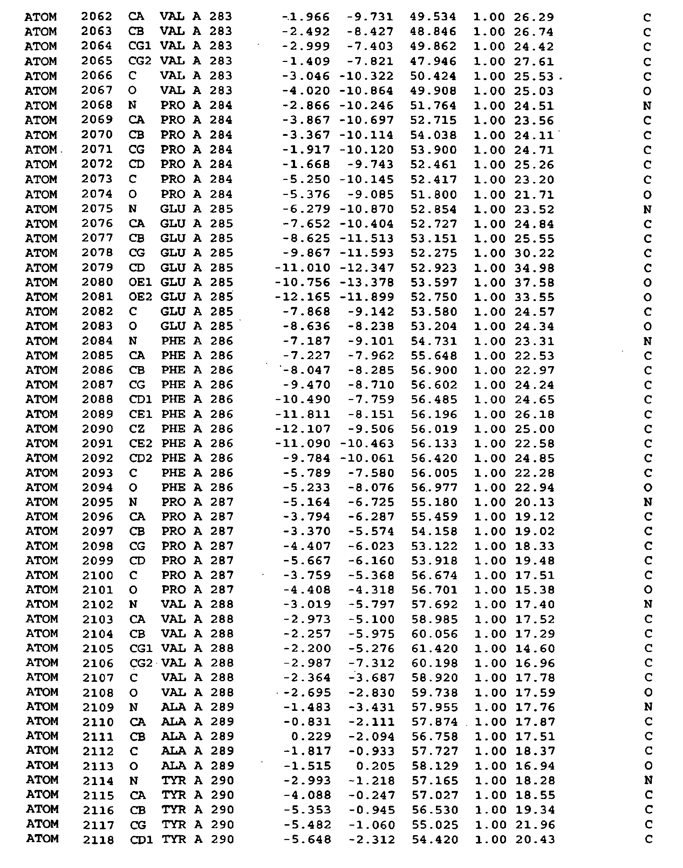

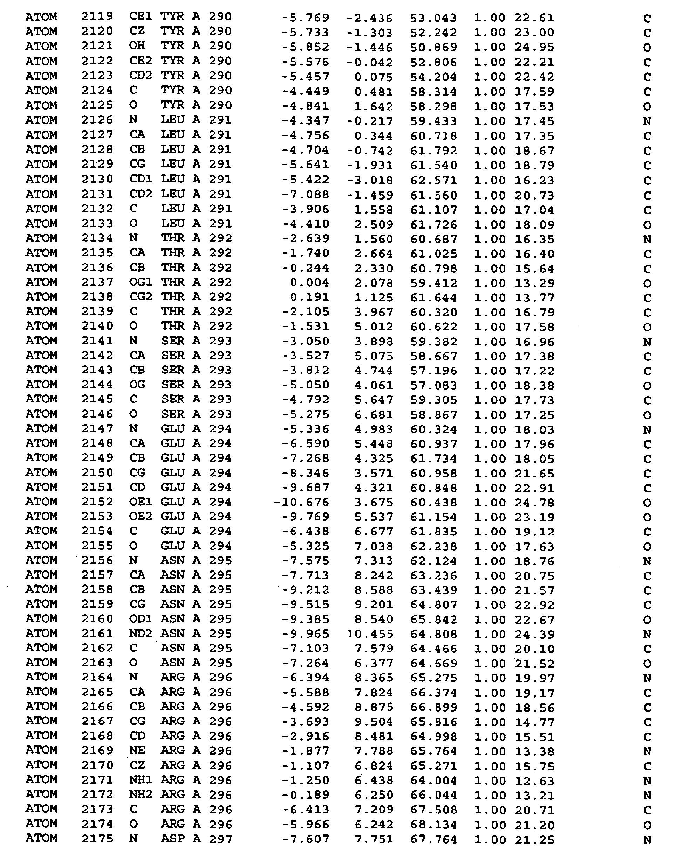

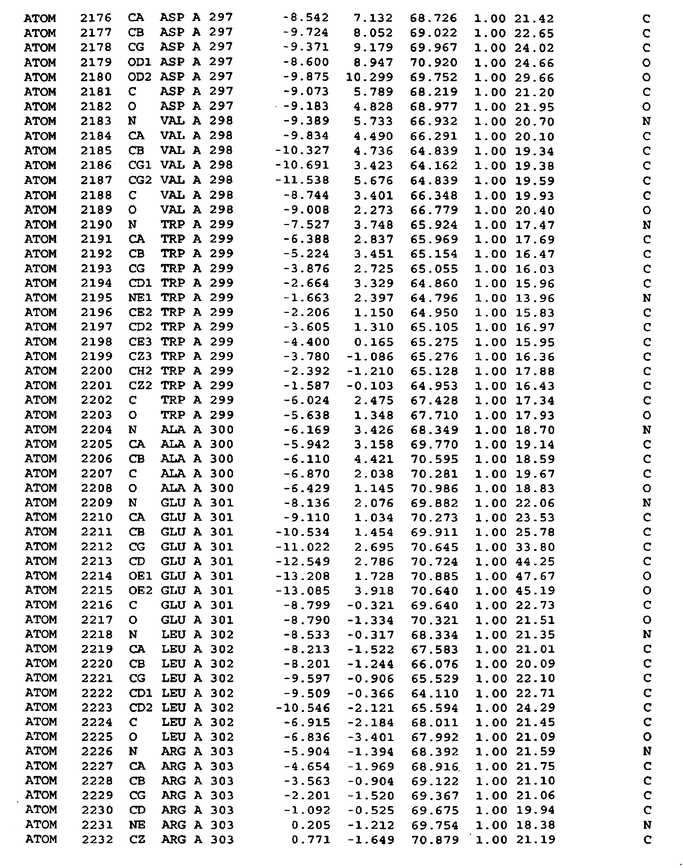

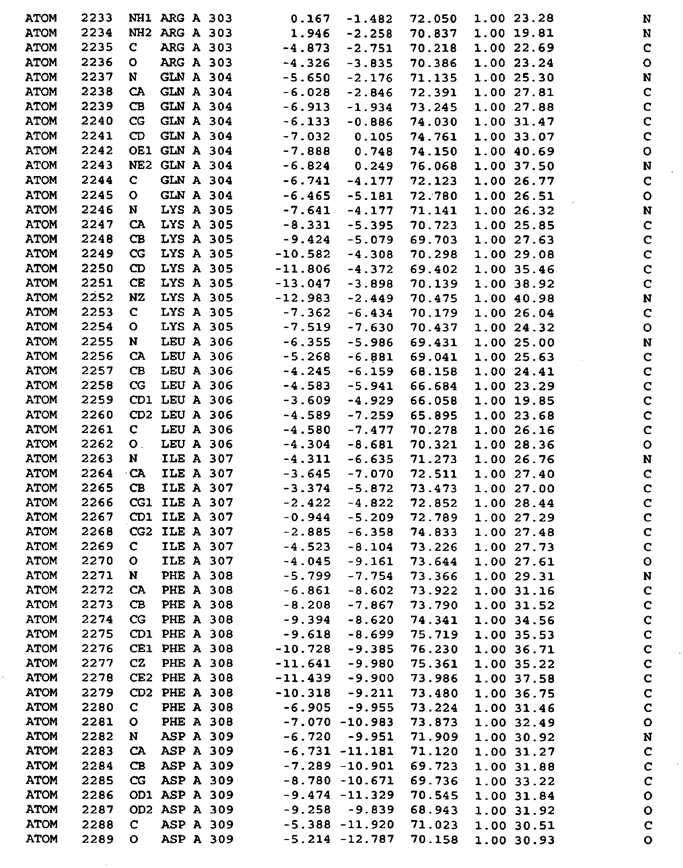

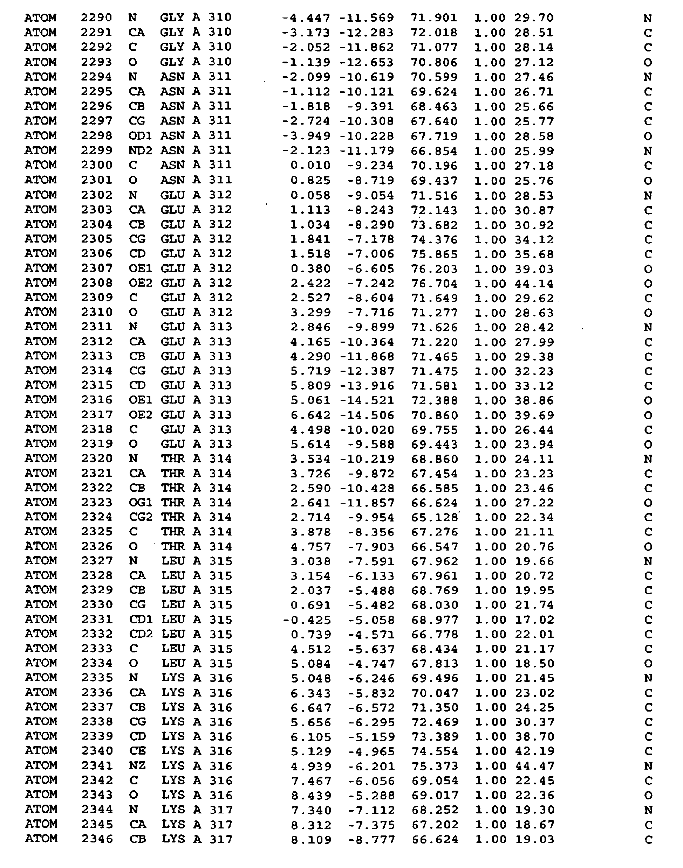

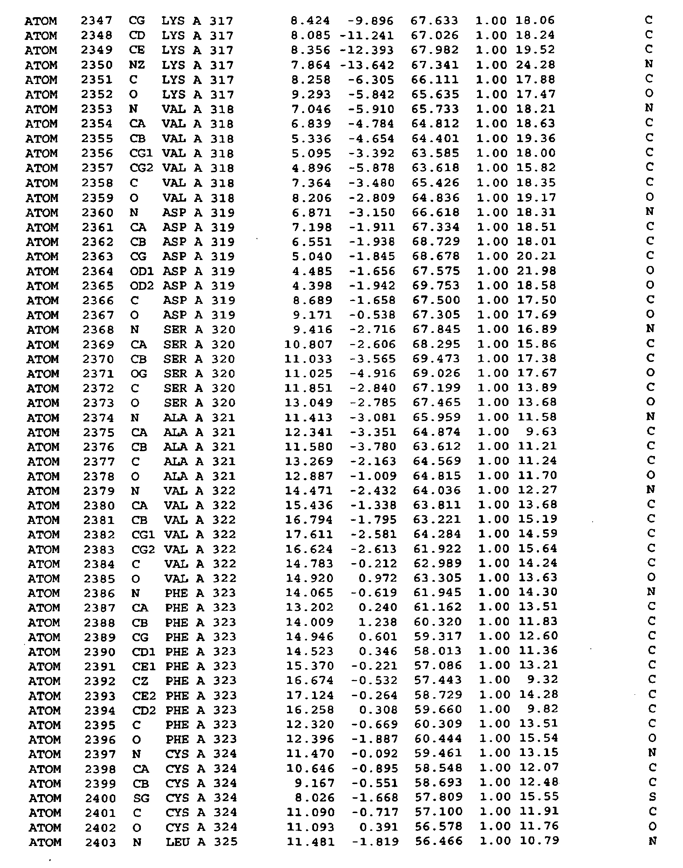

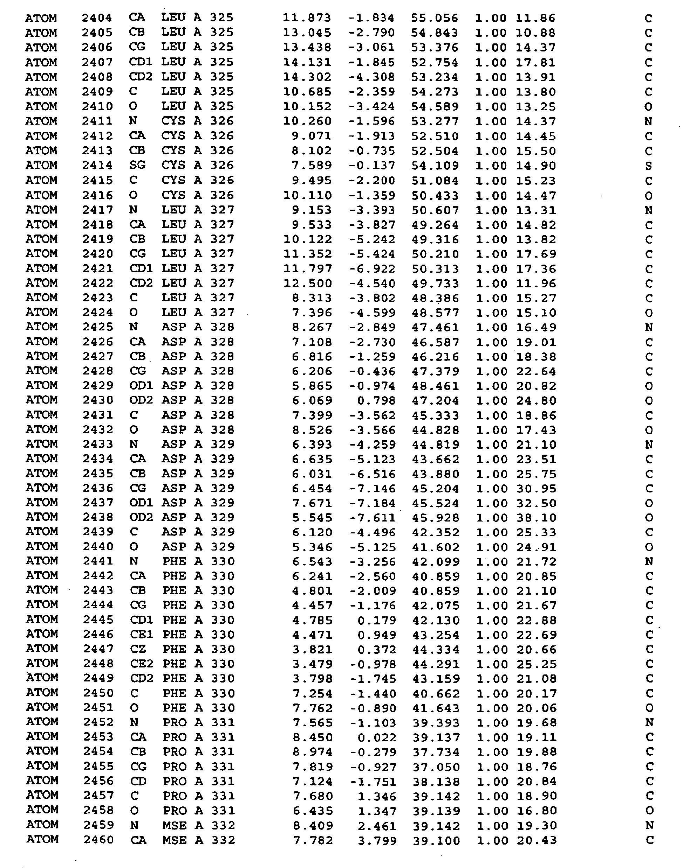

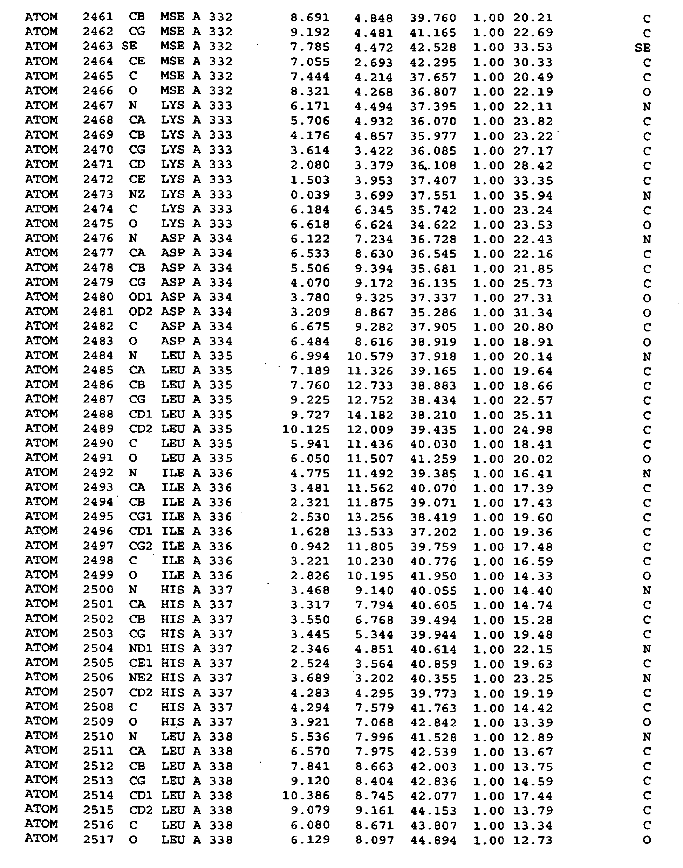

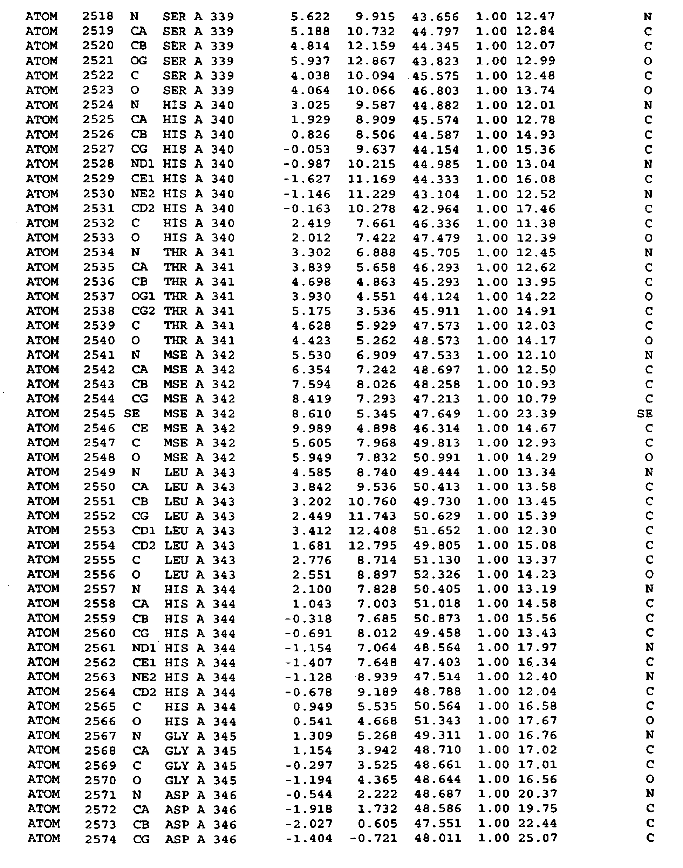

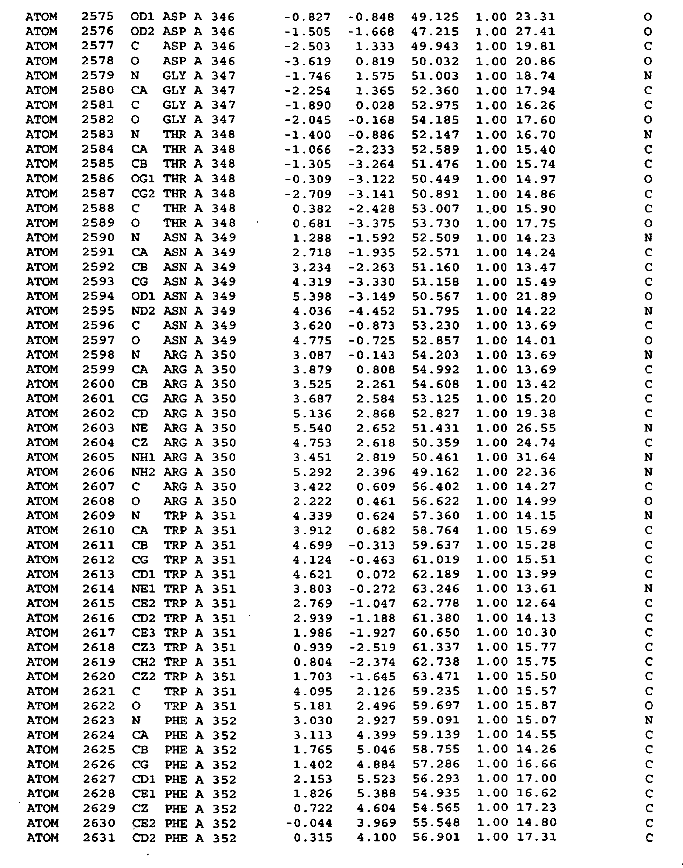

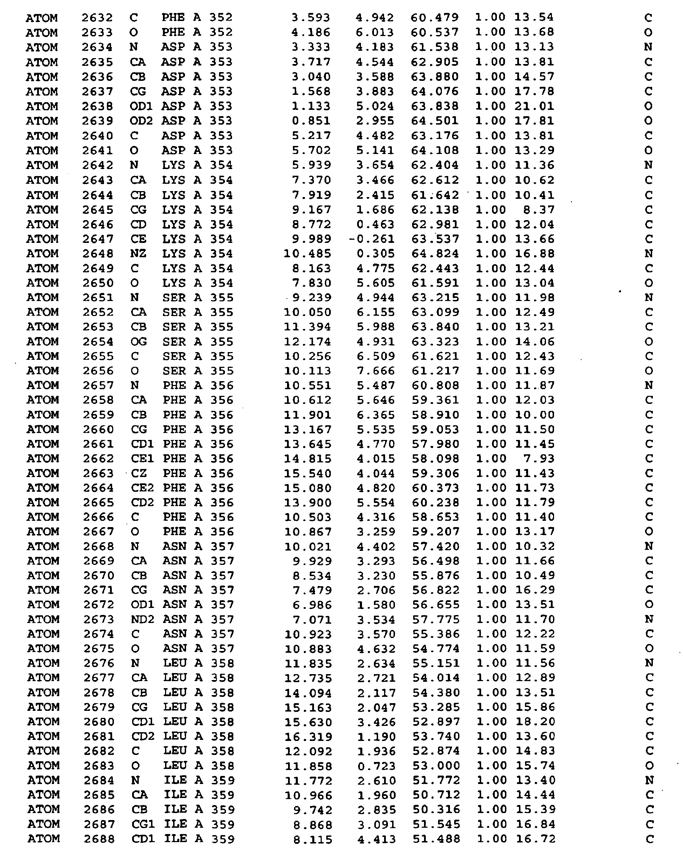

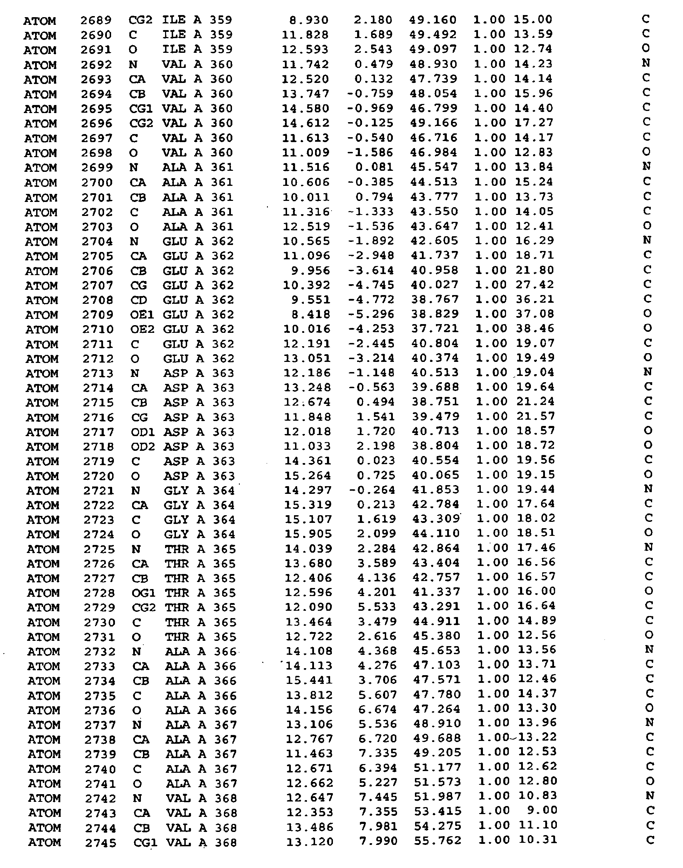

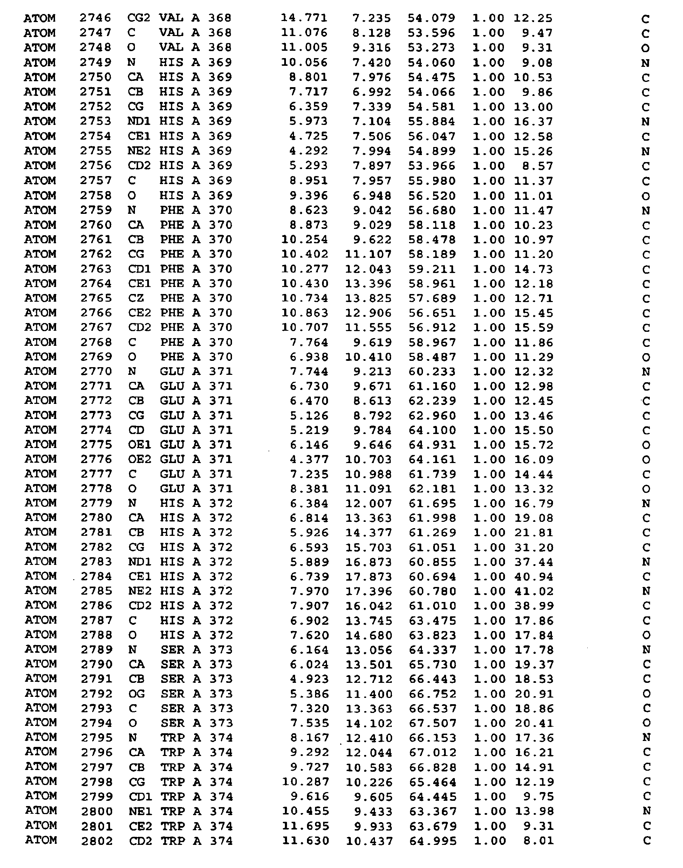

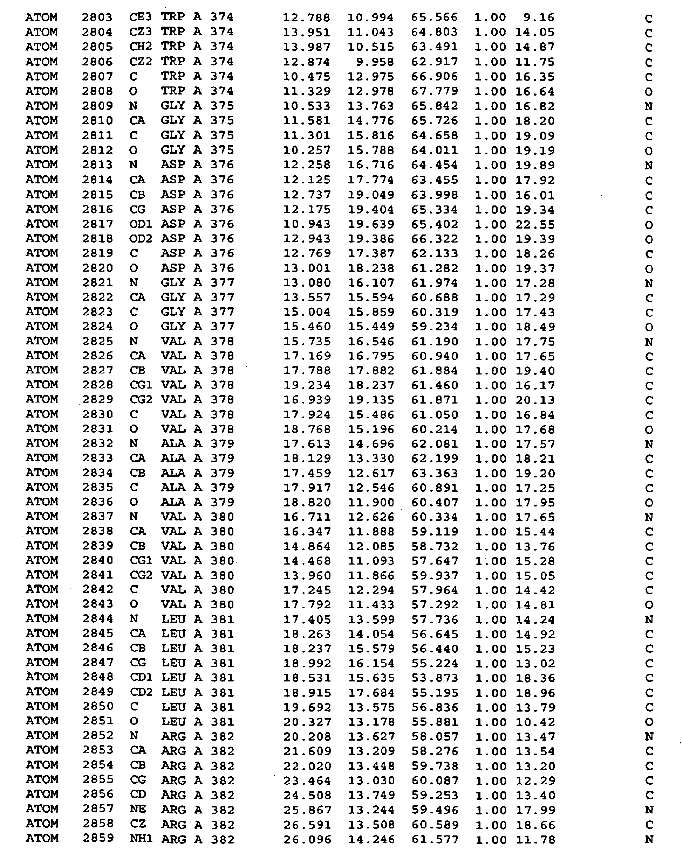

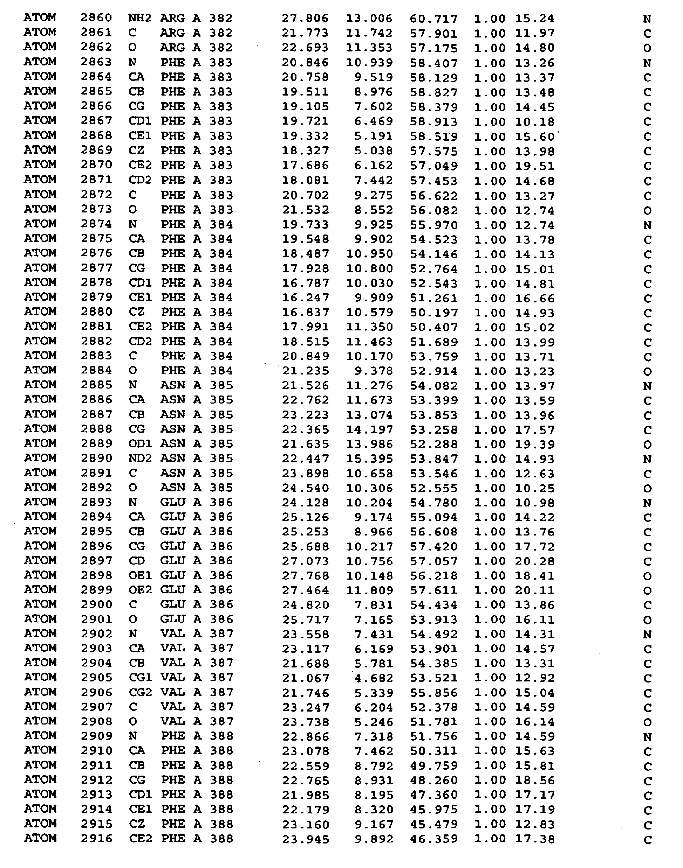

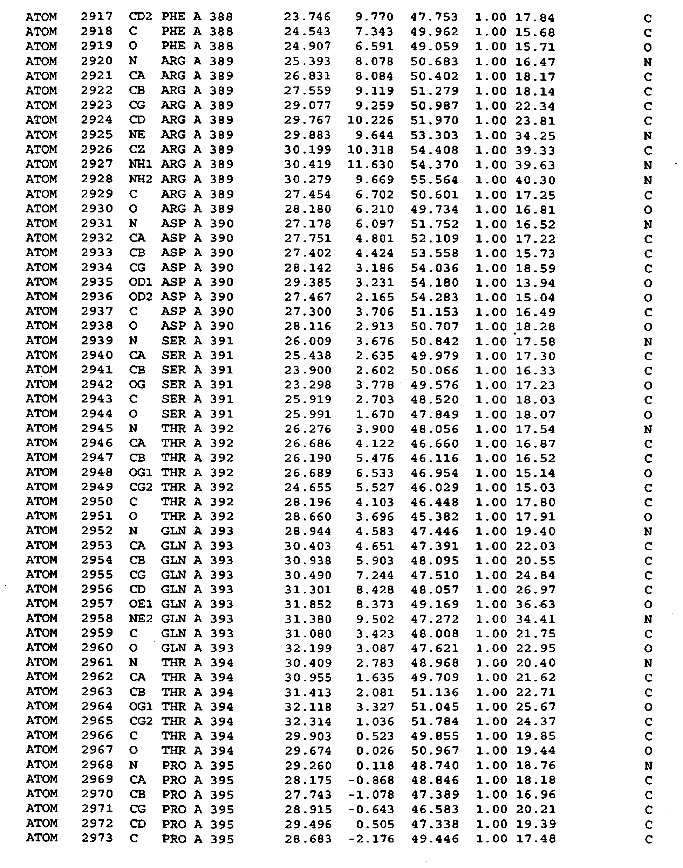

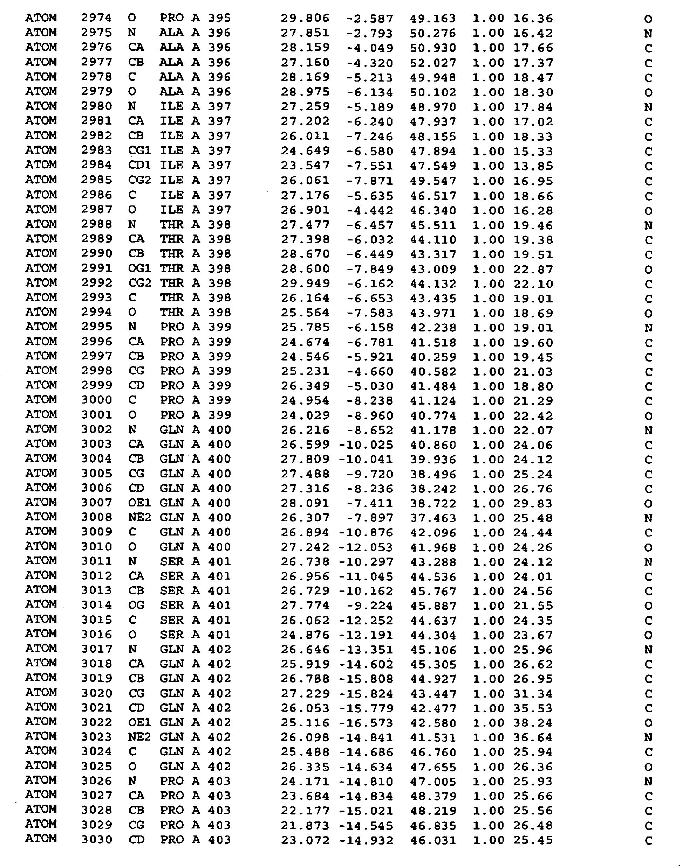

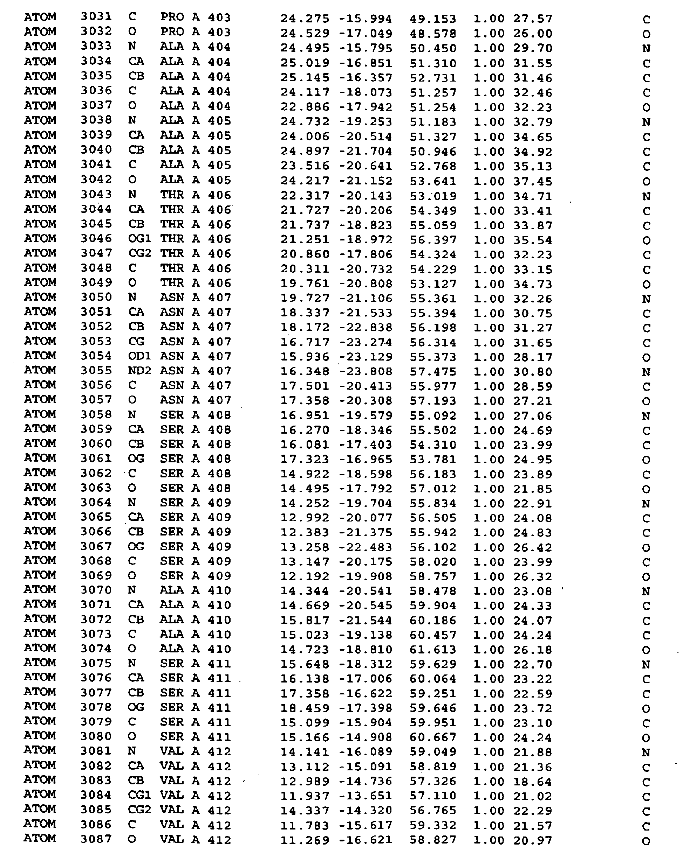

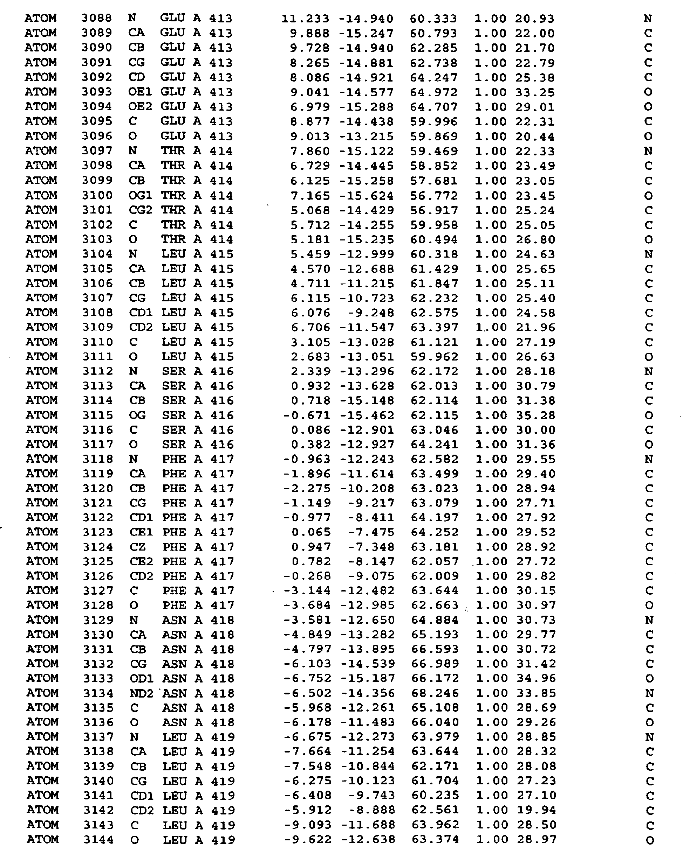

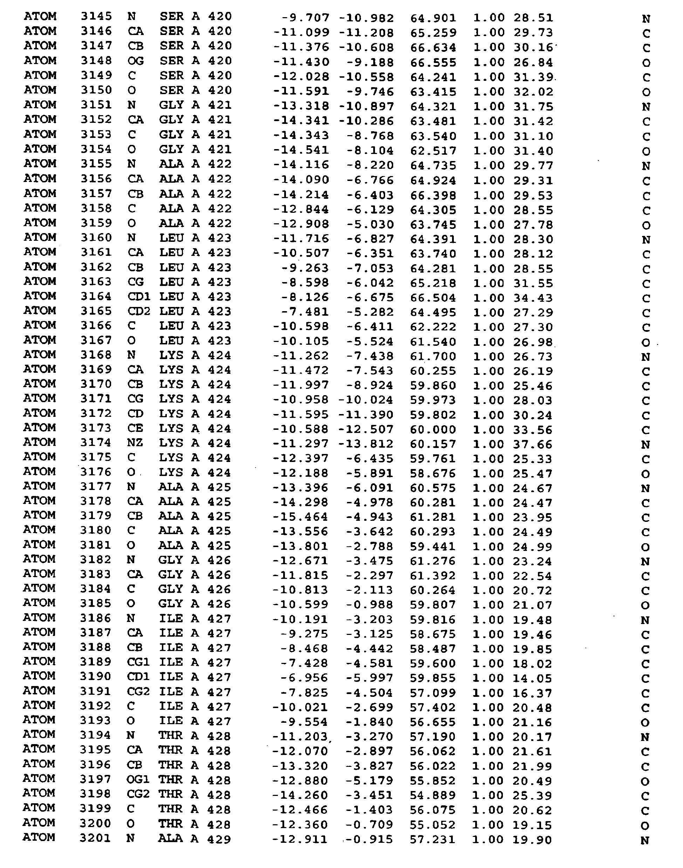

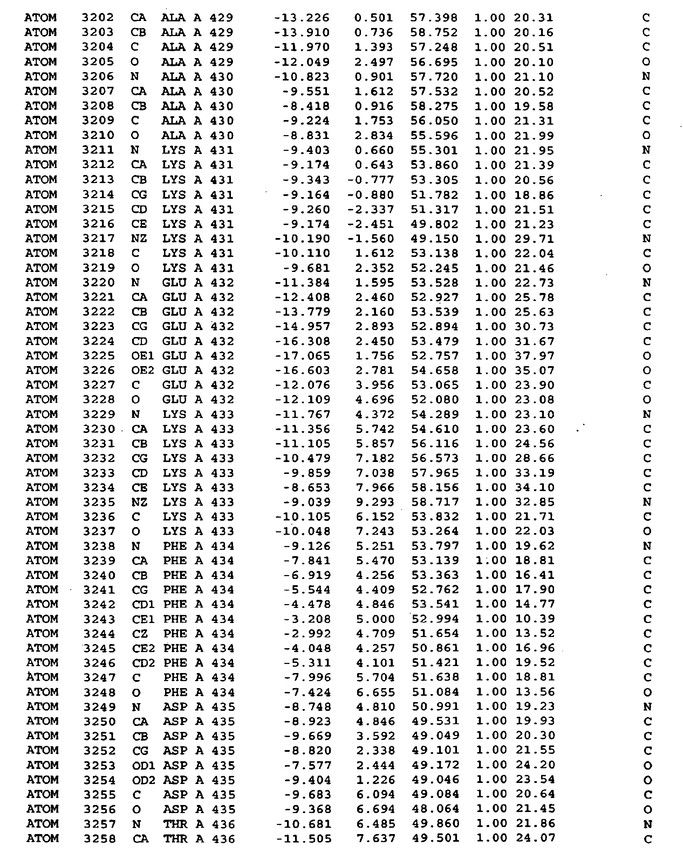

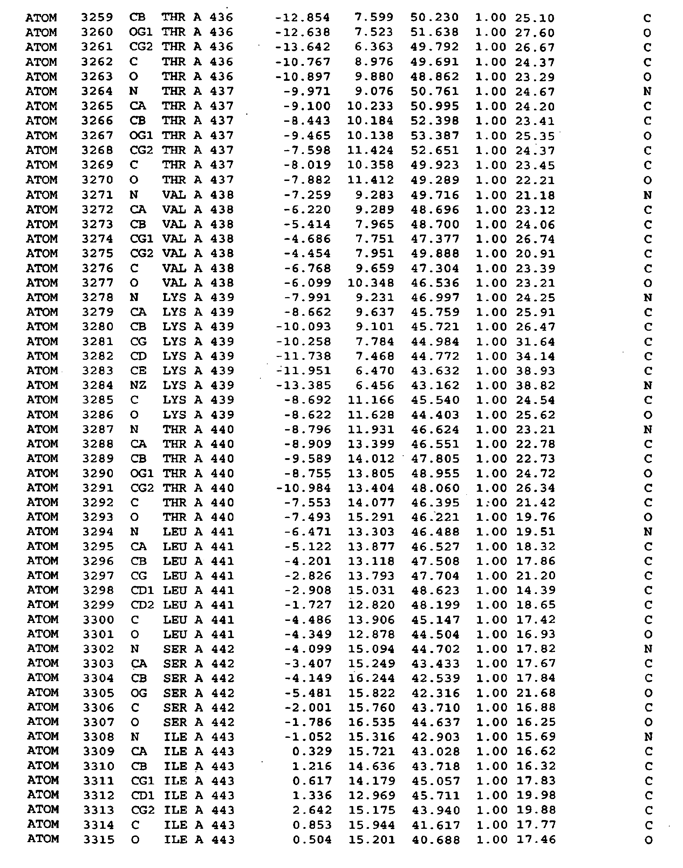

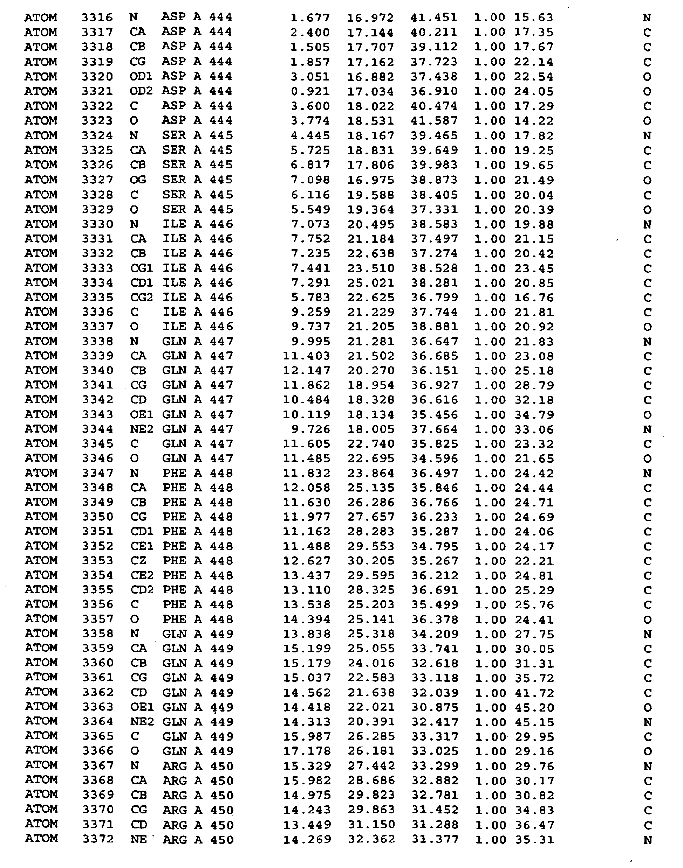

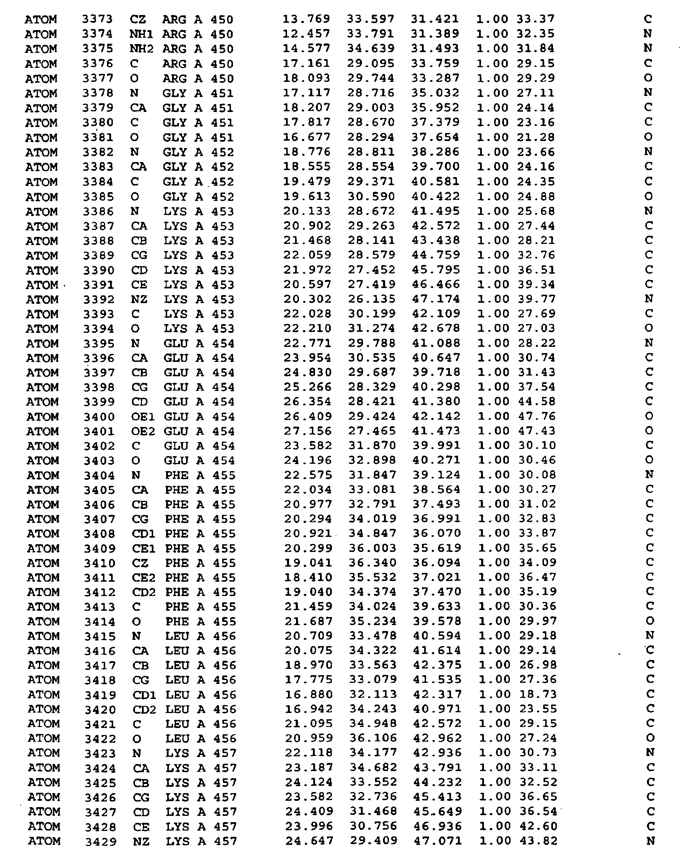

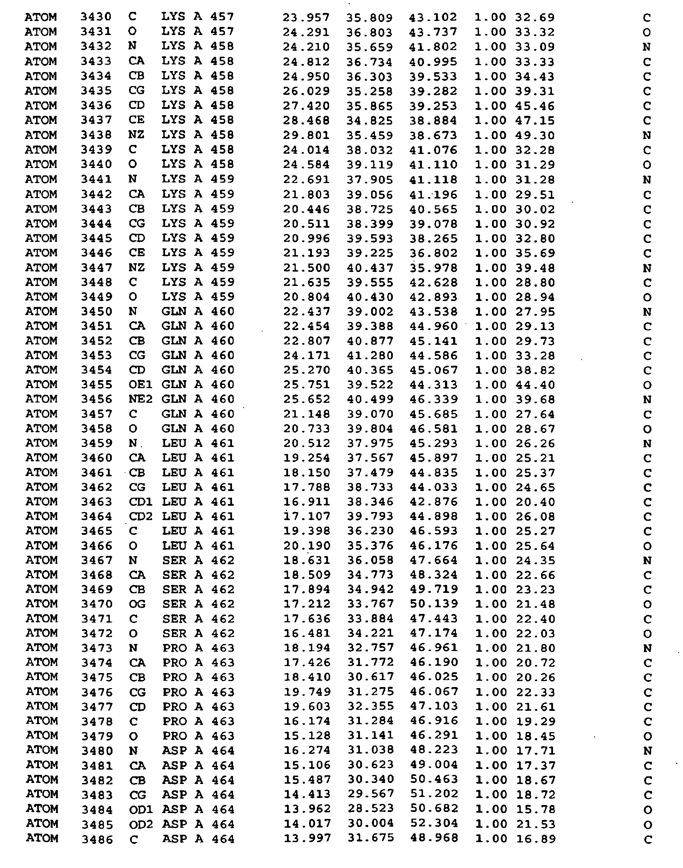

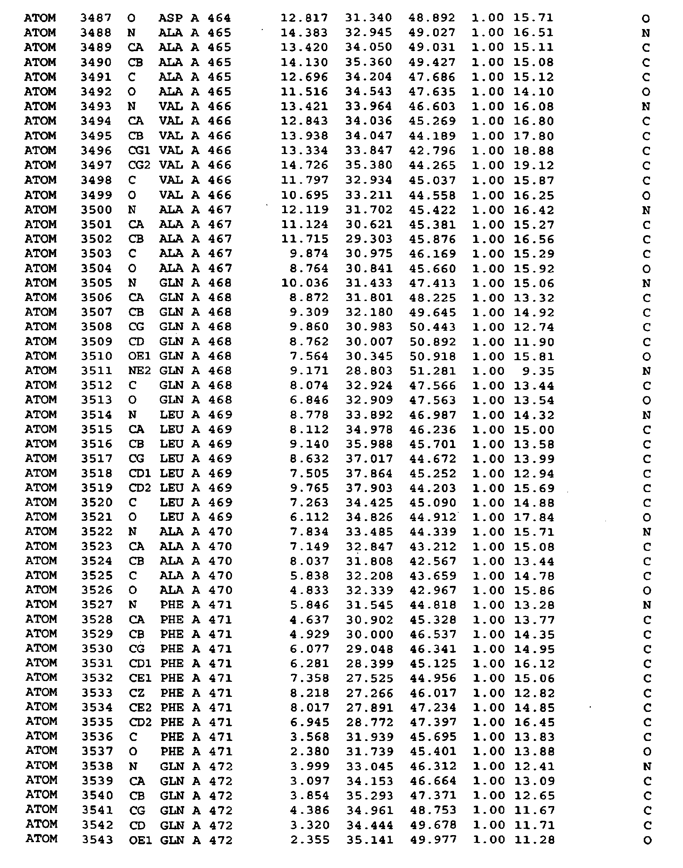

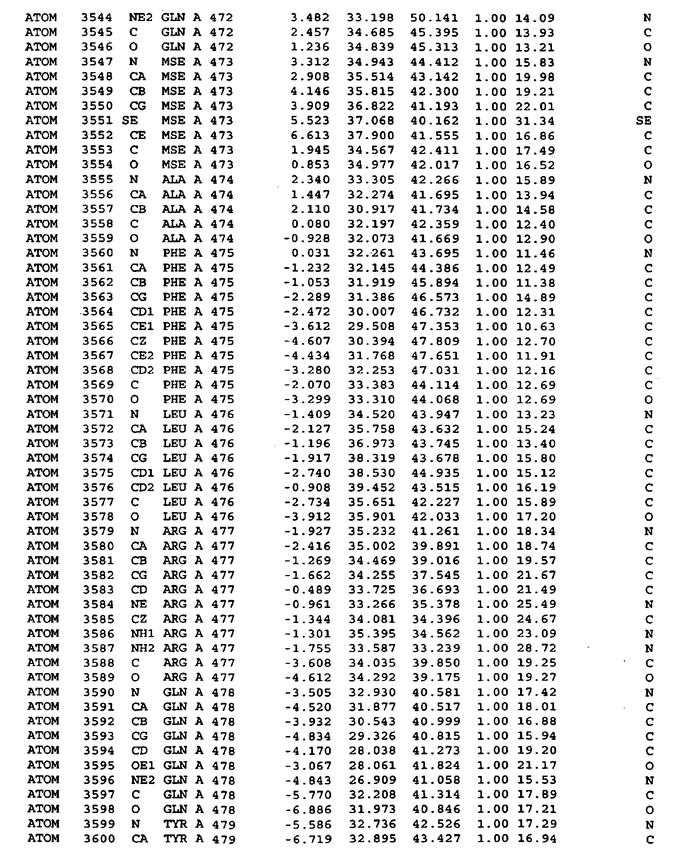

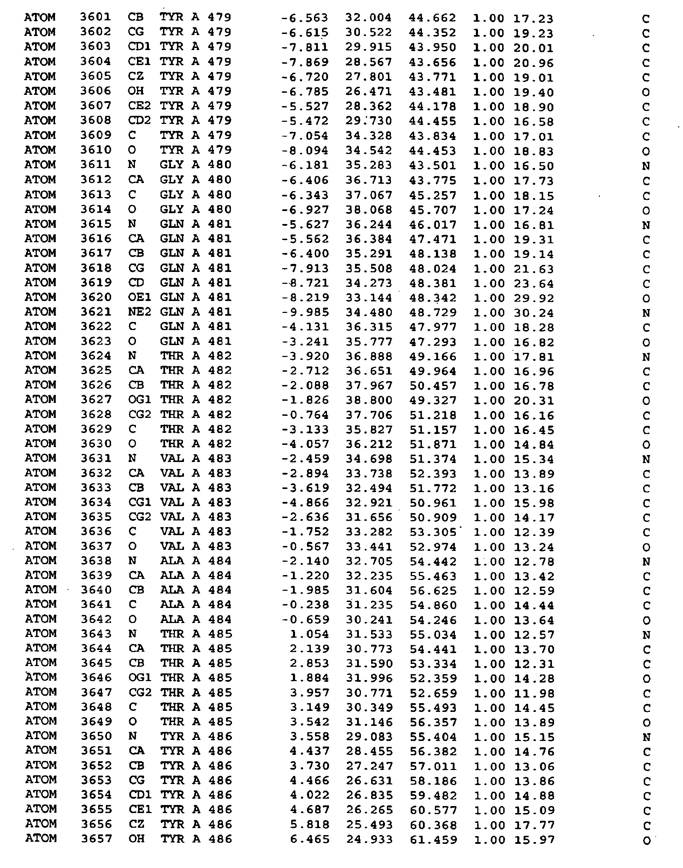

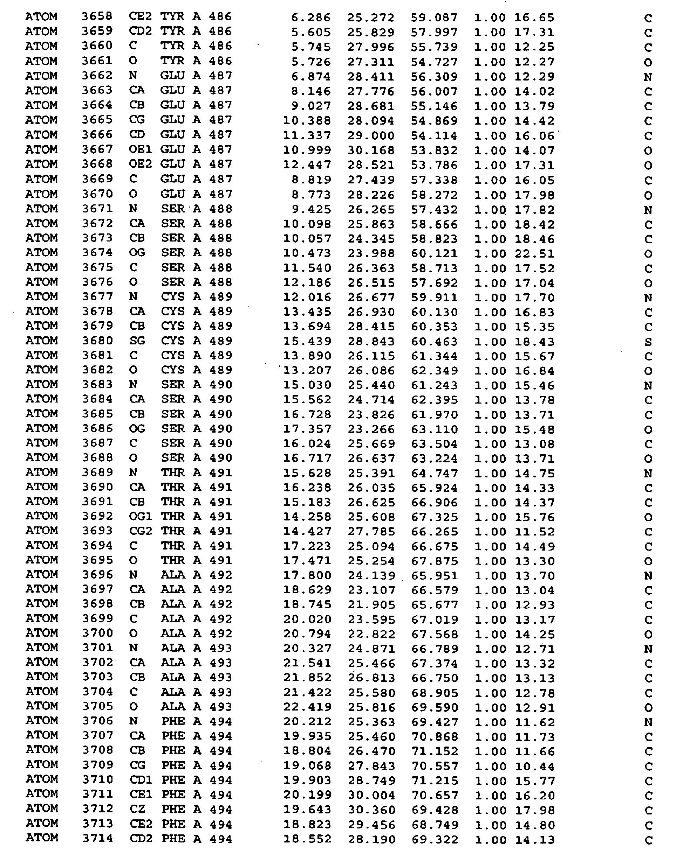

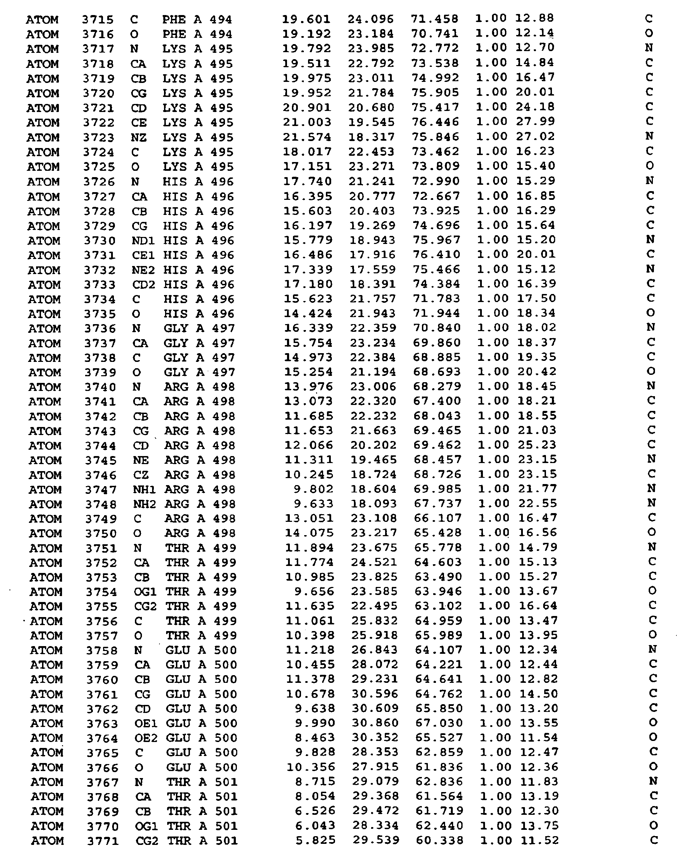

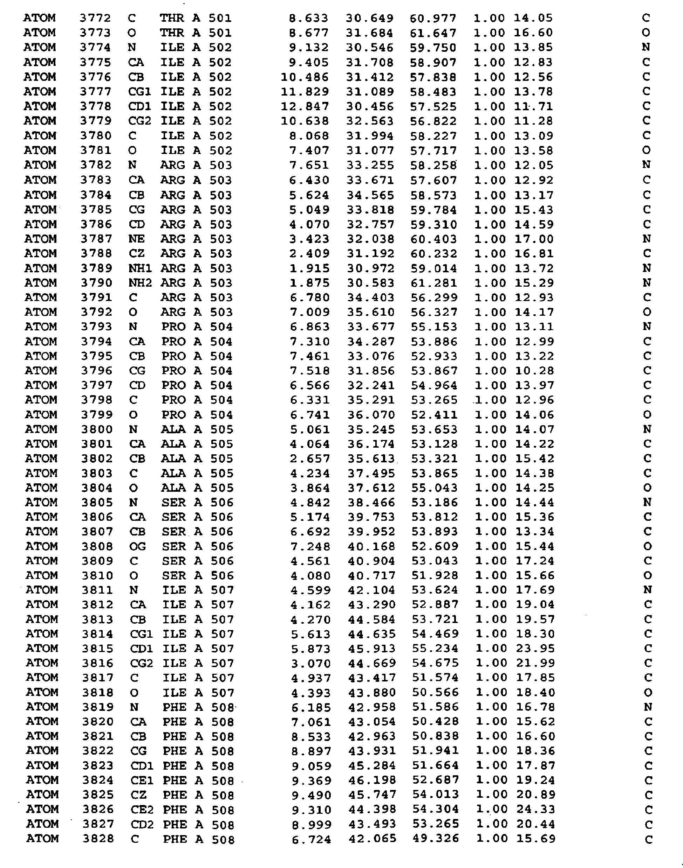

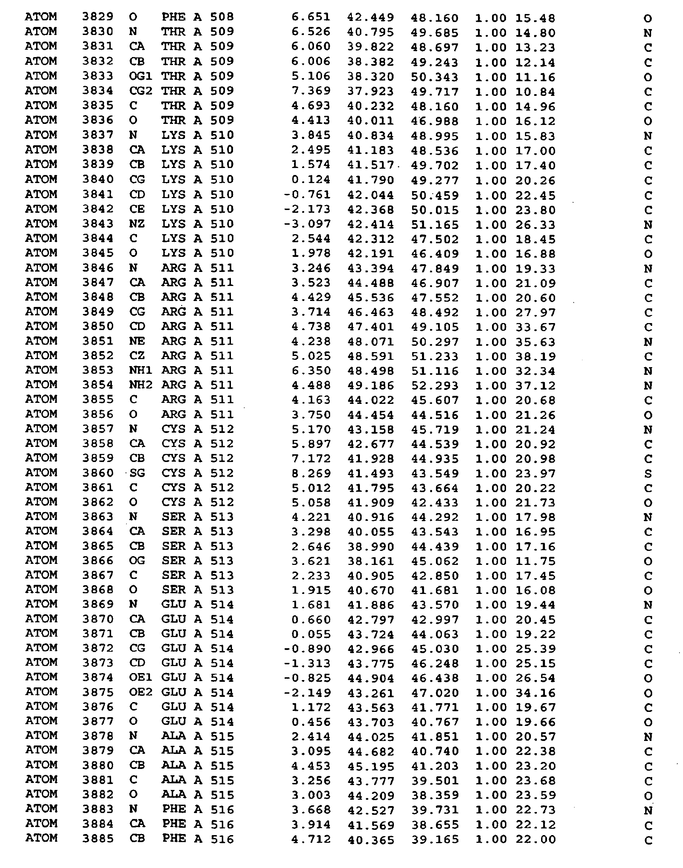

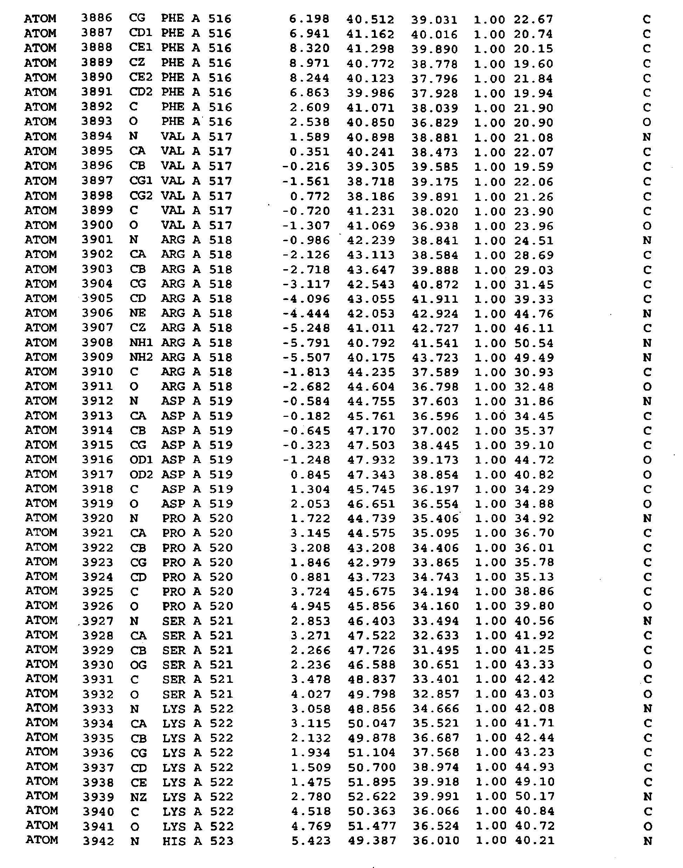

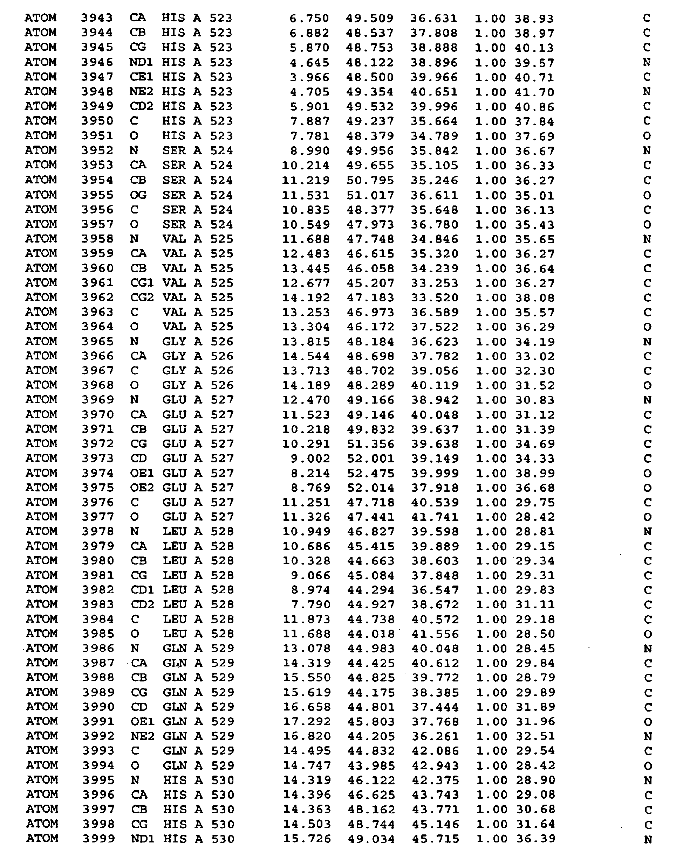

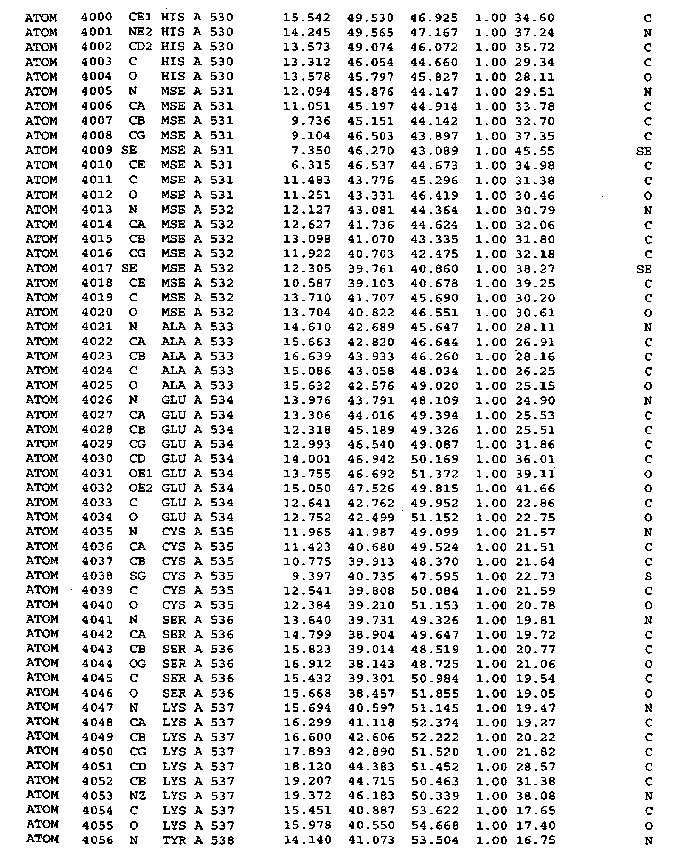

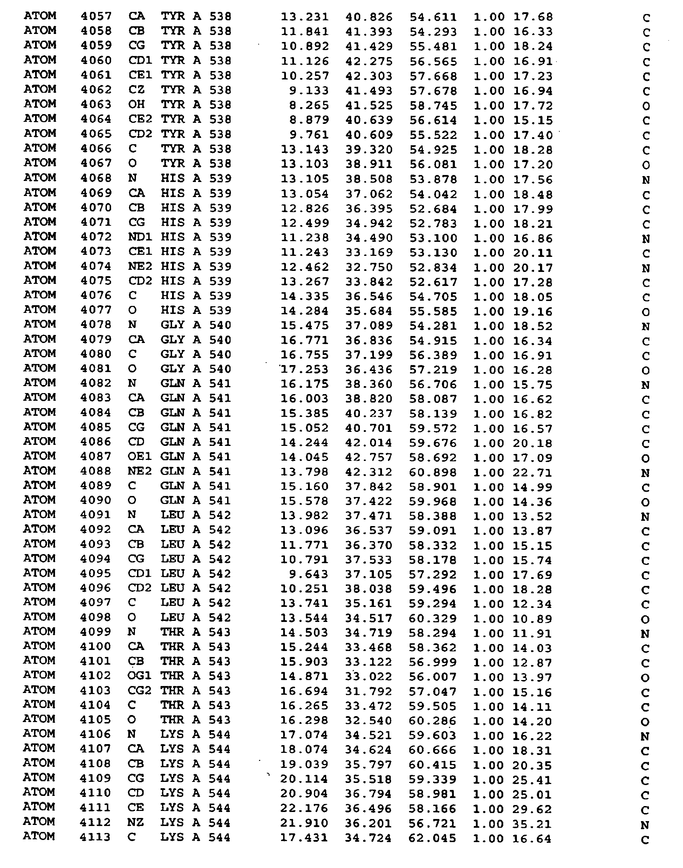

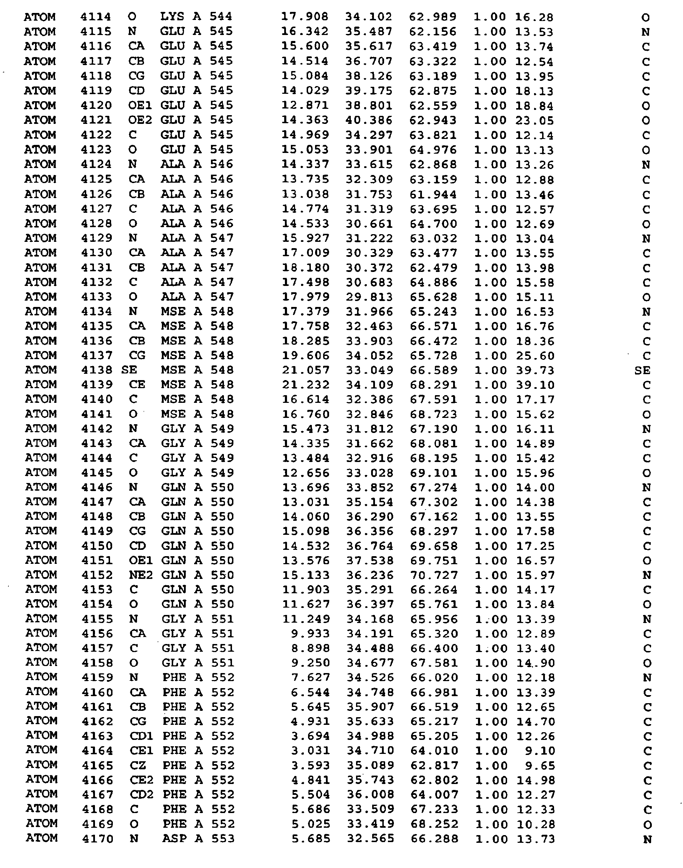

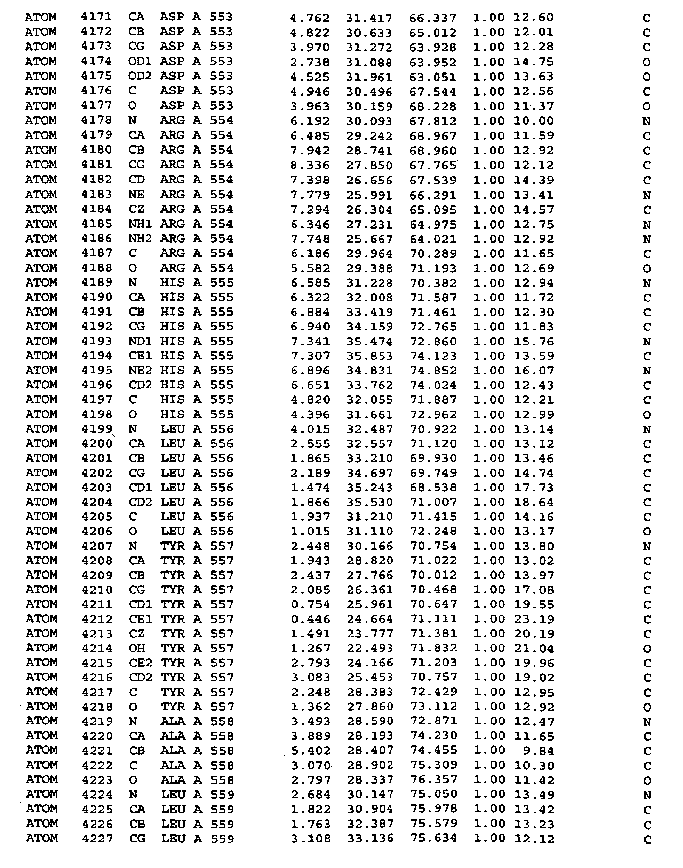

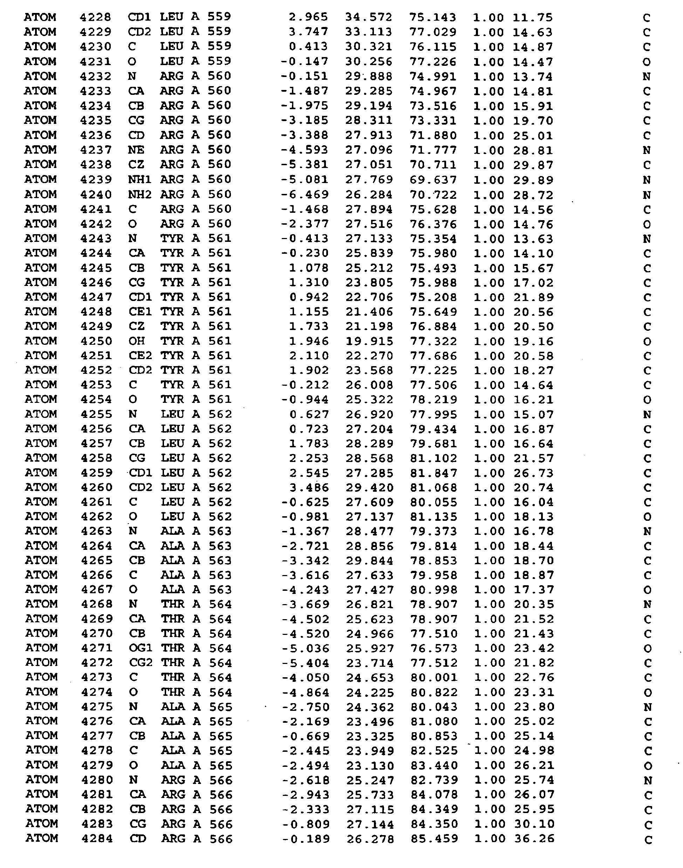

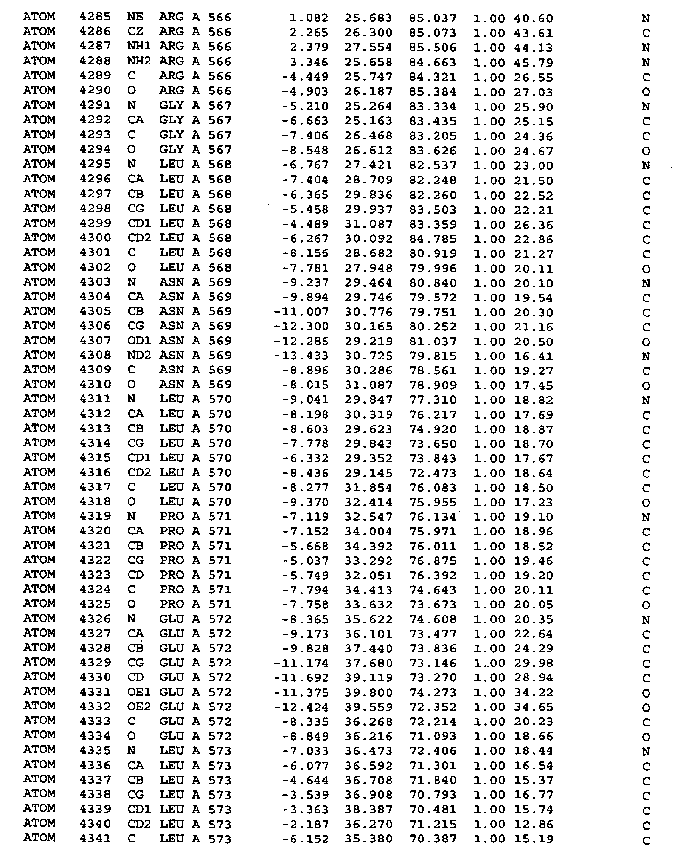

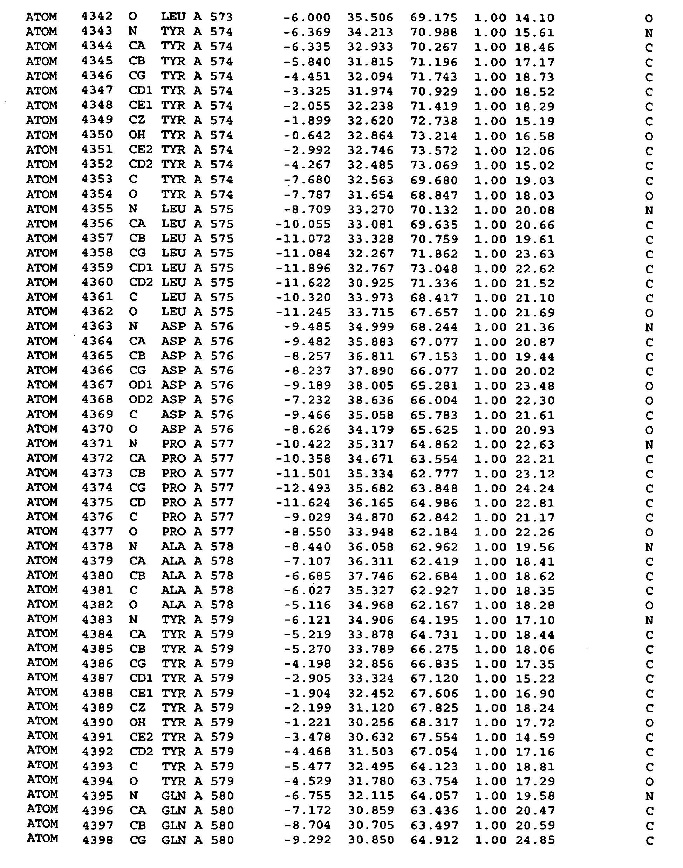

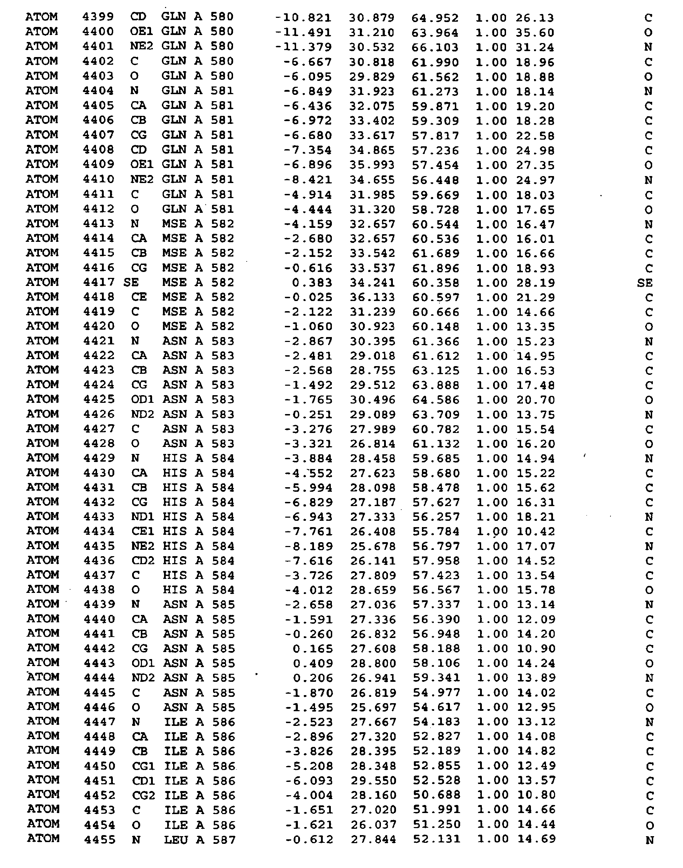

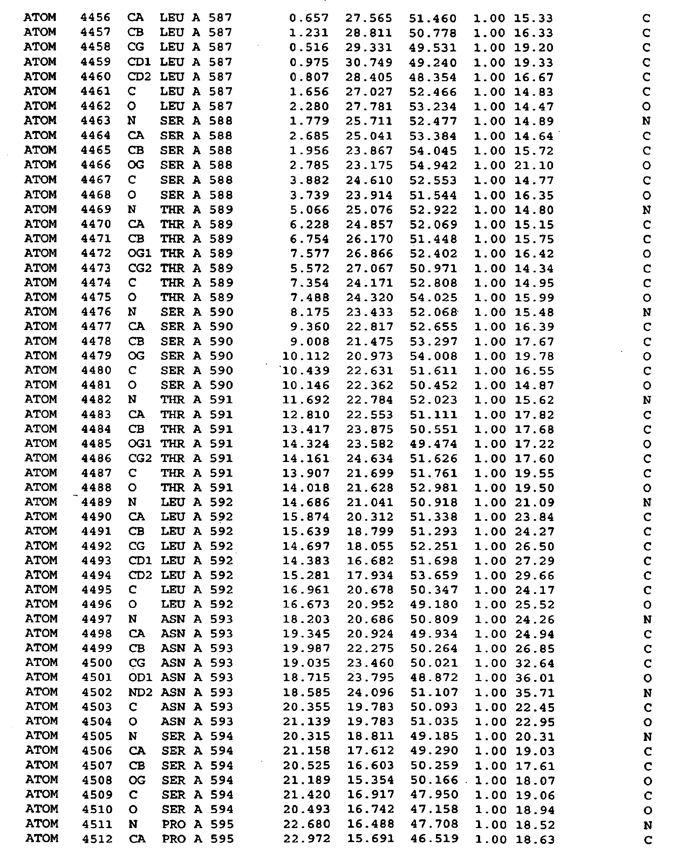

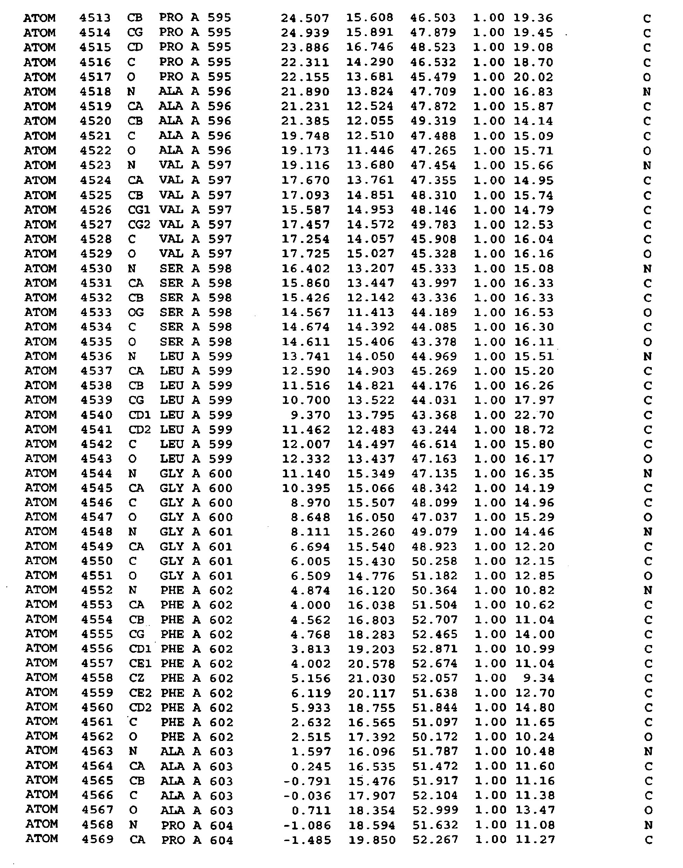

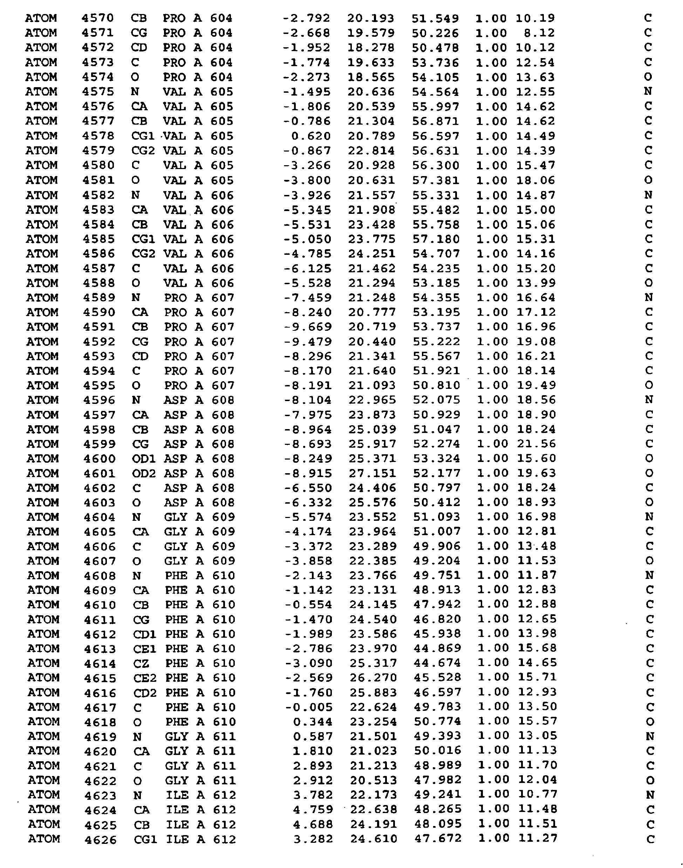

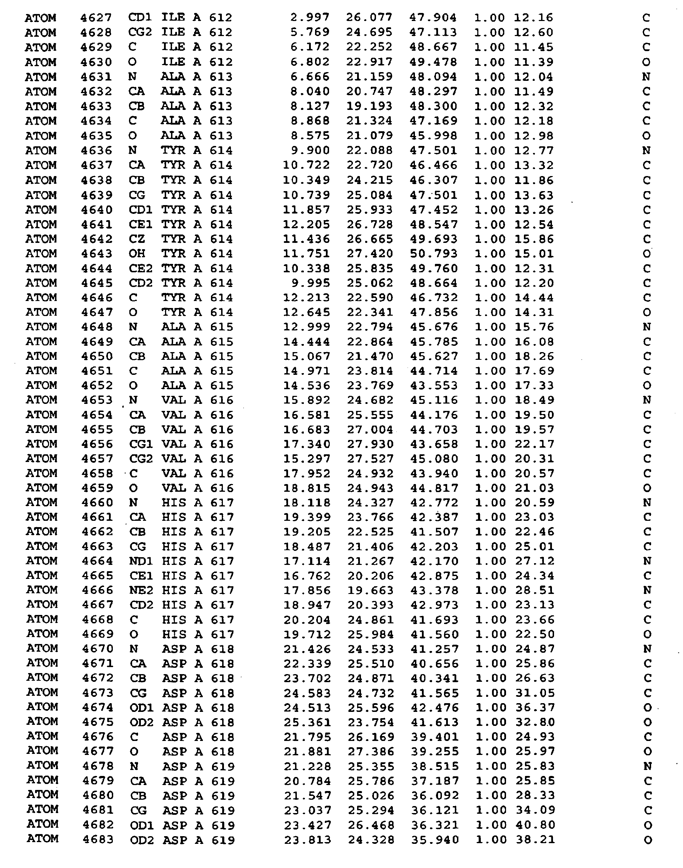

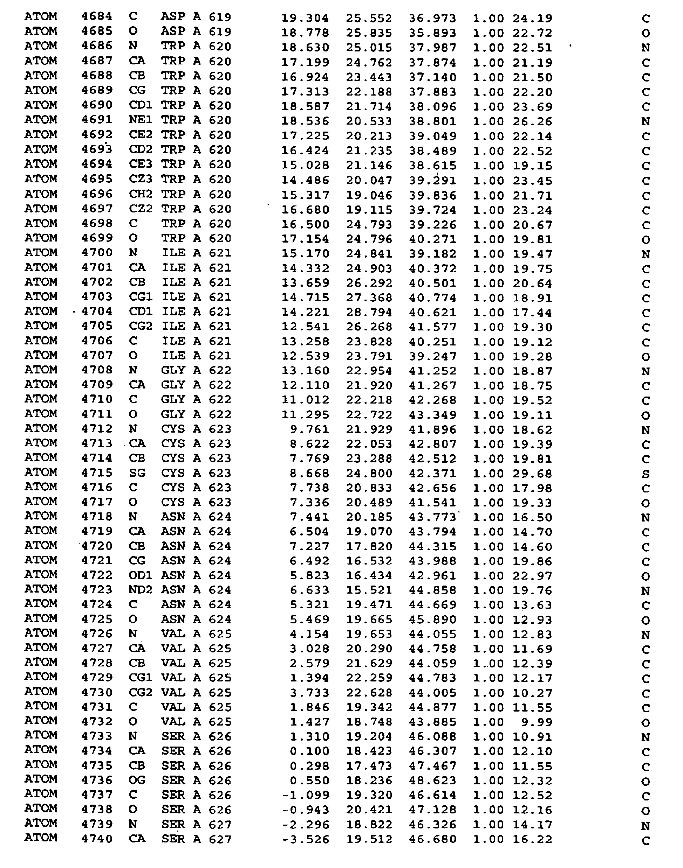

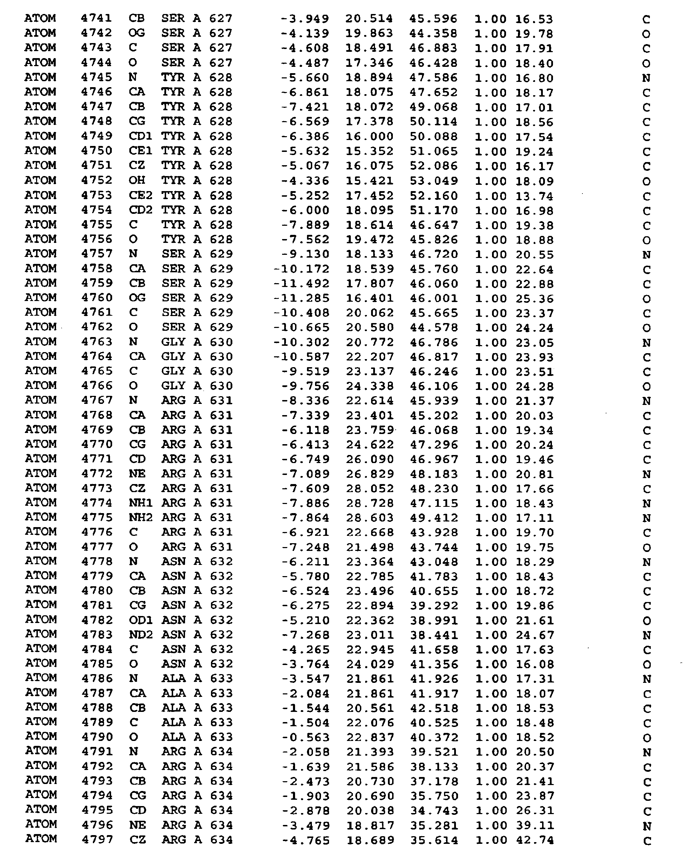

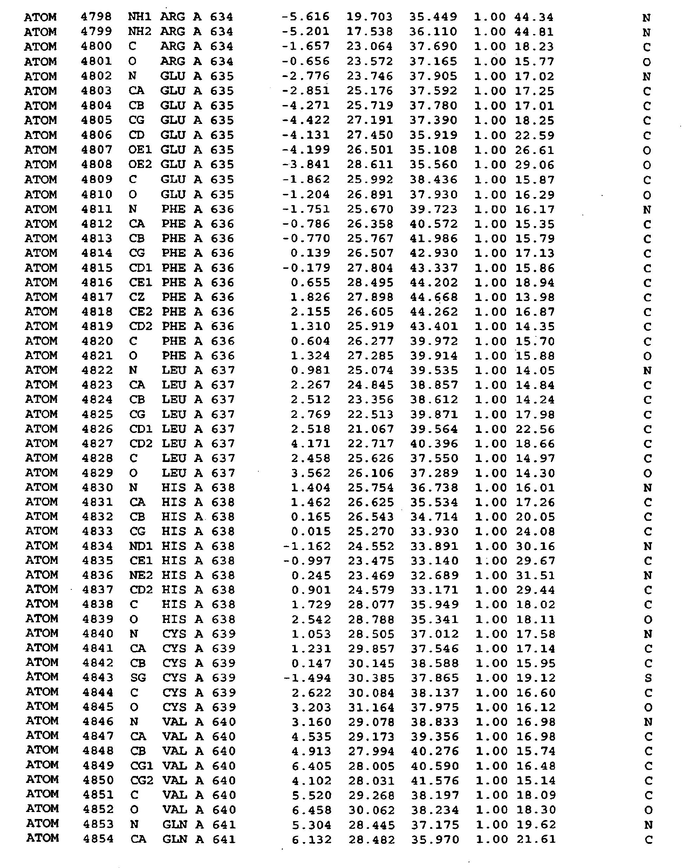

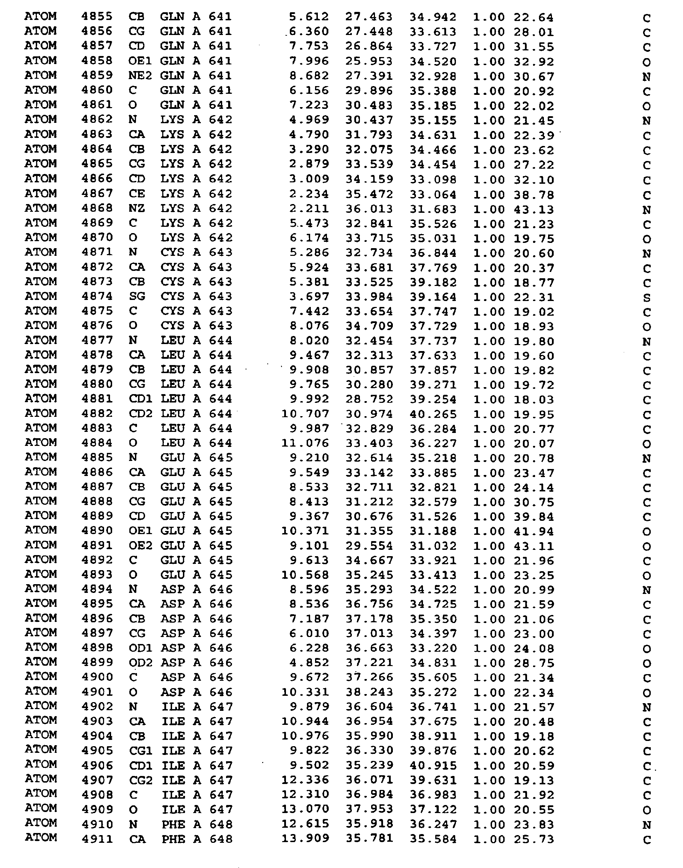

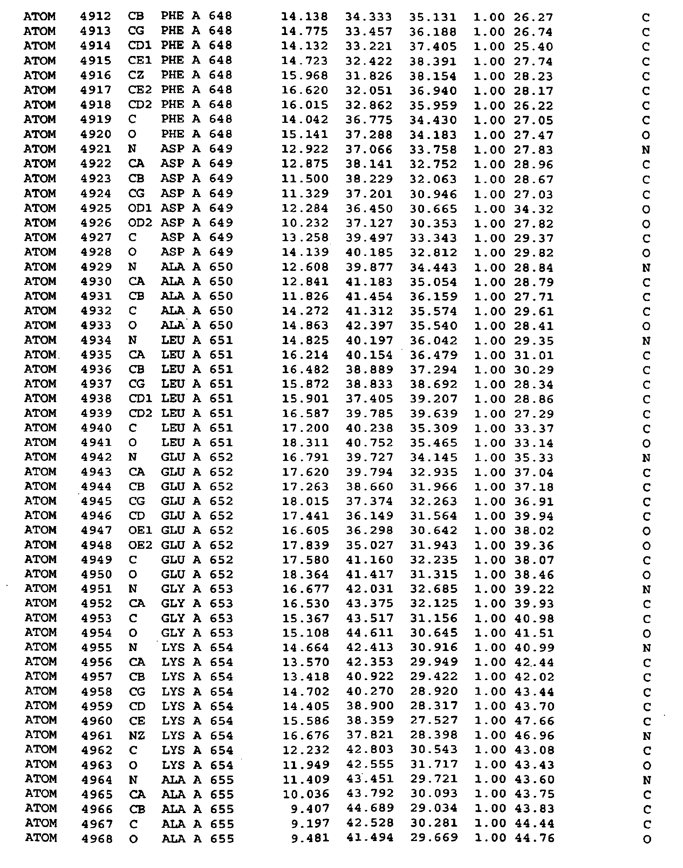

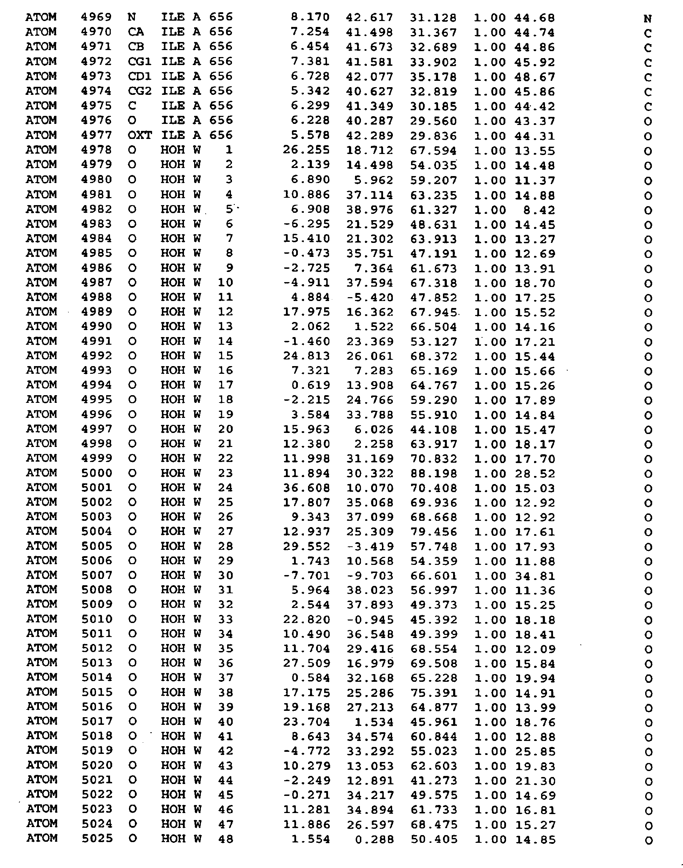

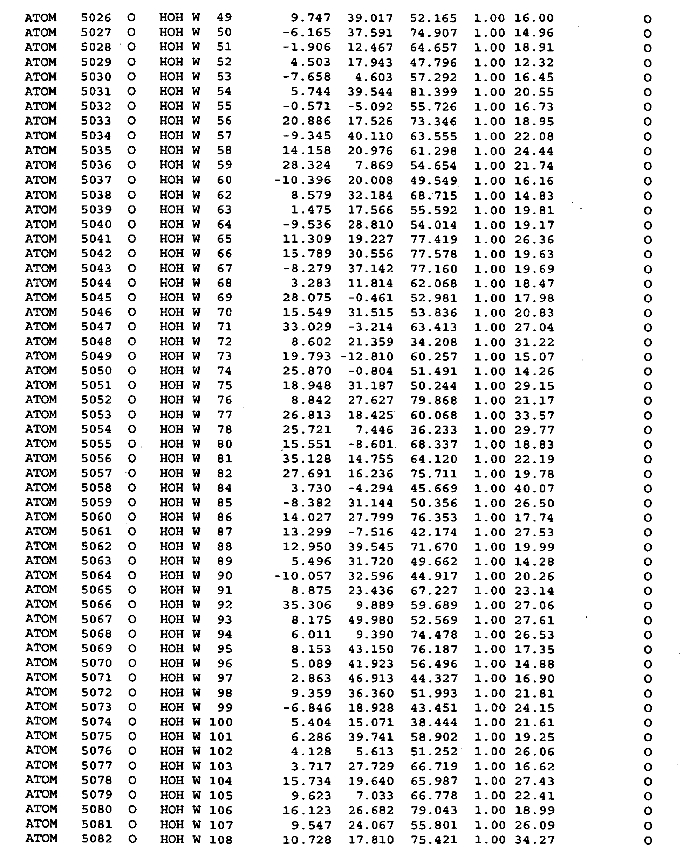

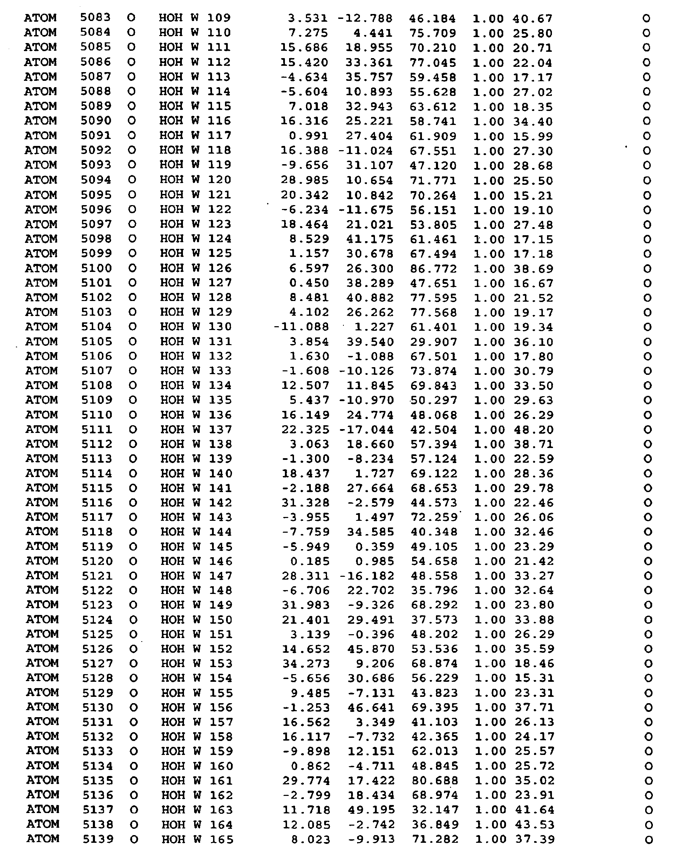

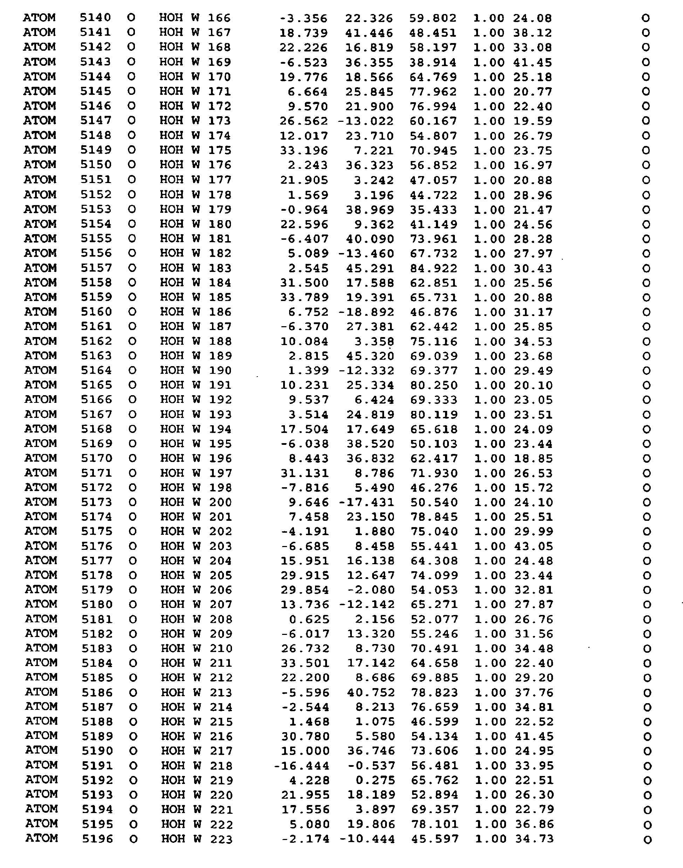

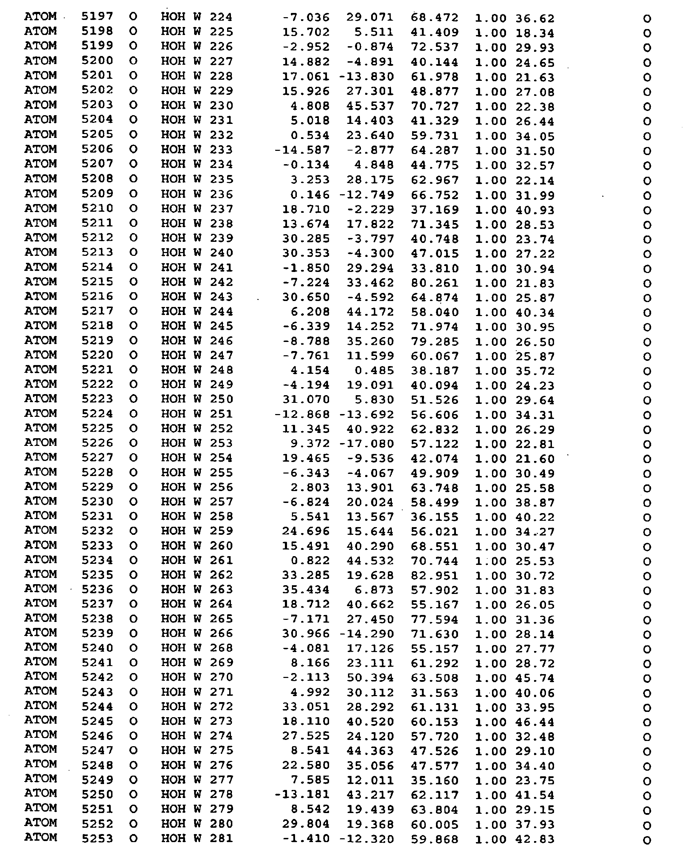

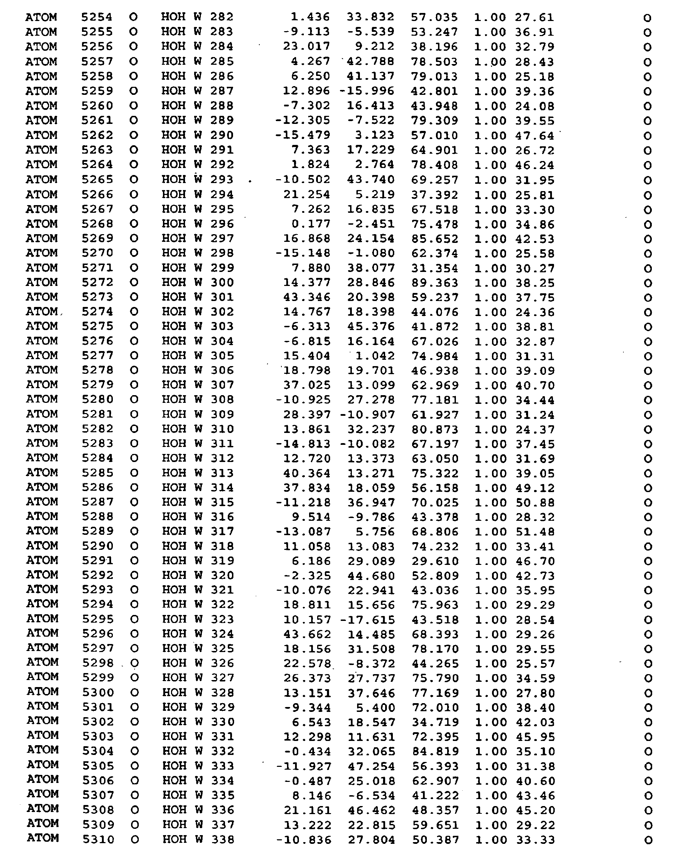

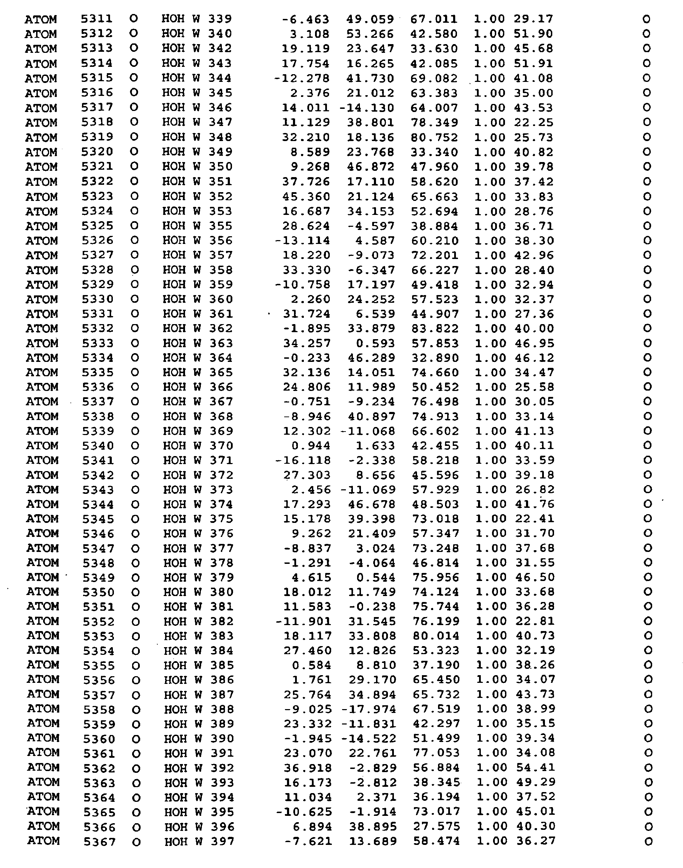

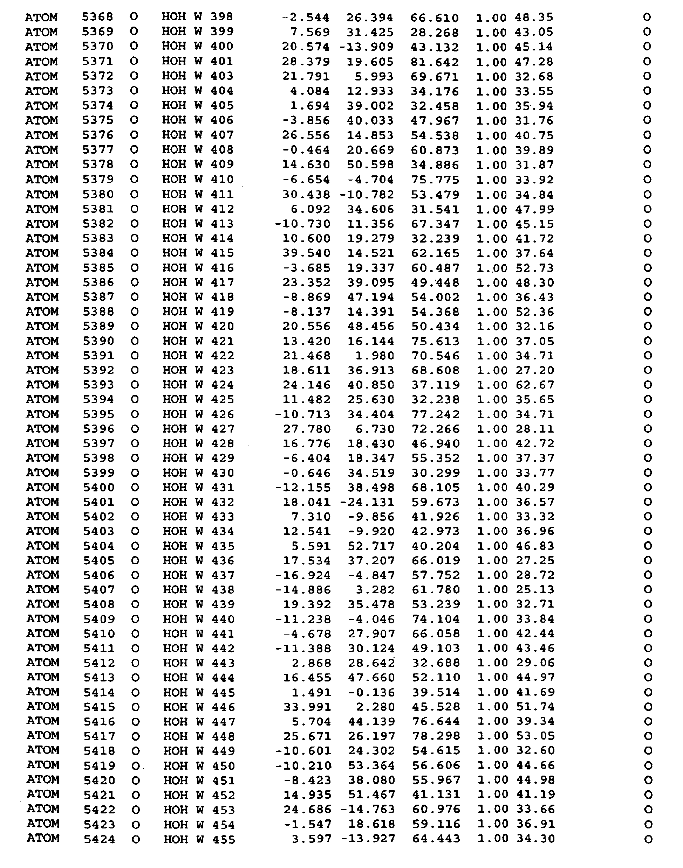

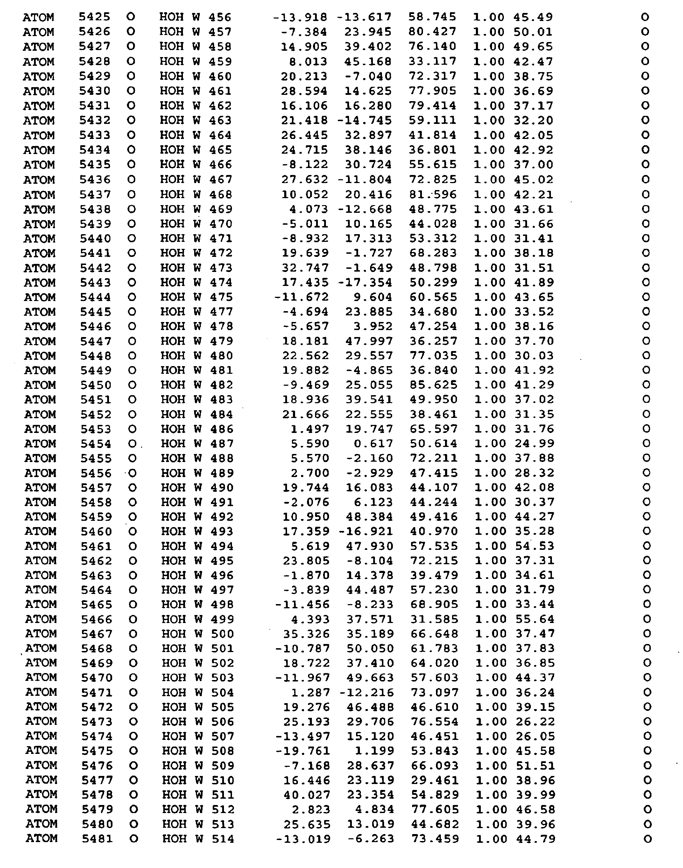

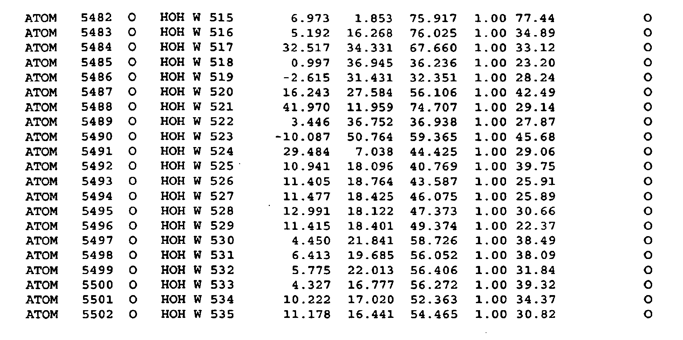

Thus, the present invention also provides a method of identifying compounds that can bind to protein CPT-2, comprising the steps of: applying a 3-dimensional molecular modeling algorithm to the atomic coordinates of protein CPT-2 shown in Fig. 4 or 5 to determine the spatial coordinates of the binding pocket of protein CPT-2; and electronically screening the stored spatial coordinates of a set of candidate compounds against the spatial coordinates of the protein CPT-2 binding pocket to identify compound that can bind to protein CPT-2.

In an embodiment of the crystals, co-crystals, method and process hereinbefore described, said CPT-2 protein is rat CPT-2 protein. Furthermore, said rat CPT-2 protein may be the protein of Seq ID No. 2.

For purposes of clarity and discussion, the crystals of the invention will be described by reference to specific CPT-2 exemplary apo crystals and co-crystals. Those skilled in the art will appreciate that the principles described herein are generally applicable to crystals of any mammalian CPT-2, including, but not limited to the CPT-2 of Seq ID No. 2.

As used herein, "apo crystal" refers to crystals of CPT-2 formed without addition of site-specific ligands.

### Brief description of the Figures:

**Figure 1****.** Structure of rat CPT-2 with ST1326 bound to the active site. The ligand ST1326 and its surrounding *fofc* simulated annealing electron density map (contoured at 2.5 σ) are depicted in pink in A-C. **A,** ST1326 binds at the interface of the amino-terminal (orange) and carboxy-terminal (cyan) domains of rat CPT-2. **B,** the central β-strands (blue) are surrounded by α-helices (green), the CPT-2-specific insert (red) protrudes from the amino-terminal domain. **C,** same as **B** but rotated 90° to the back.
**Figure 2****:** Amino acid sequence alignment of rat CPT-2 (rCPT-2) and human CPT-1 (hCPT-1). Secondary structure elements (S.S.) are indicated. The residue numbering corresponds to the rCPT-2 precursor and its mitochondrial import sequence is italicized. The CPT-2 specific insert (amino acids 179-208) is underlined. Keey residues of the acylcarnitine binding site of rCPT-2 are in bold letters and are labeled with * when fully conserved in hCPT-1. Residues that have been reported to be mutated in CPT-2 deficiency are printed red.
**Figure 3****. A,** stereo figure of the active site tunnel viewed perpendicular to the domain interface. Key active site residues are depicted in yellow. The co-crystallized ST1326 is shown in pink, a CoA molecule (blue) was modeled based on the CoA coordinates from the CrAT-CoA complex structure (PDB code 1t7q). **B,** Fischer projection of ST1326 with atom numbering.
**Figure 4****:** Coordinates of apo1 (C222₁) crystal
**Figure 5****:** Coordinates of apo1 (P4₃2₁2) crystal

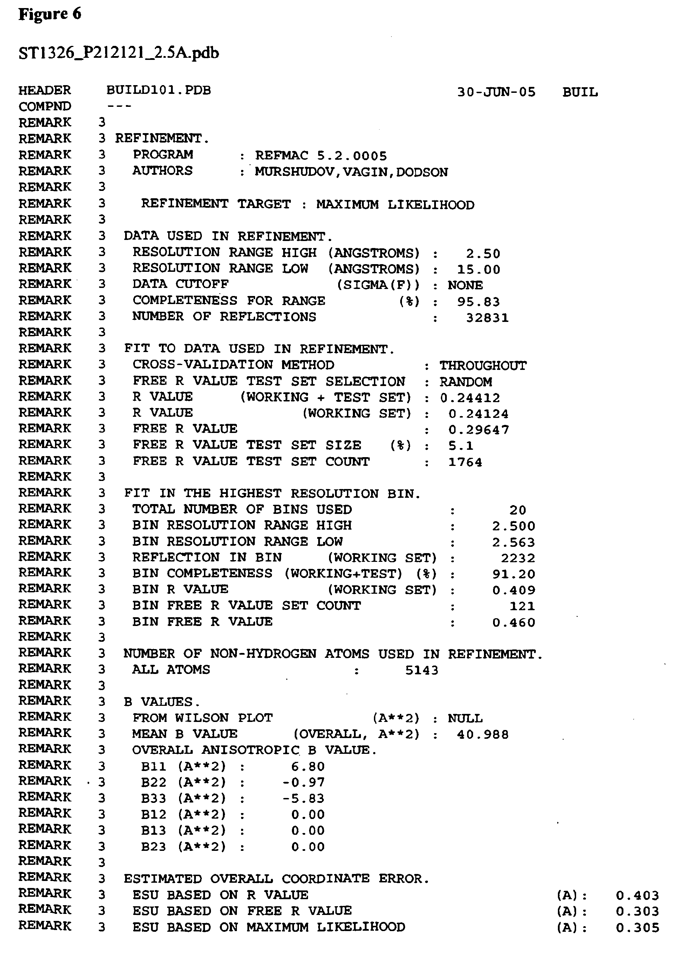

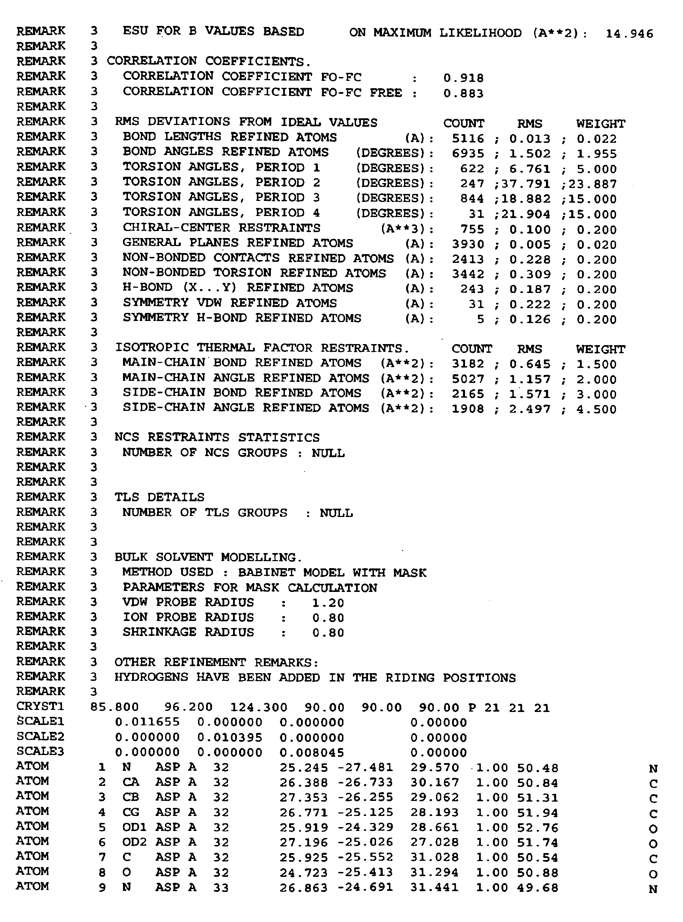

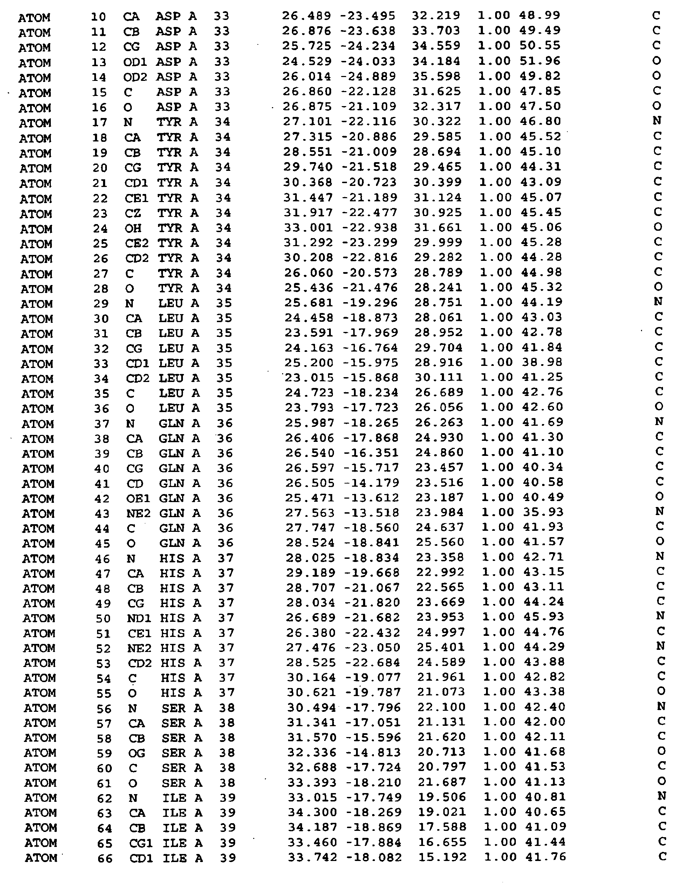

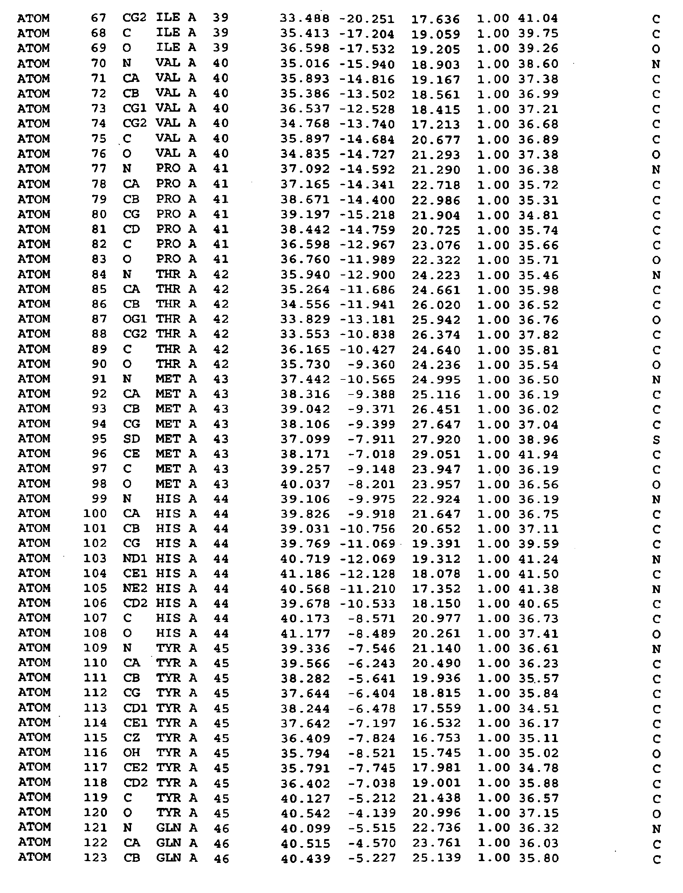

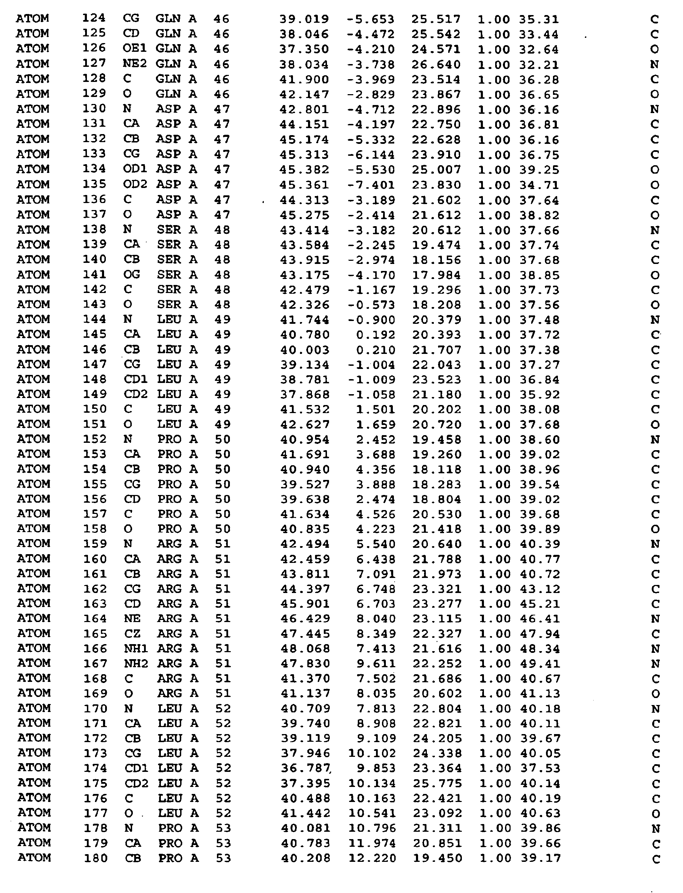

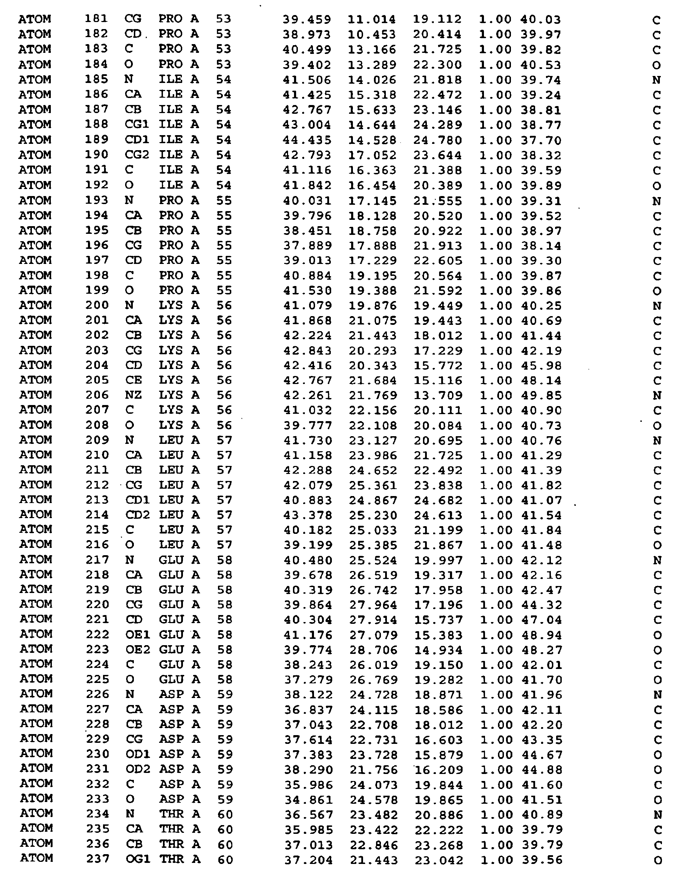

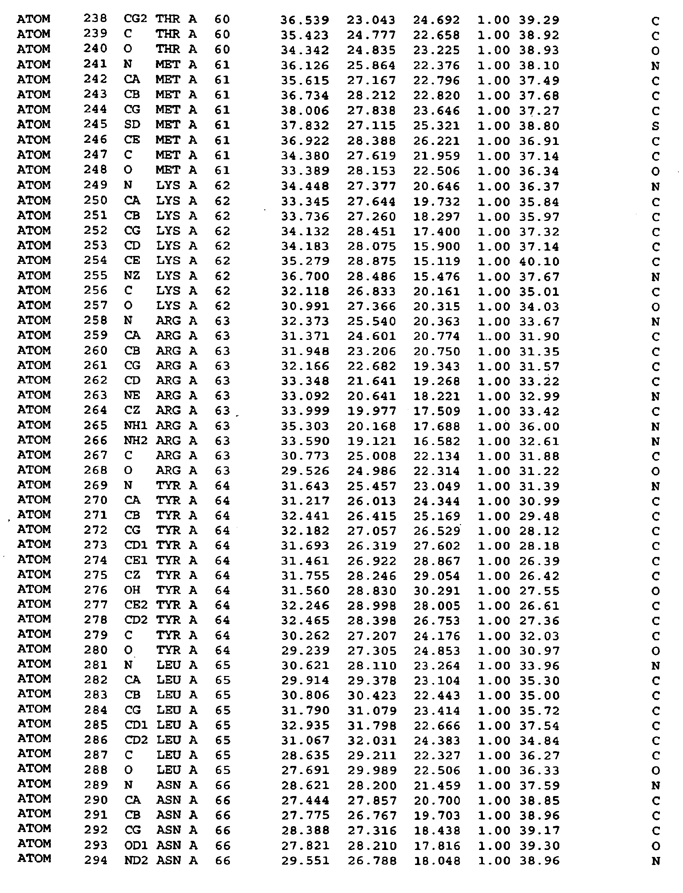

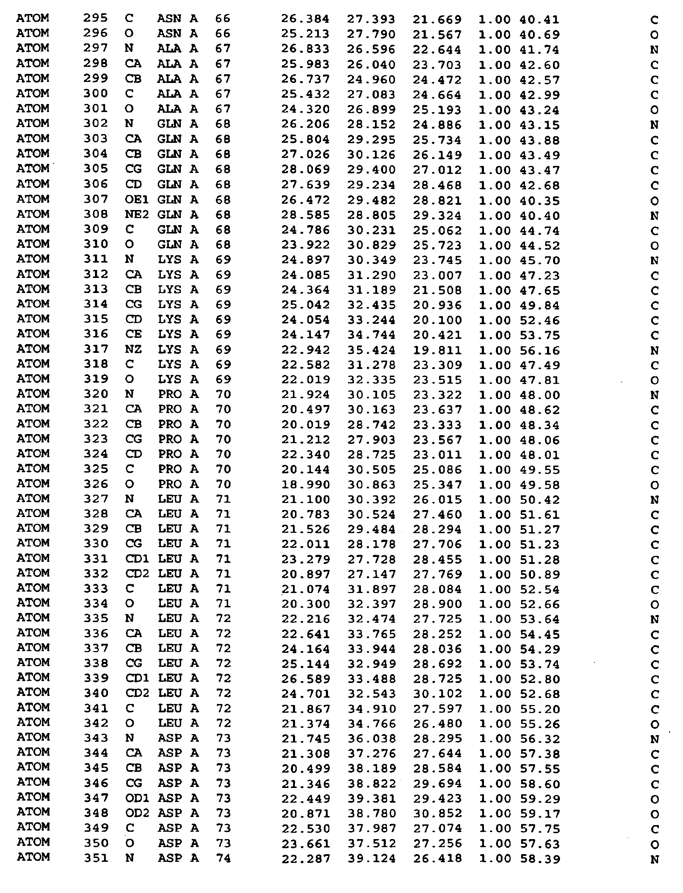

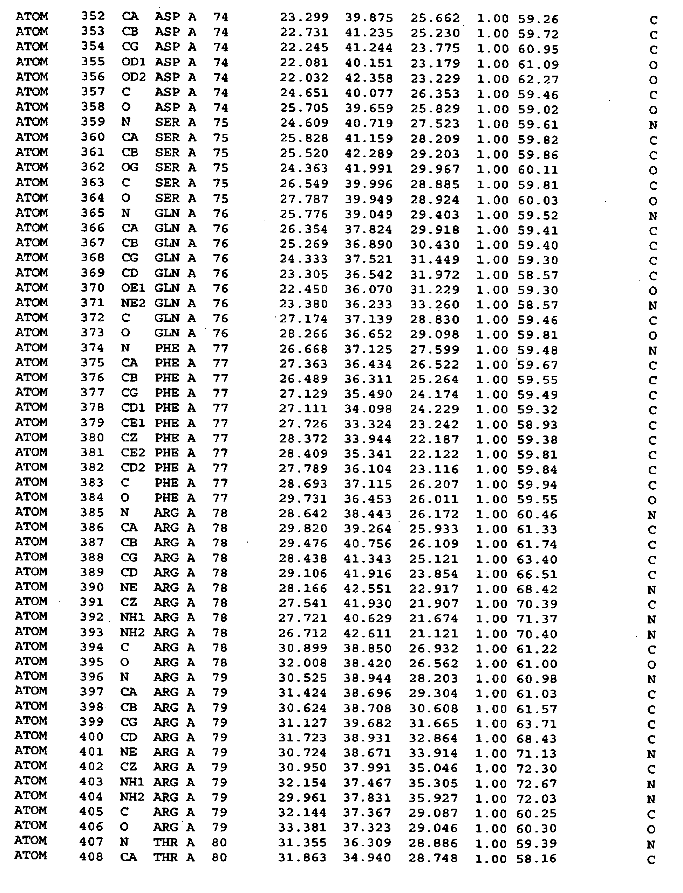

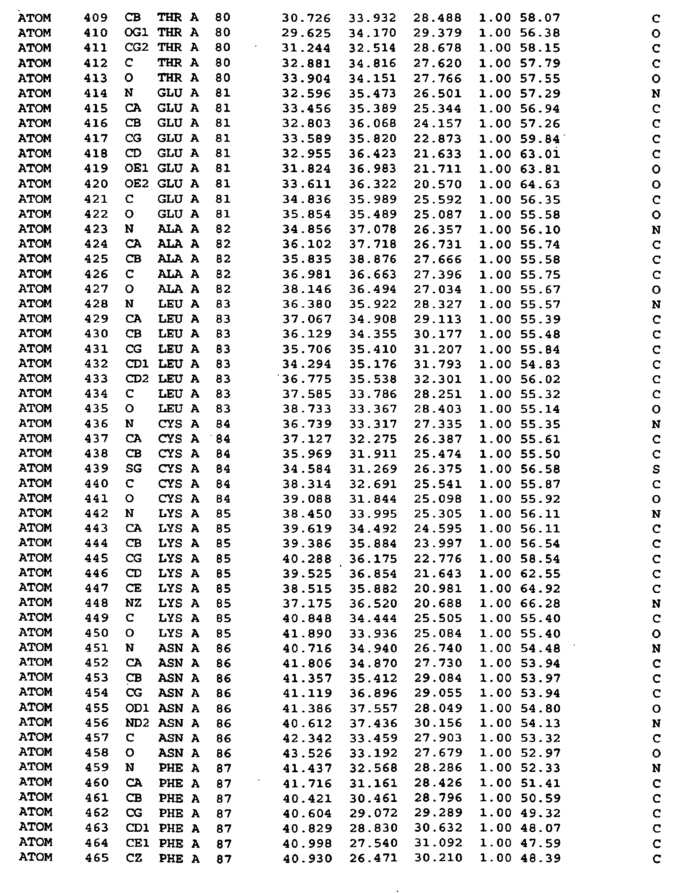

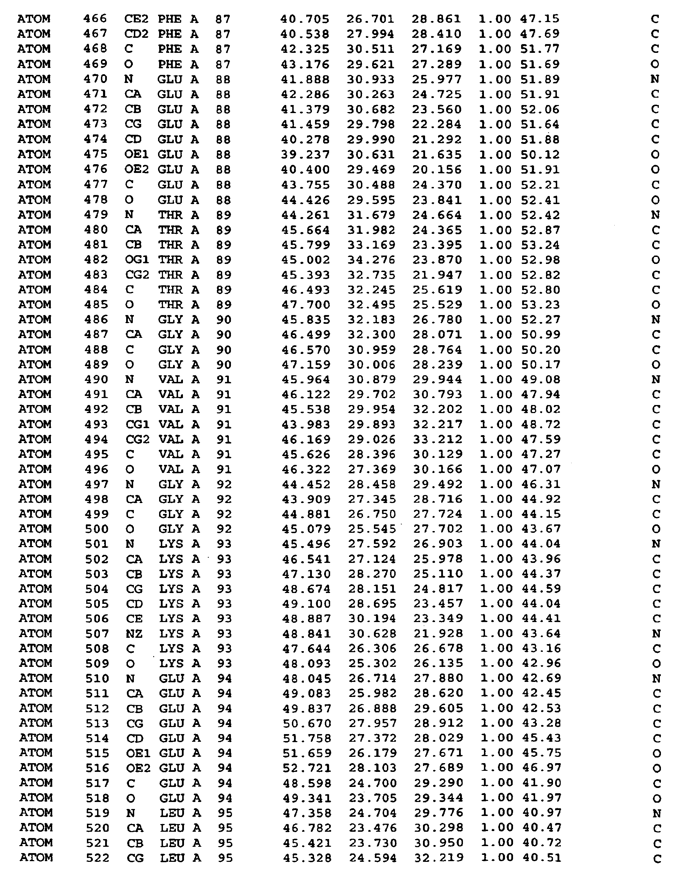

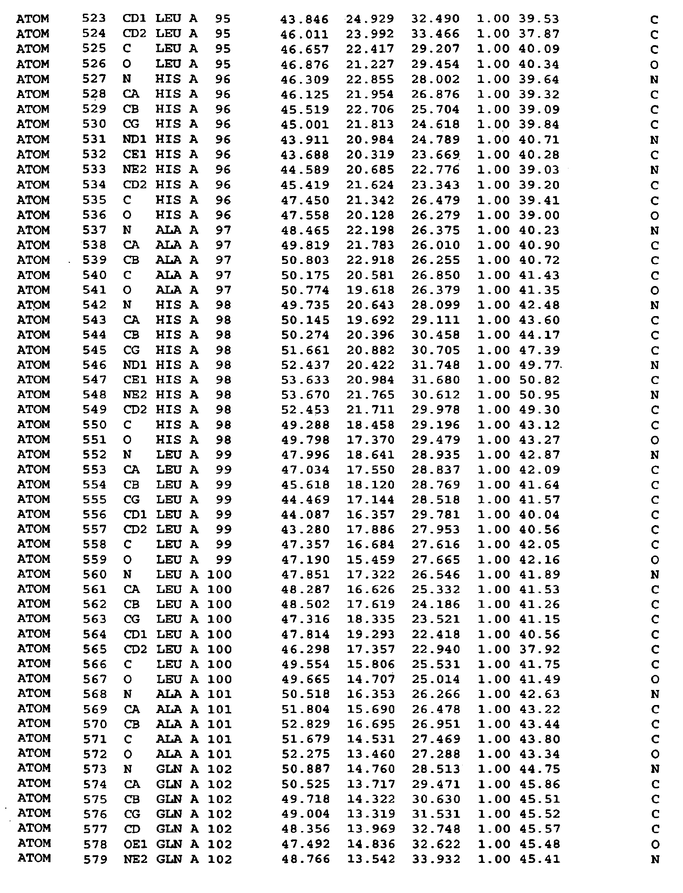

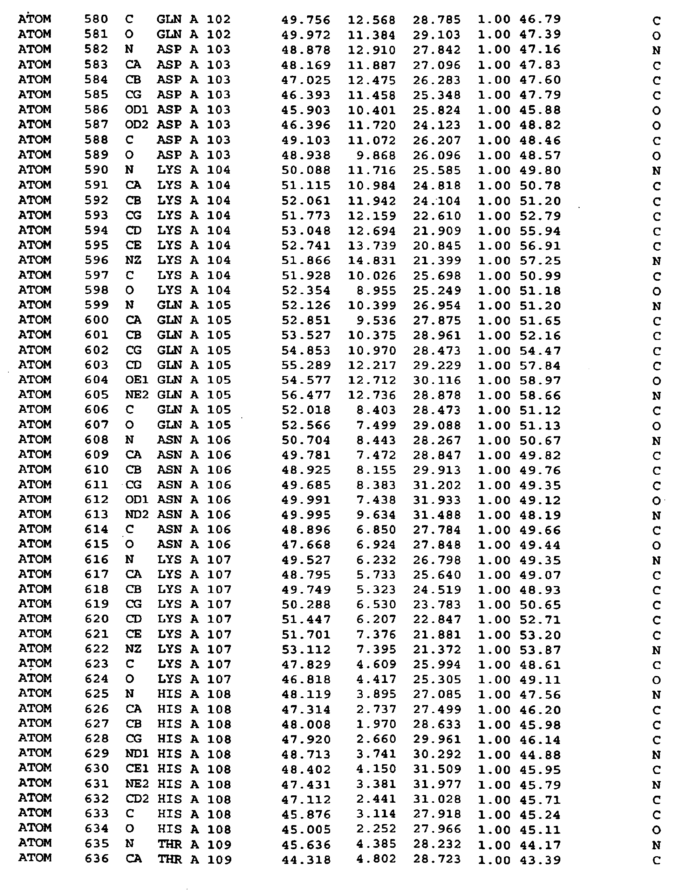

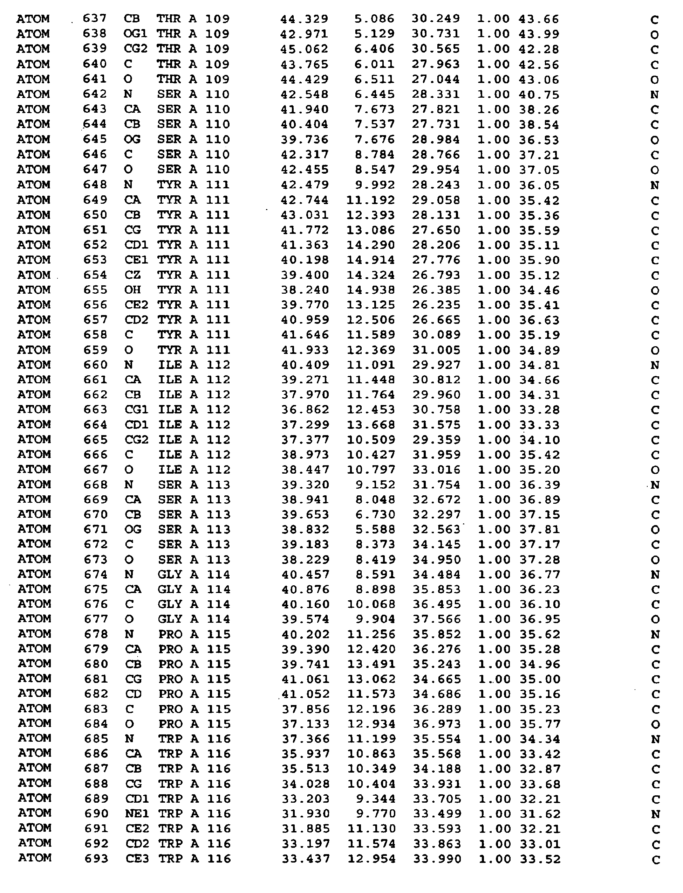

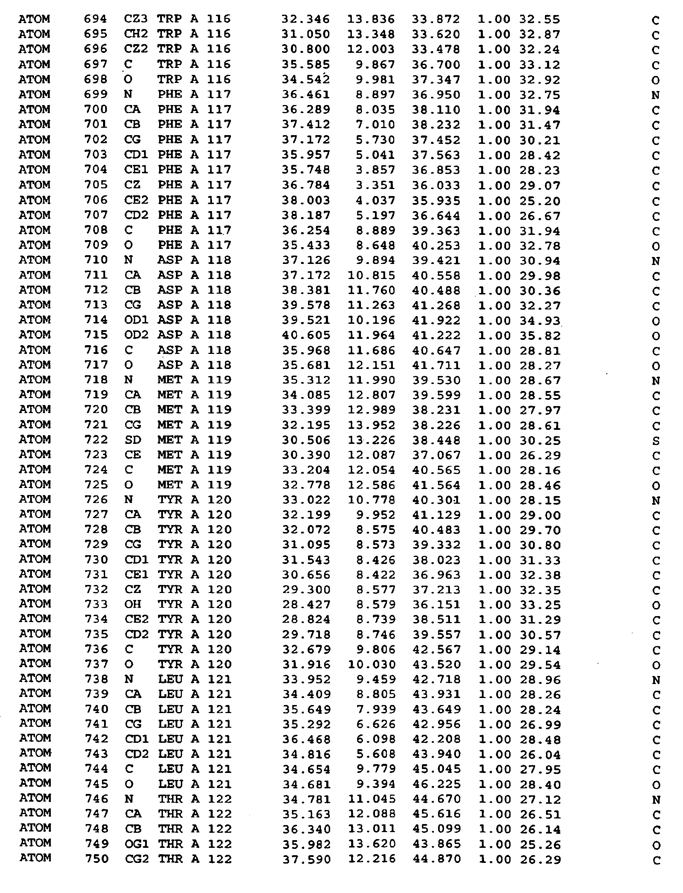

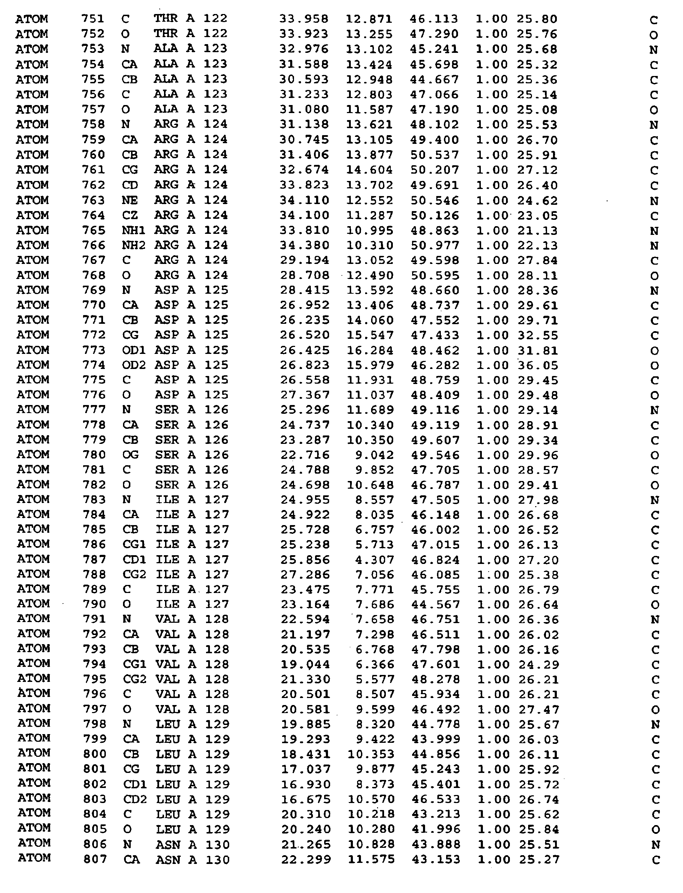

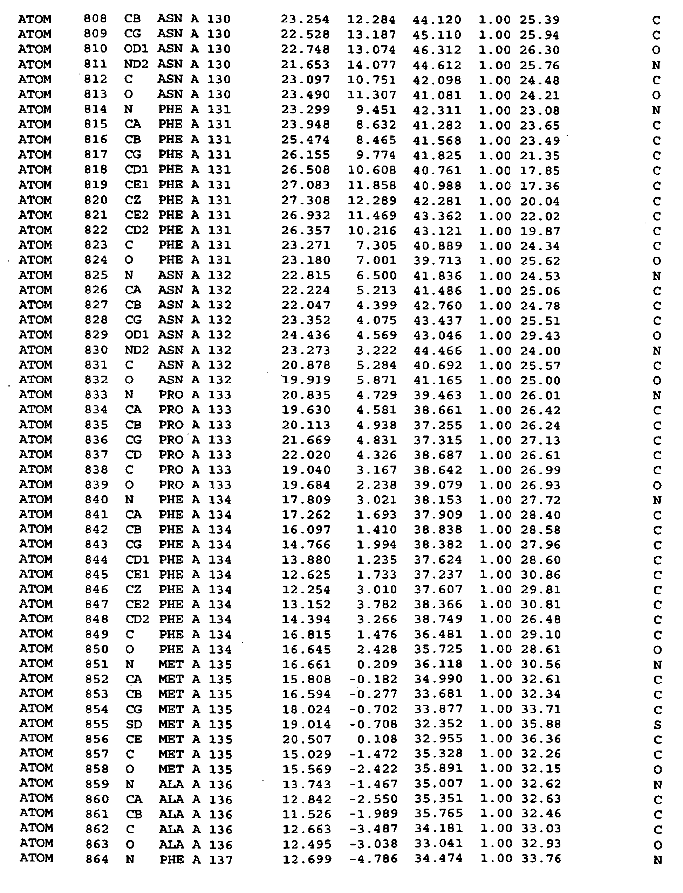

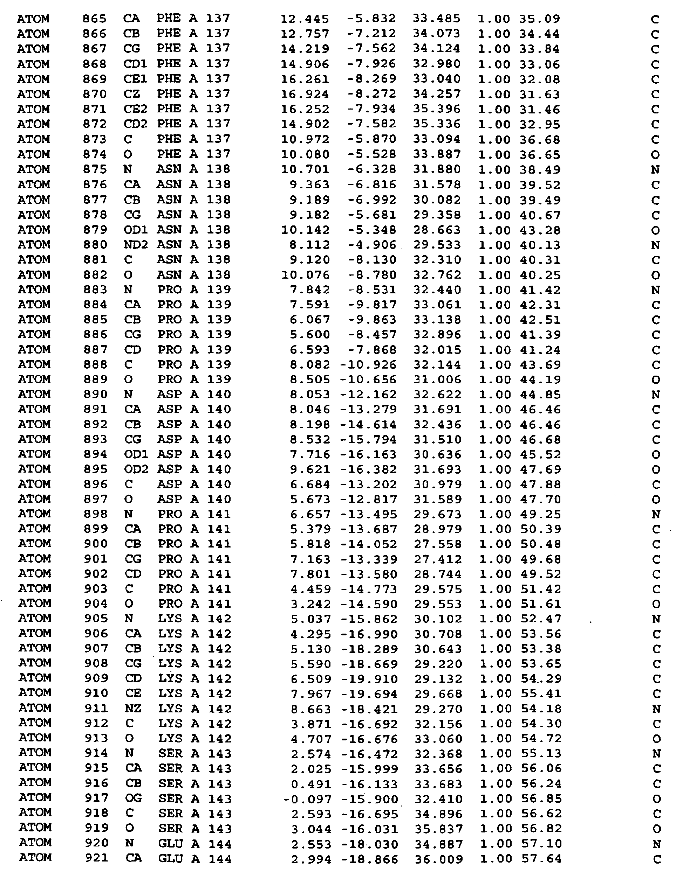

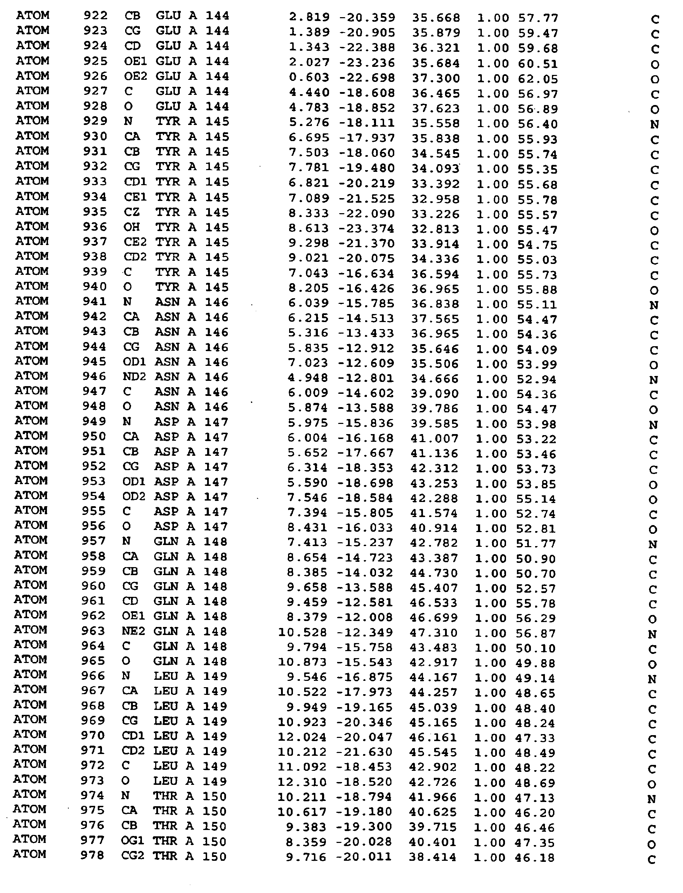

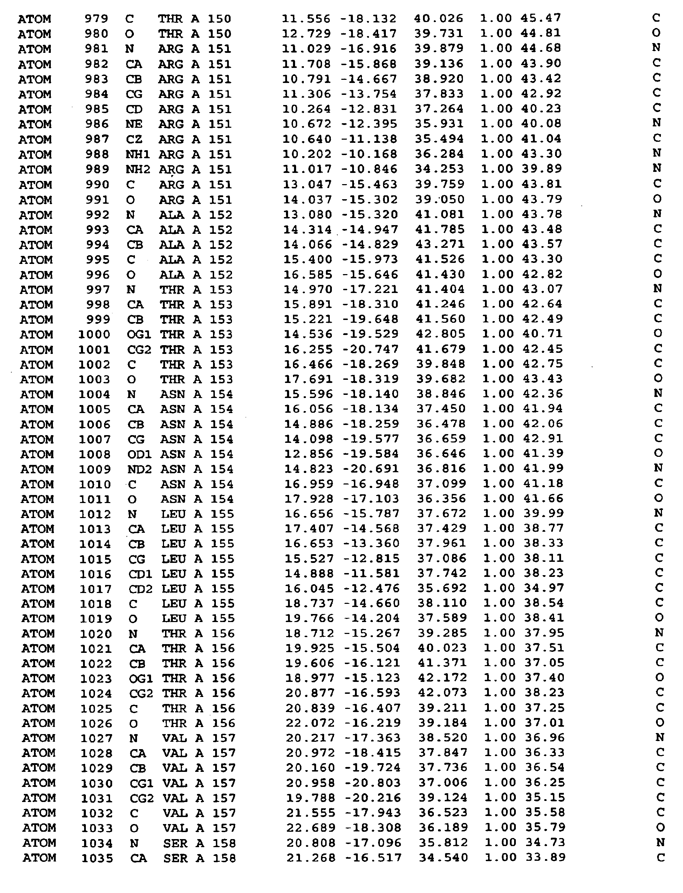

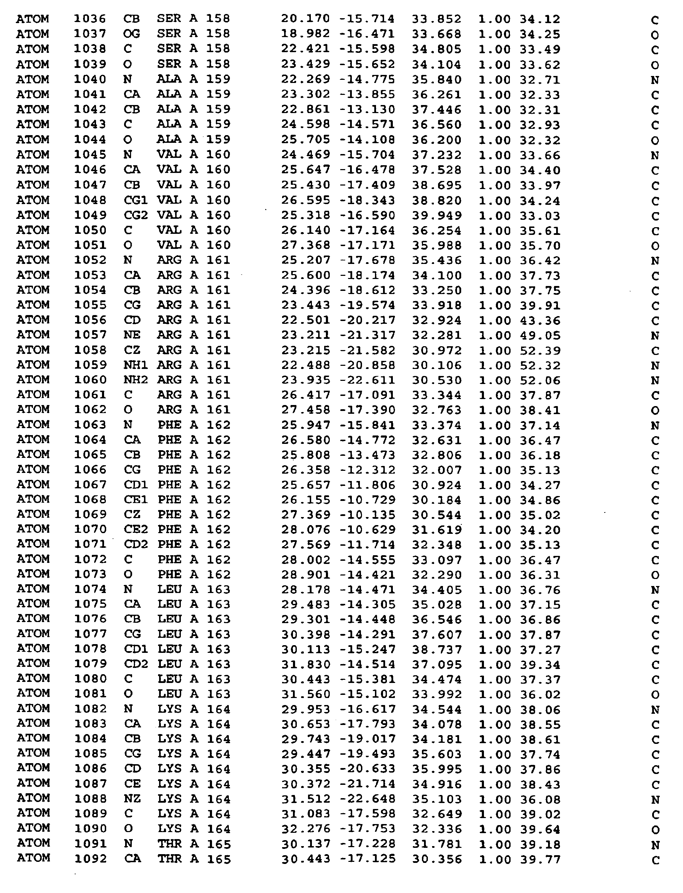

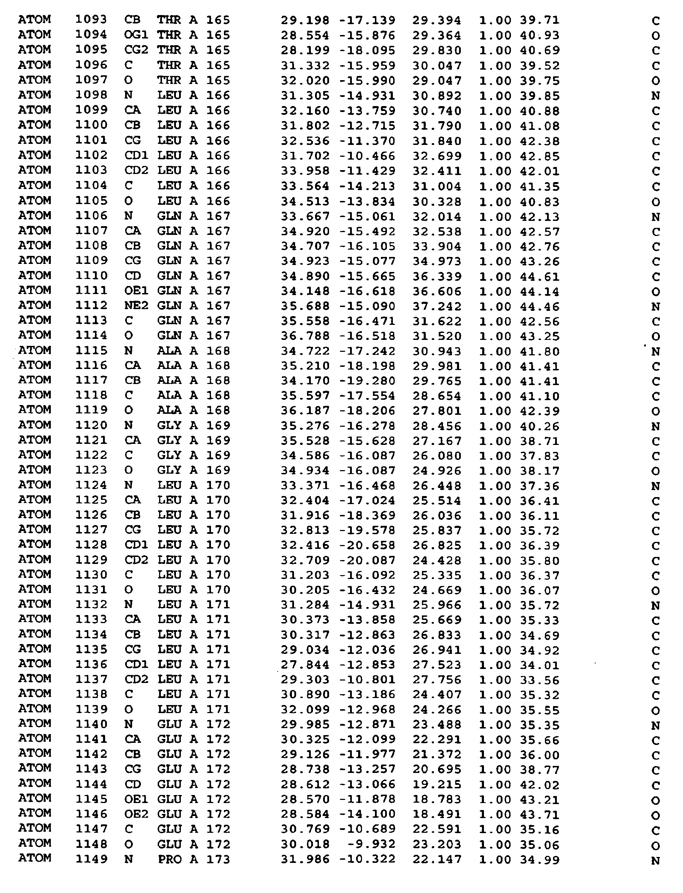

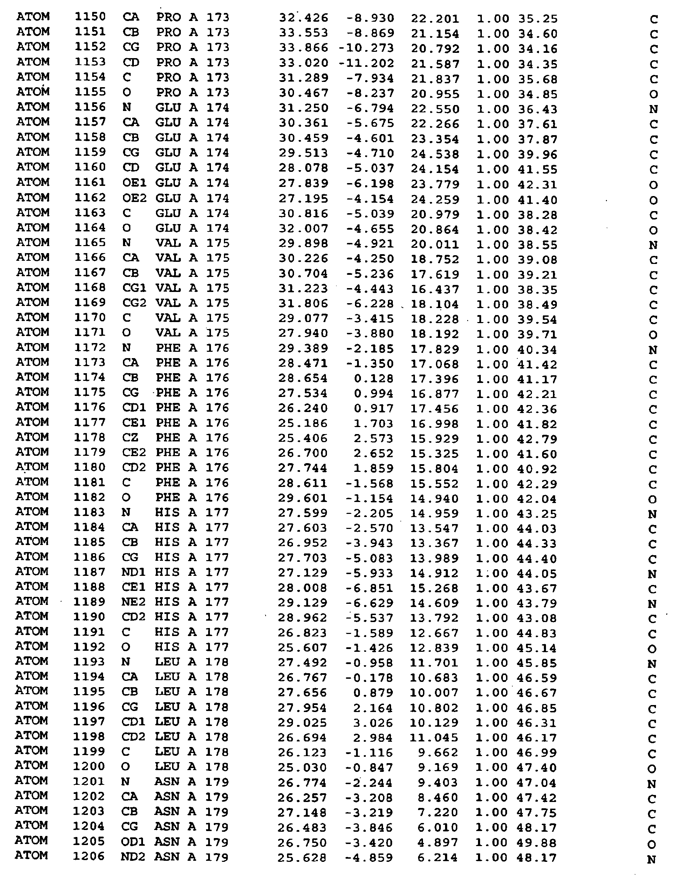

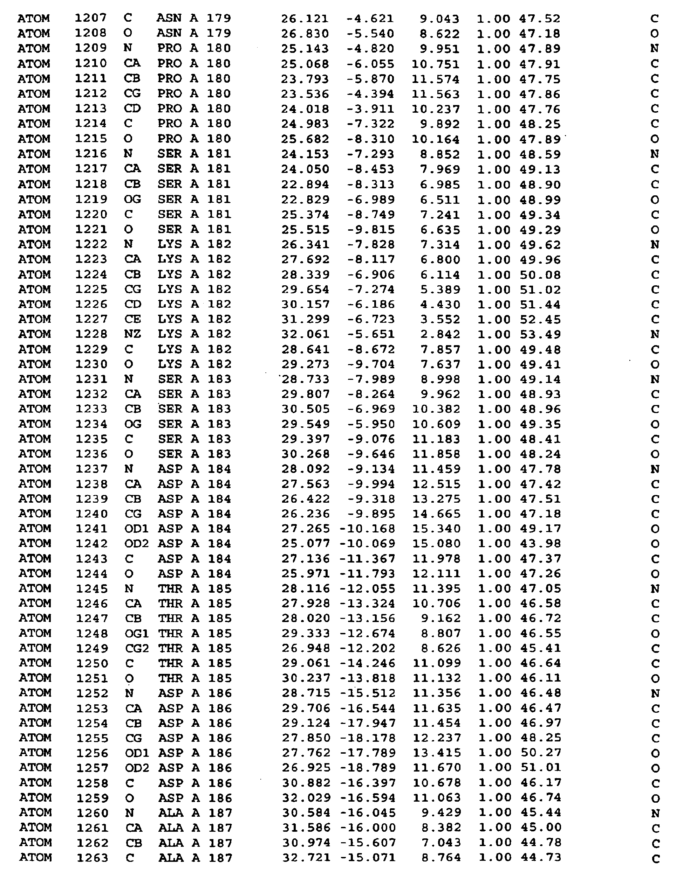

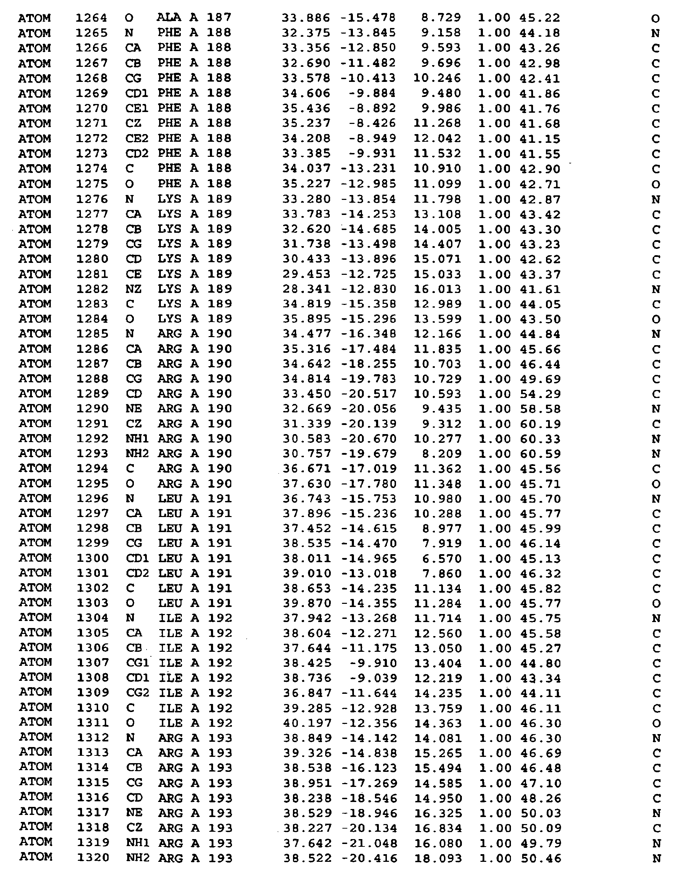

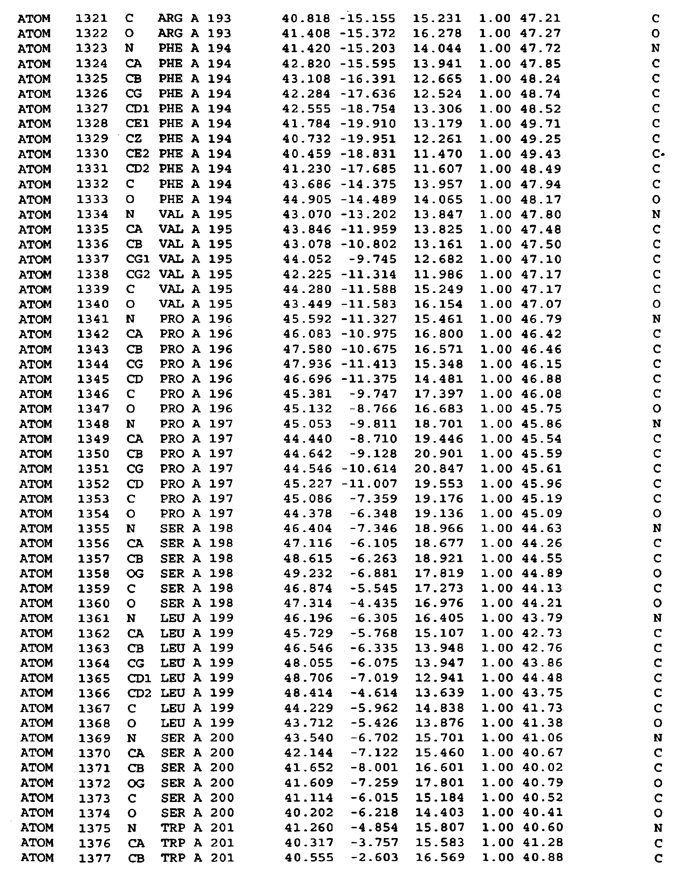

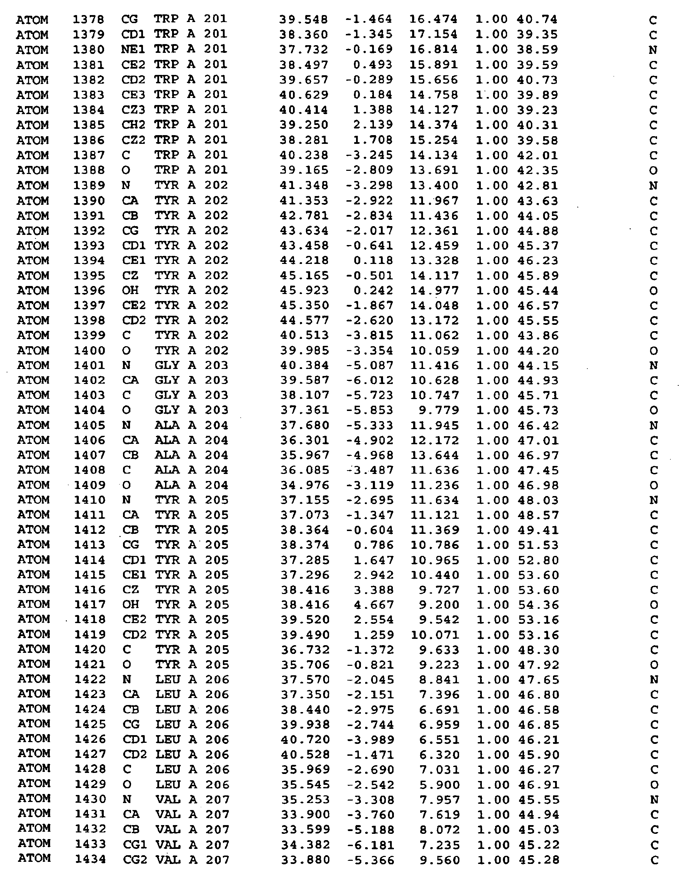

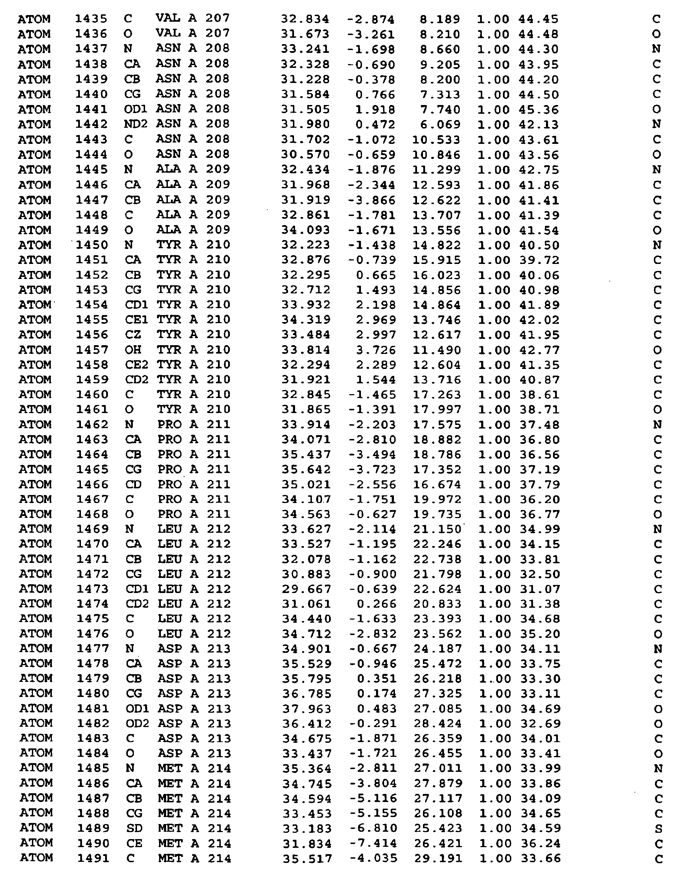

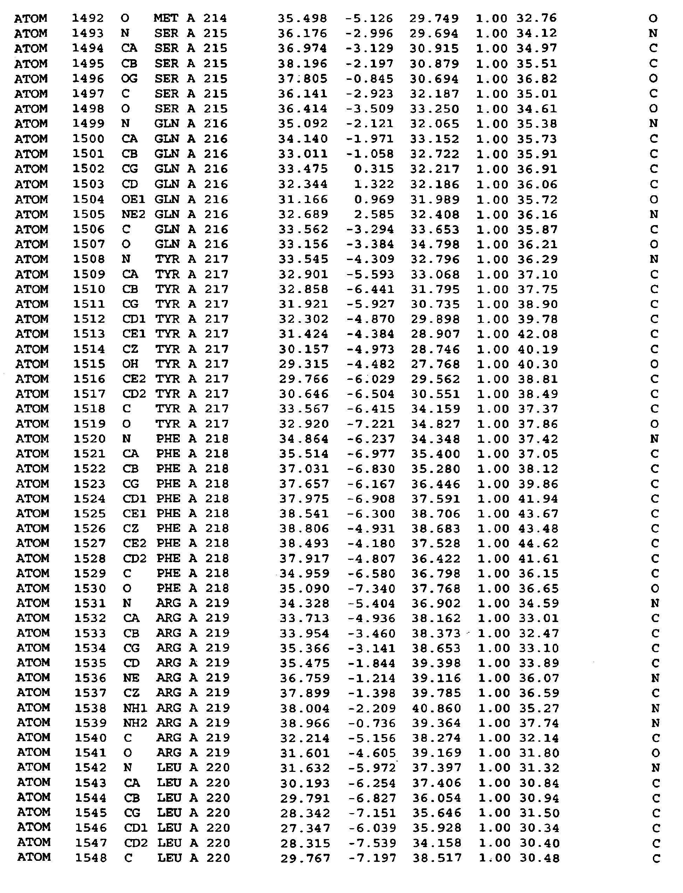

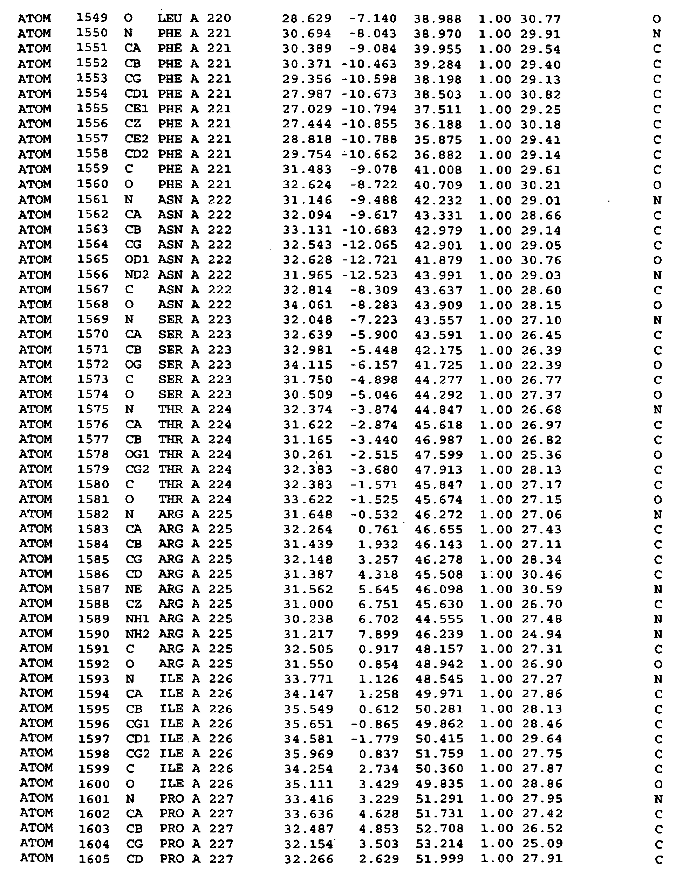

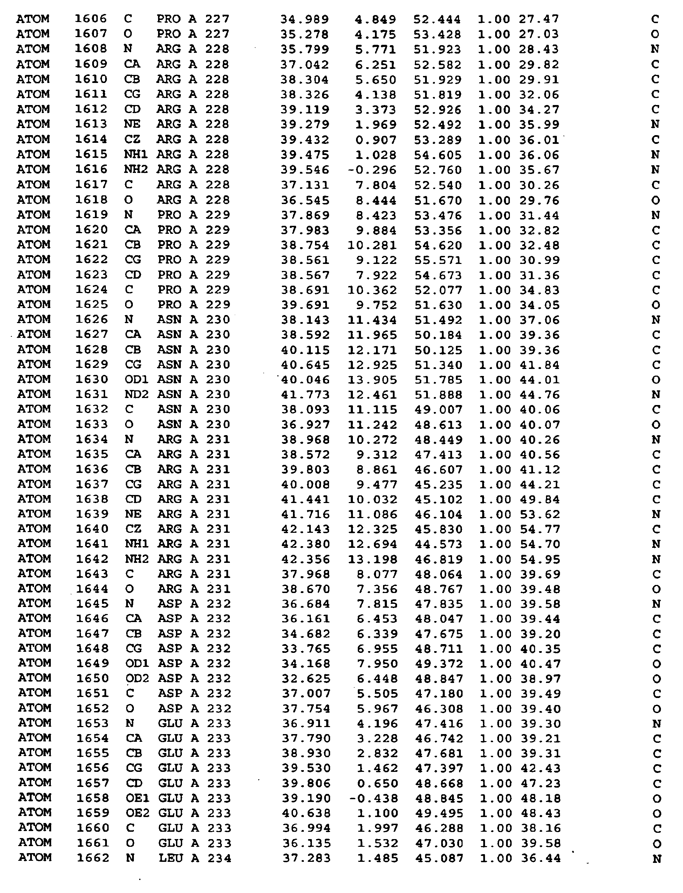

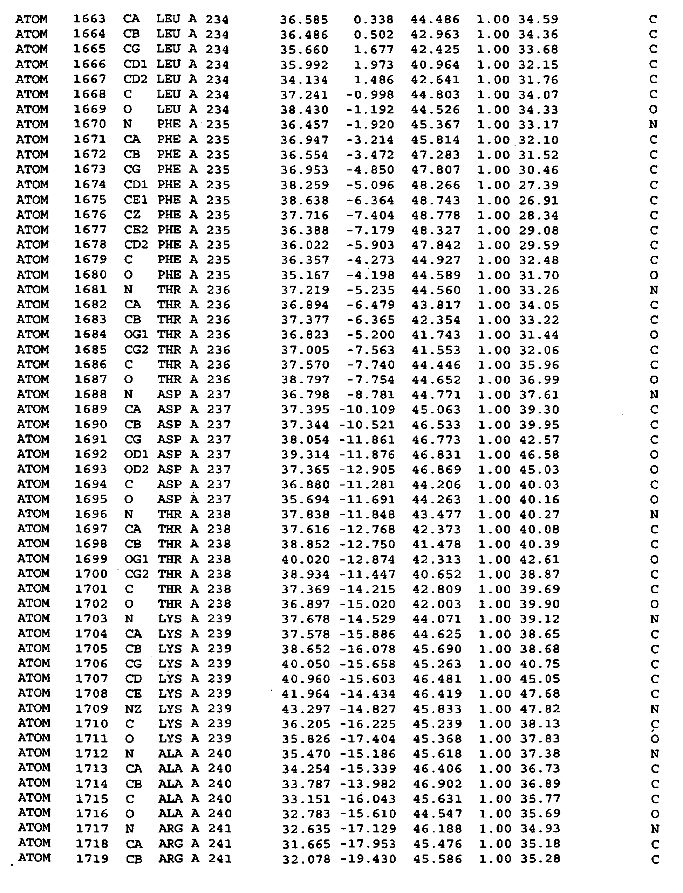

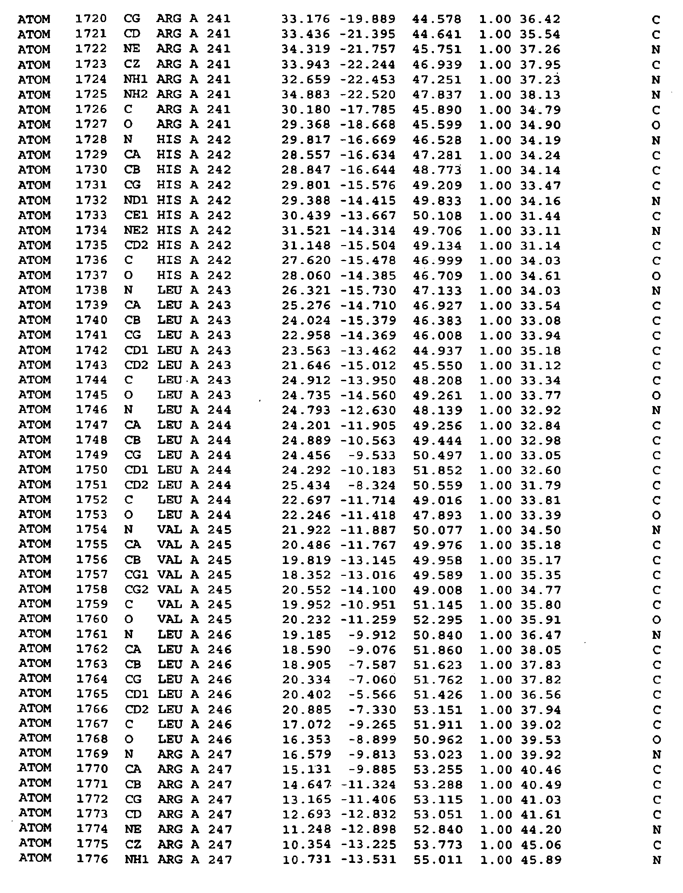

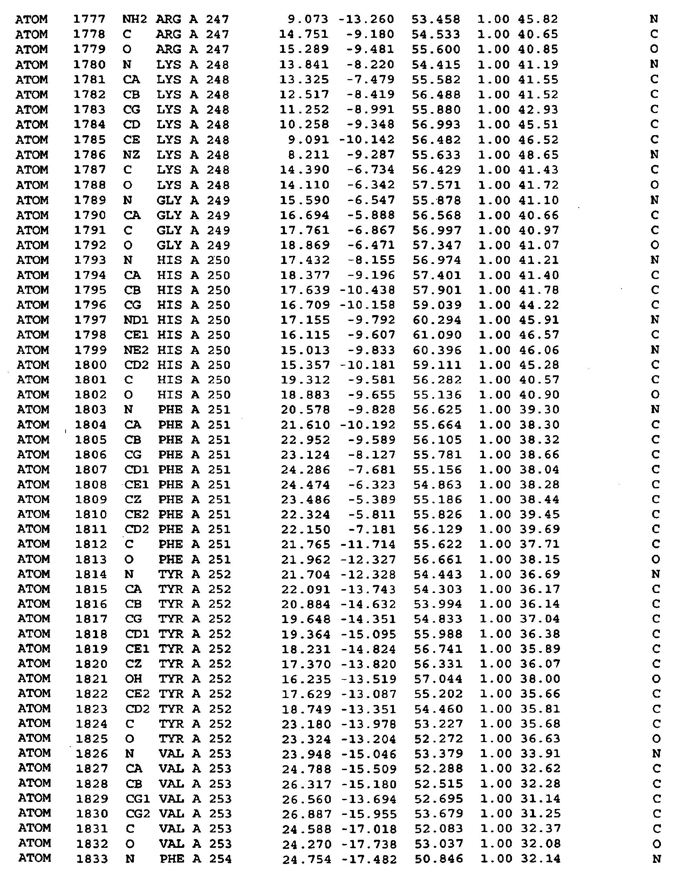

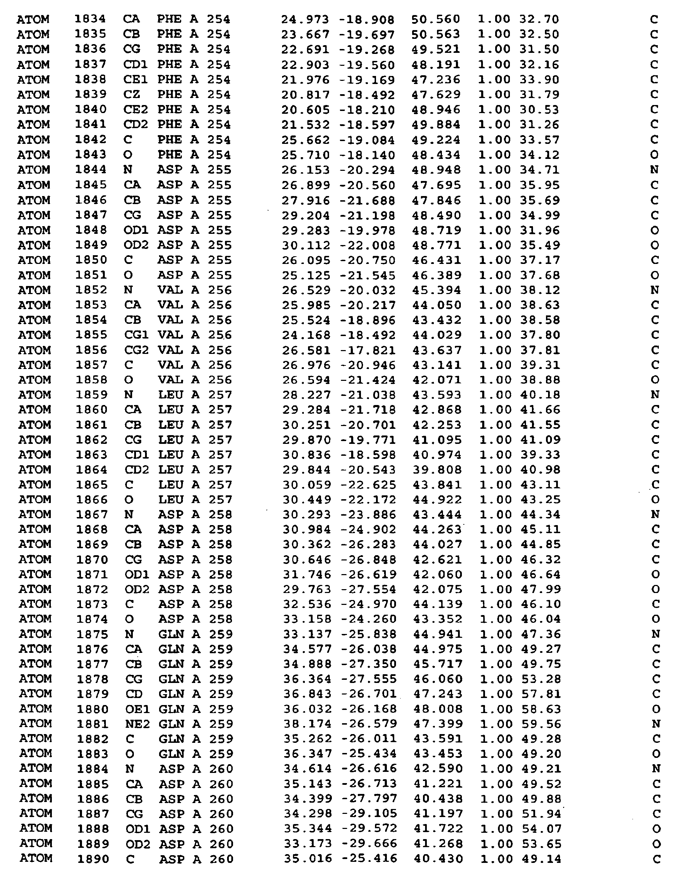

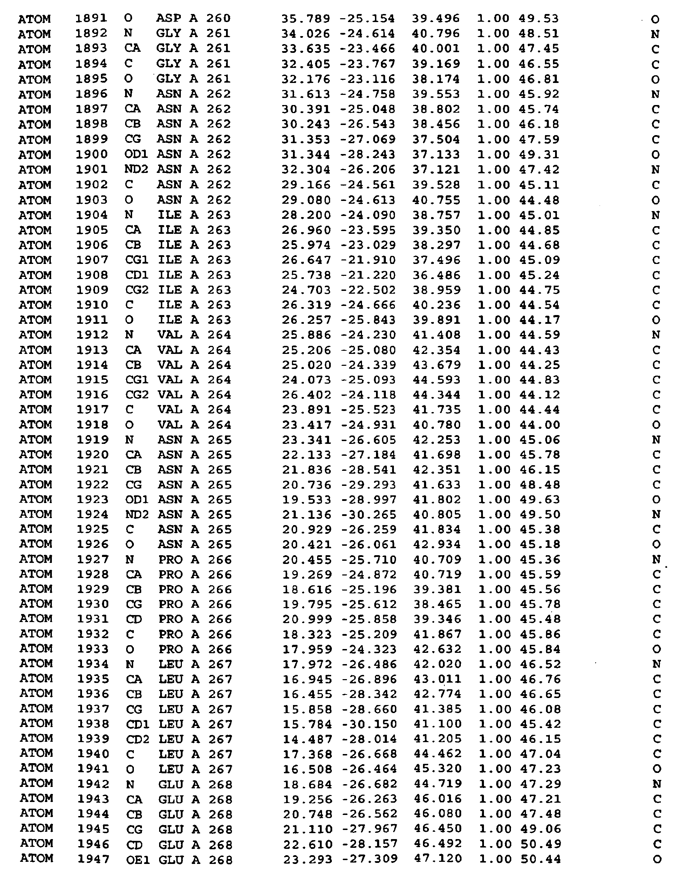

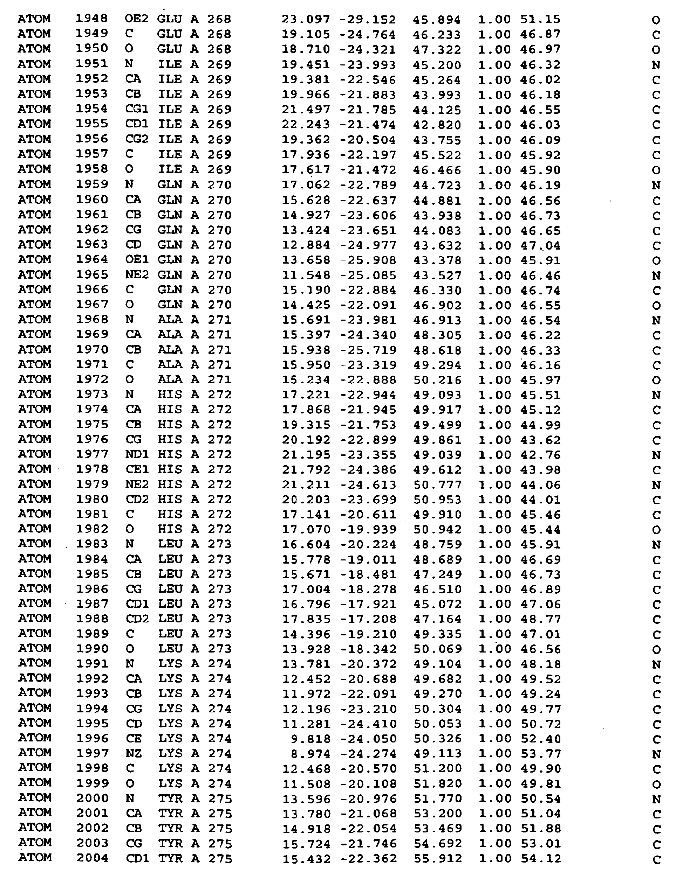

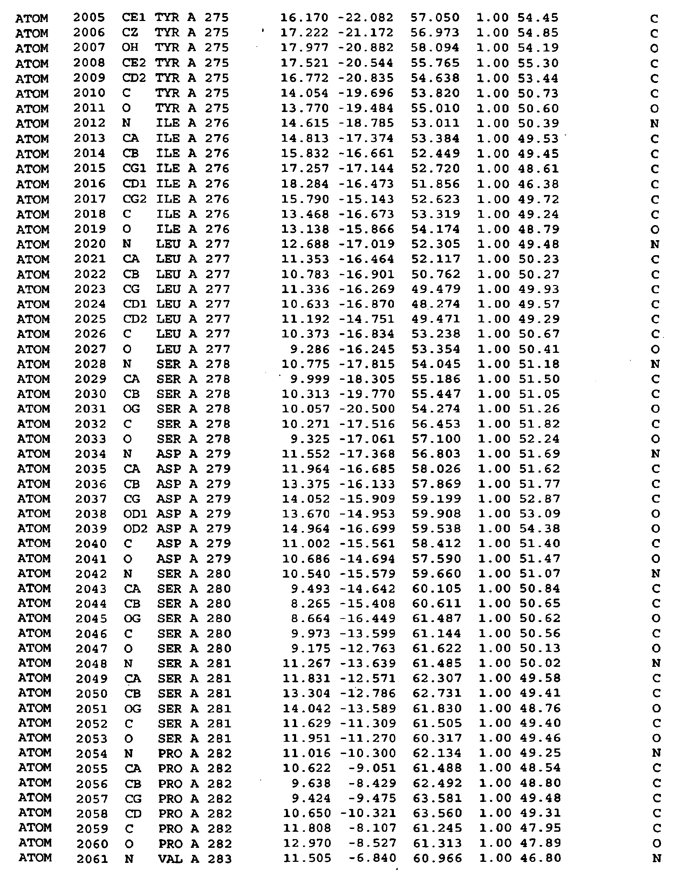

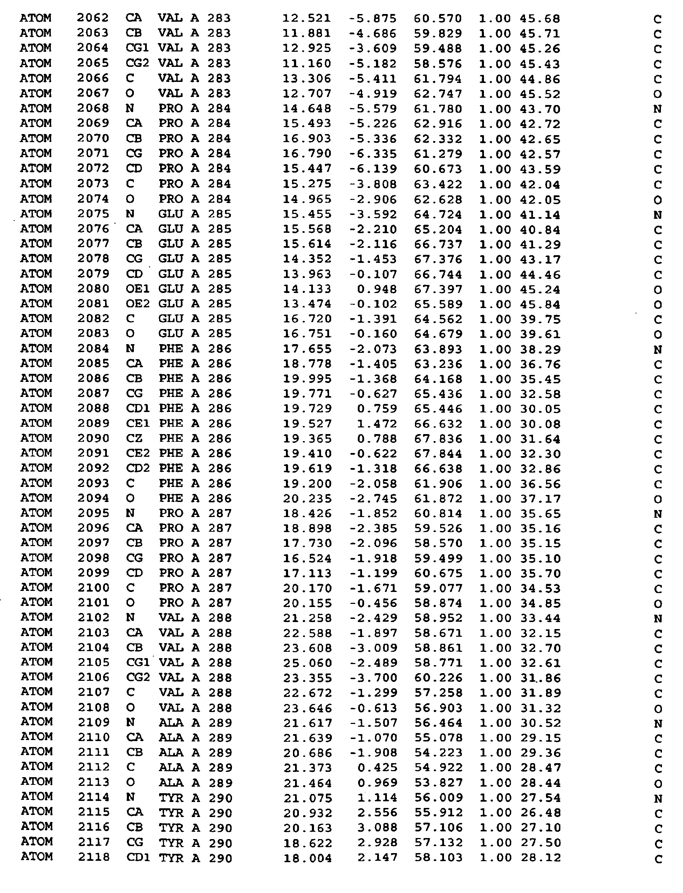

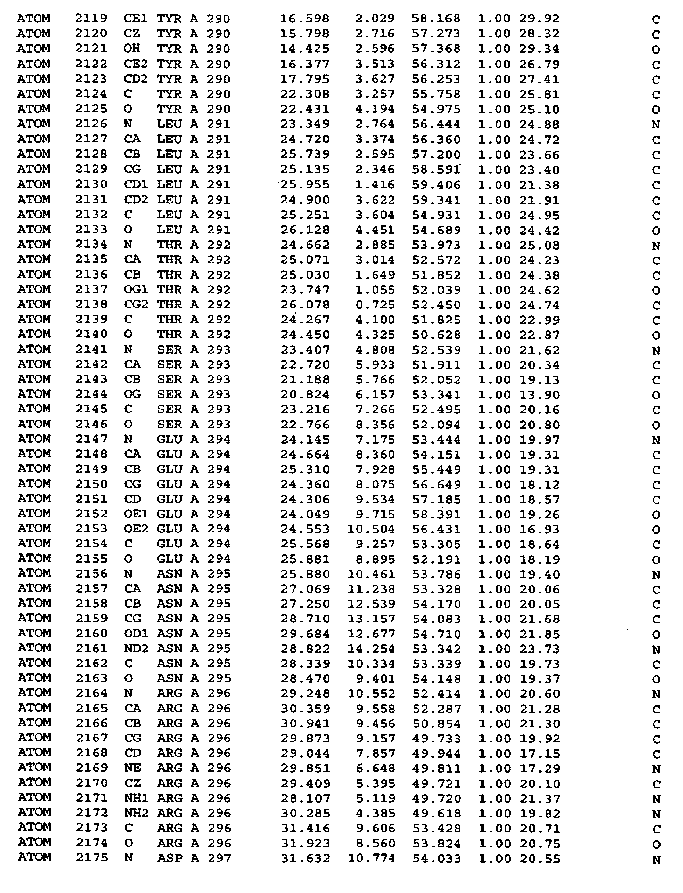

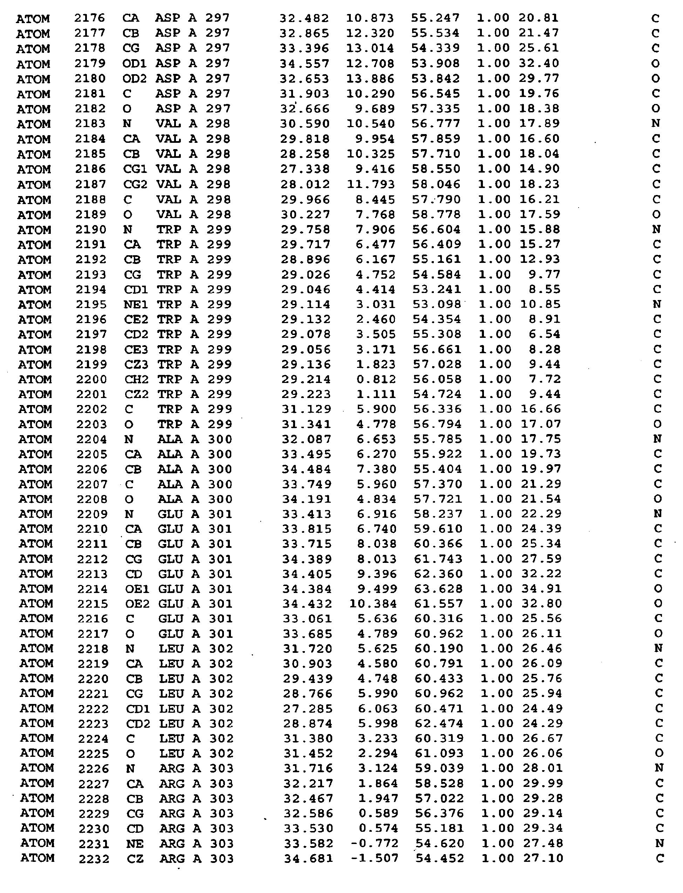

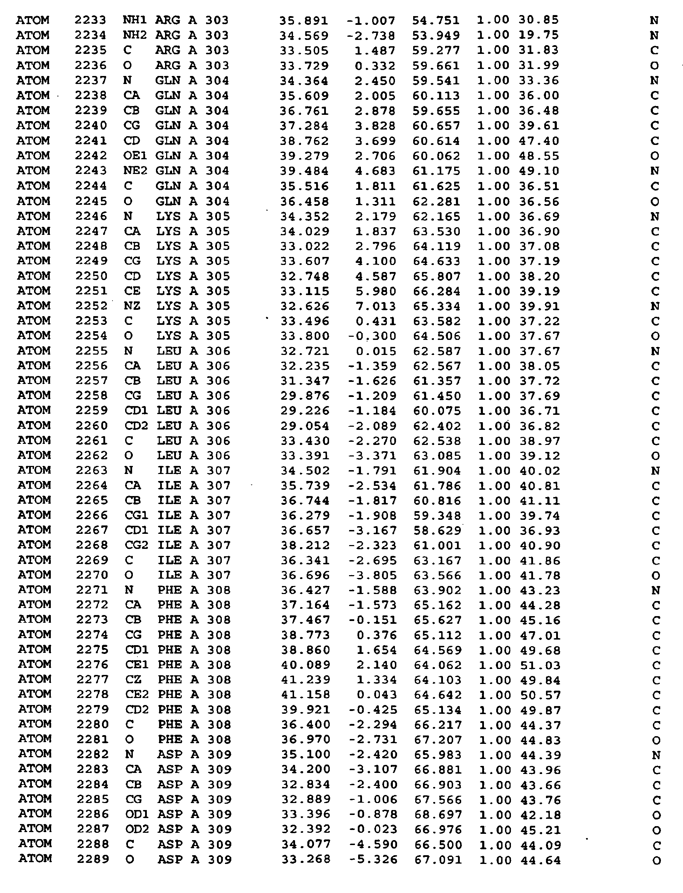

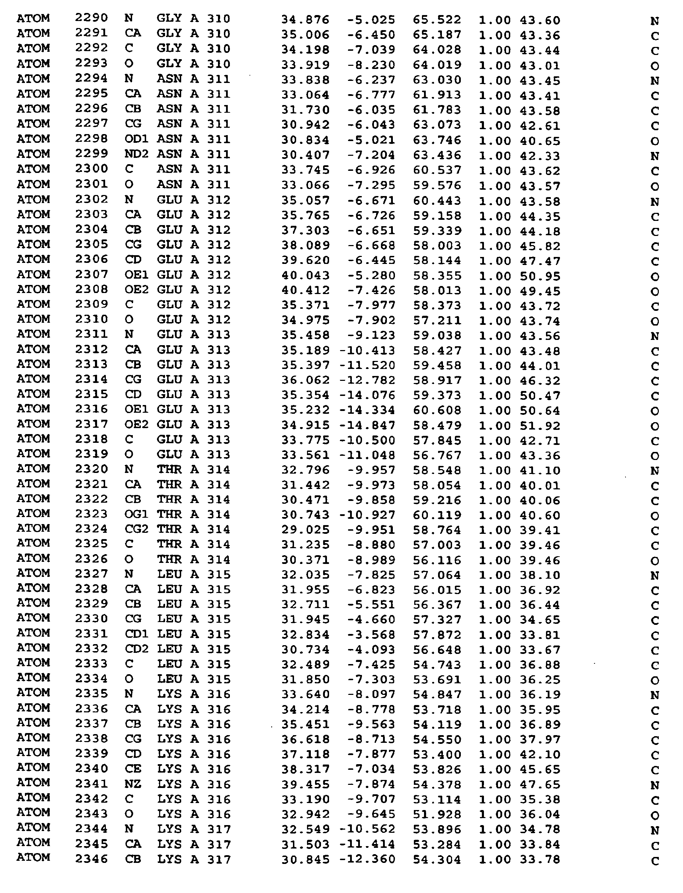

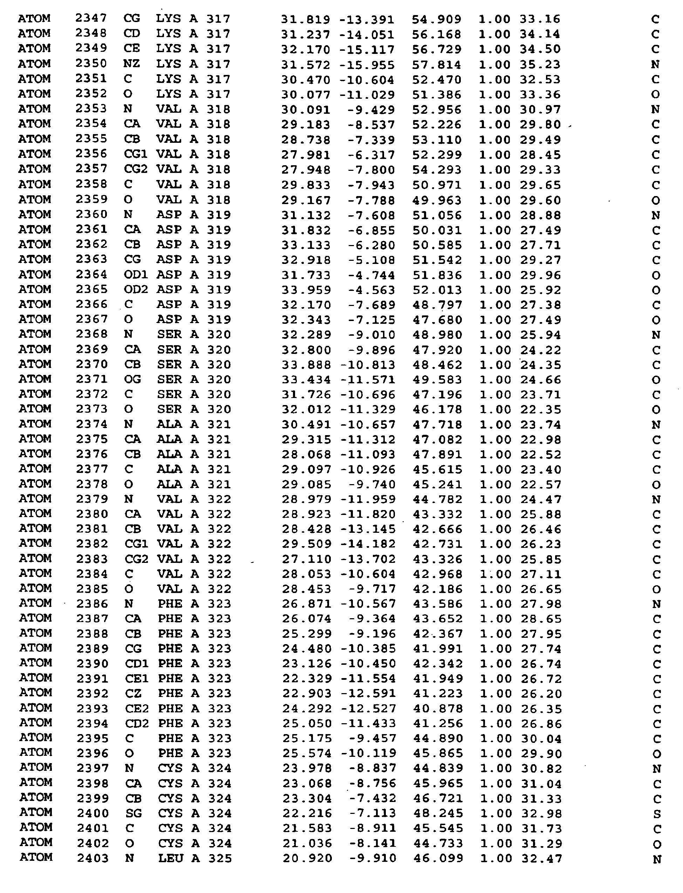

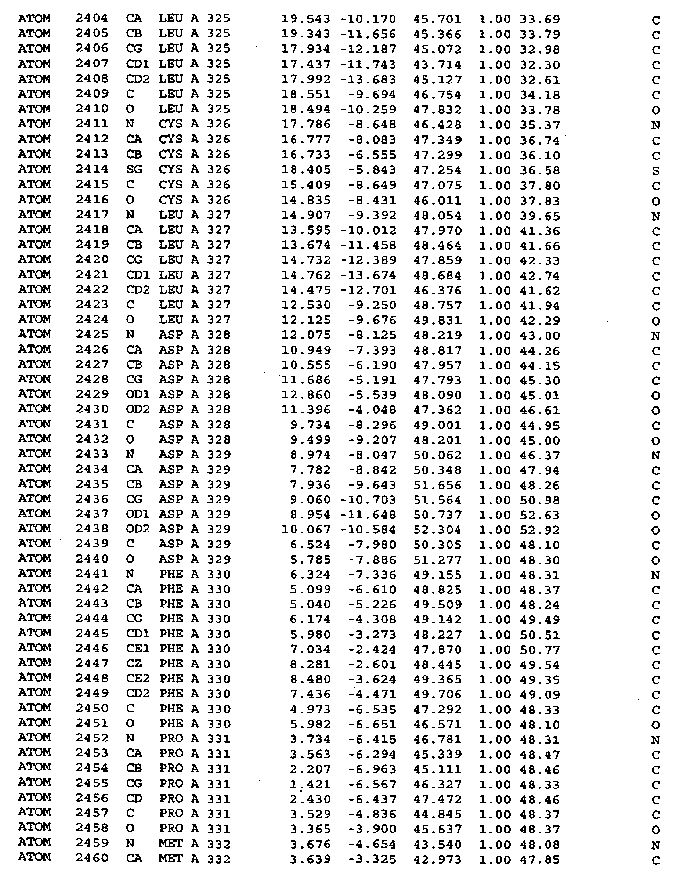

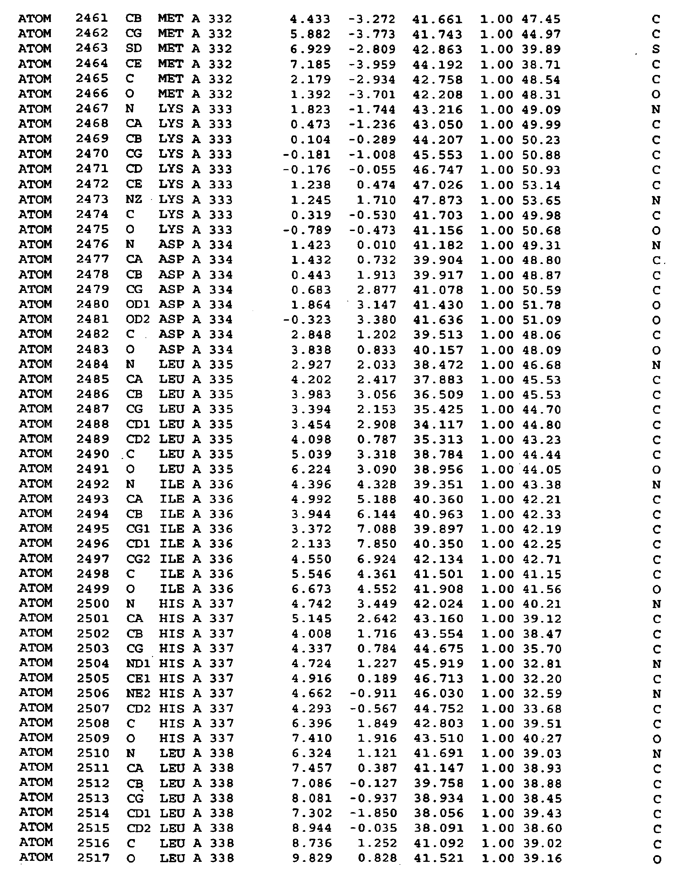

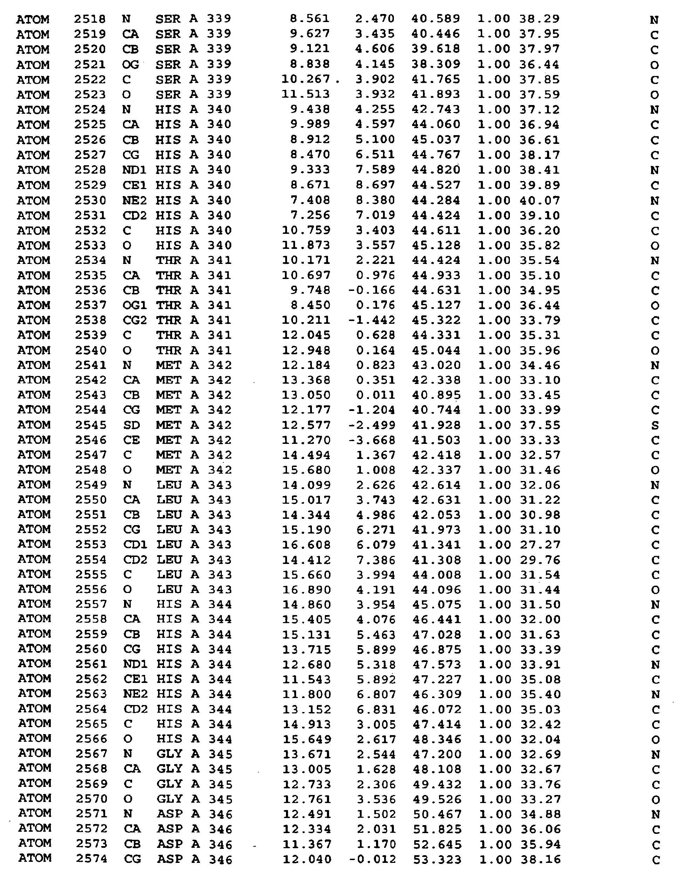

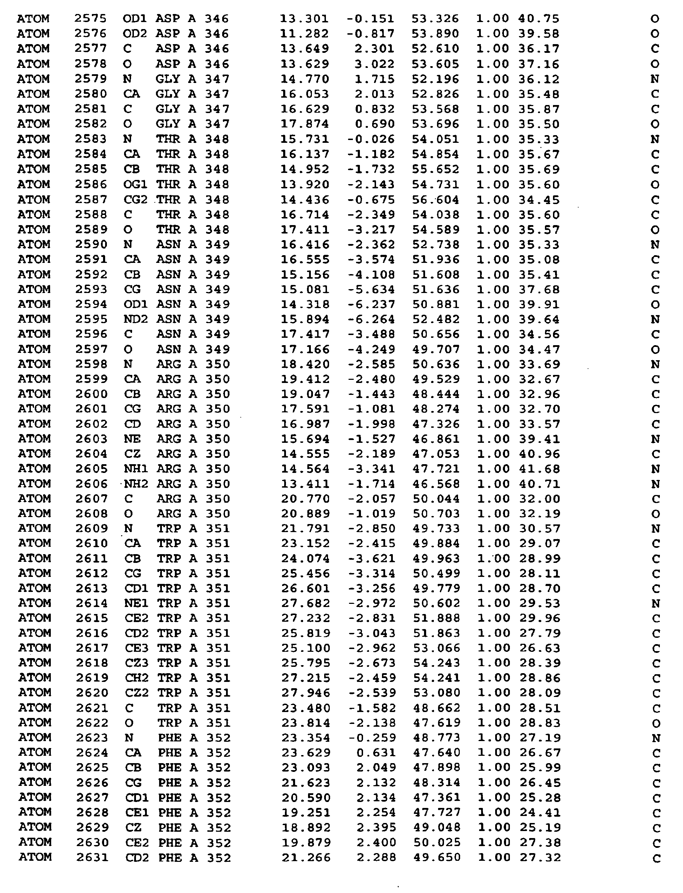

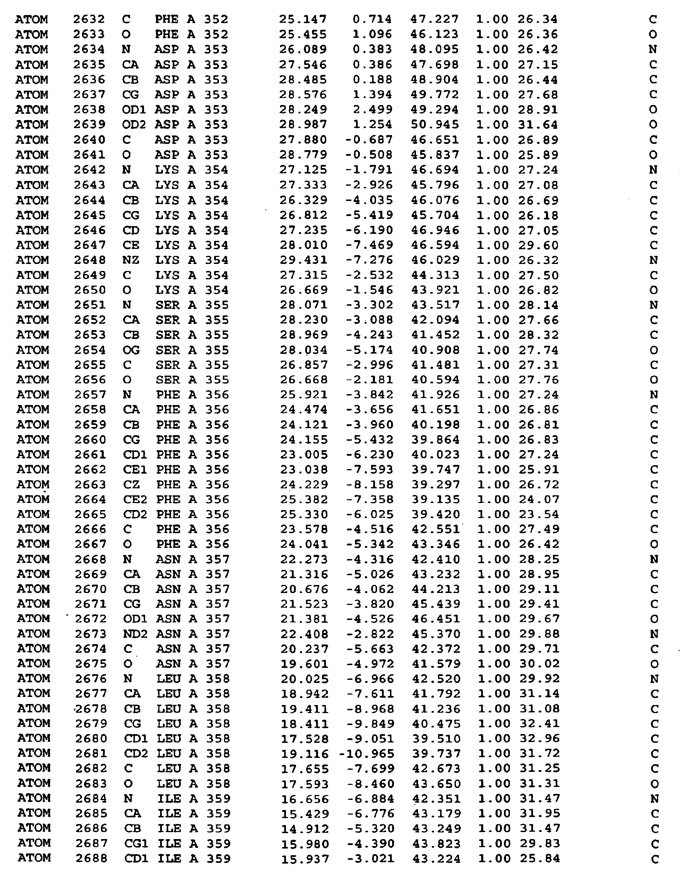

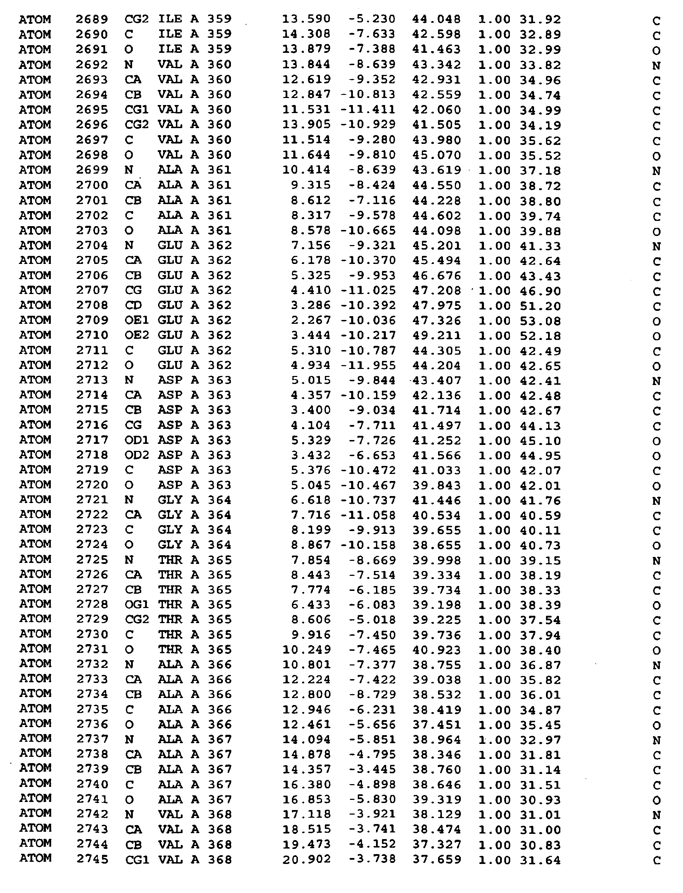

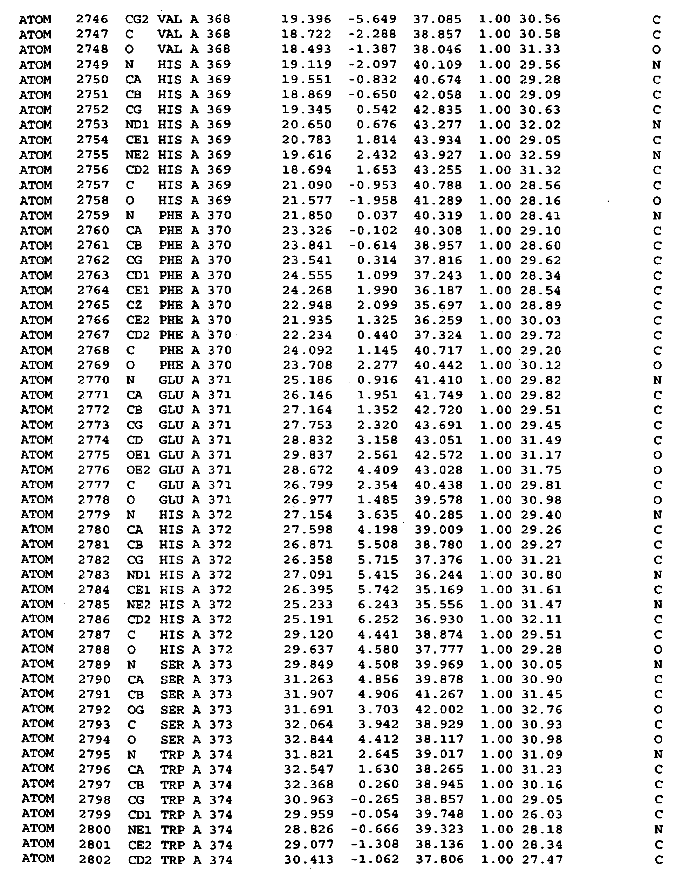

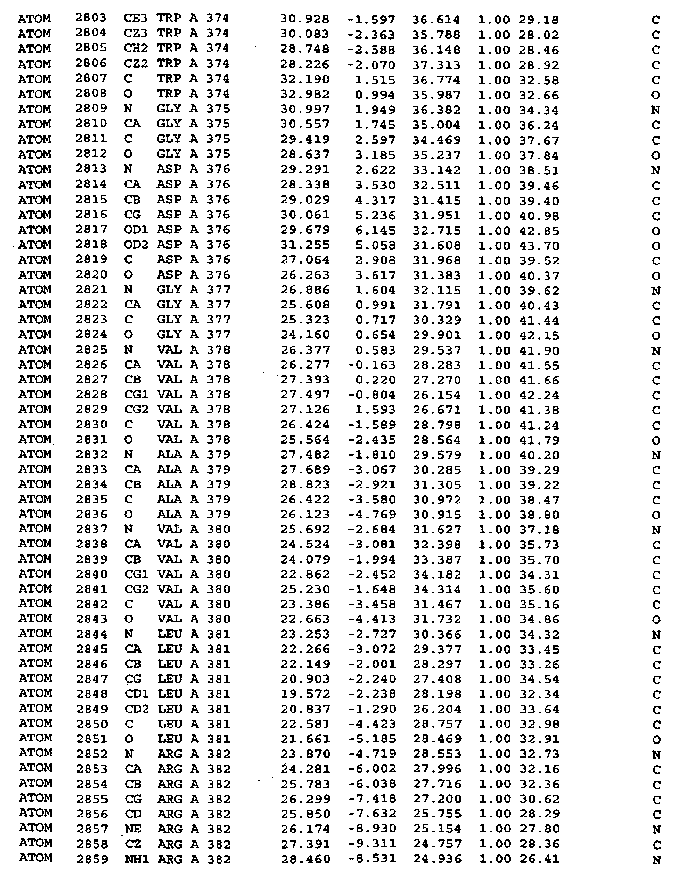

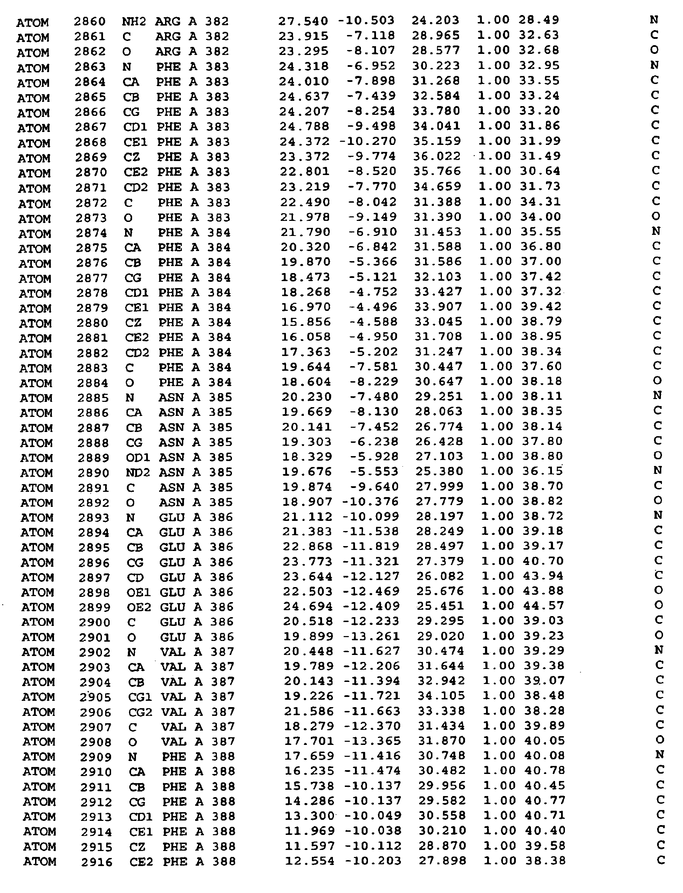

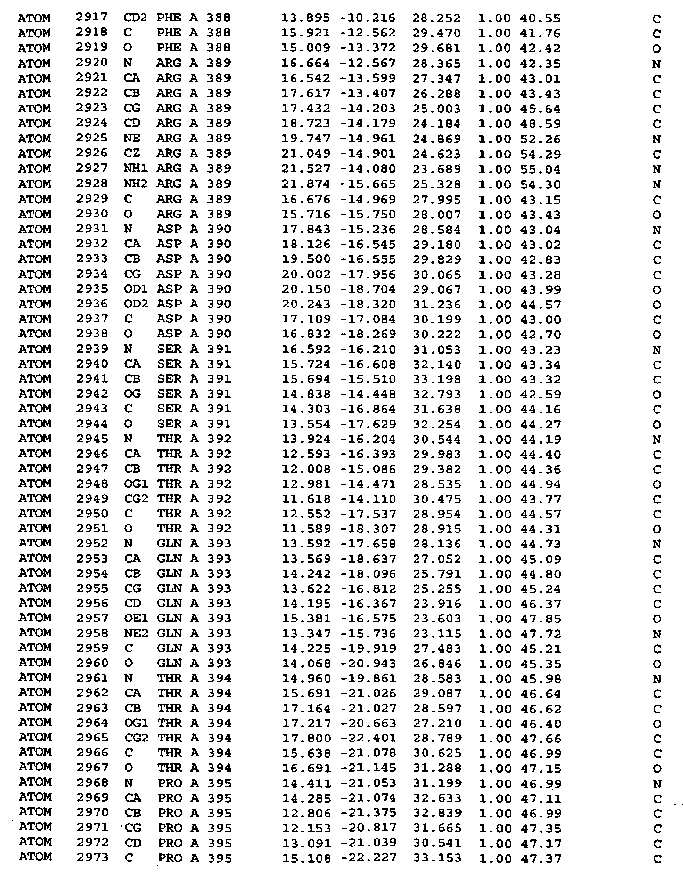

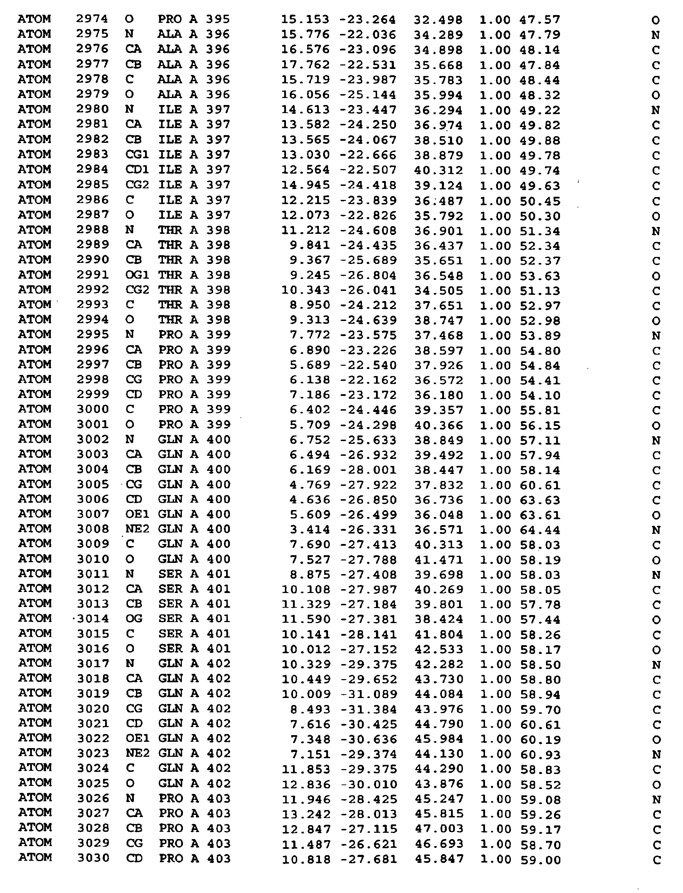

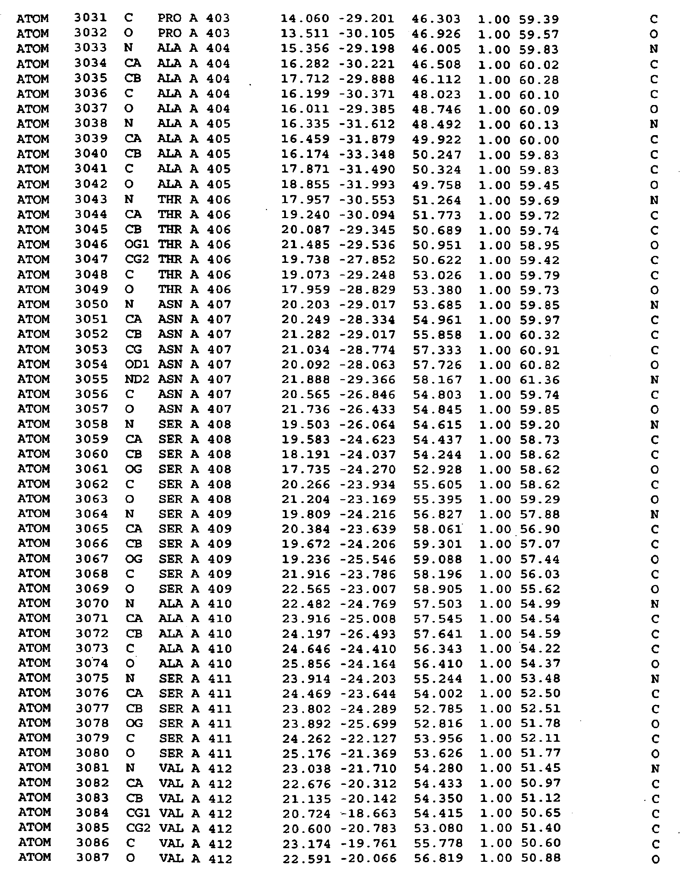

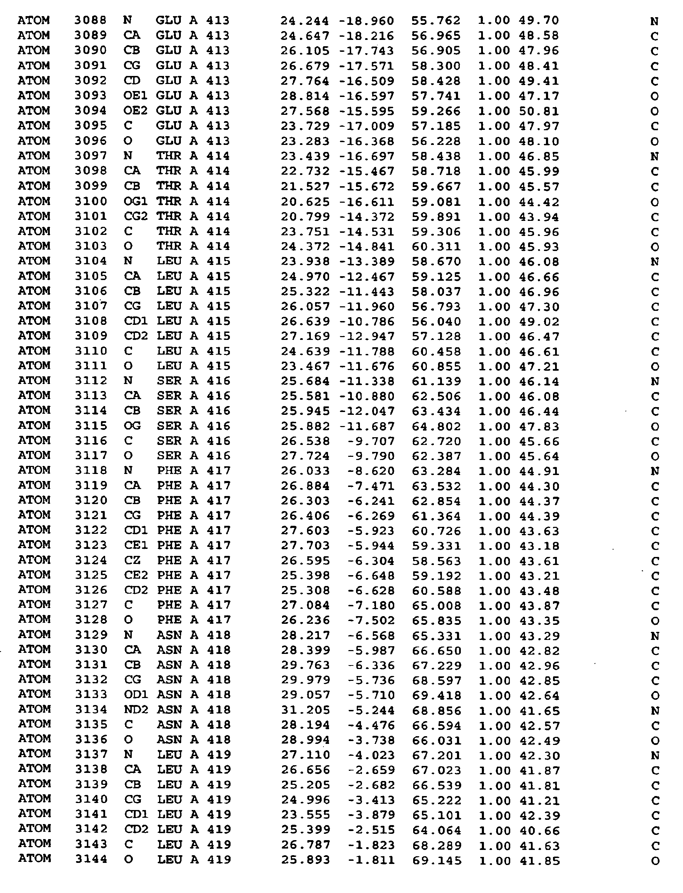

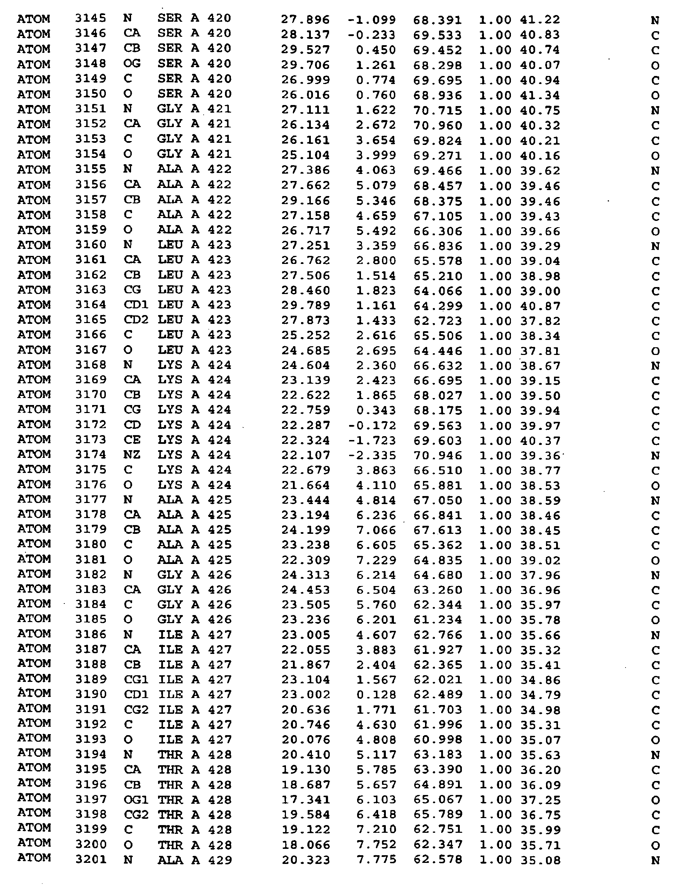

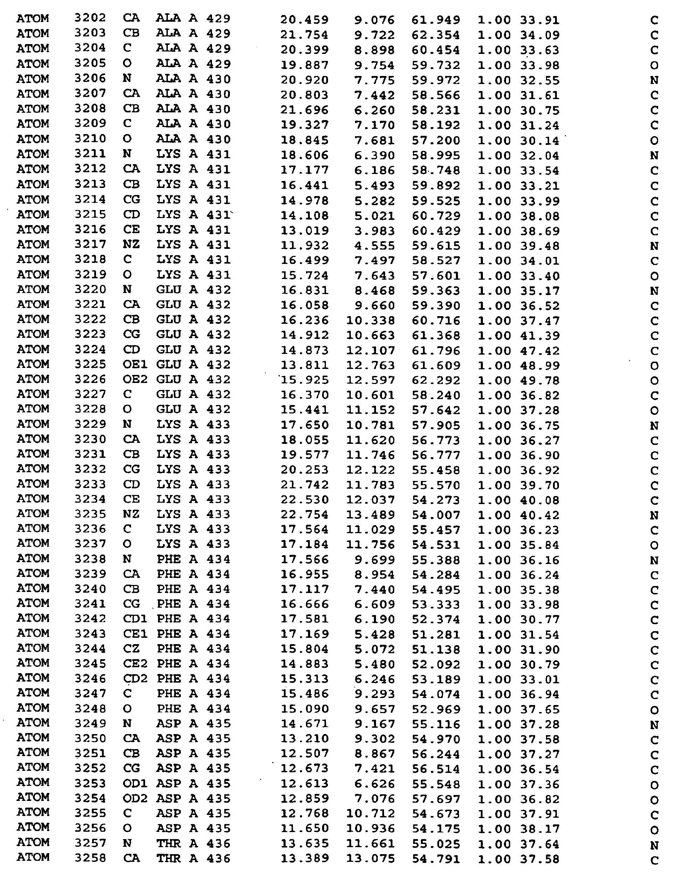

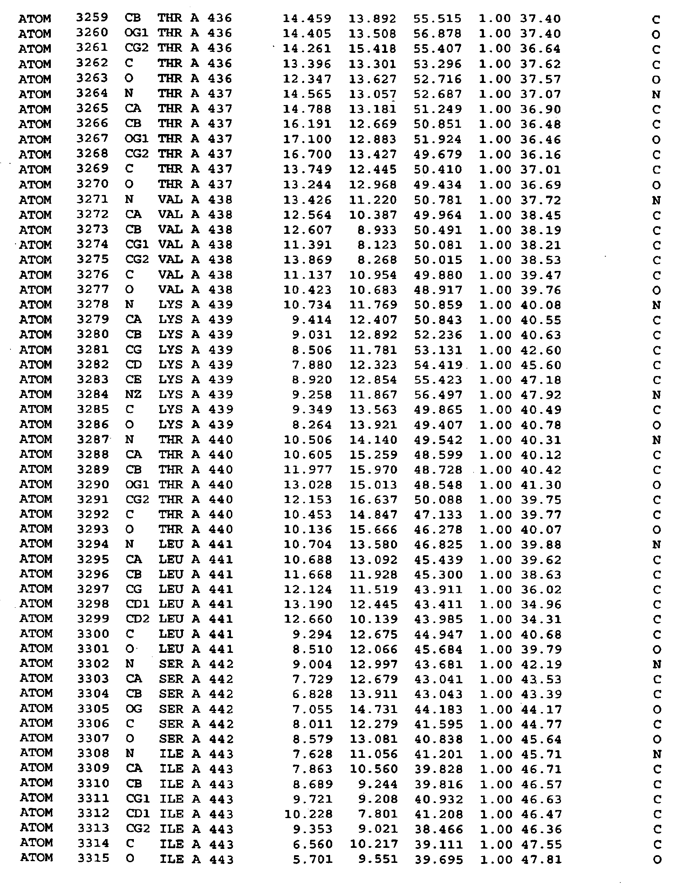

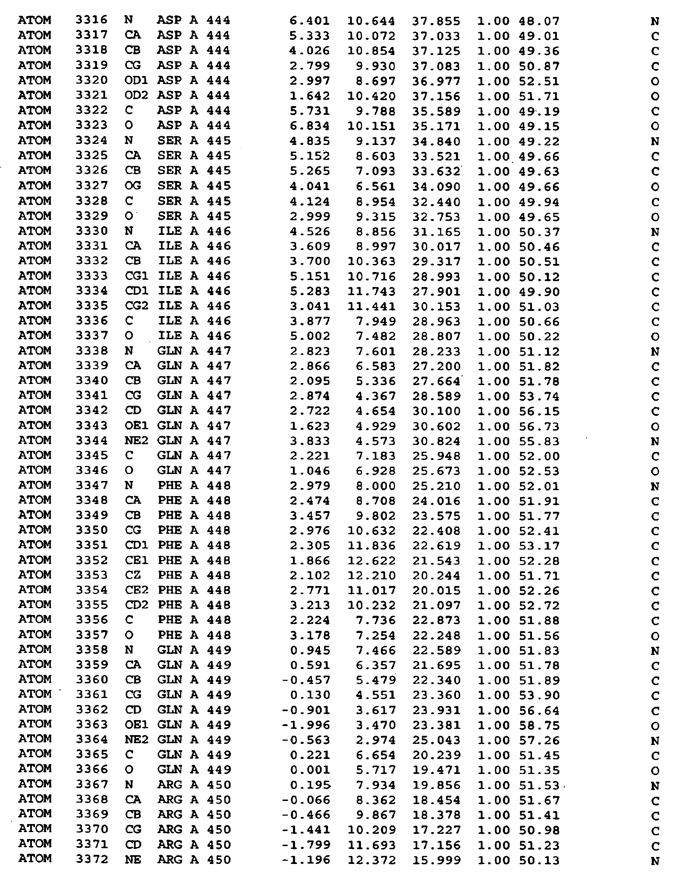

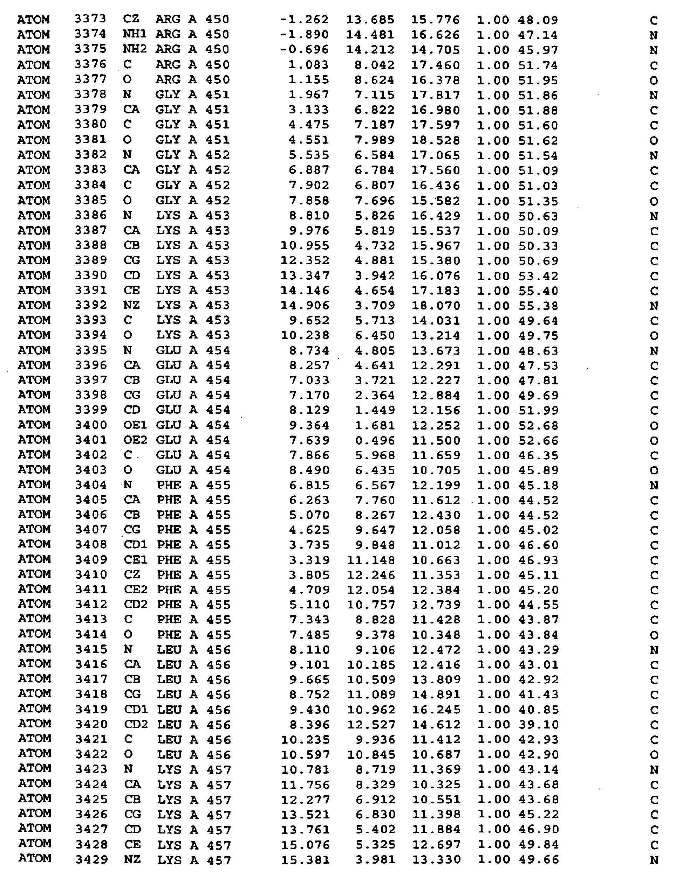

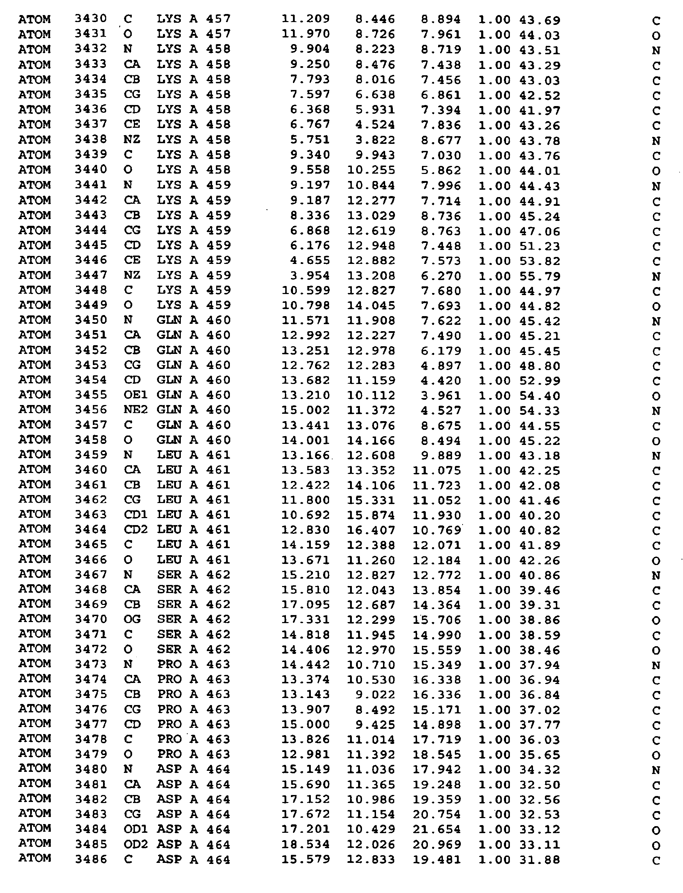

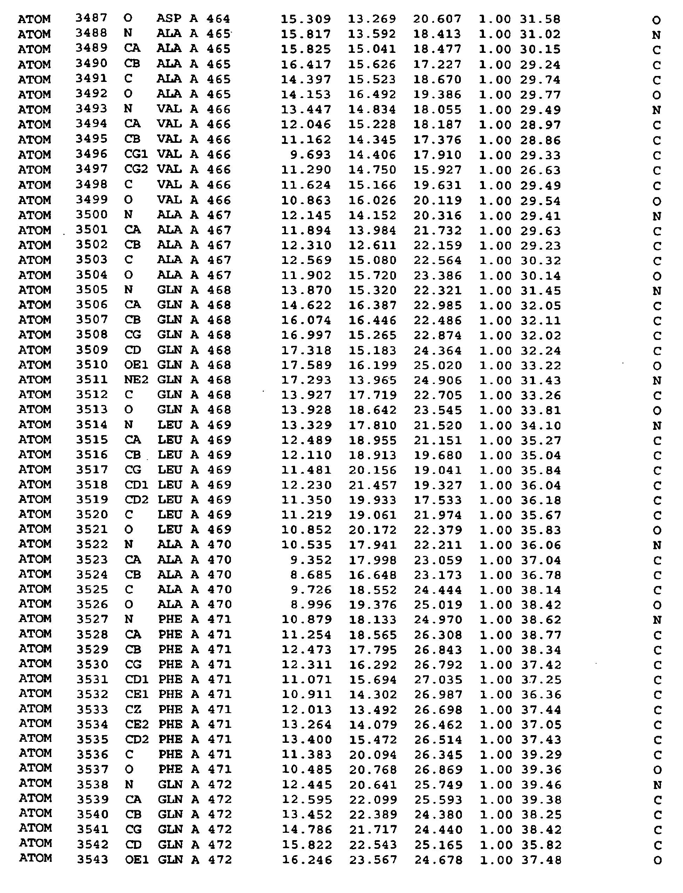

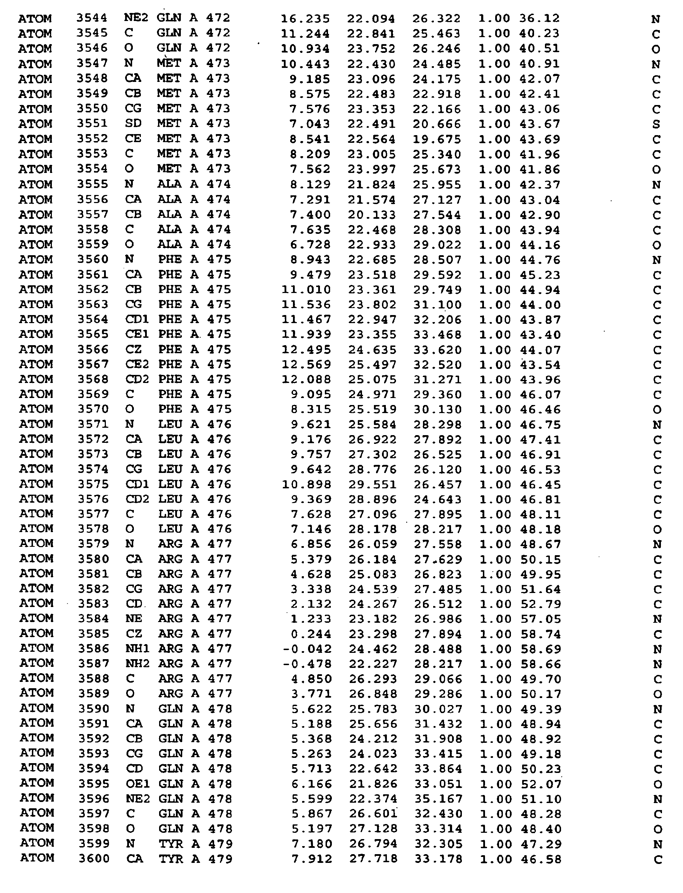

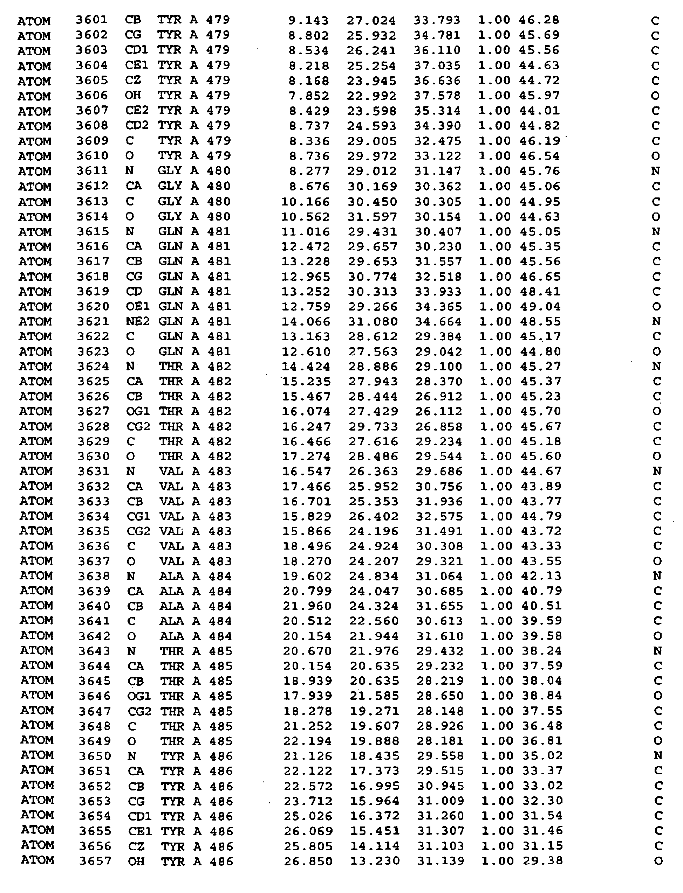

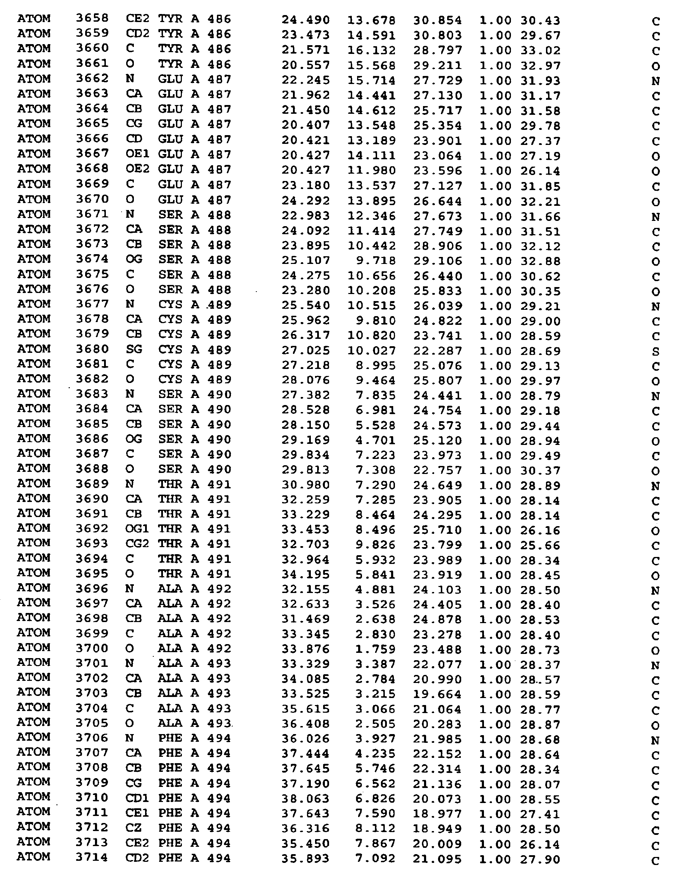

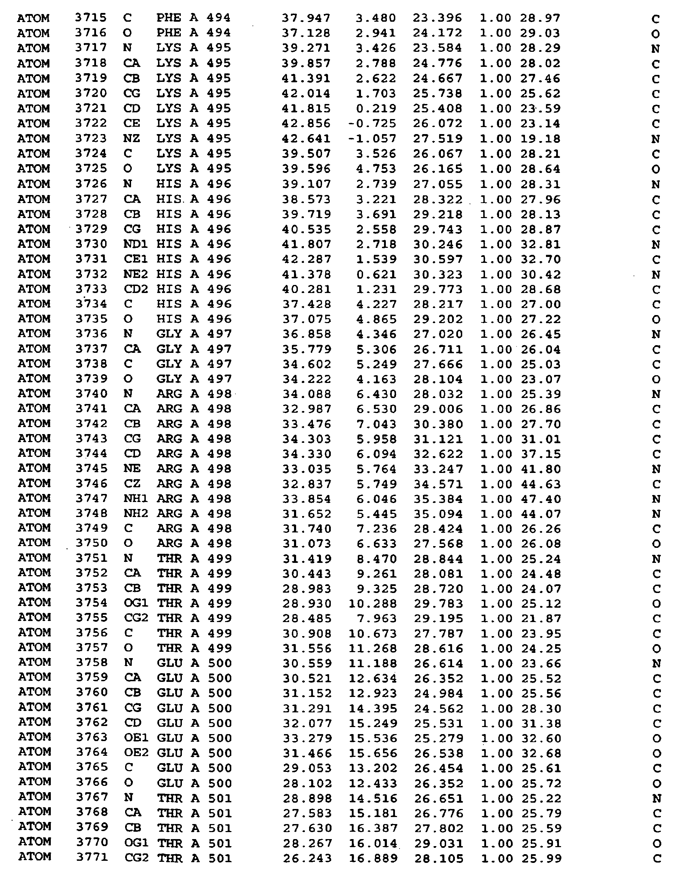

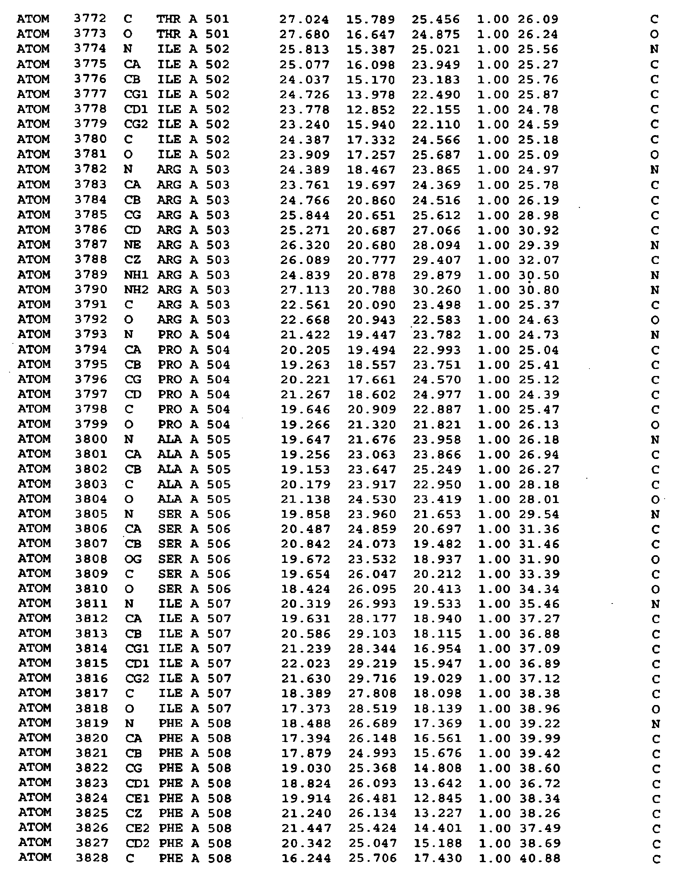

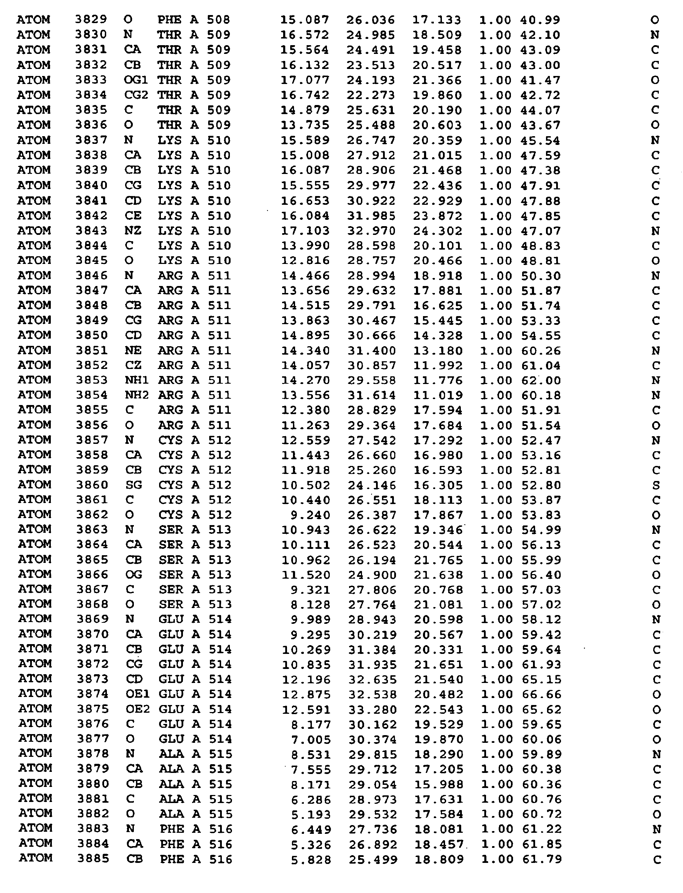

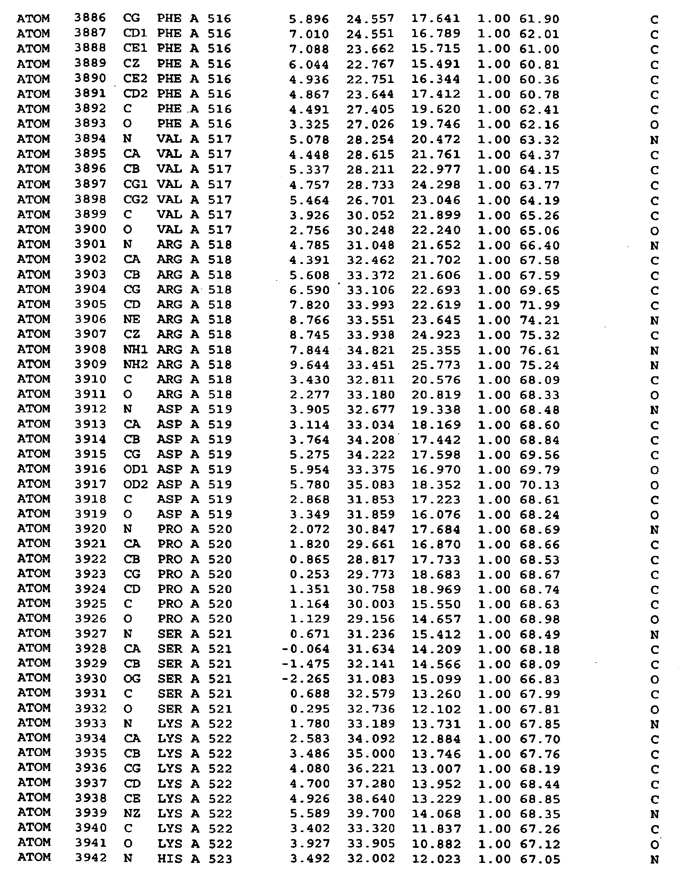

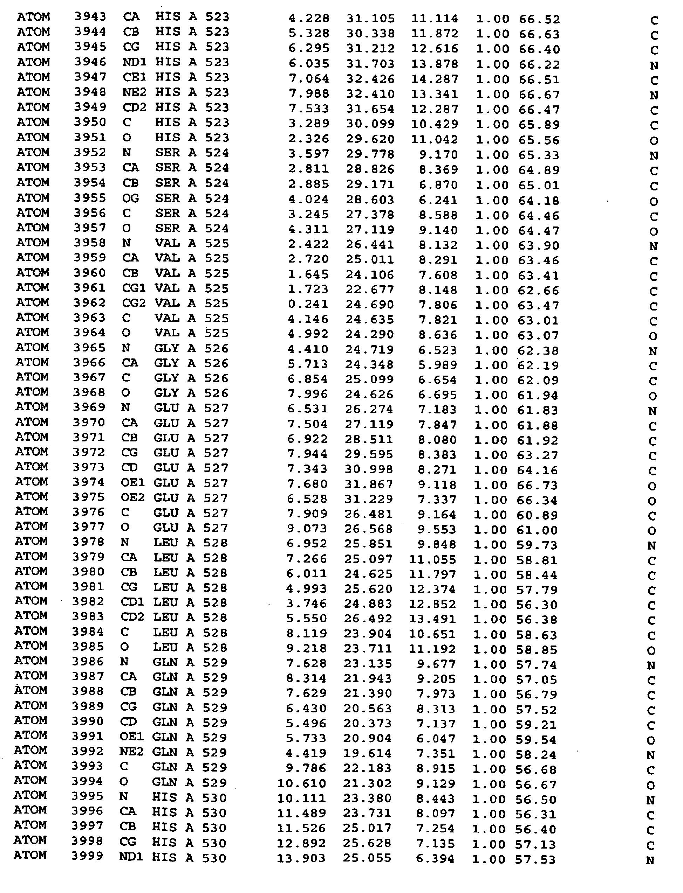

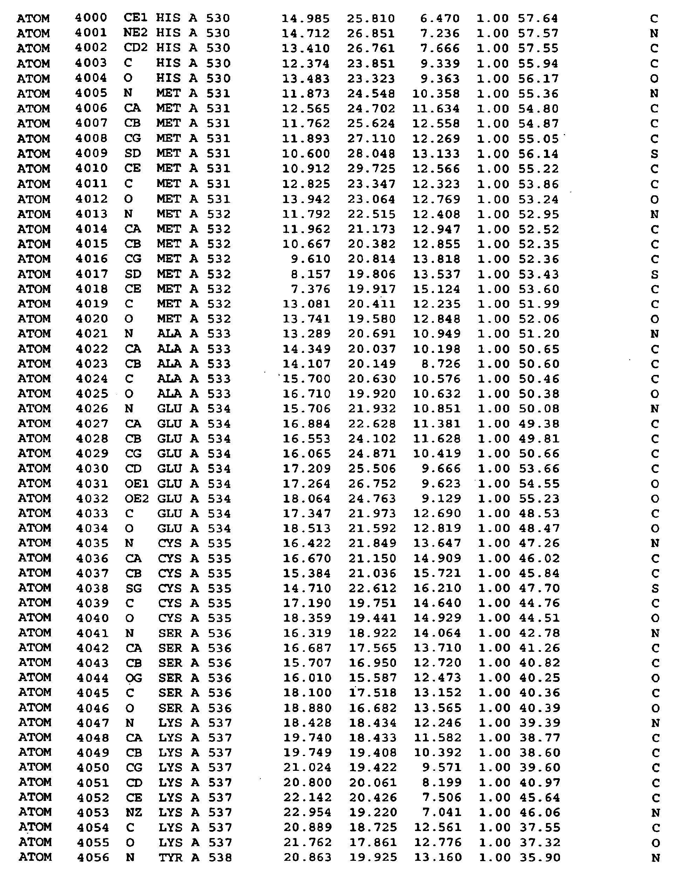

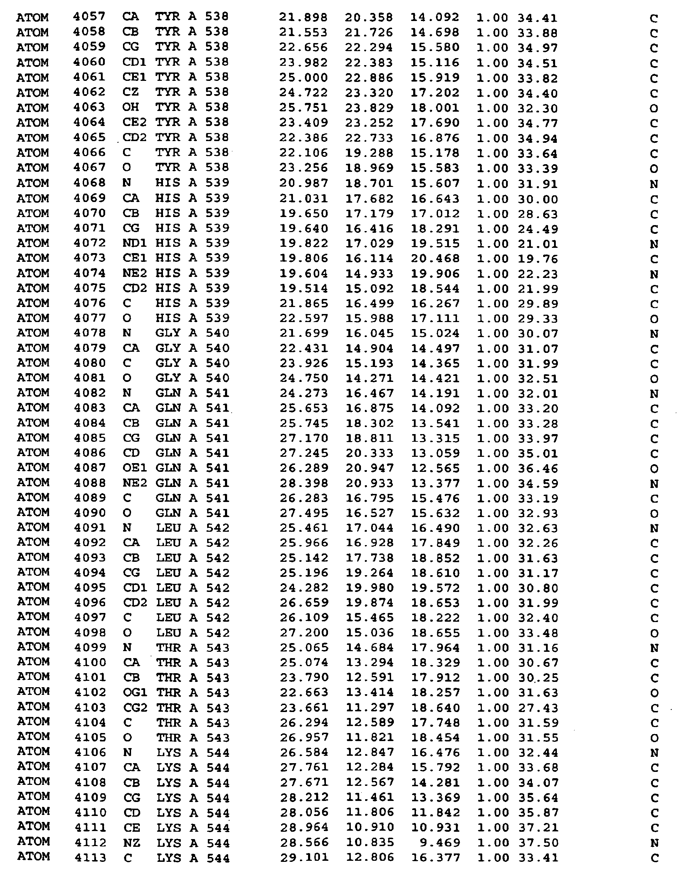

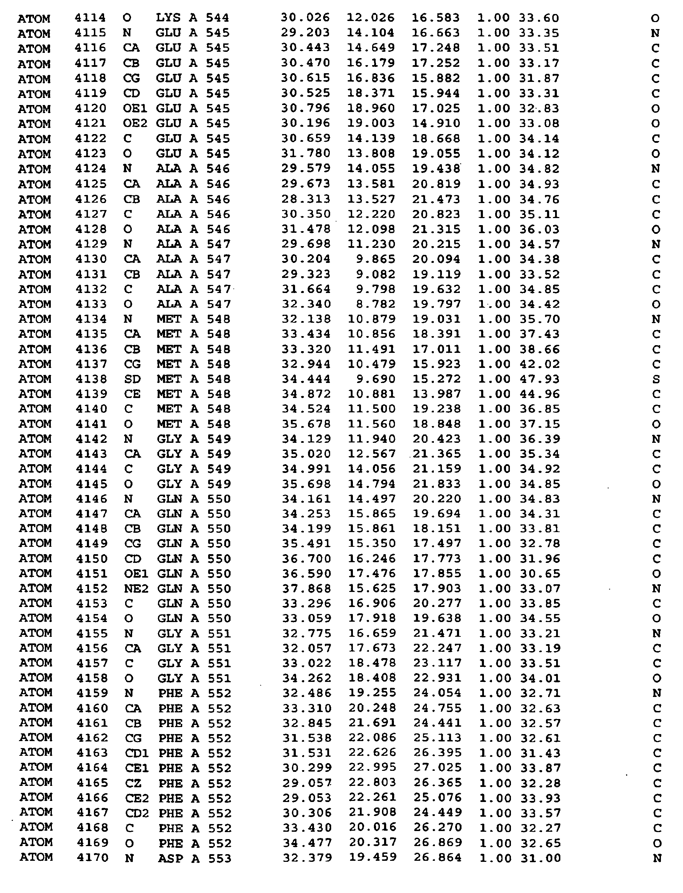

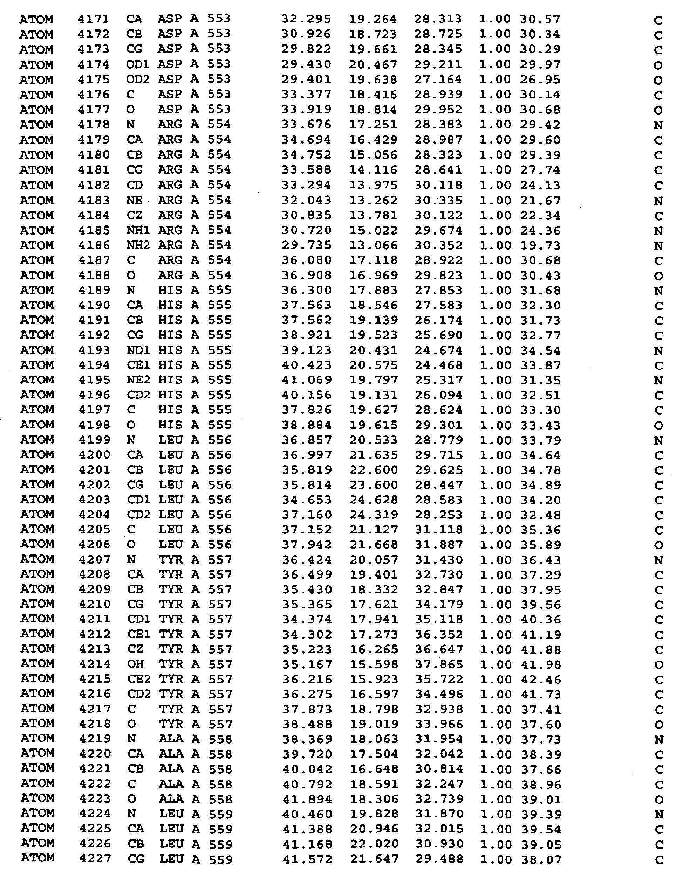

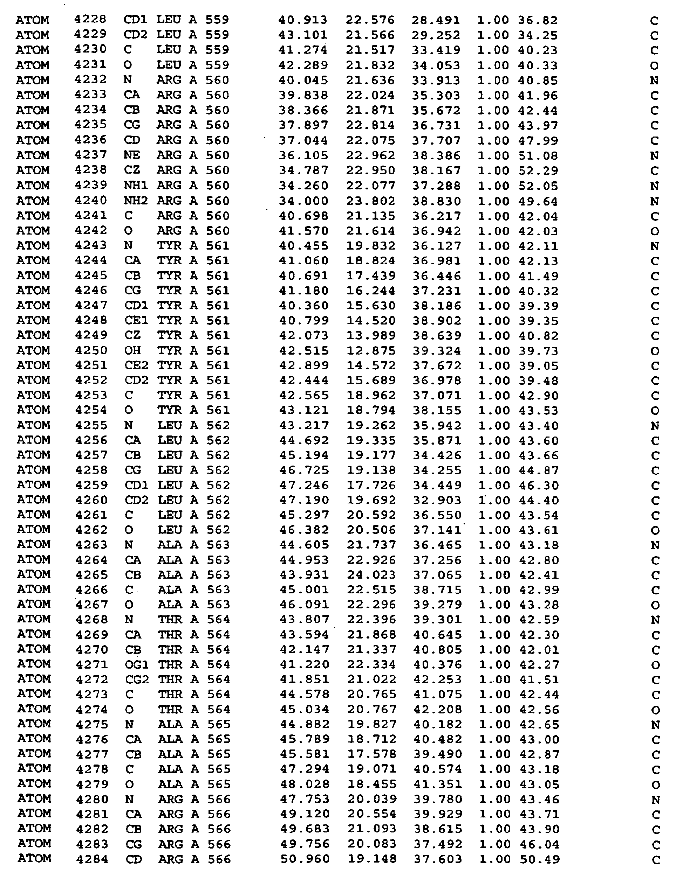

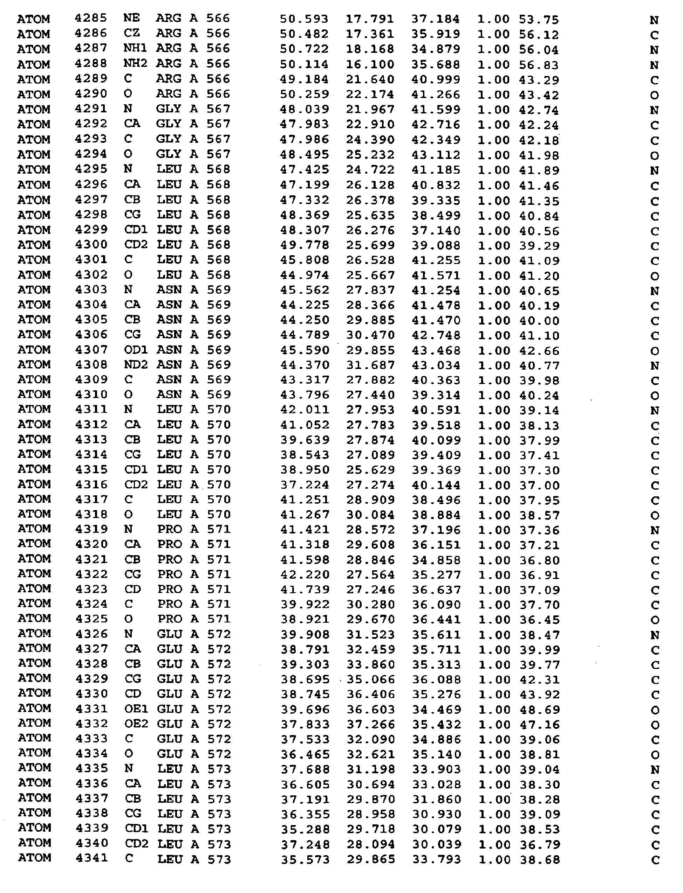

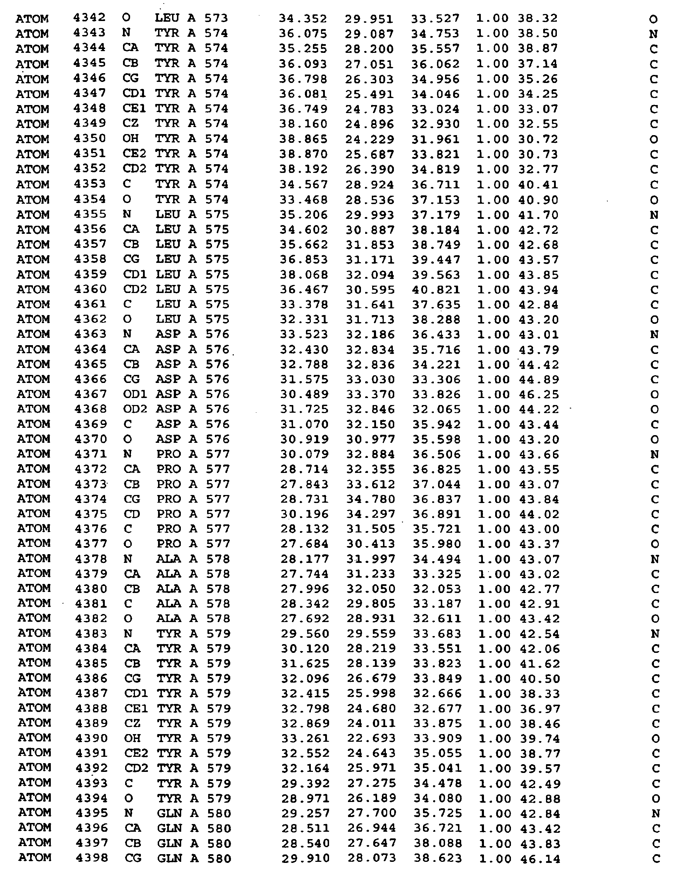

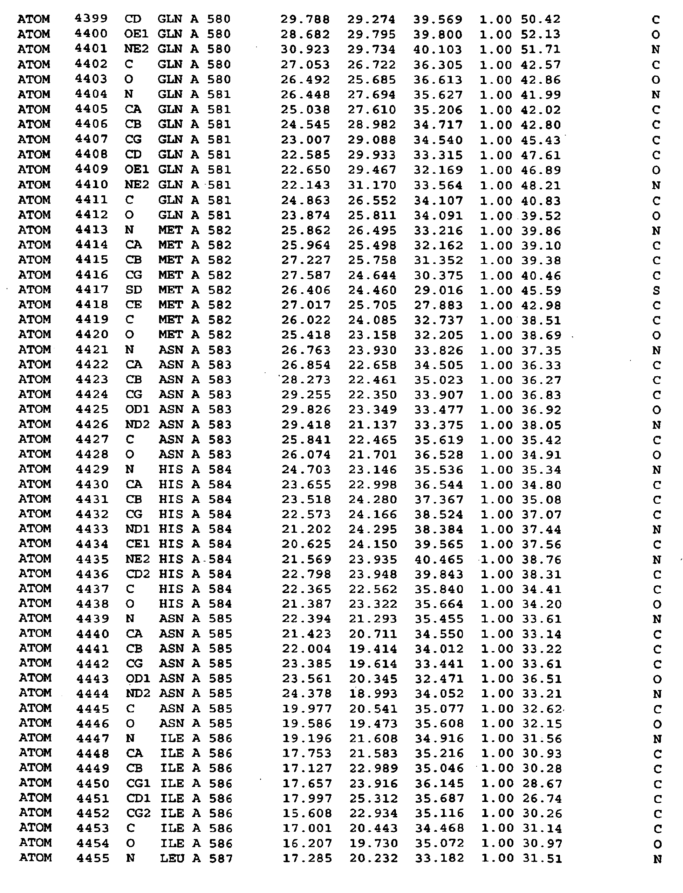

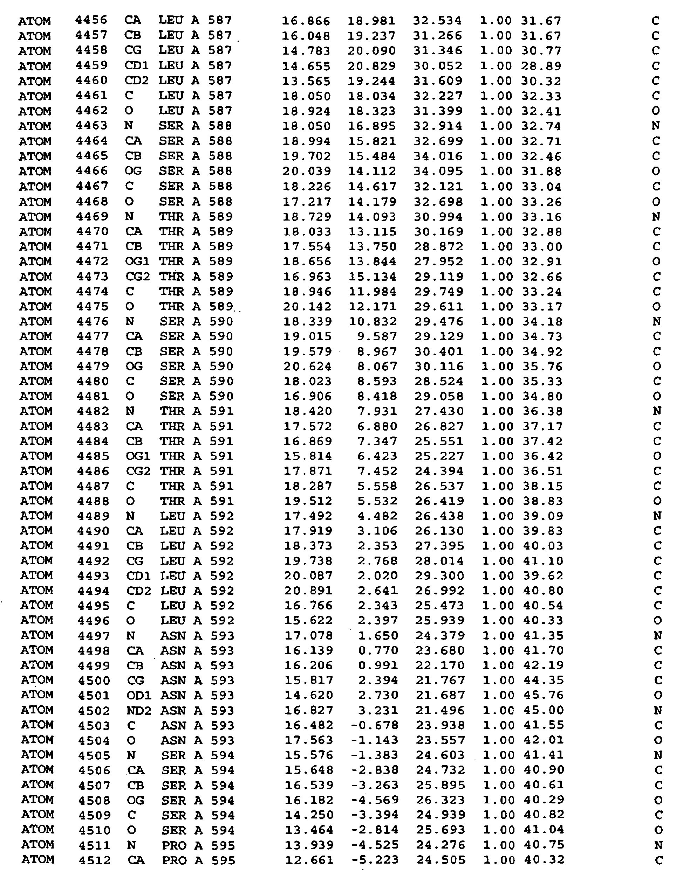

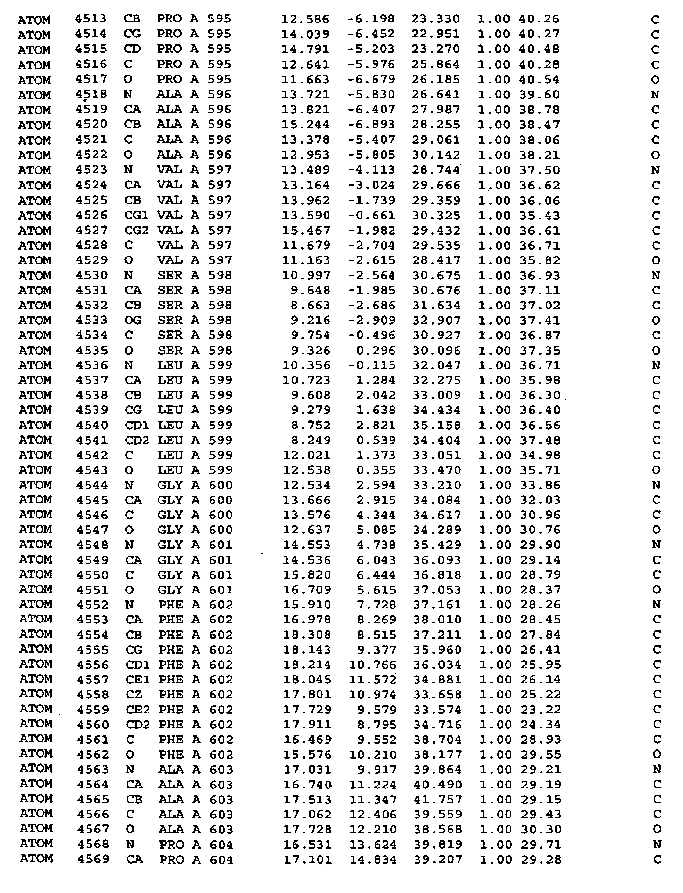

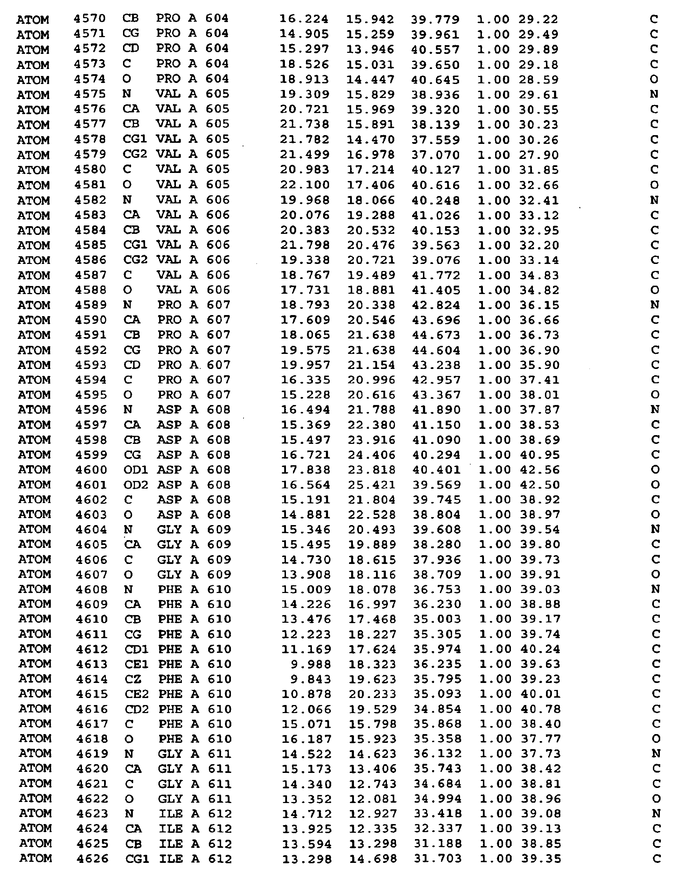

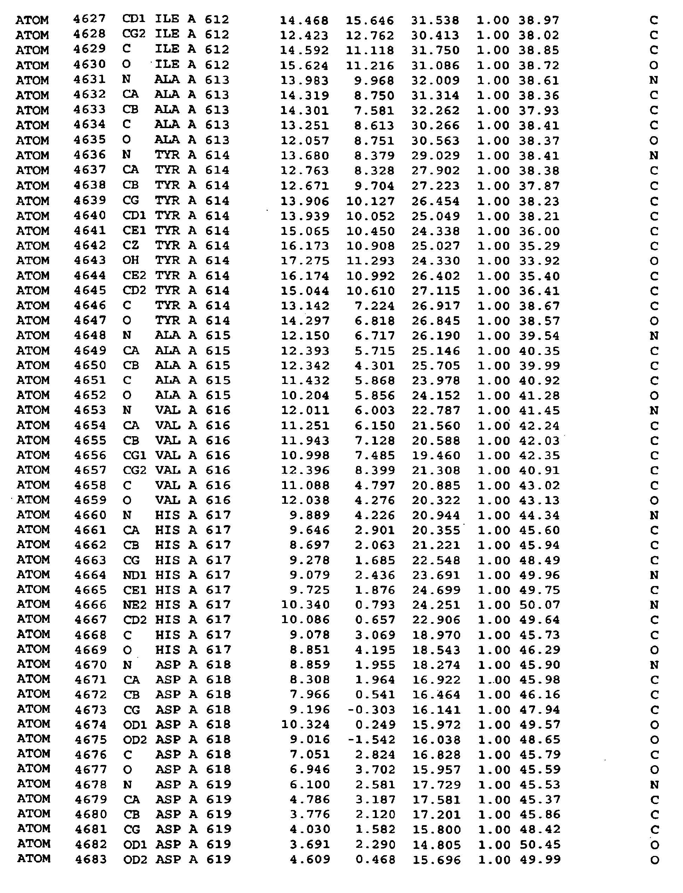

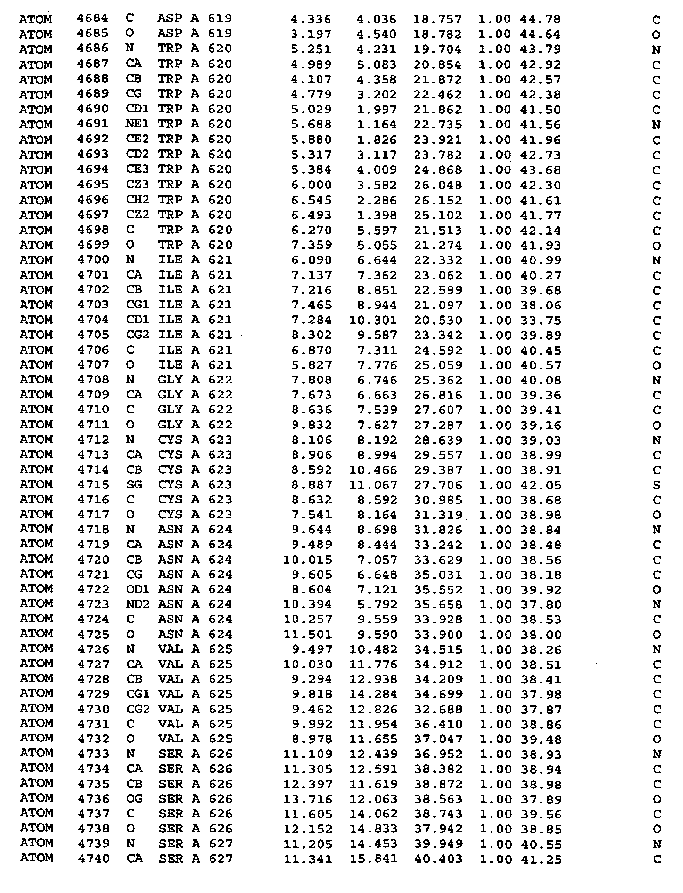

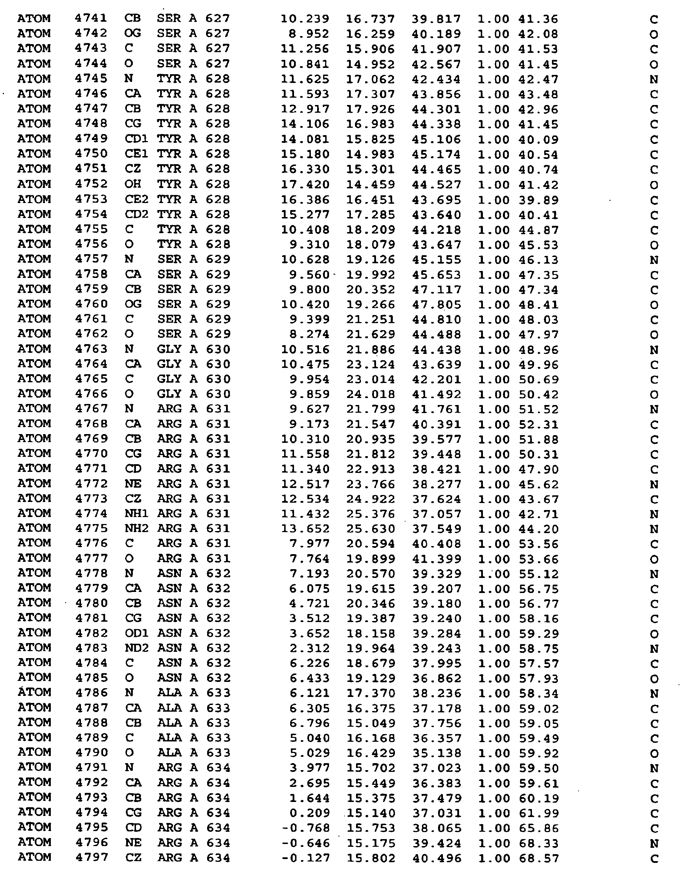

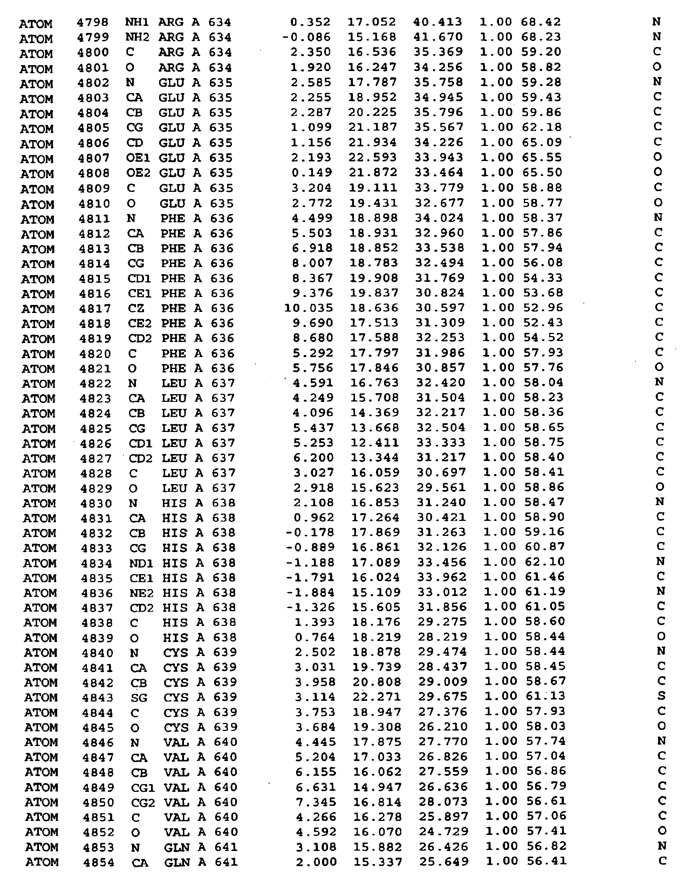

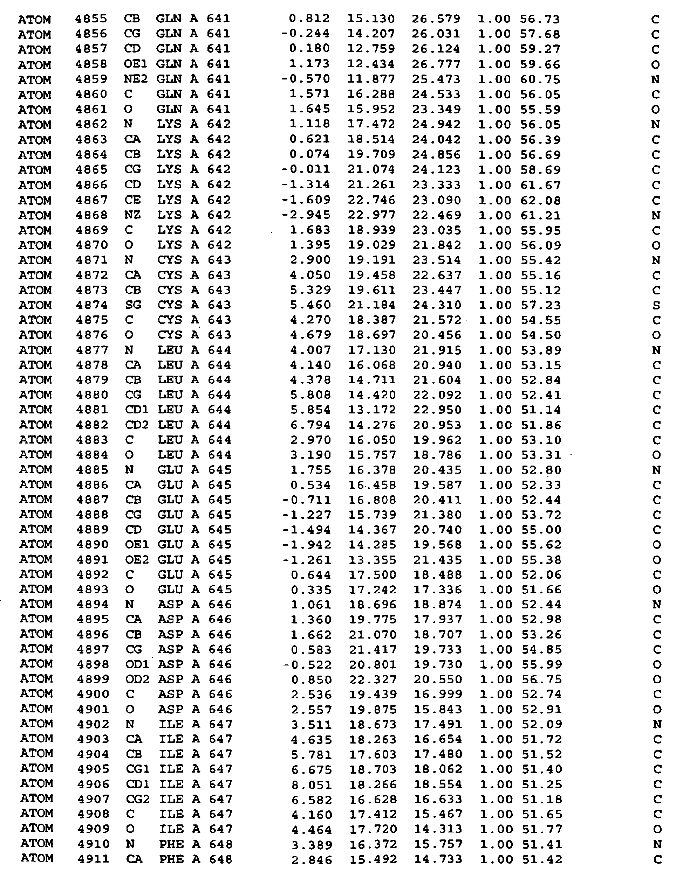

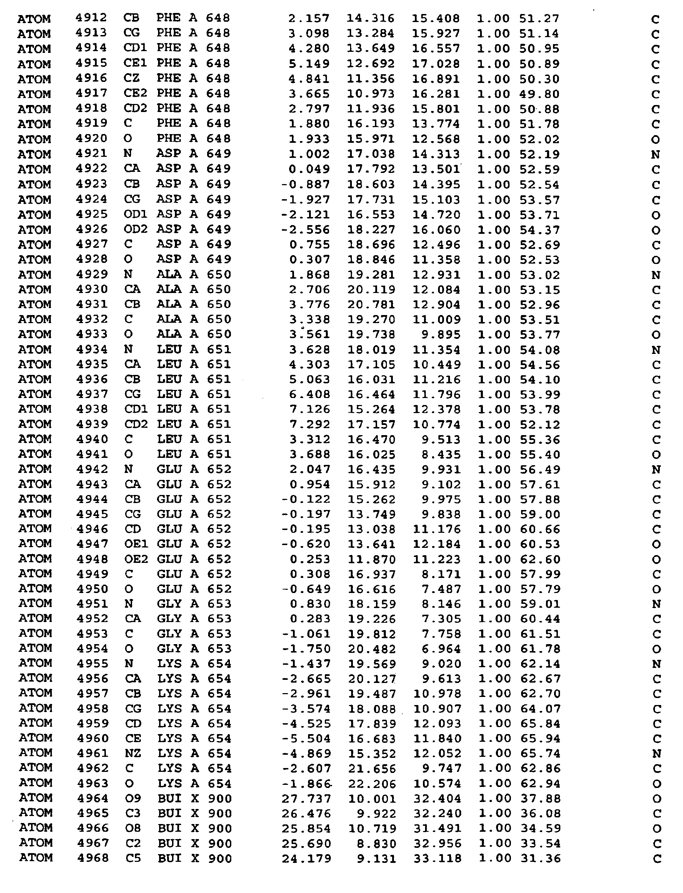

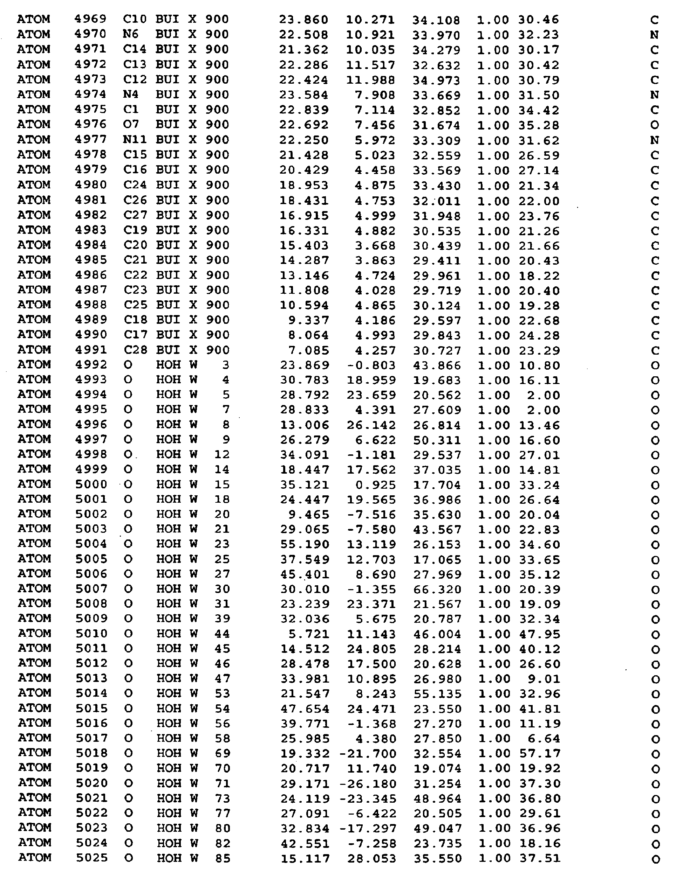

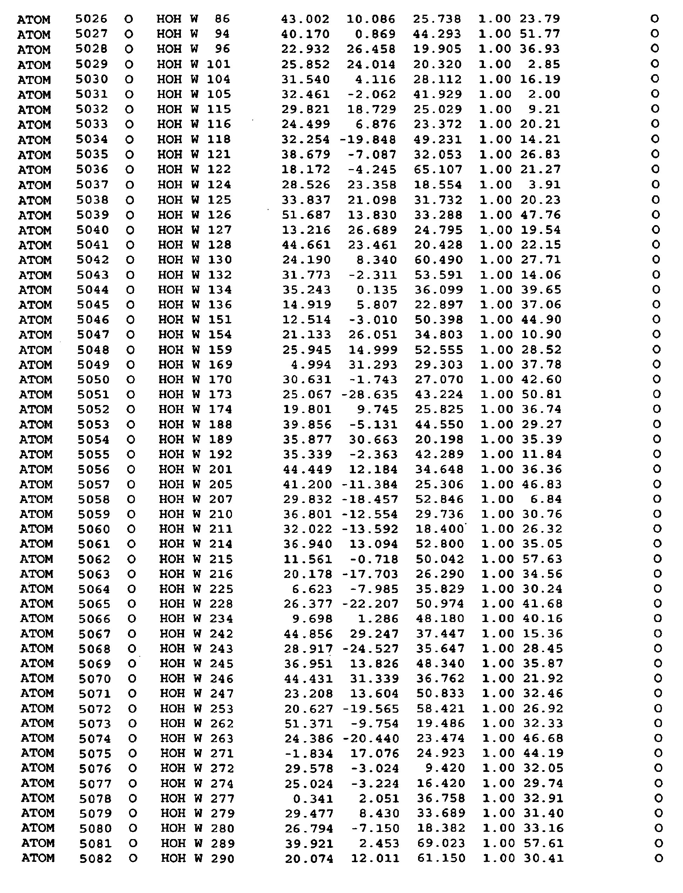

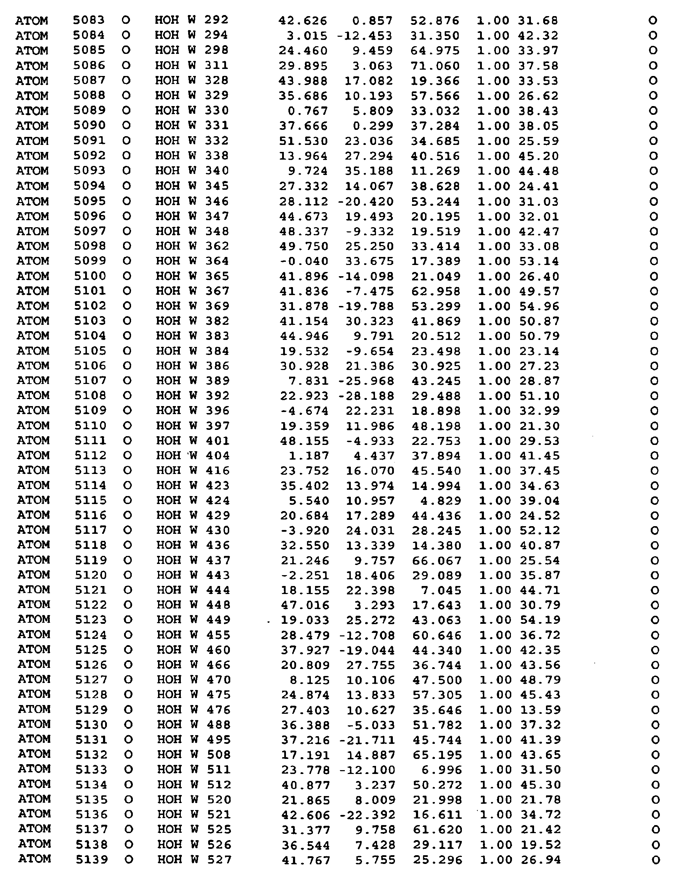

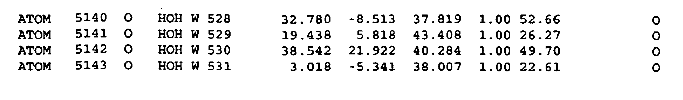

**Figure 6****:** Coordinates of CPT-2/ST 1316 co-crystal

### Examples

### Example 1: Protein expression and purification

The DNA coding for amino acids 27-658 of rat CPT-2 (a kind gift by Dr. V. A Zammit, Hannah Research Institute, Ayr, Scotland) was subcloned into a Novagen pET28a vector. This construct was used to express full-length CPT-2 (exempt from the mitochondrial import sequence, amino acids 1-26 of the CPT-2 precurser) in *E. coli* strain BL21DE3 at 20°C. After cell disruption the lysate (in 50 mM HEPES/ NaOH pH 8, 0.15 M NaCl, 5 mM TCEP, 10 mM MgCl₂, 30 mg/l DNase I, 30 Tbs./l Roche Complete protease inhibitor) was adjusted with 0.1 % (v/v) Triton-X-100. Solubilization and centrifugation (30,000 g, 45 min) was followed by immobilized metal affinity chromatography on a Ni-NTA resin. The detergent was exchanged to 1 % (v/v) n-octyl-β-D-glucopyranoside (βOG) on the column. The eluate from the IMAC step was subjected to a size exclusion chromatography column equilibrated with 25 mM Tris/ HCl pH 8, 0.15 M NaCl, 2 mM TCEP, 1 % (v/v) βOG, 0.02 % (w/v) NaN₃). ESI-MS confirmed the identity of CPT-II and showed that the protein was modified by amino-terminal α-N-gluconoylation (Geoghegan, K.F. et al. Spontaneous alpha-N-6-phosphogluconoylation of a "His tag" in Escherichia coli: the cause of extra mass of 258 or 178 Da in fusion proteins. Anal. Biochem., 267, 169-184 (1999)) (data not shown). The His-tag was not cleavable with thrombin. A 10 1 fermentation (45 g of biomass) yielded 30 mg of electrophoretically pure, monomeric and monodisperse protein.

### Example 2: Protein crystallization

Crystals of apo CPT-2 and the ST1326 [(R)-*N*-tetradecylcarbamoyl-aminocarnitine] complex (in 33 mM stock solution in H₂O/NaOH) were obtained at a protein concentration of 15 mg/ml (in 25 mM Tris/HCl pH 8.0, 150 mM NaCl, 2 mM TCEP, 1% (w/v) n-octyl-β-D-glucopyronoside and 0.02 % (w/v) NaN₃) and 21°C using hanging drops with 25 % (w/v) PEG 1500 or the modified microbatch (D'Arcy, A., et al., The advantages of using a modified microbatch method for rapid screening of protein crystallization conditions. Acta Cryst. D59, 396-399 (2003)) method with 0.15 M DL-malic acid pH 7.0, 20 % (w/v) PEG 3350, respectively (Index 37 and 91, Hampton Research). For complex formation CPT-2 was incubated with a 3-fold molar excess of the inhibitor ST1326 for 3 h on ice prior to crystallization. The crystals were flash frozen in liquid nitrogen after 30 s soaks in mother liquor supplemented with 25 % (w/v) PEG 200 (apo) or exchanging excess mother liquor and Al's oil against 100 % (v/v) paraffin oil (ST1326).

### Example 3: Data collection and processing

Datasets for apo CPT-2 and the ST1326 complex were collected on beam line X10SA at SLS, Villigen, Switzerland. The measurements were performed at 100 K with λ = 0.97853 Å (apo, Se peak wavelength) and λ = 1.008 Å (ST1326). The datasets were processed and scaled with XDS (Kabsch, W. Automatic processing of rotation diffraction data from crystals of initially unknown symmetry and cell constants. J. Appl. Cryst., 26, 795-800 (1993)). The space group was determined as P4₃2₁2 (apo tetragonal, a = b = 67.6, c = 307.3, α = β = γ = 90°, Matthew's Coeff. = 2.4 (Matthews, B. W. J. Mol. Biol., 33, 491-497 (1968))), C222₁ (apo orthorhombic, a = 95.2, b = 97.3, c= 310.4, Matthew's Coeff. = 2.4) and P2₁2₁2₁ (ST1326, a = 85.8, b = 96.2, c = 124.3, α = β = γ = 90°, Matthew's Coeff. = 3.5). First crystals of apo CPT-2 diffracted to 1.9 Å resolution but the space group could not be unequivocally assigned. In contrast, apo crystals obtained from material that was seleno-methionine labeled by standard procedures (Chene, C. et al. Crystallization of the complex of human IFN-gamma and the extracellular domain of the IFN-gamma receptor. Proteins, 23, 591-594 (1995)) were of superior diffraction quality, which could be attributed to improved protein quality due to the different media composition and bacterial protein expression profile during labeling.

The XDS rejection statistics report 0.26 % rejected misfits which fully supports the choice of P2₁2₁2₁ as the correct space group for the complex crystal despite the rather high value for R_{merge} of 16.7%. Data statistics are summarized in Table 1. Both crystal forms contain one molecule of CPT-2 per asymmetric unit.

*Structure solution and refinement*. Molecular replacement was performed using AMoRe in the CCP4 interface (Navazza, J. AMoRe: a new package for molecular replacement, in Molecular replacement. Proceedings of the CCP4 Study Weekend, 87-90, Daresbury Laboratory, Warrington, England (1992); Collaborative Computational Project, Number 4. The CCP4 suite: Programs for protein crystallography. Acta Cryst., D50, 760-763 (1994)). For the ST1326 complex a rat CPT-2 homology model built with XSAE (C. Broger, F. Hoffmann La-Roche) based on mouse CrAT (Hsiao, Y.S., et al., Structural and biochemical studies of the substrate selectivity of carnitine acetyltransferase. J. Biol. Chem., 279, 31584-31589 (2004)) (PDB code 1t7n) served as search model. The solution was refined by simulated annealing with CNS (Brünger, A. T. X-PLOR Manual Version 3.1. New Haven, CT, USA, Yale University Press (1992)). The final model was built using iterative cycles of model building in MOLOC (Gerber, P. R. Peptidemechanics: a force field for peptides and proteins working with entire residues as small units. Biopolymers, 32, 1003-1017 (1992)), solvent building in auto BUSTER (Roversi, P., et al., Modelling prior distributions of atoms for macromolecular refinement and completion. Acta Cryst., D 56, 1316-1323 (2000)) and refinement in Refmac (Murshudov, G. N., Vagin, A A., Lebedev, A., Wilson, K S. & Dodson, E. J. Efficient anisotropic refinement of macromolecular structures using FFT. Acta Cryst., D 55, 247-255 (1999)). Despite Se-labeling the apo structure was also solved by molecular replacement using the refined complex structure as search model, followed by automated model building with Arp/wArp (Perrakis, A., et al.,. Automated protein model building combined with iterative structure refinement. Nat. Struct. Biol., 6, 458-463 (1999)) and iterative Refmac and MOLOC cycles. For the orthorhombic (tetragonal) apo structure 94.0 % (92.7 %) of the resdues lie in the most favored and 5.4 % (6.8 %) in the additionally allowed regions of the Ramachandran Plot. For the ST1326 complex structure these values are 88.2 % and 11.3 %, respectively. Arg 498 (generously allowed region) and Leu 129 as well as Asn 230 (Ramachandran outliers) account for the differences to 100 %.

Refinement statistics are summarized in Table 1. Figures with molecule representations were made with PyMol (DeLano, W.L. The PyMOL User's Manual, DeLano Scientific, San Carlos, CA, USA (2002)) and MOE (Chemical Computing Group Inc., MOE, 1010 Sherbrooke Street West, Suite 910, Montreal, Canada, H3A 2R7).

## Claims

1. A crystal of protein CPT-2, wherein the crystal has unit cell dimensions of a = b = 66Å to 70Å c = 302 Å to 312 Å; α = β = γ = 90° and the crystal has a P4₃2₁2 symmetry.

2. A crystal of protein CPT-2, wherein the crystal has unit cell dimensions of = 93 Å to 97 Å, b = 94 Å to 98 Å, c = 304 Å to 312 Å; α = β = γ = 90°, and the crystal has a C222₁ symmetry.

3. A co-crystal of protein CPT-2 and a ligand bound to the active site of CPT-2, wherein the co-crystal has unit cell dimensions of a = 83 Å to 87 Å, b = 94 Å to 98 Å, c from 121 Å to 127 Å; α = β = γ = 90°, and the crystal has a P2₁2₁2₁ symmetry.

4. A method of identifying compounds that can bind to protein CPT-2, comprising the steps of:
Applying a 3-dimensional molecular modeling algorithm to the atomic coordinates of protein CPT-2 shown in Fig. 4 or 5 to determine the spatial coordinates of the binding pocket of protein CPT-2; and
electronically screening the stored spatial coordinates of a set of candidate compounds against the spatial coordinates of the protein CPT-2 binding pocket to identify a compound that can bind to protein CPT-2.

5. A process for co-crystallizing CPT-2 with a ligand that binds to the active site, the process comprising
providing a buffered, aqueous solution of 3.75 to 50 mg/ml of CPT-2,
adding a molar excess of the ligand to the aqueous solution of CPT-2, and
growing crystals by vapor diffusion or microbatch using a buffered reservoir solution of 0 % to 30 % (w/v) PEG, wherein the PEG has an average molecular weight of 200 Da to 20,000 Da.

6. The process of claim 5, wherein the buffer reservoir additionally comprises 0 M to 2 M tri-ammonium citrate pH 7, 0 M to 1 M L-proline, 0 M to 1M trimethylamine-N-oxide, 0 M to 1 M ammonium sulfate, 0 M to 1 M lithium sulfate, 0 M to 1 M ammonium acetate, 0 M to 1 M sodium or magnesium formate, and 0 M to 1 M D/L-malic acid pH7.

## Patentansprüche

1. Ein Kristall des Proteins CPT-2, wobei das Kristall die Elementarzelldimension von a = b = 66Å bis 70Å, c = 302Å; α = β = γ = 90° hat und das Kristall eine P4₃2₁2 Symmetrie hat.

2. Ein Kristall des Proteins CPT-2, wobei das Kristall die Elementarzelldimension von a = 93Å bis 97Å, b = 94Å bis 98Å, c= 304Å bis 312Å; α = β = γ = 90° hat und das Kristall eine C222₁ Symmetrie hat.

3. Ein Co-Kristall des Proteins CPT-2 und ein Ligand, der an das aktive Zentrum von CPT-2 gebunden ist, wobei das Co-Kristall eine Elementarzelldimension von a = 83Å bis 87Å, b = 94Å bis 98Å, c=121Å bis 127Å; α = β = γ = 90° hat und das Kristall eine P2₁2₁2₁ Symmetrie hat.

4. Eine Methode zur Identifizierung von Verbindungen, die an das Protein CPT-2 binden können, umfassend die Schritte:
anwenden eines 3-dimensionalen molekularen Modellierungs-Algorithmus auf die atomaren Koordinaten des Proteins CPT-2, gezeigt in Fig, 4 oder 5, um die räumlichen Koordinaten der Bindetasche des Proteins CPT-2 zu bestimmen, und
elektronisches screenen der gespeicherten räumlichen Koordinaten von einem Set von Verbindungskandidaten gegen die räumlichen Koordinaten der Bindetasche des Proteins CPT-2, um eine Verbindung zu identifizieren, die an das Protein CPT-2 binden kann.

5. Ein Verfahren zur Co-Kristallisation von CPT-2 mit einem Liganden, der an das aktive Zentrum bindet; das Verfahren umfassend
Bereitstellen einer gepufferten wässrigen Lösung mit 3.75 bis 50 mg/ml CPT-2 Hinzufügen eines molaren Überschusses des Liganden zur wässrigen Lösung von CPT-2 und
züchten von Kristallen durch Dampfdiffusion oder Microbatch unter Verwendung einer gepufferten Reservoirlösung aus 0 % bis 30 % (w/v) PEG, wobei das PEG ein mittleres Molekulargewicht von 200 Da bis 20 000 Da hat.

6. Das Verfahren nach Anspruch 5, worbei das Pufferreservoir zusätzlich 0 M bis 2 M Triammoniumcitrat pH 7, 0 bis 1 M L-Prolin, 0 bis 1 M Trimethylamin-N-oxid, 0 bis 1 M Ammoniumsulfat, 0 bis 1 M Lithiumsulfat, 0 bis 1 M Ammoniumacetat, 0 bis 1 M Natrium oder Magnesiumformiat, und 0 bis 1 M D/L -Hydroxybernsteinsäure pH 7 umfasst.

## Revendications

1. Cristal de la protéine CPT-2, dans lequel les dimensions de la maille unitaire du cristal sont a = b = 66 Å à 70 Å, c = 302 Å à 312 Å; α = β = γ = 90° et le cristal a une symétrie P4₃2₁2.

2. Cristal de la protéine CPT-2, dans lequel les dimensions de la maille unitaire du cristal sont a = 93 Å à 97 Å, b = 94 Å à 98 Å, c = 304 Å à 312 Å; α = β = γ = 90° et le cristal a une symétrie P222₁.

3. Co-cristal de la protéine CPT-2 et d'un ligand lié au site actif de la CPT-2, dans lequel les dimensions de la maille unitaire du co-cristal sont a = 83 Å à 87 Å, b = 94 Å 98 Å, c = 121 Å à 127 Å; α = β =γ = 90° et le cristal a une symétrie P2₁2₁2₁.

4. Procédé d'identification de composés qui peuvent se lier à la protéine CPT-2, comprenant les étapes:
d'application d'un algorithme de modélisation moléculaire en 3 dimensions aux coordonnées atomiques de la protéine CPT-2 présentée sur la figure 4 ou 5 pour déterminer les coordonnées spatiales de la poche de liaison de la protéine CPT-2; et
de criblage électronique des coordonnées spatiales stockées d'une série de composés candidats par rapport aux coordonnées spatiales de la poche de liaison de la protéine CPT-2 pour identifier un composé qui peut se lier à la protéine CPT-2.

5. Procédé de co-cristallisation de CPT-2 et d'un ligand qui se lie au site actif, le procédé comprenant
la fourniture d'une solution aqueuse tamponnée de 3,75 à 50 mg/ml de CPT-2,
l'addition d'un excès molaire du ligand à la solution aqueuse de CPT-2, et
la croissance des cristaux par diffusion de vapeur ou par la technique microbatch à l'aide d'une solution réservoir tamponnée de 0 % à 30 % (m/v) de PEG, le PEG ayant une masse molaire moyenne de 200 Da à 20 000 Da.

6. Procédé selon la revendication 5, dans lequel le réservoir tamponné comprend en outre de 0 M à 2 M de citrate de triammonium pH 7, de 0 M à 1 M de L-proline, de 0 M à 1 M de N-oxyde de triméthylamine, de 0 M à 1 M de sulfate d'ammonium, de 0 M à 1 M de sulfate de lithium, de 0 M à 1 M d'acétate d'ammonium, de 0 M à 1 M de formate de sodium ou de magnésium, et de 0 M à 1 M d'acide D/L-malique pH 7.


### SEQUENCE LISTING

<110> F. Hoffmann-La Roche AG
<120> CPT-2 crystals
<130> 23284
<160> 2
<170> PatentIn version 3.3
<210> 1
   <211> 658
   <212> PRT
   <213> Rattus norvegicus
<220>
   <221> rat CPT-2
   <222> (1)..(658)
   <223> Swissprot accession No. P18886
<400> 1
   Met Met Pro Arg Leu Leu Phe Arg Ala Trp Pro Arg Cys Pro Ser Leu
   1               5                   10                  15
   Val Leu Gly Ala Pro Ser Arg Pro Leu Ser Ala Val Ser Gly Pro Asp
               20                  25                  30
   Asp Tyr Leu Gln His Ser Ile Val Pro Thr Met His Tyr Gln Asp Ser
           35                  40                  45
   Leu Pro Arg Leu Pro Ile Pro Lys Leu Glu Asp Thr Met Lys Arg Tyr
       50                  55                  60
   Leu Asn Ala Gln Lys Pro Leu Leu Asp Asp Ser Gln Phe Arg Arg Thr
   65                  70                  75                  80
   Glu Ala Leu Cys Lys Asn Phe Glu Thr Gly Val Gly Lys Glu Leu His
                   85                  90                  95
   Ala His Leu Leu Ala Gln Asp Lys Gln Asn Lys His Thr Ser Tyr Ile
               100                 105                 110
   Ser Gly Pro Trp Phe Asp Met Tyr Leu Thr Ala Arg Asp Ser Ile Val
           115                 120                 125
   Leu Asn Phe Asn Pro Phe Met Ala Phe Asn Pro Asp Pro Lys Ser Glu
       130                 135                     140
   Tyr Asn Asp Gln Leu Thr Arg Ala Thr Asn Leu Thr Val Ser Ala Val
   145                 150                     155                 160
   Arg Phe Leu Lys Thr Leu Gln Ala Gly Leu Leu Glu Pro Glu Val Phe
                   165                     170                     175
   His Leu Asn Pro Ser Lys Ser Asp Thr Asp Ala Phe Lys Arg Leu Ile
               180                 185                     190
   Arg Phe Val Pro Pro Ser Leu Ser Trp Tyr Gly Ala Tyr Leu Val Asn
           195                     200                 205
   Ala Tyr Pro Leu Asp Met Ser Gln Tyr Phe Arg Leu Phe Asn Ser Thr
       210                 215                     220
   Arg Ile Pro Arg Pro Asn Arg Asp Glu Leu Phe Thr Asp Thr Lys Ala
   225                 230                     235                 240
   Arg His Leu Leu Val Leu Arg Lys Gly His Phe Tyr Val Phe Asp Val
                   245                     250                     255
   Leu Asp Gln Asp Gly Asn Ile Val Asn Pro Leu Glu Ile Gln Ala His
               260                 265                     270
   Leu Lys Tyr Ile Leu Ser Asp Ser Ser Pro Val Pro Glu Phe Pro Val
           275                     280                 285
   Ala Tyr Leu Thr Ser Glu Asn Arg Asp Val Trp Ala Glu Leu Arg Gln
       290                 295                     300
   Lys Leu Ile Phe Asp Gly Asn Glu Glu Thr Leu Lys Lys Val Asp Ser
   305                 310                     315                 320
   Ala Val Phe Cys Leu Cys Leu Asp Asp Phe Pro Met Lys Asp Leu Ile
                   325                     330                     335
   His Leu Ser His Thr Met Leu His Gly Asp Gly Thr Asn Arg Trp Phe
               340                 345                     350
   Asp Lys Ser Phe Asn Leu Ile Val Ala     Glu Asp Gly Thr Ala Ala Val
           355                 360                 365
   His Phe Glu His Ser Trp Gly Asp Gly Val Ala Val Leu Arg Phe Phe
       370                 375                 380
   Asn Glu Val Phe Arg Asp Ser Thr Gln Thr Pro Ala Ile Thr Pro Gln
   385                 390                 395                 400
   Ser Gln Pro Ala Ala Thr Asn Ser Ser Ala Ser Val Glu Thr Leu Ser
                   405                 410                 415
   Phe Asn Leu Ser Gly Ala Leu Lys Ala Gly Ile Thr Ala Ala Lys Glu
               420                 425                 430
   Lys Phe Asp Thr Thr Val Lys Thr Leu Ser Ile Asp Ser Ile Gln Phe
           435                 440                 445
   Gln Arg Gly Gly Lys Glu Phe Leu Lys Lys Lys Gln Leu Ser Pro Asp
       450                 455                 460
   Ala Val Ala Gln Leu Ala Phe Gln Met Ala Phe Leu Arg Gln Tyr Gly
   465                 470                 475                 480
   Gln Thr Val Ala Thr Tyr Glu Ser Cys Ser Thr Ala Ala Phe Lys His
                   485                 490                 495
   Gly Arg Thr Glu Thr Ile Arg Pro Ala Ser Ile Phe Thr Lys Arg Cys
               500                 505                 510
   Ser Glu Ala Phe Val Arg Asp Pro Ser Lys His Ser Val Gly Glu Leu
           515                 520                 525
   Gln His Met Met Ala Glu Cys Ser Lys Tyr His Gly Gln Leu Thr Lys
       530                 535                 540
   Glu Ala Ala Met Gly Gln Gly Phe Asp Arg His Leu Tyr Ala Leu Arg
   545                 550                 555                 560
   Tyr Leu Ala Thr Ala Arg Gly Leu Asn Leu Pro Glu Leu Tyr Leu Asp
                   565                 570                 575
   Pro Ala Tyr Gln Gln Met Asn His Asn Ile Leu Ser Thr Ser Thr Leu
               580                 585                 590
   Asn Ser Pro Ala Val Ser Leu Gly Gly Phe Ala Pro Val Val Pro Asp
   595 600 605
   Gly Phe Gly Ile Ala Tyr Ala Val His Asp Asp Trp Ile Gly Cys Asn
   610 615 620
   Val Ser Ser Tyr Ser Gly Arg Asn Ala Arg Glu Phe Leu His Cys Val
   625 630 635 640
   Gln Lys Cys Leu Glu Asp Ile Phe Asp Ala Leu Glu Gly Lys Ala Ile
   645 650 655
   Lys Thr
<210> 2
   <211> 632
   <212> PRT
   <213> Rattus norvegicus
<220>
   <221> mature rat CPT-1
   <222> (1)..(632)
   <223> position 1 corresponds to position 27 in Seq ID No. 1
<400> 2
   Ala Val Ser Gly Pro Asp Asp Tyr Leu Gln His Ser Ile Val Pro Thr
   1 5 10 15
   Met His Tyr Gln Asp Ser Leu Pro Arg Leu Pro Ile Pro Lys Leu Glu
   20 25 30
   Asp Thr Met Lys Arg Tyr Leu Asn Ala Gln Lys Pro Leu Leu Asp Asp
   35 40 45
   Ser Gln Phe Arg Arg Thr Glu Ala Leu Cys Lys Asn Phe Glu Thr Gly
   50 55 60
   Val Gly Lys Glu Leu His Ala His Leu Leu Ala Gln Asp Lys Gln Asn
   65 70 75 80
   Lys His Thr Ser Tyr Ile Ser Gly Pro Trp Phe Asp Met Tyr Leu Thr
   85 90 95
   Ala Arg Asp Ser Ile Val Leu Asn Phe Asn Pro Phe Met Ala Phe Asn
               100                 105                 110
   Pro Asp Pro Lys Ser Glu Tyr Asn Asp Gln Leu Thr Arg Ala Thr Asn
           115                 120                 125
   Leu Thr Val Ser Ala Val Arg Phe Leu Lys Thr Leu Gln Ala Gly Leu
       130                 135                 140
   Leu Glu Pro Glu Val Phe His Leu Asn Pro Ser Lys Ser Asp Thr Asp
   145                 150                 155                 160
   Ala Phe Lys Arg Leu Ile Arg Phe Val Pro Pro Ser Leu Ser Trp Tyr
                   165                 170                 175
   Gly Ala Tyr Leu Val Asn Ala Tyr Pro Leu Asp Met Ser Gln Tyr Phe
               180                 185                 190
   Arg Leu Phe Asn Ser Thr Arg Ile Pro Arg Pro Asn Arg Asp Glu Leu
           195                 200                 205
   Phe Thr Asp Thr Lys Ala Arg His Leu Leu Val Leu Arg Lys Gly His
       210                 215                 220
   Phe Tyr Val Phe Asp Val Leu Asp Gln Asp Gly Asn Ile Val Asn Pro
   225                 230                 235                 240
   Leu Glu Ile Gln Ala His Leu Lys Tyr Ile Leu Ser Asp Ser Ser Pro
                   245                 250                 255
   Val Pro Glu Phe Pro Val Ala Tyr Leu Thr Ser Glu Asn Arg Asp Val
               260                 265                 270
   Trp Ala Glu Leu Arg Gln Lys Leu Ile Phe Asp Gly Asn Glu Glu Thr
           275                 280                 285
   Leu Lys Lys Val Asp Ser Ala Val Phe Cys Leu Cys Leu Asp Asp Phe
       290                 295                 300
   Pro Met Lys Asp Leu Ile His Leu Ser His Thr Met Leu His Gly Asp
   305                 310                 315                 320
   Gly Thr Asn Arg Trp Phe Asp Lys Ser Phe Asn Leu Ile Val Ala Glu
   325 330 335
   Asp Gly Thr Ala Ala Val His Phe Glu His Ser Trp Gly Asp Gly Val
   340 345 350
   Ala Val Leu Arg Phe Phe Asn Glu Val Phe Arg Asp Ser Thr Gln Thr
   355 360 365
   Pro Ala Ile Thr Pro Gln Ser Gln Pro Ala Ala Thr Asn Ser Ser Ala
   370 375 380
   Ser Val Glu Thr Leu Ser Phe Asn Leu Ser Gly Ala Leu Lys Ala Gly
   385 390 395 400
   Ile Thr Ala Ala Lys Glu Lys Phe Asp Thr Thr Val Lys Thr Leu Ser
   405 410 415
   Ile Asp Ser Ile Gln Phe Gln Arg Gly Gly Lys Glu Phe Leu Lys Lys
   420 425 430
   Lys Gln Leu Ser Pro Asp Ala Val Ala Gln Leu Ala Phe Gln Met Ala
   435 440 445
   Phe Leu Arg Gln Tyr Gly Gln Thr Val Ala Thr Tyr Glu Ser Cys Ser
   450 455 460
   Thr Ala Ala Phe Lys His Gly Arg Thr Glu Thr Ile Arg Pro Ala Ser
   465 470 475 480
   Ile Phe Thr Lys Arg Cys Ser Glu Ala Phe Val Arg Asp Pro Ser Lys
   485 490 495
   His Ser Val Gly Glu Leu Gln His Met Met Ala Glu Cys Ser Lys Tyr
   500 505 510
   His Gly Gln Leu Thr Lys Glu Ala Ala Met Gly Gln Gly Phe Asp Arg
   515 520 525
   His Leu Tyr Ala Leu Arg Tyr Leu Ala Thr Ala Arg Gly Leu Asn Leu
   530 535 540
   Pro Glu Leu Tyr Leu Asp Pro Ala Tyr Gln Gln Met Asn His Asn Ile
   545 550 555 560
   Leu Ser Thr Ser Thr Leu Asn Ser Pro Ala Val Ser Leu Gly Gly Phe
   565 570 575
   Ala Pro Val Val Pro Asp Gly Phe Gly Ile Ala Tyr Ala Val His Asp
   580 585 590
   Asp Trp Ile Gly Cys Asn Val Ser Ser Tyr Ser Gly Arg Asn Ala Arg
   595 600 605
   Glu Phe Leu His Cys Val Gln Lys Cys Leu Glu Asp Ile Phe Asp Ala
   610 615 620
   Leu Glu Gly Lys Ala Ile Lys Thr
   625 630